# EUROPEAN PATENT APPLICATION

(11) **EP 4 834 540 A2**
(43) Date of publication of application: **04.11.2026**
(21) Application number: 24913733.2
(22) Date of filing: 27.12.2024
(51) Int. Cl.: B25J 9/00, B25J 19/00, A44B 11/26, B25J 9/16, B25J 13/00, B25J 13/08, A61H 3/00, A61B 5/11

(54) **WALKING ASSISTANCE WEARABLE ROBOT FOR ESTIMATING PHYSICAL ABILITY OF WEARER AND SUPPORTING SELECTIVE MUSCLE-STRENGTHENING EXERCISE, AND WEARABLE ROBOT SYSTEM**

(30) Priority: 29.12.2023 KR 20230197474; 27.02.2024 KR 20240027753; 02.05.2024 KR 20240058711; 04.10.2024 KR 20240135132; 15.10.2024 KR 20240140662; 22.10.2024 KR 20240144698; 05.11.2024 KR 20240155696; 21.11.2024 KR 20240167846; 22.11.2024 KR 20240168938
(71) Applicant: WIRobotics Inc., Cheonan-si, Chungcheongnam-do 31253 (KR)
(72) Inventor: CHOI, Byung June, Yongin-si, Gyeonggi-do 16856 (KR); KIM, Yong-Jae, Cheonan-si, Chungcheongnam-do 31166 (KR); LEE, Youn Baek, Yongin-si, Gyeonggi-do 17103 (KR); ROH, Changhyun, Seongnam-si, Gyeonggi-do 13540 (KR); LIM, Bok Man, Hwaseong-si, Gyeonggi-do 18444 (KR); LEE, Junghoon, Yongin-si, Gyeonggi-do 16838 (KR); CHOI, Yun Seok, Suwon-si, Gyeonggi-do 16509 (KR); JOUNG, Miok, Ansan-si, Gyeonggi-do 15478 (KR); HWANG, Jihwan, Suwon-si, Gyeonggi-do 16509 (KR); YUN, Hyukjin, Suwon-si, Gyeonggi-do 16663 (KR); LEE, Jewoo, Yongin-si, Gyeonggi-do 16942 (KR); JEON, Jihyeon, Yongin-si, Gyeonggi-do 16942 (KR); KIM, Do-yun, Yongin-si, Gyeonggi-do 16942 (KR); PARK, Jihyun, Yongin-si, Gyeonggi-do 16942 (KR); MIN, Deulle, Yongin-si, Gyeonggi-do 16942 (KR); LIM, Sungyun, Yongin-si, Gyeonggi-do 16942 (KR)
(74) Representative: Cabinet Beau de Loménie
(86) International application number: PCT/KR2024/021371
(87) International publication number: WO 2025/143929

(57) **Abstract**

The present invention relates to a walking assistance wearable robot for assisting a walking exercise of a wearer, the walking assistance wearable robot comprising: a fixing unit mountable on a body part of the wearer; and a drive unit mountable on the fixing unit, wherein the drive unit is configured to calculate a degree of change in a state trajectory of the wearer and calculate an output torque based thereon, and to calculate, in a range at or below a threshold including a time point at which a sign of the calculated output torque changes, the output torque as a value at or below the threshold for a predetermined time, wherein the predetermined time is 0.5 seconds or more and 1 second or less.

## Description

### TECHNICAL FIELD

The present invention relates to a walking assistance wearable robot and a wearable robot system for estimating a wearer's physical ability and supporting selective muscle strengthening exercise.

### BACKGROUND ART

Wearable robots have been developed since the 1960s, and have been developed as wearable robots for muscle strength augmentation for muscle strength augmentation and body protection targeting high-load, high-risk workers, as wearable assistance devices for assisting locomotion and performing rehabilitation and posture correction targeting the elderly and patients, and as prosthetic legs and prosthetic arms that substitute for body functions targeting the disabled.

Wearable robots, having started in non-commercial fields such as military/welfare for the disabled after the 1960s, have been developed since 2010 with their business scope expanded to the commercial fields of rehabilitation/industry/health.

Since a wearable robot must move in a state of being worn by a person, when its weight is heavy and its volume is large, it not only hinders the person's movement but also may rather injure the person when control is erroneous. Accordingly, although the weight may be heavy and movement may be inconvenient, it was first utilized in fields where wearer needs are great, such as assisting high-load, high-risk workers in tasks that a person cannot perform, or assisting a person who cannot walk to be able to walk.

The degree of walking, balance, and muscle strength ability due to aging has large individual differences from person to person. For efficient assistance of a wearable robot device and maximization of exercise effects, physical ability evaluation is necessary, and individual customization through this is necessary.

However, conventional physical function and walking ability evaluation requires a separate measurement protocol and an expert who is familiar therewith in a state where a wearable device is not worn, so that much cost and time are consumed for the measurement, and there is room for the occurrence of errors in the measurement, which makes the measurement difficult.

Accordingly, if a wearer's physical ability can be confirmed with a wearable device without separate walking ability measurement, it not only helps in operating group exercises by level, but also enables proposal and automation of individually customized exercise programs even when exercising alone. Hereinafter, there are provided a wearable device and method capable of estimating a wearer's physical ability using a wearable walking exercise robot using a backdrivable actuator.

In addition, among people placed in a work environment requiring walking assistance, there are many people who repeat, for a long time, complex motions in which a walking motion and a work motion that is not a walking motion are mixed. For example, in the case of a street cleaner, there are many cases of repeating various motions while moving, such as a task of walking for a long time and collecting trash located on the floor and boarding a vehicle. At this time, in a motion not requiring walking assistance, there is a need to deactivate the device torque so as not to hinder the worker's motion, and to activate the device torque only during walking so as to perform walking assistance.

In the case of US11,590,383B2, which is a conventional technology, it includes a process of estimating a user's walking phase and planning/determining an assistance torque suitable therefor in advance, and identifying one or more walking segments of the walking phase corresponding to a selected lower-body muscle. Finally, it uses a strategy of applying a resistance impulse torque that hinders the motion to the corresponding walking segment.

A scheme including such a process of identifying the walking phase and the walking segment has lower recognition accuracy during irregular walking, so that it becomes difficult to apply torque to the intended walking segment, and as a result, it is difficult to achieve the purpose of selective muscle strengthening. In addition, a pre-planned, abrupt waveform torque trajectory form that does not reflect the wearer's every-moment movement in real time abruptly hinders natural walking, and may act as a factor that hinders walking stability and comfort.

In particular, a pre-planned, abrupt waveform torque trajectory form that does not reflect the wearer's every-moment movement in real time may be dangerous to the elderly or the walking-impaired, and as a result, it is difficult to apply a high load (for the purpose of increasing the exercise effect) in a safe manner.

In addition, while the conventional technology considered only resistance force for muscle strengthening, the present invention also includes content in which it is possible to increase walking speed using assistance force so as to strengthen ankle muscle strength spaced apart from the hip at a distance.

Conventional walking assistance wearable robots have mainly used, in general, a scheme of applying a rigid frame structure to the vicinity of the waist and the hip joint in order to stably fix the wearer's body. Such a structure has an advantage of being able to enhance stability when the user performs various movements in a state of wearing the robot, but had a problem of restricting the wearer's movement and causing inconvenience. In particular, when the rigid frame surrounds the side of the hip joint, interference may occur depending on the wearer's body shape or body size, which may cause discomfort, and it became a factor that increases the wearer's fatigue during long-time use.

The rigid frame structure of conventional wearable robots requires adjustment customized to the body shape, and there were many cases where the wearing feeling was felt differently due to body shape differences among users. Such problems acted as a great obstacle factor in the commercialization and dissemination of wearable robots, and additional structural and design changes for providing customization according to various body shapes were required.

Due to this, problems also occurred in which the weight of the product increased and the overall system became complex. In order to solve such problems, attempts were made in some wearable robots to enhance the wearer's convenience by applying a soft material or a flexible structure. However, since rigid elements still remained in some portions of the frame, there was a limit in not being able to provide complete flexibility for the user's various movements and in implementing a perfect wearing feeling according to body shape.

In particular, since interference at the hip joint region could not be completely eliminated, there existed difficulty in implementing a natural assistance function according to the wearer's movement. Accordingly, in the present invention, there is proposed a wearable robot that removes the rigid frame in the lateral direction of the hip joint and applies a soft waist wearing unit, thereby being able to effectively assist the wearer's walking exercise while minimizing interference according to body shape or body size.

The present invention focuses on configuring a flexible waist wearing unit so that the user can comfortably wear it regardless of body shape, and optimizing the connection structure between the waist wearing unit and the drive unit so as to provide a natural assistance function during walking exercise.

Based on such a technical background, an object of the present invention is to provide a soft and stable thigh wearing unit designed so that the wearer can maintain comfort even when used for a long time and can naturally follow the movement of the human body. The thigh wearing unit of the present invention is intended to maximize the wearing feeling through a lightweight structure and ergonomic design, and to improve the performance of the walking assistance robot by supporting smooth sliding and rotating motions with the thigh frame joint portion.

### SUMMARY OF THE INVENTION

### TECHNICAL PROBLEM

The present invention may provide an apparatus, method, and system capable of estimating or evaluating a wearer's physical ability in order to maximize the efficient assistance and exercise effect of a wearable robot.

The present invention may provide an individually customized exercise program based on estimation of a wearer's physical ability using a wearable robot.

The present invention may provide a service that, in order to solve various problems of conventional methods of evaluating walking and physical ability, quantifies physical ability based on the device power of a wearable robot, and through this, estimates physical ability in a more convenient manner.

The present invention lies in generating and providing a graphical user interface that displays a wearer's real-time walking information, statistical information according to accumulated walking information, and a walking analysis report based on the wearer's walking analysis information using a wearable robot capable of estimating the wearer's walking ability during walking exercise.

The present invention may estimate the walking ability of a wearer of a wearable robot based on a deep learning model, and may provide at least one of a report according to the estimated walking ability and an individually customized exercise program.

A conventional walking assistance technique based on feedforward control estimates the user's current walking phase and speed, plans an assistance torque suitable therefor in advance, and then applies it. That is, in the case of the conventional technology, control is performed in a manner of estimating the current walking phase based on the user's periodic walking movement and then applying an assistance force suitable for the walking phase.

This approach method has no problem during periodic walking motions, but it is difficult to rapidly respond to abrupt motion changes, so that a user-device mismatch occurs.

In addition, in the case of the conventional technology, an additional biosignal sensor (EMG) may be used in order to grasp the user's walking motion in advance, but there is a problem that separate sensor signal processing is required and the sensor must be brought into contact with the user's skin.

In addition, an irregular walking pattern change may be caused by a walking environment change such as turning during walking, ascending/descending stairs/slopes, and the like. Conventionally, the wearer's work motion and walking environment were estimated, and a pre-planned assistance torque was changed and applied in accordance with the environment. However, this is a scheme that depends on walking environment recognition performance and is vulnerable to complex walking environments and abrupt walking pattern changes.

A conventional walking assistance wearable robot based on a scheme of recognizing walking speed, cadence, and number of steps may take at least 1 step to 2 steps (about 0.5 seconds or more) for a control rule update. In the case of a worker, there are cases of performing an abrupt motion immediately after stopping during walking, so that a mismatch between walking assistance and work motion may occur.

While conventional various fitness exercises are difficult for the elderly to easily learn and require correction/help from a professional trainer for checking a correct exercise posture, walking exercise can be performed simply and safely without the need to separately learn an exercise posture. However, walking exercise has a disadvantage that it is difficult to give various exercise effects and a high exercise load. In addition, as a result of conventional research, even adding weight to the pelvis position (waist) or the ankle is not effective for walking (improvement) training, so that it is difficult to achieve walking posture improvement and left/right balance correction during walking with conventional walking exercise.

Accordingly, if lower-body muscle strength can be (selectively) strengthened through walking exercise, the advantages of walking exercise can be utilized while the disadvantages can be minimized, and therefore, the present invention intends to selectively strengthen lower-body muscle strength during walking exercise using a wearable robot.

The present invention intends to provide a wearable robot that can correspond to various body shapes and sizes and that is easy for the wearer to put on and take off by himself/herself.

In addition, it is intended to provide a wearable robot that can be easily carried when the wearable robot is not used.

Since a wearable robot must be closely attached to the user's body to assist movement, when the weight and volume are large, it not only restricts the user's free movement and causes discomfort in the wearing feeling, but also there is a risk that the user may be injured when control is erroneous. Due to such problems, conventional wearable robots were mainly utilized for special purposes such as industrial, military, or medical use, and there was a limit in developing them to a level usable by the general public in daily life.

Among conventional technologies, a walking assistance device of Company H and a device having a similar structure are characterized by drive units arranged on both sides of the hip joint and a battery and control unit arranged on the back, and were designed so that the waist wearing unit supports the device. However, such a structure has problems in that the weight of the device increases, and due to the fixed frame form and size, it cannot effectively support users having various body shapes and sizes, and the device slides down or the degree of close contact with the body decreases. In addition, the scheme in which the waist wearing unit supports the reaction of the assistance force causes great discomfort to the user's waist.

Meanwhile, a soft exoskeleton applying a fabric material similar to clothing and a cable actuator shows a notable attempt to improve the wearing feeling, but due to the cable structure, the wearing feeling worsens because of a pressing sensation in contact with the skin in the process of assisting the joint, and there is a problem that the drive efficiency is lowered. There also exists a limit in that the torque transmission efficiency decreases due to friction of the cable, and assistance is possible only in one direction.

A device using a single actuator and applying a differential structure is an attempt to symmetrically transmit the output of the actuator to both legs, and intended to fix the wearing unit through a joint structure adapted to the wearer's body. However, it requires a complex joint structure requiring rigidity in order to align the hip joint axis and the rotation center of the actuator, and due to this, problems occur in that the weight becomes heavy and the structure becomes complex.

Accordingly, the present invention intends to solve the following problems.

The weight of the wearable robot is made lightweight so that the wearer does not feel a sense of weight even when carrying the device around, and the wearer's motion is not restricted so as to enable free movement. Through this, there is no inconvenience at all in daily motions such as getting in and out of a car, motions of sitting or spreading the legs, and sitting on a chair or lying down.

It is designed to be able to correspond to users having various body shapes and sizes, so that the wearer can easily put on and take off the device by himself/herself. In addition, when the device is not used, it can be folded or separated into a miniaturized form and put into a bag to be carried.

The wearing feeling is maximized by improving so that the device is closely attached to the wearer's thigh and waist and does not slide down, and the assistance force of the device does not give excessive pressure to the waist or skin.

Beyond simply assisting walking, it provides flexibility and adaptability of motion that can effectively support the wearer's various motions (walking, ascending stairs, running, and the like), and enables individually customized assistance suited to the user's state and needs.

In order to solve such problems, the present invention proposes a wearable robot including a thigh wearing unit that is lightweight and designed to be user-customized. The thigh wearing unit is softly attached closely to the user's thigh so that the wearing feeling is excellent, and is designed to naturally conform to the wearer's movement by supporting flexible sliding and rotating motions with the thigh frame joint portion. Through this, the disadvantages of conventional technology are supplemented, and implementation of a mobility-level wearable robot usable even by the general public in daily life is enabled.

Conventional wearable robots are heavy in weight and large in volume, so that there is a possibility of restricting the wearer's movement or causing inconvenience. The present invention intends to solve this by making the frame structure lightweight and designing it ergonomically so that the wearer can move naturally.

The shock and vibration that occur in the process of transmitting the assistance force cause inconvenience to the wearer and lower the efficiency of the device. The present invention intends to solve this by effectively suppressing shock and vibration through a urethane bearing and a sliding mechanism so as to provide a smooth and stable assistance force.

Conventional technology has a limit in that it does not sufficiently reflect the user's individual walking characteristics (walking, ascending stairs, sitting, and the like) or causes inconvenience in specific motions. The present invention intends to provide user-customized assistance through a 2-stage and 2.5-stage sliding mechanism and to flexibly correspond to various walking characteristics.

The left-right tilting phenomenon that may occur when the thigh frame slides may impair the stability of the motion and lower the durability of the device. The present invention intends to prevent the left-right tilting phenomenon during sliding by implementing a left-right symmetric structure and a certain angle in the thigh cover and the thigh inner member.

Conventional wearable robots may cause a sense of rejection when worn by not immediately reacting to the wearer's movement or by restricting the range of motion of the joint. The present invention intends to provide natural and comfortable walking support by implementing a structure that conforms to the user's movement and reacts agilely.

Since a wearable robot is a device worn and moved by a person, when the battery usage time is short, the utility is limited. The present invention intends to minimize power consumption through a lightweight structure and an efficient design.

### TECHNICAL SOLUTION

In a wearable robot capable of estimating a physical ability of a wearer during a walking exercise, the wearable robot comprises a fixing unit that may be mounted on a body part; and a drive unit that may be mounted on the fixing unit; and the drive unit may estimate the physical ability of the wearer based on a power of the drive unit due to the walking exercise of the wearer.

Further, the drive unit may estimate the physical ability of the wearer based on a value obtained by normalizing the power of the drive unit by a torque.

Further, the drive unit may calculate a ratio of power to torque of the drive unit by dividing the power of the drive unit by an RMS (Root Mean Square) torque of the drive unit.

Further, the drive unit may sense a hip angle of the wearer and may calculate an angular velocity based on the sensed hip angle; and may calculate the power of the drive unit by a product of the calculated angular velocity and the torque.

Further, the calculated angular velocity may be calculated based on walking of the wearer in a case where the wearer uses a backdrivable actuator.

Further, the torque may, in a case where the drive unit calculates a plurality of motion state values based on the calculated angular velocity, be calculated as a weighted sum of the selected motion state value by selecting at least one motion state value among the calculated plurality of motion state values.

Further, the drive unit may accumulate the calculated ratio of power to torque of the drive unit for each walking.

Further, the calculated plurality of motion state values may be stored by repeating a shift operation through a state trajectory memory buffer.

In a method of estimating a physical ability of a wearer during walking using a wearable robot comprising a drive unit, the method comprises a step of accumulating, for each walking, the ratio of power to torque of the drive unit calculated by the drive unit; and a step of calculating a statistic on the accumulation.

In a method of estimating a physical ability of a wearer during walking using a wearable robot comprising a drive unit, the method comprises a step of receiving, from a server device, statistical information on an accumulation obtained by accumulating, for each walking, the ratio of power to torque of the drive unit calculated by the drive unit; and a step of displaying the received statistic on the accumulation.

In a method of estimating a physical ability of a wearer during walking using a wearable robot comprising a drive unit, the method comprises a step of suggesting exercise information based on a database according to a correlation between the ratio of power to torque of the drive unit calculated by the drive unit and a wearer's physical condition.

In a method of estimating a physical ability of a wearer during walking using a wearable robot comprising a drive unit, the method comprises a step of suggesting exercise information based on a deep learning model trained using, as training data, the ratio of power to torque of the drive unit calculated by the drive unit and a wearer's physical condition.

In a method of estimating a physical ability of a wearer during walking using a wearable robot comprising a drive unit, the method comprises a step of predicting an exercise effect based on a deep learning model trained using, as training data, at least any one or more of the ratio of power to torque of the drive unit calculated by the drive unit, a wearer's physical condition, wearable robot usage information, and an exercise effect.

Further, the wearer's physical condition may be a condition considering at least any one or at least two or more of the wearer's age, gender, height and weight, and exercise ability.

In a method of estimating a physical ability of a wearer during walking using a wearable robot comprising a drive unit, the method comprises a step of receiving, from a server device, exercise information based on a database according to a correlation between the ratio of power to torque of the drive unit calculated by the drive unit and a wearer's physical condition; and a step of displaying the received exercise information.

In a server device for managing an exercise of a wearable robot wearer, the server device may receive, from a first user terminal, walking information of a wearer wearing a wearable robot interworking with the first user terminal, and may transmit the received walking information of the wearer to a second user terminal.

Further, the server device may receive, from the second user terminal, feedback information based on the walking information of the wearer received by the second user terminal.

Further, the feedback information based on the walking information of the wearer received by the second user terminal may comprise an exercise intensity based on the walking information of the wearer.

Further, the feedback information based on the walking information of the wearer received by the second user terminal comprises information for controlling, in real time, an exercise intensity based on the walking information of the wearer.

In a system for managing an exercise of a wearer wearing a wearable robot capable of estimating a physical ability of the wearer during a walking exercise, the system comprises a first user terminal that outputs analysis information on the exercise of the wearer; a server device that receives the analysis information on the exercise of the wearer output by the first user terminal; and a second user terminal that receives the analysis information on the exercise of the wearer transmitted by the server device.

Further, the server device may receive, from the second user terminal, feedback information based on information on the exercise of the wearer transmitted to the second user terminal, and may transmit the received feedback information to the first user terminal.

Further, the feedback information received by the server device comprises an exercise intensity based on the walking information of the wearer.

Further, the analysis information on the exercise of the wearer output by the first user terminal is based on the ratio of power to torque calculated by the wearable robot.

Further, the analysis information on the exercise of the wearer output by the first user terminal is at least any one or more of an agility or a lower body muscular strength of the wearer.

A method of generating a graphical user interface for providing information on walking of a wearable robot wearer according to an embodiment of the present invention may be a method of generating a graphical user interface for providing information on walking of a wearable robot wearer, the method comprising a step of receiving real-time walking information of the wearer from the wearable robot; and a step of generating a graphical user interface based on the received real-time walking information of the wearer.

Further, the graphical user interface may be a method of generating a graphical user interface for providing information on walking of a wearable robot wearer, in which the graphical user interface comprises at least one of a state information display area of the wearable robot, a display area of the received real-time walking information of the wearer, an exercise start/end selection area, a walking map selection area, and a robot exercise setting area.

Further, the graphical user interface may be a method of generating a graphical user interface for providing information on walking of a wearable robot wearer, in which the graphical user interface comprises an area displaying information on walking of a plurality of wearable robot wearers, and the area comprises a robot exercise setting area capable of controlling each of the plurality of wearable robots.

Further, the graphical user interface may be a method of generating a graphical user interface for providing information on walking of a wearable robot wearer, in which a position of the robot exercise setting area may be fixed.

Further, the state information display area of the wearable robot may be a method of generating a graphical user interface for providing information on walking of a wearable robot wearer, in which the state information display area of the wearable robot comprises at least one of a usage time information display area of the wearable robot, a battery remaining amount information display area, and a torque gauge bar information display area, and the torque gauge bar is displayed in real time in proportion to a torque value applied by the wearable robot to the wearer based on a hip angle of the wearer.

Further, the display area of the received real-time walking information of the wearer may be a method of generating a graphical user interface for providing information on walking of a wearable robot wearer, in which the display area of the received real-time walking information of the wearer comprises at least one of a distance information display area of the wearer, a number of steps information display area, a speed information display area, and a consumed calorie information display area.

Further, the robot exercise setting area may be a method of generating a graphical user interface for providing information on walking of a wearable robot wearer, in which the robot exercise setting area is an area for setting at least one of a walking mode and a torque intensity, the walking mode comprises at least one torque transfer mode, each of the torque transfer modes is a mode for transferring a torque corresponding to a walking purpose, and the torque intensity comprises at least one of a plurality of levels providing a differential torque.

Further, the robot exercise setting area may be a method of generating a graphical user interface for providing information on walking of a wearable robot wearer, in which an area for setting the walking mode and the torque intensity may be separated.

Further, the step of generating a graphical user interface based on the received real-time walking information of the wearer may be a method of generating a graphical user interface for providing information on walking of a wearable robot wearer, the step comprising a step of receiving, from a user terminal, input information based on the walking map selection area; a step of collecting current location information of the wearer after receiving the input information; a step of generating a walking map based on the collected current location information of the wearer and a map development tool; and a step of generating a graphical user interface displaying the walking map based on the generated walking map and the received real-time walking information of the wearer.

Further, the graphical user interface displaying the walking map may be a method of generating a graphical user interface for providing information on walking of a wearable robot wearer, in which the graphical user interface displaying the walking map is at least one of a walking map display area comprising the current location information and distance information of the wearer and a walking time information display area of the wearer.

Further, the step of receiving real-time walking information of the wearer from the wearable robot may be a method of generating a graphical user interface for providing information on walking of a wearable robot wearer, the step comprising a step of transmitting at least one of the walking mode and torque intensity information set in a user terminal to the wearable robot; and a step of receiving, after the transmitting, the real-time walking information of the wearer generated in the wearable robot based on at least one of the set walking mode and torque intensity information.

Further, the step of transmitting at least one of the set walking mode and torque intensity information may be a method of generating a graphical user interface for providing information on walking of a wearable robot wearer, the step comprising a step of displaying, on the user terminal, a graphical user interface displaying the robot exercise setting area; and a step of receiving an input of a setting of at least one of the walking mode and the torque intensity from the user terminal and transmitting it to the wearable robot.

A method of generating a graphical user interface for providing information on walking of a wearable robot wearer according to an embodiment of the present invention may be a method of generating a graphical user interface for providing information on walking of a wearable robot wearer, the method comprising a step of receiving walking information of the wearer from the wearable robot; a step of generating walking analysis information of the wearer based on the received walking information of the wearer; and a step of generating a graphical user interface based on the generated walking analysis information of the wearer.

Further, the graphical user interface may be a method of generating a graphical user interface for providing information on walking of a wearable robot wearer, in which the graphical user interface is a graphical user interface displaying statistical information according to accumulated walking information of the wearer, and the graphical user interface displaying the statistical information according to the accumulated walking information of the wearer comprises at least one of a category selection area, a statistical information display area according to the accumulated walking information, an entire walking information display area of a selected date, and a per-walking record information display area of a selected date.

Further, the statistical information display area according to the accumulated walking information may be a method of generating a graphical user interface for providing information on walking of a wearable robot wearer, in which the statistical information display area according to the accumulated walking information may represent the statistical information according to the accumulated walking information by a bar or a chart, may comprise an average trend line representing an average of the statistical information according to the accumulated walking information displayed by the bar or the chart, the bar or the chart may have a plurality of areas according to dates, and the plurality of areas have relative sizes in proportion to the statistical information according to the accumulated walking information.

Further, the entire walking information display area of the selected date may be a method of generating a graphical user interface for providing information on walking of a wearable robot wearer, in which the entire walking information display area of the selected date comprises at least one of a number-of-steps information display area, a distance information display area, a consumed calorie information display area, and a walking time information display area.

Further, the per-walking record information display area of the selected date may be a method of generating a graphical user interface for providing information on walking of a wearable robot wearer, in which the per-walking record information display area of the selected date comprises a plurality of areas displaying the per-walking record information of the selected date, and comprises an area displaying per-walking detailed record information based on input information of at least one of the plurality of areas.

Further, the graphical user interface may be a method of generating a graphical user interface for providing information on walking of a wearable robot wearer, in which the graphical user interface is a graphical user interface displaying a walking analysis report of the wearer, and the graphical user interface displaying the walking analysis report of the wearer comprises at least one of a comprehensive walking score and age information display area, a statistical information display area according to accumulated walking information, a walking index diagramming area, and a walking detailed analysis information display area.

Further, the statistical information display area according to the accumulated walking information may be a method of generating a graphical user interface for providing information on walking of a wearable robot wearer, in which the statistical information display area according to the accumulated walking information may represent the statistical information according to the accumulated walking information by a bar or a chart, may comprise an average trend line representing an average of the statistical information according to the accumulated walking information displayed by the bar or the chart, the bar or the chart may have a plurality of areas according to dates, and the plurality of areas have relative sizes in proportion to the statistical information according to the accumulated walking information.

Further, the walking index diagramming area may be a method of generating a graphical user interface for providing information on walking of a wearable robot wearer, in which the walking index diagramming area may represent a walking ability of the wearer by a figure so as to compare a score per walking index of the wearer with a score per walking index according to an age average, and the score per walking index is a score of speed, agility, muscular strength, stability, and balance.

Further, the walking detailed analysis information display area may be a method of generating a graphical user interface for providing information on walking of a wearable robot wearer, in which the walking detailed analysis information display area comprises at least one of the score display area per walking index and a feedback display area according to the score per walking index.

Further, the step of generating the walking analysis information of the wearer may be a method of generating a graphical user interface for providing information on walking of a wearable robot wearer, the step comprising a step of receiving the walking information of the wearer from the wearable robot; a step of generating physical/exercise ability information of the wearer based on the received walking information of the wearer; and a step of generating the walking analysis information of the wearer based on at least one of the generated physical/exercise ability information of the wearer and statistical information according to accumulated walking information of the wearer stored in an external server.

Further, the step of receiving the walking information of the wearer may be a method of generating a graphical user interface for providing information on walking of a wearable robot wearer, the step comprising a step of transmitting at least one of the walking mode and torque intensity information set in a user terminal to the wearable robot; a step of receiving, after the transmitting, the real-time walking information of the wearer generated in the wearable robot based on at least one of the set walking mode and torque intensity information; and a step of receiving a quantified physical/exercise ability value of the wearer generated based on the received real-time walking information of the wearer.

Further, the step of transmitting at least one of the set walking mode and torque intensity information may be a method of generating a graphical user interface for providing information on walking of a wearable robot wearer, the step comprising a step of displaying, on the user terminal, a graphical user interface displaying the robot exercise setting area; and a step of receiving an input of a setting of at least one of the walking mode and the torque intensity at the user terminal and transmitting at least one of the set walking mode and torque intensity information to the wearable robot.

Further, the step of generating a graphical user interface based on the generated walking analysis information of the wearer may be a method of generating a graphical user interface for providing information on walking of a wearable robot wearer, in which the step is preceded by a step of receiving an input of an exercise end at a user terminal and transmitting the received exercise end information to the wearable robot.

Further, it may be a method of generating a graphical user interface for providing information on walking of a wearable robot wearer, in which, after receiving the input of the exercise end information, in a case where a necessary condition for generating the walking analysis information of the wearer is not satisfied, a graphical user interface displaying a notification that the walking analysis information has not been generated is generated.

A method of generating a graphical user interface for providing information on walking of a wearable robot wearer according to an embodiment of the present invention may be a method of generating a graphical user interface for providing information on walking of a wearable robot wearer, the method comprising a step of acquiring information on walking of the wearer; a step of training a deep learning model based on the acquired information on walking of the wearer; a step of estimating walking analysis information of the wearer based on the trained deep learning model; and a step of generating a graphical user interface based on the estimated walking analysis information of the wearer.

Further, the graphical user interface may be a method of generating a graphical user interface for providing information on walking of a wearable robot wearer, in which the graphical user interface is a graphical user interface displaying a walking ability estimation report of the wearer, and the graphical user interface displaying the walking ability estimation report of the wearer comprises at least one of an estimated comprehensive walking score display area, a basic information display area of the wearer, an estimated walking index diagramming area, and an estimated walking detailed analysis information display area.

Further, the estimated walking index diagramming area may be a method of generating a graphical user interface for providing information on walking of a wearable robot wearer, in which the estimated walking index diagramming area may represent the estimated walking ability of the wearer by a figure so as to compare an estimated score per walking index of the wearer with a score per walking index according to an age average, and the estimated score per walking index is a score of speed, agility, muscular strength, stability, and balance.

Further, the estimated walking detailed analysis information display area may be a method of generating a graphical user interface for providing information on walking of a wearable robot wearer, in which the estimated walking detailed analysis information display area comprises at least one of the estimated score display area per walking index and a feedback display area according to the estimated score per walking index.

Further, the graphical user interface may be a method of generating a graphical user interface for providing information on walking of a wearable robot wearer, in which the graphical user interface is a graphical user interface displaying customized program information of the wearer, and the graphical user interface displaying the customized program information of the wearer comprises at least one of a suggested customized program display area, a comprehensive walking score display area, a walking index diagramming area, and a detailed content display area of the suggested program.

Further, the comprehensive walking score display area may be a method of generating a graphical user interface for providing information on walking of a wearable robot wearer, in which the comprehensive walking score display area represents an expected walking score of the wearer that is expected to be improved after performing the suggested customized program.

Further, the walking index diagramming area may be a method of generating a graphical user interface for providing information on walking of a wearable robot wearer, in which the walking index diagramming area may represent a walking ability of the wearer by a figure so as to compare an estimated score per walking index of the wearer and a score per walking index according to an age average with a score per walking index of the expected walking score of the wearer, and the score per walking index is a score of speed, agility, muscular strength, stability, and balance.

Further, the step of training the deep learning model may be a method of generating a graphical user interface for providing information on walking of a wearable robot wearer, in which, in a case where the acquired information on walking of the wearer is of a plurality of different forms, the acquired information on walking of the wearer is combined into multimodal data, and a deep learning model is trained based on the combined multimodal data.

Further, the step of acquiring information on walking of the wearer may be a method of generating a graphical user interface for providing information on walking of a wearable robot wearer, the step comprising a step of receiving walking information of the wearer from the wearable robot; a step of generating walking analysis information of the wearer based on the received walking information of the wearer; and a step of acquiring the information on walking of the wearer based on the generated walking analysis information of the wearer.

Further, the step of generating the walking analysis information of the wearer may be a method of generating a graphical user interface for providing information on walking of a wearable robot wearer, the step comprising a step of receiving the walking information of the wearer from the wearable robot; a step of generating physical/exercise ability information of the wearer based on the received walking information of the wearer; and a step of generating the walking analysis information of the wearer based on at least one of the generated physical/exercise ability information of the wearer and statistical information according to accumulated walking information of the wearer stored in an external server.

Further, the step of receiving the walking information of the wearer may be a method of generating a graphical user interface for providing information on walking of a wearable robot wearer, the step comprising a step of transmitting at least one of the walking mode and torque intensity information set in a user terminal to the wearable robot; a step of receiving, after the transmitting, the real-time walking information of the wearer generated in the wearable robot based on at least one of the set walking mode and torque intensity information; and a step of receiving a quantified physical/exercise ability value of the wearer generated based on the received real-time walking information of the wearer.

Further, the step of transmitting at least one of the set walking mode and torque intensity information may be a method of generating a graphical user interface for providing information on walking of a wearable robot wearer, the step comprising a step of displaying, on the user terminal, a graphical user interface displaying the robot exercise setting area; and a step of receiving an input of a setting of at least one of the walking mode and the torque intensity at the user terminal and transmitting at least one of the set walking mode and torque intensity information to the wearable robot.

A non-transitory computer-readable storage medium storing one or more programs configured to be executed by one or more processors to generate a graphical user interface displaying information on walking of a wearable robot wearer according to an embodiment of the present invention may be a non-transitory computer-readable storage medium, in which the one or more programs comprise instructions to perform at least one of the above-described methods.

An apparatus for generating a graphical user interface for providing information on walking of a wearable robot wearer according to an embodiment of the present invention may be an apparatus for generating a graphical user interface for providing information on walking of a wearable robot wearer, the apparatus comprising an input unit and a communication unit; a display unit; one or more processor units; and a memory unit that stores one or more programs configured to be executed by the one or more processors - the one or more programs comprising instructions for performing at least one of the above-described methods -.

The present invention is, in a walking assistance wearable robot for assisting a walking exercise of a wearer, a walking assistance wearable robot comprising a fixing unit that may be mounted on a body part of the wearer; and a drive unit that may be mounted on the fixing unit; and the drive unit is configured to calculate a degree of change of a state trajectory of the wearer accumulated during a predetermined time and to calculate an output torque based thereon.

Further, the predetermined time is at or above 0.5 seconds and at or below 1 second.

Further, the drive unit may be configured to, in a case where a movement distance of the state trajectory of the wearer during the predetermined time is at or below a first threshold, not calculate the output torque or calculate it as a value at or below a threshold, and may be configured to, in a case where the movement distance of the state trajectory of the wearer during the predetermined time is at or above a second threshold, calculate the output torque as a value at or above a threshold.

Further, the first threshold and the second threshold are configured as the same value, or the second threshold is configured as a value larger than the first threshold.

Further, the drive unit is configured to, in a case where the movement distance of the state trajectory of the wearer during the predetermined time is at or below a first threshold, change a value of a torque intensity adjustment parameter for calculating the output torque to at or below a threshold or change it to 0.

Further, the drive unit is configured to gradually change the value of the torque intensity adjustment parameter for calculating the output torque to at or below a threshold or 0.

Further, the drive unit is configured to, in a case where the movement distance of the state trajectory of the wearer during the predetermined time is at or above a second threshold, restore a value of the torque intensity adjustment parameter for calculating the output torque to a set value.

Further, the drive unit is configured to gradually restore the value of the torque intensity adjustment parameter for calculating the output torque to a set value.

The present invention is, in a walking assistance wearable robot for assisting a walking exercise of a wearer, a walking assistance wearable robot comprising a fixing unit that may be mounted on a body part of the wearer; and a drive unit that may be mounted on the fixing unit; and the drive unit is configured to calculate an output torque in a case where a movement distance of the state trajectory of the wearer accumulated during a predetermined time is at or above a threshold and a number of consecutive steps after a stop of the wearer is at or above a predetermined number of steps.

Further, the drive unit is configured to, in a case where the movement distance of the state trajectory of the wearer during the predetermined time is at or above a threshold, restore a value of the torque intensity adjustment parameter for calculating the output torque to a set value.

Further, the drive unit is configured to gradually restore the value of the torque intensity adjustment parameter for calculating the output torque to a set value.

Further, the drive unit is configured to count a number of consecutive steps after a stop of the wearer by counting a point in time at which a motion state value of the wearer is 0.

Further, the drive unit is configured to count a number of consecutive steps after a stop of the wearer by counting a point in time at which a target body part on one side and a target body part on the other side of the wearer cross.

Further, the drive unit is configured to count a number of consecutive steps after a stop of the wearer by counting a point in time at which a motion state value of the wearer has a predetermined value.

The present invention is, in a walking assistance wearable robot for assisting a walking exercise of a wearer, a walking assistance wearable robot comprising a fixing unit that may be mounted on a body part of the wearer; and a drive unit that may be mounted on the fixing unit; and the drive unit is configured to calculate a degree of change of the state trajectory of the wearer and to calculate an output torque based thereon, and, in a range at or below a threshold including a point in time at which a sign of the calculated output torque changes, to calculate the output torque as a value at or below a threshold during a predetermined time.

Further, the predetermined time is at or above 0.5 seconds and at or below 1 second.

Further, the drive unit may be configured to, in a case where a movement distance of the state trajectory of the wearer during the predetermined time is at or below a first threshold, not calculate the output torque or calculate it as a value at or below a threshold, and may be configured to, in a case where the movement distance of the state trajectory of the wearer during the predetermined time is at or above a second threshold, calculate the output torque as a value at or above a threshold.

Further, the first threshold and the second threshold are configured as the same value, or the second threshold is configured as a value larger than the first threshold.

Further, the drive unit is configured to, in a case where the movement distance of the state trajectory of the wearer during the predetermined time is at or below a first threshold, change a value of a torque intensity adjustment parameter for calculating the output torque to at or below a threshold or change it to 0.

The present invention is, in a method of calculating an output torque of a drive unit of a walking assistance wearable robot for assisting a walking exercise of a wearer, a method comprising a step of calculating a movement distance of the state trajectory of the wearer accumulated during a predetermined time; a step of counting a number of consecutive steps after a stop of the wearer; and a step of calculating an output torque in a case where the calculated movement distance of the state trajectory of the wearer is at or above a threshold and the counted number of consecutive steps after the stop is at or above a predetermined number of steps.

The present invention is, in a method of calculating an output torque of a drive unit of a walking assistance wearable robot for assisting a walking exercise of a wearer, a method comprising a step of calculating a movement distance of the state trajectory of the wearer accumulated during a predetermined time; a step of calculating an output torque based on the calculated movement distance of the state trajectory of the wearer; and a step of calculating, in a range at or below a threshold including a point in time at which a sign of the calculated output torque changes, the output torque as a value at or below a threshold during a predetermined time.

In a walking assistance wearable robot for assisting a walking exercise of a wearer that receives target muscle information to be exercised from a user terminal, the walking assistance wearable robot comprises a fixing unit that may be mounted on a body part of the wearer; and a drive unit that may be mounted on the fixing unit; and the drive unit is configured to set a parameter based on the received target muscle information and to calculate an output torque based thereon.

Further, the drive unit is configured to calculate an angular velocity based on walking of the wearer, to calculate a motion state value based on the calculated angular velocity, and to calculate the output torque based thereon.

Further, the set parameter is a parameter representing at least one piece of information among output timing information of the torque to be calculated, intensity adjustment information of the torque to be calculated, filter information for calculating the output torque, or asymmetry information for calculating the output torque.

Further, the output timing information of the torque to be calculated comprises position information of the calculated motion state value.

Further, the intensity adjustment information of the torque to be calculated may be expressed by a torque intensity adjustment parameter, and the torque intensity adjustment parameter is a negative number in a case where the drive unit outputs a resistance torque, and is a positive number in a case where the drive unit outputs an assistance torque.

Further, the drive unit is configured to calculate the output torque by multiplying a weighted sum of the calculated motion state values by the torque intensity adjustment parameter.

Further, it may further comprise a memory unit, the drive unit is configured to be able to calculate an output torque according to a set exercise mode, the memory unit is configured to store a parameter according to the exercise mode as a lookup table, and the set parameter is configured as a parameter according to the target muscle information received from the user terminal based on the lookup table.

Further, the lookup table is composed of a combination of at least one of output timing information of the torque to be calculated, intensity adjustment information of the torque to be calculated, filter information for calculating the output torque, or asymmetry information for calculating the output torque.

Further, it may further comprise a memory unit, the memory unit may store an exercise mode based on the received target muscle information and a parameter according to the exercise mode in a form of a lookup table, the drive unit may select an exercise mode based on the received target muscle information, and is configured to calculate the output torque based on a parameter according to the selected exercise mode.

Further, the drive unit may receive the parameter value from the user terminal, and is configured to calculate the output torque based thereon.

The present invention is, in a walking assistance wearable robot system for assisting a walking exercise of a wearer, the system comprising the user terminal and the wearable robot, in which the wearable robot may receive target muscle information to be exercised from the user terminal, is configured to set a parameter based on the received target muscle information, to calculate an output torque based thereon, and to transmit information related to the walking exercise to the user terminal, and the user terminal is configured to receive the transmitted information related to the walking exercise.

Further, the wearable robot is configured to calculate an angular velocity based on walking of the wearer, to calculate a motion state value based on the calculated angular velocity, and to calculate the output torque based thereon.

Further, the set parameter is a parameter representing at least one piece of information among output timing information of the torque to be calculated, intensity adjustment information of the torque to be calculated, filter information for calculating the output torque, or asymmetry information for calculating the output torque.

Further, the output timing information of the torque to be calculated comprises position information of the calculated motion state value.

Further, the intensity adjustment information of the torque to be calculated may be expressed by a torque intensity adjustment parameter, and the torque intensity adjustment parameter is a negative number in a case where the drive unit outputs a resistance torque, and is a positive number in a case where the drive unit outputs an assistance torque.

Further, the wearable robot is configured to calculate the output torque by multiplying a weighted sum of the calculated motion state values by the torque intensity adjustment parameter.

Further, the wearable robot is configured to be able to calculate an output torque according to a set exercise mode, is configured to store a parameter according to the exercise mode as a lookup table, and the set parameter is configured as a parameter according to the target muscle information received from the user terminal based on the lookup table.

Further, the lookup table is composed of a combination of at least one or more of at least one piece of information among output timing information of the torque to be calculated, intensity adjustment information of the torque to be calculated, filter information for calculating the output torque, or asymmetry information for calculating the output torque.

Further, the wearable robot may store an exercise mode based on the received target muscle information and a parameter according to the exercise mode in a form of a lookup table, may select an exercise mode based on the received target muscle information, and is configured to calculate the output torque based on a parameter according to the selected exercise mode.

Further, the wearable robot may receive the parameter value from the user terminal, and is configured to calculate the output torque based thereon.

Further, the user terminal is configured to provide audiovisual feedback information based on the received information related to the walking exercise, and the audiovisual feedback information comprises exercise score information of the wearer, walking performance information of the wearer, or exercise information of the wearer.

Further, the walking performance information comprises at least one of speed information of the wearer, posture information of the wearer, stride information of the wearer, regularity information of the wearer, asymmetry information of the wearer, and walking score information of the wearer.

Further, the exercise information comprises at least one of exercise elapsed time information per exercise mode of the wearer, accumulated exercise time information of the wearer, and current exercise mode exercise elapsed time information of the wearer.

Further, the exercise score information is configured to be calculated based on the movement distance of the state trajectory of the wearer.

Further, the exercise score information is configured to be calculated based on a median value or an average value of the movement distance of the state trajectory of the wearer during a predetermined time.

Further, the user terminal is configured to provide the calculated exercise score information after normalizing it to a score of 100 points.

The present invention is, in a walking assistance wearable robot for assisting a walking exercise of a wearer that receives target exercise information to be exercised from a user terminal, a walking assistance wearable robot comprising a fixing unit that may be mounted on a body part of the wearer; and a drive unit that may be mounted on the fixing unit; and the drive unit is configured to set a parameter based on the received target exercise information and to calculate an output torque based thereon.

The present invention is, in a walking assistance wearable robot system for assisting a walking exercise of a wearer, the system comprising the user terminal and the wearable robot, in which the wearable robot may receive target exercise information to be exercised from the user terminal, is configured to set a parameter based on the received target exercise information, to calculate an output torque based thereon, and to transmit information related to the walking exercise to the user terminal, and the user terminal is configured to receive the transmitted information related to the walking exercise.

The present invention is, in a waist wearing unit of a walking assistance wearable robot for assisting a walking exercise of a wearer, a waist wearing unit comprising a waist wearing unit that may be mounted on a waist of the wearer; and a drive unit that may be mounted on the waist wearing unit; in which the waist wearing unit comprises a waist wearing unit member; a waist wearing buckle unit connected to the waist wearing unit member; the drive unit comprises a drive unit fixing frame positioned on both sides of the drive unit; and the waist wearing buckle unit is configured to be able to be coupled to and decoupled from the drive unit fixing frame.

Further, the drive unit fixing frame is configured to have an inclination of at or above 0 degrees and at or below 30 degrees with respect to a vertical plane of the drive unit.

Further, the waist wearing buckle unit comprises a drive unit fixing member, the drive unit fixing member further comprises an inner connecting member, the drive unit fixing member further comprises a button member positioned on the inner connecting member; and an elastic member positioned inside the waist wearing buckle unit; and, upon coupling of the waist wearing buckle unit and the drive unit fixing frame, the button member is configured to move in a traveling direction of the drive unit fixing frame, and accordingly the elastic member is configured to contract, and, upon coupling of the button member and the drive unit fixing frame, the elastic member relaxes to be configured to prevent separation of the drive unit fixing frame to the button member.

Further, the button member is configured to have an inclined portion, and, upon coupling of the waist wearing buckle unit and the drive unit fixing frame, the drive unit fixing frame is configured to contact the inclined portion of the button member and move in a traveling direction.

Further, the waist wearing buckle unit comprises a drive unit fixing member, the drive unit fixing member further comprises an inner connecting member, and further comprises a button member positioned on the inner connecting member; a handle member positioned on the inner connecting member; and an elastic member positioned inside the waist wearing buckle unit; and, upon decoupling of the waist wearing buckle unit and the drive unit fixing frame, the handle member moves in the traveling direction of the drive unit fixing frame at the time of the coupling, and, according to the movement of the inner connecting member in the traveling direction, the elastic member moves in the traveling direction, so that the coupling of the button member and the drive unit fixing frame is configured to be released.

Further, the waist wearing unit member and the waist wearing buckle unit are configured to be fixed by a band.

Further, the waist wearing unit member and the waist wearing buckle unit are configured to be fixed by Velcro.

The present invention is, in a waist wearing unit of a walking assistance wearable robot for assisting a walking exercise of a wearer, a waist wearing unit comprising a waist wearing unit that may be mounted on a waist of the wearer; and a drive unit that may be mounted on the waist wearing unit; in which the waist wearing unit comprises a waist wearing unit member; a waist wearing buckle unit connected to the waist wearing unit member; and the waist wearing buckle unit further comprises a drive unit fixing member that may be coupled to the drive unit; and a swing member connected to the drive unit fixing member.

Further, the swing member is configured to connect the waist wearing unit member and the waist wearing buckle unit.

Further, the swing member is configured to be able to swing at an angle of at or above 0 degrees and at or below 30 degrees with respect to a pre-swing posture.

Further, the drive unit comprises a drive unit fixing frame positioned on both sides of the drive unit, and the waist wearing buckle unit is configured to be able to be coupled to and decoupled from the drive unit fixing frame.

Further, the drive unit fixing frame is configured to have an inclination of at or above 0 degrees and at or below 30 degrees with respect to a vertical plane of the drive unit.

Further, the waist wearing buckle unit further comprises a button member positioned on an inner side of the waist wearing buckle unit; and an elastic member positioned inside the waist wearing buckle unit; and, upon coupling of the waist wearing buckle unit and the drive unit fixing frame, the button member is configured to move in a traveling direction of the drive unit fixing frame, and accordingly the elastic member is configured to contract, and, upon coupling of the button member and the drive unit fixing frame, the elastic member relaxes to be configured to prevent separation of the drive unit fixing frame to the button member.

Further, the button member is configured to have an inclined portion, and, upon coupling of the waist wearing buckle unit and the drive unit fixing frame, the drive unit fixing frame is configured to contact the inclined portion of the button member and move in a traveling direction.

Further, the waist wearing buckle unit further comprises an inner connecting member, and further comprises a button member positioned on the inner connecting member; a handle member positioned on the inner connecting member; and an elastic member positioned inside the waist wearing buckle unit; and, upon decoupling of the waist wearing buckle unit and the drive unit fixing frame, the handle member moves in the traveling direction of the drive unit fixing frame at the time of the coupling, and, according to the movement of the inner connecting member in the traveling direction, the elastic member moves in the traveling direction, so that the coupling of the button member and the drive unit fixing frame is configured to be released.

Further, the waist wearing unit member and the waist wearing buckle unit are configured to be fixed by a band.

Further, the waist wearing unit member and the waist wearing buckle unit are configured to be fixed by Velcro.

The present invention is, in a walking assistance wearable robot for assisting a walking exercise of a wearer, a walking assistance wearable robot comprising a waist wearing unit that may be mounted on a waist of the wearer; and a drive unit that may be mounted on the fixing unit; in which the waist wearing unit comprises a waist wearing unit member; a waist wearing buckle unit connected to the waist wearing unit member; the waist wearing buckle unit further comprises a drive unit fixing member that may be coupled to the drive unit; and a swing member connected to the drive unit fixing member; the drive unit comprises a drive unit fixing frame positioned on both sides of the drive unit; the drive unit fixing frame has an inclination of at or above 0 degrees and at or below 30 degrees with respect to a vertical plane of the drive unit; and the waist wearing unit member and the waist wearing buckle unit are configured to be connected by the swing member.

Further, the drive unit fixing frame is configured to have an inclination of at or above 5 degrees and at or below 20 degrees with respect to a vertical plane of the drive unit.

The present invention is, in a wearable robot for assisting a walking exercise, a wearable robot comprising a waist wearing unit that may be mounted on a waist of a wearer; and a drive unit that is coupled to the waist wearing unit to assist walking of the wearer; in which the waist wearing unit comprises a waist wearing member that may be worn in close contact with the waist of the wearer; and a waist wearing buckle unit connected to the waist wearing member to couple with the drive unit; and the waist wearing buckle unit comprises a drive unit fixing portion that may couple with the drive unit; and a swing member connected to the drive unit fixing member to permit a relative movement between the drive unit and the waist wearing unit.

Further, the swing member is configured to connect the waist wearing unit member and the waist wearing buckle unit.

Further, the swing member is configured to be able to swing at an angle of at or above 0 degrees and at or below 30 degrees with respect to a pre-swing posture.

The present invention is, in a thigh wearing unit of a walking assistance wearable robot for assisting a walking exercise of a wearer, a thigh wearing unit comprising a waist wearing unit that may be mounted on a waist of the wearer; a thigh wearing unit that may be mounted on a thigh of the wearer; a drive unit connected to the waist wearing unit; and a thigh frame connecting the drive unit and the thigh wearing unit; in which the thigh frame comprises a thigh frame joint portion; the thigh wearing unit comprises a fixing clip portion; and the fixing clip portion is configured to be able to fix or separate the thigh frame joint portion.

Further, the thigh wearing unit comprises a thigh frame Release button for switching the thigh frame joint portion from a fixed state to a released state.

Further, the thigh frame Release button further comprises a thigh frame Release elastic member, and the thigh frame Release elastic member is configured to provide a return force so as to be able to automatically return to an original position after operation of the thigh frame Release button.

Further, the thigh wearing unit further comprises a thigh band portion; and a thigh buckle portion, and the thigh band portion is connected to the thigh buckle portion and is configured to be able to adjust a length to match a circumference of the thigh of the wearer.

Further, the thigh frame joint portion is designed in a round shape so as to be rotatable, and is configured to rotate flexibly according to a thigh movement of the wearer.

Further, the thigh wearing unit comprises a thigh attachable/detachable frame, the thigh attachable/detachable frame further comprises a sliding rail member, and the thigh frame joint portion is configured to be able to move along the sliding rail member.

Further, the thigh wearing unit comprises an impact prevention member, and the impact prevention member is positioned on an inner side of the thigh wearing unit and is configured to be able to mitigate an impact or vibration occurring during a sliding operation of the thigh frame.

Further, the sliding rail member further comprises a linear guide member on the thigh attachable/detachable frame, and the linear guide member is configured to support the sliding movement of the thigh frame joint portion.

Further, the impact prevention member is configured to absorb impact energy occurring while the thigh frame joint portion slides.

The present invention is, in a thigh frame of a walking assistance wearable robot for assisting a walking exercise of a wearer, a thigh frame comprising a waist wearing unit that may be mounted on a waist of the wearer; a thigh wearing unit that may be mounted on a thigh of the wearer; a drive unit connected to the waist wearing unit and comprising an actuator; and a thigh frame connecting the drive unit and the thigh wearing unit; in which the thigh frame comprises a joint member connecting the drive unit and the thigh frame; a thigh cover member connected to the joint member; and a thigh inner member that slides according to the walking exercise of the wearer inside the thigh cover member.

Further, the joint member is connected in series with the actuator and is configured to transfer an assistance force generated from the actuator to the thigh frame.

Further, the joint member is configured to enable a swing operation coinciding with a walking direction of the wearer and a swing operation in a direction perpendicular to the walking direction.

Further, the joint member comprises a joint cap member serving as a cover of the joint member; and a joint shaft member serving as a shaft of the joint member; and the joint member is coupled with the joint cap member and the joint shaft member to be configured to enable a swing operation coinciding with a walking direction of the wearer and a swing operation in a direction perpendicular to the walking direction.

Further, the thigh cover member is configured to comprise a rail structure of a predetermined angle formed symmetrically left and right inside the thigh cover member.

Further, the thigh inner member comprises a structure of a predetermined angle formed symmetrically left and right, and is configured to prevent a left-right tilting phenomenon when sliding inside the thigh cover member.

Further, it further comprises a bearing end member, the thigh inner member is coupled with the bearing end member, and the bearing end member is configured to prevent left-right play upon sliding of the thigh inner member, and to distribute a load applied to the thigh frame upon generation of the assistance force of the actuator.

Further, the thigh inner member comprises a 2-stage bearing member, and the 2-stage bearing member is characterized in that it operates perpendicular to a sliding direction of the thigh inner member to maintain a left-right balance and to prevent a left-right tilting that may occur upon sliding.

Further, the thigh inner member further comprises a 2-stage auxiliary bearing member, and the 2-stage auxiliary bearing member is characterized in that it is configured to, together with the 2-stage bearing member, prevent a left-right movement of the thigh inner member and to maintain a constant Gap upon sliding.

Further, the thigh inner member comprises a 2.5-stage bearing member, and the 2.5-stage bearing member is configured such that the thigh inner member slides inward upon a stair-climbing, sitting, or squatting operation of the wearer.

Further, the thigh inner member further comprises a Distal return elastic member, and the Distal return elastic member is characterized in that it assists so that the thigh inner member can return to an initial state after a sliding operation.

Further, the thigh cover member comprises a rail structure having a constant angle symmetrical left and right inside the thigh cover member, and the rail structure is characterized in that it is configured to suppress a left-right tilting phenomenon while the thigh inner member slides.

Further, the thigh cover member performs a sliding guide role of the thigh inner member in a process of transferring the assistance force generated by the actuator to the thigh inner member, and the left-right symmetrical rail structure formed on the inner side of the thigh cover member is configured to prevent left-right tilting while the thigh inner member slides.

Further, the thigh inner member is coupled with the thigh wearing unit and is configured to be slidable so that a length value can change to match a leg length of the wearer, and the sliding operation is characterized in that it is divided into 2-stage and 2.5-stage sliding and can be adjusted according to an operation of the wearer.

Further, the thigh inner member comprises a 2-stage return elastic band member, and the 2-stage return elastic band member is configured to provide an elastic force so that the thigh inner member can return to an original position after a sliding operation.

Further, the thigh frame comprises a bearing cover member, and the bearing cover member is configured to perform a role of protecting a bearing disposed on an inner side of the thigh inner member so that it does not separate during sliding.

Further, the thigh frame comprises a front urethane bearing member and a rear urethane bearing member, and the front urethane bearing member and the rear urethane bearing member are configured to suppress vibration and noise occurring during a sliding operation of the thigh inner member and to maintain a left-right balance of the thigh frame.

Further, the front urethane bearing member and the rear urethane bearing member are characterized in that they are disposed at the same angle as an inner rail of the thigh cover member so as to be configured to minimize vibration that may occur during a sliding process.

Further, the thigh inner member comprises a 2.5-stage bearing member and a Distal return elastic member, and the Distal return elastic member is configured to provide an elastic force so that the 2.5-stage bearing member can return to an original position after sliding.

The present invention is, in a walking assistance wearable robot for assisting a walking exercise of a wearer, a walking assistance wearable robot comprising a waist wearing unit that may be mounted on a waist of the wearer; a thigh wearing unit that may be mounted on a thigh of the wearer; a drive unit connected to the waist wearing unit and comprising an actuator; and a thigh frame connecting the drive unit and the thigh wearing unit; in which the thigh frame comprises a joint member connecting the drive unit and the thigh frame; a thigh cover member connected to the joint member; and a thigh inner member that slides according to the walking exercise of the wearer inside the thigh cover member.

The above object is achieved, according to an embodiment of the present invention, by a wearable robot comprising a first fixing unit fixed to a body part on one side of a joint portion; a second fixing unit fixed to a body part on the other side of the joint portion; a drive unit fixed to the first fixing unit; and a connecting member that connects the drive unit and the second fixing unit and transfers a driving force provided from the drive unit to the second fixing unit for movement of the joint portion; in which the connecting member has a length adjusted in correspondence with a gap between the drive unit and the second fixing unit that varies according to an operation magnitude of the joint portion, or comprises a plurality of unit members arranged in a row to form the connecting member and an interlocking portion that connects the plurality of unit members so that the plurality of unit members interlock with each other.

Here, it may further comprise a rotation joint portion respectively coupled to left and right both sides of the drive unit and rotating in a front-rear direction about a left-right direction rotation axis, and the connecting member may be hinge-coupled to a lower end portion of the rotation joint portion so as to rotate in a left-right direction about a front-rear direction rotation axis.

Here, the drive unit comprises two actuators; and a main body housing accommodating the actuators therein, and the connecting members on both sides may rotate by respectively receiving power transferred from the actuators.

Here, the drive unit comprises a single actuator; an actuator frame surrounding and accommodating the actuator therein and rotating in a horizontal axial direction; and a main body housing accommodating the actuator and the actuator frame therein, and the connecting member disposed on one side of the main body portion rotates by receiving power transferred from the actuator, and the connecting member disposed on the other side of the drive unit may be connected to the actuator frame and rotate.

Here, the drive unit may further comprise a bearing mounted on an outer side of the actuator frame to rotate the actuator frame with respect to the main body housing.

Here, it may further comprise a first fixing unit fixing the drive unit to a waist; and a second fixing unit fixing the connecting member to the thigh.

Here, the connecting member may be formed of a plurality of links to be bendable.

Here, an upper end of the connecting member may be hinge-coupled so as to rotate in a left-right direction about a front-rear direction rotation axis.

Here, the connecting member may comprise a first member supported by the drive unit, a second member movably connected to the first member, and a third member movably connected to the second member and supported by the second fixing unit.

Further, a second slit capable of guiding the movement of the third member may be formed on one surface of the second member, and a first slit communicating with the second slit in an overlapping state with the second member may be formed on one surface of the first member.

Further, it may comprise an elastic member elastically supporting the second member or the third member in a contraction direction.

Further, a frictional force of the second member with respect to the first member and a frictional force of the third member with respect to the second member may be set to be different from each other.

Further, the second member may further comprise a roller that performs a rolling motion on a contact surface with the first member.

Further, the third member may further comprise a sliding pad that has a relatively larger frictional force than the roller and performs a sliding motion on a contact surface with the second member.

Further, the connecting member may comprise a first length adjustment portion and a second length adjustment portion arranged in a row between the drive unit and the second fixing unit.

Further, the first length adjustment portion has a length adjusted within a partial region of a movable range of the joint portion, and the second length adjustment portion may have a length adjusted in a remaining region exceeding the partial region of the movable range of the joint portion.

Further, the connecting member may comprise a permanent magnet capable of fixing a position in a state where the third member has moved in a direction in which it expands on the second member.

Further, the permanent magnet may comprise a first permanent magnet provided at a second end portion of the second member and a second permanent magnet provided at a position corresponding to the first permanent magnet of the third member.

A first end portion refers to an end portion of the first member or the second member with respect to an overlapping direction when the first member (141) and the second member (142) overlap, and a second end portion may refer to an end portion of the first member or the second member with respect to a direction opposite to the overlapping direction.

Further, the connecting member may further comprise an elastic member that has one end portion fixed to the first member and is fixed to the third member in a state where the other end portion is supported by the second member.

Further, the second member may comprise a first pulley supporting the elastic member.

Further, the interlocking portion may comprise a first cable that has one end portion fixed to the first member and the other end portion fixed to the third member in a state of being supported by a second end portion of the second member, and a second cable that has one end portion fixed to the first member and the other end portion fixed to the third unit member in a state of being supported by a first end portion of the second member.

Further, a second pulley supporting the first cable is disposed at a second end portion of the second member, and a third pulley supporting the second cable may be disposed at a first end portion of the second member.

Further, the interlocking portion may comprise a rack disposed along a longitudinal direction on the first member, a pinion disposed at a contraction-side end portion of the second member and engaging with the rack, a first pulley rotating together with the pinion, a second pulley disposed at an expansion-side end portion of the second member, and a belt that has both end portions fixed to the third member in a state of being wound around the first pulley and the second pulley.

Further, a second slit capable of guiding the movement of the third member may be formed on one side of the second member, and a first slit communicating with the second slit in an overlapping state with the second member may be formed on one side of the first member.

Further, in a case where the first fixing unit is worn on a waist, the waist wearing unit is preferably composed of a waist belt and a waist wearing frame. Both ends of the waist belt couple with both ends of the waist wearing frame, and a length of the waist belt may be adjusted according to a waist size.

Further, the waist wearing frame preferably comprises a detachment button, a lower mechanism portion, and an upper mechanism portion, and one surface of the main body housing may comprise a lower hook and an upper hook.

Further, in a case where the second fixing unit is worn on a thigh to function as a thigh wearing unit, the thigh wearing unit may be composed of a strap portion and a plate.

Further, the strap portion may comprise a second button that couples with one end of the strap portion.

Further, both ends of the strap portion may couple with both ends of the plate.

Further, at a coupling portion of both ends, a length of the strap portion may be adjusted or the strap portion may be separated from the plate.

Further, the plate may comprise a plate frame and a first button.

An operation assistance method according to an embodiment of the present invention comprises a step of sequentially storing motion state values in a state trajectory memory buffer; a step of selecting at least one motion state value among the motion state values stored in the state trajectory memory buffer; a step of determining an assistance force using the selected motion state value; and a step of outputting the determined assistance force.

Further, the step of storing the motion state values may store only a preset number of motion state values in a FIFO (First In First Out) manner.

Further, the motion state value may be a sensing value obtained by measuring a motion state at regular time intervals or a conversion value of the sensing value using a preset formula.

Further, in the step of determining the assistance force, the assistance force may be determined by a weighted sum of the selected motion state values.

Further, in the step of selecting the at least one motion state value, a motion state value stored at a predetermined position among the motion state values stored in the state trajectory memory buffer may be selected.

Further, it may further comprise, prior to the step of selecting the at least one motion state value, a step of calculating a state trajectory movement distance by summing differences between the motion state values sequentially stored in the state trajectory memory buffer; and a step of changing the predetermined position according to the state trajectory movement distance in the step of selecting the at least one motion state value.

Further, the larger the state trajectory movement distance, the more the predetermined position is changed toward a first storage position of a memory array in the state trajectory memory buffer, and the smaller the state trajectory movement distance, the more the predetermined position may be changed toward a last storage position of the memory array.

Further, the motion state value may be a conversion value obtained by converting a sensed hip angle value (q0) using a conversion formula SO=Asin(q0/2), and A may be a constant.

Further, an operation assistance system according to an embodiment of the present invention may comprise a state trajectory memory buffer that sequentially stores motion state values and a determination unit that determines an assistance force using one or more motion state values among the motion state values stored in the state trajectory memory buffer.

Further, the state trajectory memory buffer may be a finite-size memory buffer that stores the motion state values in a FIFO (First In First Out) manner.

Further, the state trajectory memory buffer may store motion state values for a user's movement during recent several seconds.

Further, the determination unit may select a motion state value stored at a predetermined position of a memory array in the state trajectory memory buffer, and change the predetermined position according to a change of the motion state values stored in the state trajectory memory buffer and select a motion state value stored at the changed position.

Further, an operation assistance program according to an embodiment of the present invention may be a program stored in a medium for being combined with hardware to execute respective steps of the operation assistance method according to an embodiment of the present invention.

Further, a safety mode according to an embodiment of the present invention may comprise a step of detecting an operation angle or an operation speed of the drive unit, and a step of stopping motor rotation of the drive unit to operate the safety mode.

### ADVANTAGEOUS EFFECTS

According to the present invention, it comprises a fixing unit that may be mounted on a body part; and a drive unit that may be mounted on the fixing unit; and the drive unit may estimate a physical ability of the wearer based on a power of the drive unit due to a walking exercise of the wearer.

According to the present invention, a method of estimating a physical ability of a wearer during walking using a wearable robot comprising a drive unit comprises a step of accumulating, for each walking, a ratio of power to torque of the drive unit calculated by the drive unit; and a step of calculating a statistic on the accumulation, and through this, a statistic on the accumulation of the ratio of power to torque of the drive unit calculated by the drive unit for each walking can be calculated and provided.

According to the present invention, it comprises a step of receiving, from a server device, a statistic on an accumulation obtained by accumulating, for each walking, a ratio of power to torque of the drive unit calculated by the drive unit; and a step of displaying the calculated statistic on the accumulation, and through this, the calculated statistic on the accumulation can be provided to the wearer.

According to the present invention, using a wearable walking exercise robot using a backdrivable actuator, the physical ability of the wearer can be grasped in real time using a power-torque ratio generated by the apparatus during a walking exercise.

According to the present invention, a graphical user interface displaying walking analysis information of a wearable robot wearer can be expected to have the following effects.

According to the present invention, the wearable robot, using a backdrivable actuator, quantifies a physical/exercise ability value of the wearer using a power-torque ratio generated by the apparatus during a walking exercise based on at least one of a walking mode and a torque intensity set in a user terminal, generates physical/exercise ability information of the wearer based on the quantified physical/exercise ability value, generates walking analysis information of the wearer based on the generated physical/exercise ability information of the wearer, and, based on this, can provide a graphical user interface displaying statistical information according to accumulated walking of the wearer or a walking analysis report.

According to the present invention, by inputting, as training data, walking information of the wearer and the like based on a deep learning model, a walking ability of the wearable robot wearer can be estimated, or customized exercise program information can be provided.

According to the present invention, while being provided as a graphical user interface displaying information on walking of the wearer such as real-time walking information of the wearer, statistical information according to accumulated walking information of the wearer, a walking analysis report, a walking ability estimation report, and customized program information, the physical/exercise ability information of the wearer can be intuitively recognized.

According to the present invention, based on intuitive materials such as a walking analysis report generated based on information on walking of the wearer, the wearable robot wearer or an administrator can set at least one of an appropriate walking mode and torque intensity in a robot exercise process of the wearable robot wearer.

The effects of the present invention are not limited to the effects mentioned above, and other effects not mentioned will be clearly understood by those skilled in the art from the description below.

The present invention rapidly reduces and deactivates a device torque during an operation other than walking and smoothly activates the device (applies torque) when at or above a threshold number of walking steps, thereby being able to assist smooth long-time work.

The present invention increases a torque deactivation (torque zero) section in a section where a sign of the device torque changes, thereby being able to efficiently use energy at a moment when walking assistance is needed, and at the same time, can assist the wearer to perform natural walking assistance.

The present invention can reduce a possibility that a movement direction of the wearer and the device are mismatched, and can efficiently use energy during long-time work by reducing the output torque value.

Further, by increasing the torque zero section, it can be advantageous for battery consumption and durability improvement.

Further, an impact is repeatedly applied to the device upon stepping during walking, and in this case, if a low torque is applied through zero torque shaping, an impulse can be relatively reduced.

The present invention provides an effect of being able to perform selective muscle strengthening exercise through a walking exercise by time-shifting and inverting a smooth interaction torque profile using a wearable walking exercise robot.

The present invention has an effect of sequentially storing recent (within 1 to 2 seconds) leg movement state information of the wearer in a buffer memory and performing output feedback control using the stored state information, thereby achieving specific muscle strengthening and various exercise effects.

The present invention has an effect of strengthening various walking functions through a walking exercise using a wearable device.

The present invention has an effect of achieving an exercise intensity (muscle stimulation) that is difficult to achieve through a walking exercise using a wearable device, such as Hip, Knee, ankle.

In the present invention, the waist wearing unit is made of a Soft material, so that it can flexibly respond to a movement of the wearer. Due to this, the wearer can naturally perform various postures and operations, so that an effect of minimizing a burden can be provided even when assisting a highly active work or walking exercise.

The Soft waist wearing unit of the present invention can be flexibly worn regardless of a body shape or body size of the wearer, so that various users can comfortably wear it regardless of gender, body shape, or body size. In particular, since it automatically adheres closely according to a body shape of the wearer, additional adjustment is not necessary, and it has an effect of providing a user-customized wearing feel.

The present invention does not use a Rigid frame, so that unnecessary pressure or interference does not occur on a hip joint side or a waist portion of the wearer. Such a Soft waist wearing unit does not give discomfort to the wearer even upon long-time wearing and provides a stable wearing feel, so that the user feels less fatigue and can use it for a long time.

The Soft waist wearing unit of the present invention is naturally compatible with a movement of the user, so that interference does not occur according to a posture or movement during wearing. Due to this, the wearable robot can smoothly assist an operation of the wearer, and the user can receive smooth assistance even during a walking exercise.

By removing the Rigid frame of the present invention, an overall weight is reduced, so that the user can carry and use it without burden. The weight reduction of the product improves the wearing feel and is easy to store or transport, so that an effect of maximizing convenience of use can be provided.

The present invention can provide an effect of improving the disadvantages of an existing wearable robot through a single actuator and an optimized structural design, and significantly improving the wearing feel and the assistance performance.

The present invention uses a single actuator to make a system lightweight and slim in design, thereby preventing a weight increase and a system volume expansion and enabling an efficient operation.

A single actuator can symmetrically assist a leg on the opposite side based on a supporting foot in accordance with a walking characteristic of a person, so that, compared to an existing technology using an individual actuator per each joint, it can simplify a structure, reduce heat loss, and reduce manufacturing cost.

Further, by canceling a rotational force and a rotational reaction force of the actuator inside the actuator frame, the user feels only a reaction to the assistance force, so that discomfort upon wearing is reduced and the wearing feel is significantly improved.

The present invention is designed by disposing an apparatus main body including a single actuator on a front of an abdomen of the wearer, so that a Rigid frame is not necessary on a hip joint side. Due to this, interference does not occur regardless of a body shape and size of the user, so that an effect of making a movement of the wearer more free is provided.

The thigh wearing unit of the present invention is designed so that the user can easily mount and remove the apparatus. It is configured in a left-right symmetrical structure and in a band and buckle form, so that the wearer can quickly wear and detach it with a simple operation. Such a characteristic enables the walking assistance robot to be conveniently used in various situations, thereby being able to enhance user convenience.

The thigh wearing unit of the present invention can adjust a length to match a thigh body shape and circumference of the wearer, so that it is suitable for users having various body sizes and shapes. Due to this, the apparatus closely adheres accurately to the thigh of the user, and has an effect of enhancing stability by preventing a problem of sliding down or separating during wearing.

The thigh wearing unit of the present invention is designed in a form enclosing a thigh frame joint structure, so that it can reduce a frame distortion phenomenon that may occur during walking. Through this, the user can maintain a free movement even in a state of wearing the apparatus, and an effect of not feeling discomfort in everyday operations (e.g., walking, sitting, stair-climbing, etc.) can be provided.

The thigh wearing unit of the present invention is provided with a button that can easily attach to and detach from the thigh frame, so that the user can quickly assemble or disassemble the apparatus alone. The button automatically returns to an original position through an elastic member having a return force, so that a stable coupling state can be maintained.

The thigh wearing unit of the present invention is designed to softly adhere closely to the thigh of the wearer, so that it can effectively mitigate an impact and shaking occurring during walking. Due to this, fatigue is minimized even upon long-time wearing of the apparatus, and a comfortable wearing feel can be provided.

The thigh wearing unit of the present invention naturally adapts to a movement of the wearer in various use environments, and can effectively operate not only in walking assistance but also in various activities such as stair-climbing and running. Further, the apparatus is stably fixed to the thigh, so that reliability can be maintained in any environment.

### BRIEF DESCRIPTION OF DRAWINGS

FIG. 1a is a view illustrating a wearable robot according to an embodiment of the present invention.
FIG. 1b is an exploded perspective view of a drive unit of a wearable robot according to an embodiment of the present invention.
FIG. 1c is a view illustrating a left-right directional hinge movement of a connecting member according to an embodiment of the present invention.
FIG. 1d is a view illustrating a sliding operation of a connecting member according to an embodiment of the present invention.
FIG. 1e is a view illustrating a link operation of a connecting member according to an embodiment of the present invention.
FIG. 1f is a view illustrating an operation during walking of a wearable robot according to an embodiment of the present invention.
FIG. 1g is a view illustrating an operation of a wearable robot according to a motion and a posture of a wearer according to an embodiment of the present invention.
FIG. 2 is a view for illustrating a walking assistance mode and a walking resistance mode according to an embodiment of the present invention.
FIG. 3 is a view for illustrating an embodiment in which a trainer collects device information of a member during a group exercise according to an embodiment of the present invention.
FIG. 4 is a view for illustrating a method of quantifying a physical/exercise ability of a wearable robot wearer according to the present invention.
FIG. 5 is a view illustrating a method of calculating a torque to provide a driving force to a wearable robot wearer using a state trajectory memory buffer according to the present invention.
FIG. 6a is a view for illustrating an operation of sequentially storing motion state values based on a state trajectory memory buffer according to an embodiment of the present invention.
FIG. 6b is a view illustrating an embodiment in which a motion state value stored in a last storage location (N) is deleted from a state trajectory memory buffer according to an embodiment of the present invention.
FIG. 6c is a view illustrating an embodiment in which a motion state value reflecting a motion state of a wearer according to a motion of the wearer is stored in a state trajectory memory buffer according to an embodiment of the present invention.
FIG. 7 is a view illustrating a result of analyzing a Pearson correlation between a walking, balance, and physical function index of a wearable robot wearer and a device power torque ratio.
FIG. 8 is a view illustrating a correlation between an age of a wearer according to the present invention and, in the case of a walking assistance mode of a wearable robot, a device power torque ratio, and a correlation between an age of a wearer according to the present invention and, in the case of a walking resistance mode of a wearable robot, a device power torque ratio.
FIG. 9 is a schematic diagram illustrating one or more network functions for performing a method of suggesting exercise information based on a database according to a correlation between a ratio of power to torque of a drive unit (230) and a physical condition of a wearer according to the present invention.
FIG. 10 is a view for illustrating a method of calculating an importance score of a category of physical information or exercise information of a wearer of a wearable robot based on a deep neural network according to an embodiment of the present invention.
FIGS. 11a to 11d are block diagrams of a system for generating a graphical user interface for providing information regarding walking of a wearable robot wearer using a wearable robot capable of estimating a walking ability of a wearer during a walking exercise according to an embodiment of the present invention.
FIG. 12 is a block diagram of a user terminal of a system for generating a graphical user interface displaying information regarding walking of a wearable robot wearer according to an embodiment of the present invention.
FIGS. 13 and 14 are views illustrating a graphical user interface showing a user/administrator and experience mode login process for accessing a system displaying information regarding walking of a wearable robot wearer according to an embodiment of the present invention.
FIG. 15a is a view illustrating a graphical user interface showing a process in which a Bluetooth connection is completed for interworking of a user terminal and a wearable robot according to an embodiment of the present invention.
FIG. 15b is a view illustrating a graphical user interface showing a process in which a Bluetooth connection between a user terminal and a wearable robot has failed according to an embodiment of the present invention.
FIG. 15c is a view illustrating a graphical user interface showing a process in which a Bluetooth connection between a user terminal and a wearable robot has been disconnected according to an embodiment of the present invention.
FIG. 16 is a view illustrating a graphical user interface showing a main screen on a user/administrator terminal after a login and a Bluetooth connection are completed according to an embodiment of the present invention.
FIG. 17a is a view illustrating a graphical user interface of a user terminal showing a rest mode before a robot exercise according to an embodiment of the present invention.
FIG. 17b is a view illustrating a graphical user interface of a user terminal in which at least one of an assistance/exercise mode and a torque intensity is set at a start of a robot exercise according to an embodiment of the present invention.
FIG. 17c is a view illustrating a graphical user interface of a user terminal displaying information regarding walking of a wearer measured during a robot exercise process at an end of a robot exercise according to an embodiment of the present invention.
FIG. 17d is a view illustrating a graphical user interface displaying a walking map of a user terminal displaying current location information and distance information of a wearer and walking time information of a wearer during a robot exercise process according to an embodiment of the present invention.
FIG. 17e is a view illustrating a graphical user interface displaying a notification that walking analysis information has not been generated when a robot exercise is ended according to an embodiment of the present invention.
FIG. 18 is a flowchart for illustrating a method of generating a graphical user interface of a user terminal showing information regarding walking of a wearable robot wearer transmitted and received among a wearable robot, a user terminal, and an external server according to an embodiment of the present invention.
FIG. 19a is a view illustrating a graphical user interface of a user terminal displaying statistical information according to accumulated walking information of a wearer based on a 'number of steps' by recording robot exercise processes according to an embodiment of the present invention.
FIG. 19b is a view illustrating a graphical user interface of a user terminal displaying statistical information according to accumulated walking information of a wearer based on a 'distance' by recording robot exercise processes according to an embodiment of the present invention.
FIG. 19c is a view illustrating a graphical user interface of a user terminal displaying statistical information according to accumulated walking information of a wearer based on a 'time' by recording robot exercise processes according to an embodiment of the present invention.
FIG. 20 is a view illustrating a graphical user interface of a user terminal displaying a walking analysis report based on walking analysis information of a wearer based on at least one of information regarding walking of a wearer obtained in a past robot exercise process and accumulated data stored in an external server according to an embodiment of the present invention.
FIG. 21 is a view for illustrating a method of estimating walking analysis information of a wearable robot wearer based on a deep learning model according to an embodiment of the present invention.
FIG. 22 is a flowchart for illustrating a process of combining information regarding different types of walking of a wearer into multimodal data and estimating walking analysis information based on a deep learning model according to an embodiment of the present invention.
FIG. 23 is a view illustrating a graphical user interface displaying a walking ability estimation report based on walking analysis information of a wearer estimated based on a deep learning model according to an embodiment of the present invention.
FIG. 24 is a view illustrating a graphical user interface displaying customized exercise program information suitable for a walking ability of a wearable robot wearer based on a deep learning model according to an embodiment of the present invention.
FIG. 25 is a view illustrating customized exercise program information suitable for a walking ability of a wearable robot wearer and an expected effect based on a deep learning model according to an embodiment of the present invention.
FIG. 26 is a view illustrating a graphical user interface showing a process for registering information of an administrator in a user terminal according to an embodiment of the present invention.
FIG. 27 is a view illustrating a graphical user interface showing a process for registering user information in an administrator terminal according to an embodiment of the present invention.
FIG. 28 is a flowchart for illustrating a process in which each piece of information of a user and an administrator is registered in a user terminal and an administrator terminal, respectively, according to an embodiment of the present invention.
FIGS. 29a and 29b are views illustrating a graphical user interface of a user terminal capable of checking and modifying account information of a user according to an embodiment of the present invention.
FIG. 30 is a view illustrating a graphical user interface of a user terminal capable of checking information of an administrator and deleting a registration according to an embodiment of the present invention.
FIG. 31 is a view illustrating a graphical user interface of a user terminal capable of checking information of a robot used by a user according to an embodiment of the present invention.
FIG. 32 is a view illustrating a graphical user interface of an administrator terminal capable of checking and modifying account information of an administrator according to an embodiment of the present invention.
FIG. 33 is a view illustrating a graphical user interface of an administrator terminal that sets a robot name (management number) of a wearable robot connected to a user terminal according to an embodiment of the present invention.
FIG. 34 is a view illustrating a graphical user interface of an administrator terminal showing a basic screen when a program tab for a 1:N group exercise is clicked according to an embodiment of the present invention.
FIG. 35 is a view illustrating a graphical user interface of an administrator terminal that generates a program for a 1:N group exercise according to an embodiment of the present invention.
FIG. 36 is a view illustrating a graphical user interface of an administrator terminal showing general information of a program generated for a 1:N group exercise according to an embodiment of the present invention.
FIG. 37 is a view illustrating a graphical user interface of an administrator terminal showing a process of selecting members participating in a program generated for a 1:N group exercise according to an embodiment of the present invention.
FIG. 38 is a view illustrating a graphical user interface of an administrator terminal showing a process of connecting, in a case where a member among members participating in a program for a 1:N group exercise is not connected to a wearable robot, the member according to an embodiment of the present invention.
FIG. 39 is a view illustrating a graphical user interface of an administrator terminal showing a process of causing a robot exercise to start for at least one or more members among members participating in a program for a 1:N group exercise according to an embodiment of the present invention.
FIG. 40 is a view illustrating a graphical user interface of an administrator terminal in which a walking mode and a torque intensity are set at a start of an exercise of each member in a program for a 1:N group exercise according to an embodiment of the present invention.
FIG. 41 is a view illustrating a graphical user interface capable of checking, through an administrator terminal, walking information of a member participating in a program for a 1:N group exercise according to an embodiment of the present invention.
FIG. 42 is a view illustrating a graphical user interface capable of checking, through an administrator terminal, basic information and a walking analysis report of one of members participating in a program for a 1:N group exercise according to an embodiment of the present invention.
FIG. 43 is a view illustrating a device control method for safely performing various tasks other than walking during walking assistance of a wearer according to the present invention.
FIG. 44 is a view for illustrating a comparison by hip state trajectory operation according to an embodiment of the present invention.
FIG. 45a is a view illustrating a state trajectory in a case where a walker is stopped and a case where a walker proceeds with walking after stopping according to an embodiment of the present invention.
FIG. 45b is a view for illustrating a state trajectory movement distance according to an embodiment of the present invention.
FIG. 45c is a view for illustrating a torque intensity adjustment parameter according to an embodiment of the present invention.
FIG. 46 is a view for illustrating a continuous number of steps counting according to an embodiment of the present invention.
FIG. 47 is a view for illustrating a walking assistance output algorithm according to the present invention.
FIG. 48 is a view for illustrating a state feedback control based assistance method according to the present invention.
FIG. 49 is a view for illustrating an output torque algorithm according to the present invention.
FIG. 50a is a view illustrating a change in a torque value when walking again after a task other than walking during walking according to the present invention.
FIG. 50b is a view illustrating a change in a torque value when performing slow walking during walking and walking again after a task other than walking according to the present invention.
FIG. 51a is a view for illustrating an embodiment of increasing a torque deactivation section in a section in which a device torque sign changes according to the present invention.
FIG. 51b is a view for illustrating, by a graph, an embodiment of increasing a torque deactivation section in a section in which a device torque sign changes according to the present invention.
FIG. 52 is a view illustrating a wearable mobility system and a user terminal according to the present invention.
FIG. 53 is a view for illustrating a walking assistance mode and a walking resistance mode according to the present invention.
FIG. 54 is a view for illustrating a method of supporting a selective muscle strengthening exercise during a walking exercise according to the present invention.
FIG. 55 is a view illustrating a user interface for selecting a target muscle to be exercised according to an embodiment of the present invention.
FIG. 56 is a view for illustrating a walking assistance output algorithm according to the present invention.
FIG. 57 is a view illustrating a lookup table of setting values matching a target muscle strength exercise mode according to an embodiment of the present invention.
FIG. 58 is a view for illustrating an output torque algorithm according to the present invention.
FIG. 59 is a view illustrating provision of user feedback information according to an embodiment of the present invention.
FIG. 60 is a view illustrating provision of hip joint visual feedback information according to an embodiment of the present invention.
FIG. 61 is a view illustrating a muscle strengthening effect of an assistance mode A according to an embodiment of the present invention.
FIG. 62 is a view illustrating a muscle strengthening effect of a resistance mode A according to an embodiment of the present invention.
FIG. 63 is a view for comparing a difference in strengthened muscle strength between a resistance mode B and a resistance mode C according to the present invention.
FIG. 64 is a view illustrating a torque and power trajectory of an assistance mode A according to the present invention.
FIG. 65 is a view illustrating a torque and power trajectory of a resistance mode A according to the present invention.
FIG. 66 is a view illustrating a torque and power trajectory of a resistance mode B according to the present invention.
FIG. 67 is a view illustrating a torque and power trajectory of a resistance mode C according to the present invention.
FIG. 68 is a view illustrating a torque and power trajectory of a resistance mode D according to the present invention.
FIG. 69 is a view illustrating a torque and power trajectory of a resistance mode E according to the present invention.
FIG. 70 is a view for illustrating a method of supporting a selective walking strengthening exercise during a walking exercise according to an embodiment of the present invention.
FIG. 71 is a view illustrating a setting value lookup table matching a target motion exercise mode according to an embodiment of the present invention.
FIG. 72 is a view illustrating a target improvement motion setting user interface according to an embodiment of the present invention.
FIG. 73 is a view illustrating a torque and power profile of an assistance mode A according to an embodiment of the present invention.
FIG. 74 is a view illustrating a torque and power profile of an assistance mode B according to an embodiment of the present invention.
FIG. 75 is a view illustrating a torque and power profile of an assistance mode C according to an embodiment of the present invention.
FIG. 76 is a view illustrating a torque and power profile of an assistance mode D according to an embodiment of the present invention.
FIG. 77 is a view illustrating an interval walking exercise program according to an embodiment of the present invention.
FIG. 78 is a view illustrating an assistance/resistance program user interface according to an embodiment of the present invention.
FIG. 79 is a view illustrating an additional control mode suitable for a situation such as downhill or uphill walking according to an embodiment of the present invention.
FIG. 80 is a view for illustrating a waist wearing unit and a waist wearing buckle unit of the present invention.
FIG. 81 is a view for illustrating a Velcro fixing embodiment of a waist wearing unit and a waist wearing buckle unit of the present invention.
FIG. 82 is a view for illustrating a drive unit fixing member according to the present invention.
FIG. 83 is a view for illustrating a drive unit fixing frame portion according to the present invention.
FIG. 84 is a view for illustrating an embodiment related to a left-right symmetric twist angle of a drive unit fixing frame portion according to the present invention.
FIG. 85 is a view for illustrating an embodiment of mounting a drive unit fixing member on a drive unit fixing frame portion according to the present invention.
FIG. 86 is a view for illustrating an embodiment related to a button member of a drive unit fixing member according to the present invention.
FIG. 87 is a view for illustrating an embodiment related to a button member handle portion of a drive unit fixing member according to the present invention.
FIG. 88 is a view for illustrating a detailed configuration of a thigh wearing unit according to the present invention.
FIG. 89 is a view for illustrating an operation of a thigh wearing unit according to an embodiment of the present invention.
FIG. 90 is a view for illustrating a thigh frame fixing joint structure according to the present invention.
FIG. 91 is a view for illustrating a thigh attachment/detachment frame according to the present invention.
FIG. 92 is a view for illustrating an attachment/detachment mechanism of a thigh wearing unit according to an embodiment of the present invention.
FIG. 93 is a view for illustrating a linear guide member according to an embodiment of the present invention.
FIG. 94 is a view for illustrating a sliding rail member according to an embodiment of the present invention.
FIG. 95 is a view for illustrating an operation of a second stage and a 2.5th stage in a coaxial second bearing structure and an auxiliary second bearing structure of a thigh frame according to an embodiment of the present invention.
FIG. 96 is a view for illustrating a second stage auxiliary bearing member of a thigh frame according to an embodiment of the present invention.
FIG. 97a is a view for illustrating a second stage bearing member according to an embodiment of the present invention.
FIG. 97b is a view for illustrating a role of a return elastic member being coupled to a 2.5th stage sliding member to assist a return according to an embodiment of the present invention.
FIG. 97c is a view for illustrating a shape of a bearing shaft member according to an embodiment of the present invention.
FIG. 98 is a view for illustrating a joint member, a joint shaft member, a second stage return elastic band member, and the like according to an embodiment of the present invention.
FIG. 99 is a view for illustrating an operation of a thigh frame having a urethane bearing structure that slides along a rail having a certain angle according to the present invention.
FIG. 100 is a view for illustrating a bearing end bracket and a bearing sliding bracket according to an embodiment of the present invention.
FIG. 101 is a view for illustrating a urethane bearing according to an embodiment of the present invention.
FIG. 102 is a view for illustrating a joint member, a joint shaft member, a second stage return elastic band member, and the like in a urethane bearing structure according to an embodiment of the present invention.
FIGS. 103 to 108 are views illustrating an operating state of a wearable robot according to an embodiment of the present invention.
FIG. 109 is an operation view of a connecting member according to a third embodiment of the present invention.
FIG. 110 is an operation view of a connecting member according to a fourth embodiment of the present invention.

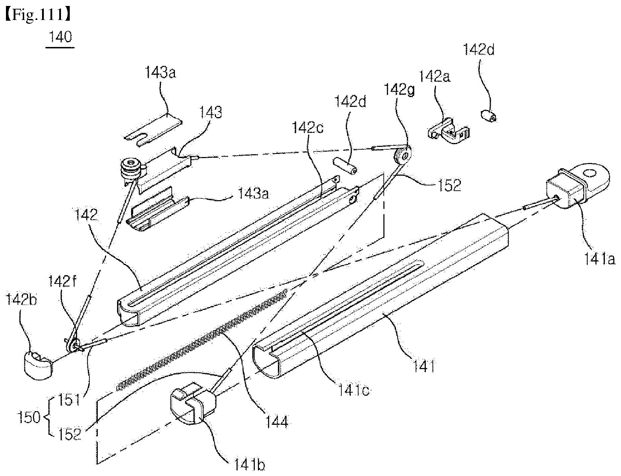

FIG. 111 is an exploded perspective view of a connecting member in a wearable robot according to an embodiment of the present invention.
FIG. 112 is a cross-sectional view of a connecting member in a wearable robot according to an embodiment of the present invention.
FIG. 113 is an enlarged view of a portion "A" of FIG. 112.
FIG. 114 is an enlarged view of a portion "B" of FIG. 112.
FIGS. 115 and 116 are views illustrating an operating state of a wearable robot according to an embodiment of the present invention.
FIG. 117 is an operation view of a connecting member of the present invention.
FIG. 118 is a perspective view of a state in which a waist belt and a waist wearing frame are coupled.
FIGS. 119 to 121 are views for illustrating a process of detaching/attaching a waist wearing unit of a wearable robot.
FIG. 122 is a perspective view of a thigh wearing unit in a wearable robot according to an embodiment of the present invention.
FIG. 123 is an exploded perspective view of the thigh wearing unit illustrated in FIG. 122.
FIG. 124 is a view for illustrating a process in which a plate and a strap unit are separated/coupled.
FIG. 125 is a view illustrating a method in which a first button operates within a plate.
FIG. 126 is a view for illustrating a process in which a plate and a connecting member are separated/coupled.
FIGS. 127 to 129 are views illustrating an elastic member connecting between a waist wearing unit and a thigh wearing unit in a wearable robot according to an embodiment of the present invention.

### DETAILED DESCRIPTION

### MODE FOR THE INVENTION

Specific details of the embodiments are included in the detailed description and the drawings.

The advantages and features of the present invention, and the methods of achieving them, will become clear with reference to the embodiments described in detail below together with the accompanying drawings. However, the present invention is not limited to the embodiments disclosed below, but may be implemented in various different forms, and the present embodiments are provided merely to make the disclosure of the present invention complete and to fully inform a person having ordinary skill in the technical field to which the present invention pertains of the scope of the invention, and the present invention is defined only by the scope of the claims. Throughout the specification, the same reference numerals refer to the same components.

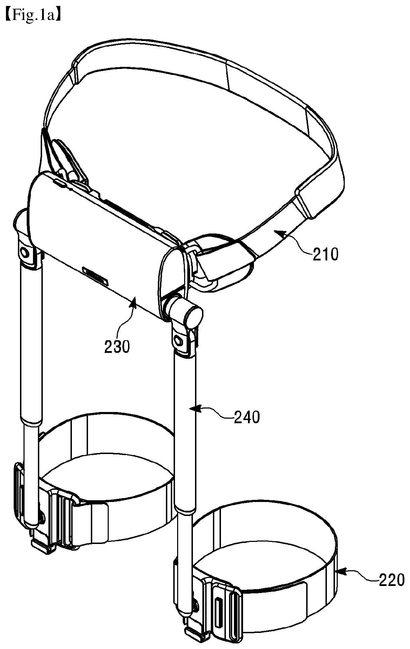

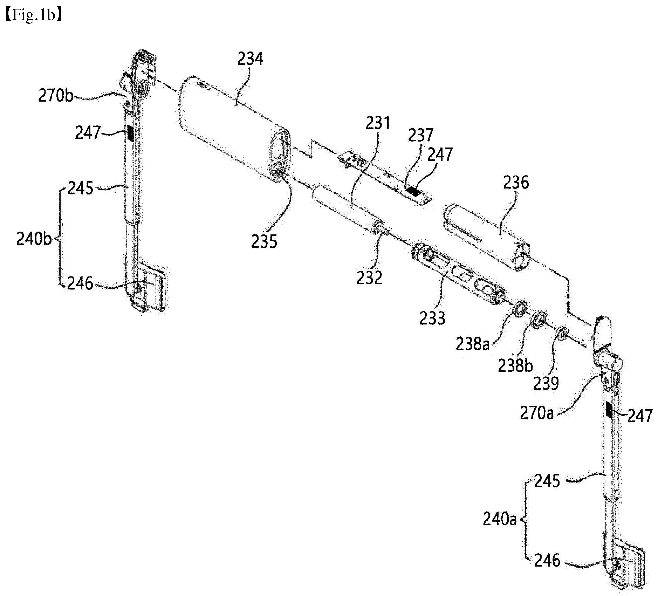

FIG. 1a is a diagram showing a wearable robot according to one embodiment of the present invention. FIG. 1b is an exploded perspective view of a drive unit of a wearable robot according to one embodiment of the present invention.

### Overall Structure of the Wearable Robot

As shown in FIG. 1a, the wearable robot according to one embodiment of the present invention includes a first fixing unit (210), a second fixing unit (220), a drive unit (230), and a connecting member (240). In addition, the first fixing unit (210) and the second fixing unit (220) of the wearable robot according to one embodiment of the present invention may be worn on a body part. According to the present invention, the wearable robot capable of estimating the physical ability of the wearer during walking exercise may include a fixing unit (including the first fixing unit (210) and the second fixing unit (220)) that may be mounted on a body part and a drive unit (230) that may be mounted on the fixing unit, and the drive unit (230) may estimate the physical ability of the wearer based on the power of the drive unit (230) due to the walking exercise of the wearer.

For convenience of description, the front direction of the wearer wearing the wearable robot is defined as the x-axis direction. In addition, both side directions of the wearer wearing the wearable robot are defined as the y-axis direction, and the vertical direction is defined as the z-axis direction.

In one embodiment of the present invention, the first fixing unit (210) may be mounted on a reference body part of the wearer, the second fixing unit (220) may be mounted on a target body part of the wearer, and the connecting member (240) connecting the drive unit (230) provided on the first fixing unit (210) side and the second fixing unit (220) may rotate about the y-axis by the driving of the drive unit (230), thereby providing an assistance force to the movement of the body part about the joint. The connecting member (240) may be divided into a first connecting member (240a) and a second connecting member (240b).

According to one embodiment of the present invention, the first fixing unit (210) and the second fixing unit (220) are each mounted on a body part, and may be formed in a form such as a band or a belt that may wrap around the body part. In addition, fixing means such as a hook-and-loop fastener that may be mutually fixed may be provided at both ends.

According to one embodiment of the present invention, the drive unit (230) is provided on the first fixing unit (210) and may be configured of a motor or an actuator for providing a rotational torque about the y-axis, and may include a reduction gear or the like for increasing the torque.

As shown in FIG. 1b, the drive unit (230) includes an actuator (231) that generates power for the assistance force, and may be mounted in front of or behind the reference body part.

According to the present invention, a battery (236) that supplies power to the actuator (231) may be mounted together with the drive unit (230), but the battery (236) may be formed separately from the drive unit (230) while including a control board (237) and may be mounted on another part of the body.

According to the present invention, the first fixing unit (210) may be configured of a typical belt (or scrap) and a buckle (or Velcro), or the like. Accordingly, the first fixing unit (210) may mount the drive unit (230) and wrap around the reference body part to mount the drive unit (230) in front of the reference body part of the wearer or behind the reference body part. At this time, the circumferential length of the first fixing unit (210) may be adjusted to fit the size of the reference body part of the wearer.

According to one embodiment of the present invention, when the drive unit (230) is mounted behind the reference body part, the connecting member (240) may be positioned behind or in front of the target body part, but is not limited thereto.

According to the present invention, the connecting member (240) is rotatably coupled to both sides of the drive unit (230), and each of them may extend along both target body parts and be mounted on the target body parts. At this time, the connecting member (240) may be disposed in front of or behind the target body part depending on the mounting position of the drive unit (230).

According to the present invention, the connecting member (240) may rotate forward and backward about a rotation axis by the operation of the actuator (231) of the drive unit (230), thereby transmitting an assistance force to the target body part.

According to one embodiment of the present invention, the connecting member (240) may be formed in a long rod shape, and the inner side surface in contact with the target body part may be formed as a curved surface according to the shape in contact with the target body part so as to be in close contact with the target body part.

As shown in FIG. 1b, the first connecting member (240a) may include an extension frame (245) that is rotatably coupled to the drive unit (230) and extends long with a rectangular or circular cross-section. In addition, the first connecting member (240a) may include a fixing frame (246). The second connecting member (240b) may include an extension frame (245) that is rotatably coupled to the drive unit (230) and extends long with a rectangular or circular cross-section. In addition, the second connecting member (240b) may include a fixing frame (246). The fixing frame (246) is fixed to the lower end of the extension frame (245), has a relatively large contact area with the target body part, has its inner side surface formed as a curved surface according to the shape in contact with the target body part, and may fix the connecting member (240) to the target body part.

According to the present invention, the second fixing unit (220) is connected to the lower end of the connecting member (240) and may fix the lower end of the connecting member (240) to the target body part. The second fixing unit (220), like the first fixing unit (210), may be configured of a belt (or strap) and a buckle (or Velcro), or the like.

In the drawing, the second fixing unit (220) is formed at the lower end of the connecting member (240), but it may be formed at another position or may be additionally formed at a position other than the lower end of the connecting member (240).

In addition, it may include a movement detection sensor (247) that detects the movement or posture of the target body part of the wearer.

According to one embodiment of the present invention, the control board (237) may receive a signal from the detection sensor to predict the movement of the wearer, and accordingly control the actuator (231) of the drive unit (230) to drive the connecting member (240), thereby assisting the movement of the target body part.

According to one embodiment of the present invention, the movement detection sensor (247) may be an inertial sensor, an angle sensor, a limit sensor, or the like. The movement detection sensor (247) may be mounted in the main body housing (234) to sense the angle of the reference body part of the wearer.

In addition, the movement detection sensor (247) may be mounted on the rotational joint portion (270a, 270b) on one side or both sides to estimate the forward-backward direction angle of the target body part of the wearer.

Alternatively, after sensing only the angle of the target body part on one side, the angle of the target body part on the other side may be calculated through the relative angle of the target body parts on both sides.

In addition, the movement detection sensor (247) may be mounted on the connecting member (240) to sense the angle of the forward-backward direction and the left-right rotation direction of the target body part of the wearer. The sensed angle of the left-right rotation direction may be used when calculating information on the balance of the wearer.

In addition, the movement detection sensor (247) may be an encoder, a resolver, a Hall sensor, or the like, that may estimate the rotation amount and direction of the motor. The movement detection sensor (247) may detect the rotation of the motor shaft to measure the rotation variation.

In one embodiment of the present invention, since the drive unit (230) including the actuator (231) is disposed in front of or behind the reference body part and the connecting member (240) is positioned in the forward-backward direction in which the target body part moves during operation, the connecting member (240) may effectively transmit the assistance force of the connecting member (240) to the target body part.

Therefore, since the assistance force may be transmitted more effectively compared to the existing structure, an actuator (231) having a relatively small power may be used, and furthermore, as described later, the assistance force may be transmitted to the target body parts on both sides using a single actuator (231), so that the weight of the device may be reduced.

### Embodiment of the Drive Unit

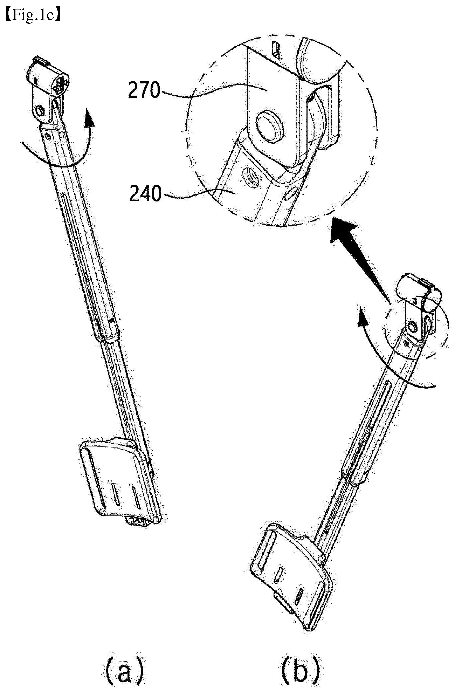

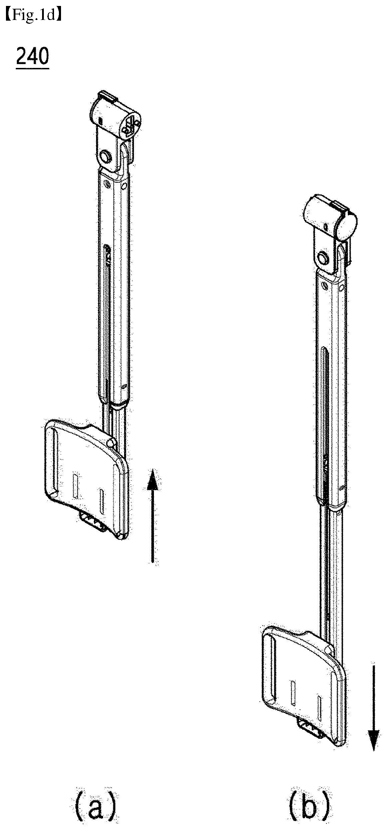

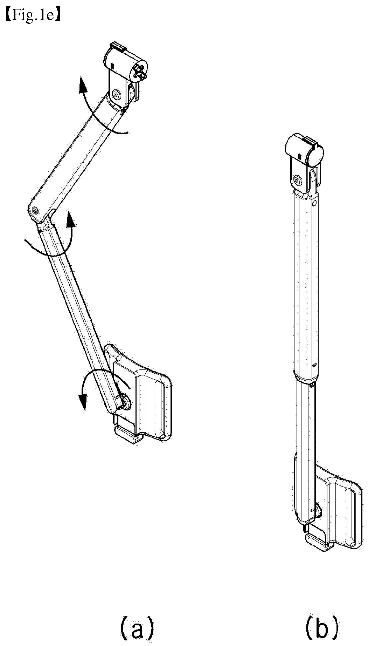

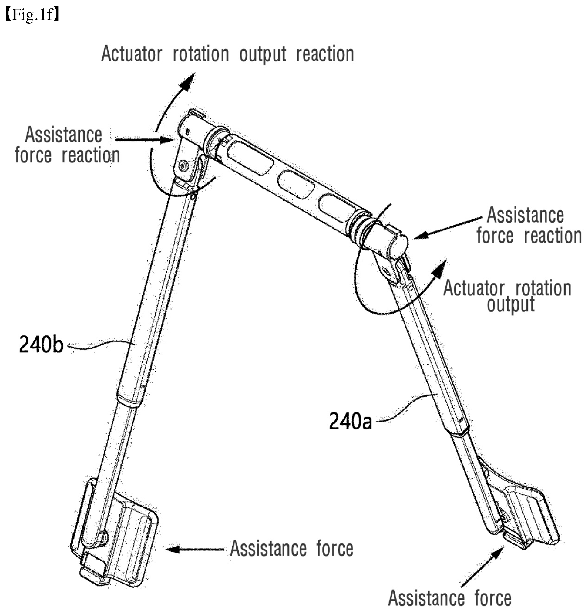

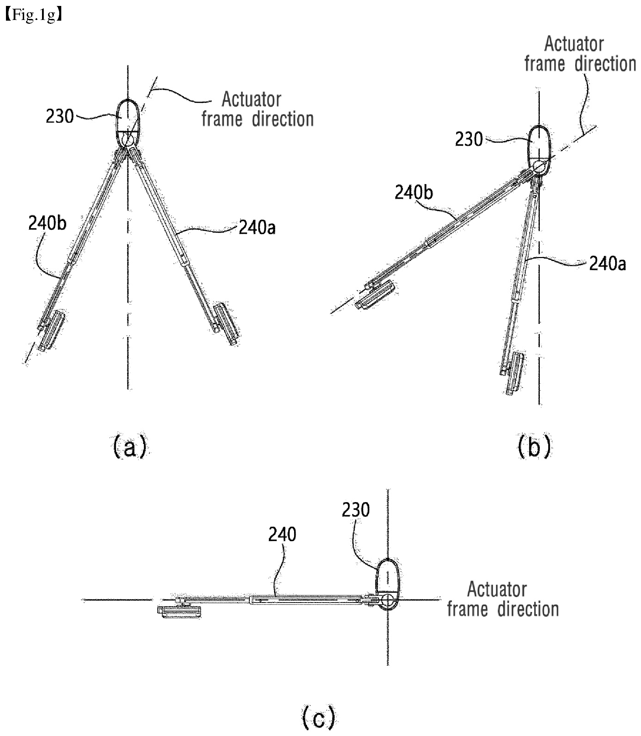

FIG. 1b is an exploded perspective view of a drive unit of a wearable robot according to one embodiment of the present invention. FIG. 1c is a diagram explaining the left-right direction hinge movement of the connecting member according to one embodiment of the present invention. FIG. 1d is a diagram explaining the sliding operation of the connecting member according to one embodiment of the present invention. FIG. 1e is a diagram explaining the link operation of the connecting member according to one embodiment of the present invention. FIG. 1f is a diagram explaining the operation during walking of the wearable robot according to one embodiment of the present invention. FIG. 1g is a diagram explaining the operation of the wearable robot according to the operation and posture of the wearer according to one embodiment of the present invention.

As shown in FIG. 1b, the drive unit (230) may include a single actuator (231), an actuator frame (233), and a main body housing (234).

According to the present invention, when the target body part is the thigh, the left thigh and the right thigh may be driven in conjunction. In this case, the objects to which the assistance force or resistance force is transmitted at the target body part may be two points, one of which is referred to as the one-side target body part and the other as the other-side target body part.

Here, the thigh is merely an example of a target body part for specifically explaining the embodiment of the drive unit, and the target body part is not limited to the thigh, and may correspond to various body parts of the human body.

According to the present invention, the actuator (231) may be configured of a rotation motor that may change the rotation direction. A connecting member (240a) mounted on the one-side target body part is connected to the motor shaft (232) of the rotation motor, so that the connecting member (240a) may rotate forward and backward about the left-right direction rotation axis by the power of the rotation motor.

According to the present invention, the actuator frame (233) is in a long cylindrical shape, accommodates the actuator (231) therein, and may rotate in the horizontal axial direction.

According to one embodiment of the present invention, the actuator frame (233) may not be separately provided, and the actuator frame (233) and the actuator (231) may be integrated to constitute the actuator (231) itself.

According to the present invention, the main body housing (234) may accommodate the actuator frame (233) therein.

In one embodiment of the present invention, the main body housing (234) may be formed with a region in which a cylindrical hole (235) is formed in the horizontal direction so as to accommodate the actuator frame (233) therein and a region in which the battery (236) and the control board (237) are mounted, separately.

According to one embodiment of the present invention, a first bearing (238a) and a second bearing (238b) may be mounted between the outer side of the actuator frame (233) and the cylindrical hole (235) so that the actuator frame (233) rotates in the axial direction within the cylindrical hole (235) formed in the main body housing (234).

According to one embodiment of the present invention, the actuator (231) located inside the actuator frame (233) may be fixed inside the actuator frame (233). Therefore, when the actuator frame (233) rotates, the actuator (231) may also rotate together.

According to one embodiment of the present invention, a second connecting member (240b) mounted on the other-side target body part is connected to the other end of the actuator frame (233), so that when the actuator frame (233) rotates, the second connecting member (240b) may also rotate forward and backward about the left-right direction rotation axis together.

As shown in the drawing, a rotational joint portion (270b) may be coupled to the other end of the actuator frame (233), and the connecting member (240b) may be fixed to the rotational joint portion (270b). In addition, a rotational joint portion (270a) may also be coupled to the motor shaft (232) of the rotation motor.

In one embodiment of the present invention, a bush (239) is separately coupled to the end of the motor shaft (232), and the rotational joint portion (270a) is coupled to the bush (239).

According to one embodiment of the present invention, a second bearing (238b) may be separately mounted between the outer side surface of the motor shaft (232) and the cylindrical hole (235) of the main body housing (234) or between the outer side surface of the bush (239) and the cylindrical hole (235) of the main body housing (234).

According to the present invention, the connecting member (240) may be hinge-coupled to the lower end of the rotational joint portion (270) on both left and right sides so as to be rotatable in the left-right direction about the forward-backward direction rotation axis.

According to the present invention, one side of the upper end of the rotational joint portion (270) may be coupled to the other end of the actuator frame (233) or the bush (239). The lower end of the rotational joint portion (270) may be formed in a shape in which both sides extend in a plate shape (a 'U' shape when viewed from the side), so that the upper end of the connecting member (240) may be hinge-coupled between the two side plates so as to be rotatable in the left-right direction about the forward-backward direction rotation axis.

Therefore, as shown in FIG. 1c, the connecting member (240) may rotate in the left-right direction between the two side plates about the upper end of the connecting member (240).

Therefore, the connecting member (240) may rotate in the left-right direction by the hinge coupling, and may also rotate forward and backward about the left-right direction rotation axis by the rotation of the rotational joint portion (270). In addition, the length of the connecting member (240) may be variable.

In one embodiment of the present invention, the drive unit (230) is mounted on the reference body part.

Therefore, the straight-line distance between both sides of the drive unit (230) and the lower end of the connecting member (240) may change according to various postures or movements of the wearer, such as when sitting on a chair, bending the waist, or raising an arm. At this time, if the length of the connecting member (240) is fixed, the connecting member (240) may restrict (interfere with) the movement of the wearer. At this time, in one embodiment of the present invention, the length of the connecting member (240) is made variable so as to conform to the posture or movement of the wearer, thereby preventing the connecting member (240) from restricting the movement of the wearer.

As shown in FIG. 1d, the connecting member (240) may be formed of a plurality of frames that overlap and perform sliding movement. Therefore, the length of both ends of the connecting member (240) may be variable so as to conform to the posture or movement of the wearer.

As shown in FIG. 1e, the connecting member (240) may be formed of a plurality of links and may be configured to bend or unfold so as to conform to the movement of the wearer. At this time, the links may be link-coupled so as to bend in the forward-backward direction or bend in the left-right direction. Therefore, since the bending amount of the links varies so as to conform to the posture or movement of the wearer, the connecting member (240) may be prevented from restricting the movement of the wearer.

The wearable robot according to one embodiment of the present invention configured as described above supports and generates an assistance force in a manner that assists the one-side target body part on the basis of the other-side target body part.

That is, the connecting member (240b) of the other-side target body part is connected to the actuator frame (233). In addition, the connecting member (240a) of the one-side target body part is connected to the motor shaft of the actuator (231) so as to directly receive the rotational force of the actuator (231), and may rotate directly by the output of the actuator (231).

Therefore, when the actuator (231) generates power, the connecting member (240a) of the one-side target body part operates, on the basis of the other-side target body part, in a direction that spreads the one-side target body part forward or pulls it backward.

Therefore, as shown in FIG. 1f, the output generated by the actuator (231) is transmitted to the one-side target body part through the connecting member (240a) of the one-side target body part, and a reaction force against the rotational force (output) of the actuator (231) may be transmitted as an assistance force to the other-side target body part. That is, using a single actuator (231), the assistance force may be transmitted to the target body parts on both sides simultaneously with rotational forces in mutually opposite directions. At this time, the rotational output of the actuator (231) acting on the connecting member (240a) of the one-side target body part and the rotational reaction force acting on the connecting member (240b) of the other-side target body part against the rotational force of the actuator (231) cancel each other out at the actuator frame (233), so that only the reaction force against the assistance force may be transmitted to the wearer. Therefore, only a small force is transmitted to the drive unit (230), so that the repulsive force felt by the wearer is small, and the wearing comfort may be structurally improved.

In addition, as shown in FIG. 1g, the actuator frame (233) may rotate forward and backward about the left-right direction rotation axis within the cylindrical hole (235) of the main body housing (234). At this time, the actuator (231) fixed inside the actuator frame (233) may also rotate together with the actuator frame (233).

Therefore, with respect to various postures and movements of the wearer, the rotation-direction reference position of the actuator frame (233) may change so as to conform on the basis of the main body housing (234) whose position is fixed to the reference body part, so that the wearing comfort of the wearer may be improved. For example, as shown in (a) of FIG. 1g, (b) of FIG. 1g, and (c) of FIG. 1g, even when the angles that the connecting member (240a) and the connecting member (240b) form with the horizontal plane are different from each other according to the posture of the wearer, the operation reference angle of the actuator frame (233) with respect to the main body housing (234) whose position is mounted on the reference body part may change so as to conform according to the posture.

In addition, as described above, the connecting member (240) is hinge-coupled and is also rotatable left and right, and the length of the connecting member (240) is formed to be variable or is formed in a link structure. Accordingly, with respect to various postures of the wearer, the connecting member (240) does not restrict the movement but moves so as to conform, thereby improving the assistance force transmission efficiency and wearing comfort of the wearable robot.

For example, as shown in (a) of FIG. 1g when walking on flat ground, as shown in (b) of FIG. 1g when going up or down stairs, and as shown in (c) of FIG. 1g when sitting on a chair, the operation reference angle of the actuator frame (233) with respect to the main body housing (234) whose position is fixed to the waist may change so as to conform according to the posture. As shown in (c) of FIG. 1g, when sitting on a chair, since there is no relative movement of both legs, the actuator frame (233) merely undergoes free rotation within the main body housing (234) without the output of the actuator (231) (without generation of the assistance force). In addition, as described above, the connecting member (240) is hinge-coupled and is also rotatable left and right, and the length of the connecting member (240) is formed to be variable or is formed in a link structure, so that, with respect to various postures of the wearer (such as a posture of bending the waist in the forward-backward direction or sitting on a chair) and postures of the thigh that spreads or closes left and right, etc., the connecting member (240) does not restrict the movement but moves so as to conform, thereby improving the assistance force transmission efficiency and wearing comfort of the wearable robot. In the embodiment described above, the structure is one in which the assistance force is directly transmitted by the single actuator (231) to the connecting member (a) fixed to the one-side thigh, and the assistance force is transmitted to the connecting member (b) fixed to the other-side thigh by the reaction force of the supported leg, but although not shown, two actuators may be provided and each actuator may be configured to rotate the connecting members (240) on both sides, respectively. At this time, since the drive unit (230) is fixed to the waist of the wearer and the connecting member (240) is disposed in front of or behind the thigh, the force transmission efficiency of the assistance force is good, so that an actuator of relatively small power may be used.

The embodiment described above is a structure in which the assistance force is directly transmitted by the single actuator (231) to the connecting member (240a) fixed to the one-side target body part, and the assistance force is transmitted to the connecting member (240b) fixed to the other-side target body part by the reaction force of the supported target body part, but although not shown, two actuators may be provided and each actuator may be configured to rotate the connecting members (240) on both sides, respectively. At this time, since the drive unit (230) is fixed to the reference body part of the wearer and the connecting member (240) is disposed in front of or behind the target body part, the force transmission efficiency of the assistance force is good, so that an actuator of relatively small power may be used.

### Walking Assistance and Walking Resistance

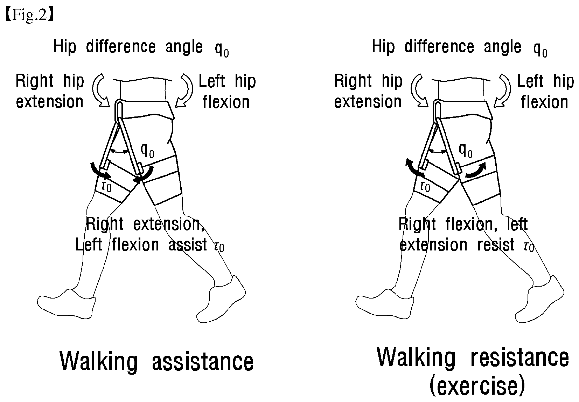

FIG. 2 is a diagram for explaining a walking assistance mode and a walking resistance mode according to one embodiment of the present invention. As shown in FIG. 2, the angle value (q0, Hip difference angle) between the one-side target body part and the other-side target body part of the wearer may be a motion state value described later.

In the case of the walking assistance mode according to one embodiment of the present invention, by assisting the movement of the leg of the wearable robot wearer, the muscle force consumption during walking may be reduced, or the wearer may be guided to a correct walking posture with a fast and wide stride. With reference to FIG. 2, in the direction from right to left, a force that pushes (extension) the leg of the wearable robot wearer is applied, and with reference to FIG. 2, in the direction from left to right, a force that pulls (flexion) the leg of the wearable robot wearer is applied, so that as a result the wearer may reduce the muscle force consumption during walking. That is, in the case of the walking assistance mode according to the present invention, it is possible to provide a walking assistance effect by applying a forward-direction torque to the movement of the leg of the wearable robot wearer.

In the case of the walking resistance (exercise) mode according to one embodiment of the present invention, it is possible to provide an exercise effect by applying a reverse-direction torque (resistance torque) to the movement of the leg of the wearable robot wearer. With reference to FIG. 2, in the direction from right to left, a force that pulls (flexion) the leg of the wearable robot wearer is applied, and with reference to FIG. 2, in the direction from left to right, a force that pushes (extension) the leg of the wearable robot wearer is applied, so that as a result the wearer consumes muscle force during walking and may have an exercise effect.

According to one embodiment of the present invention, in the case of the walking assistance mode, since the direction of the device hip angular velocity and the direction of the torque provided by the drive unit (230) are the same, transmission of power from the device to the wearable robot wearer occurs, and the power value may be calculated as a positive number. On the other hand, in the case of the walking resistance (exercise) mode, since the direction of the device hip angular velocity and the direction of the torque provided by the drive unit (230) are opposite, transmission of power from the wearer to the device occurs, so that the power value may be calculated as a negative number. That is, during walking assistance, positive power values may be mainly calculated, and during walking resistance, negative power values may be mainly calculated.

### Embodiment of Physical Information Output

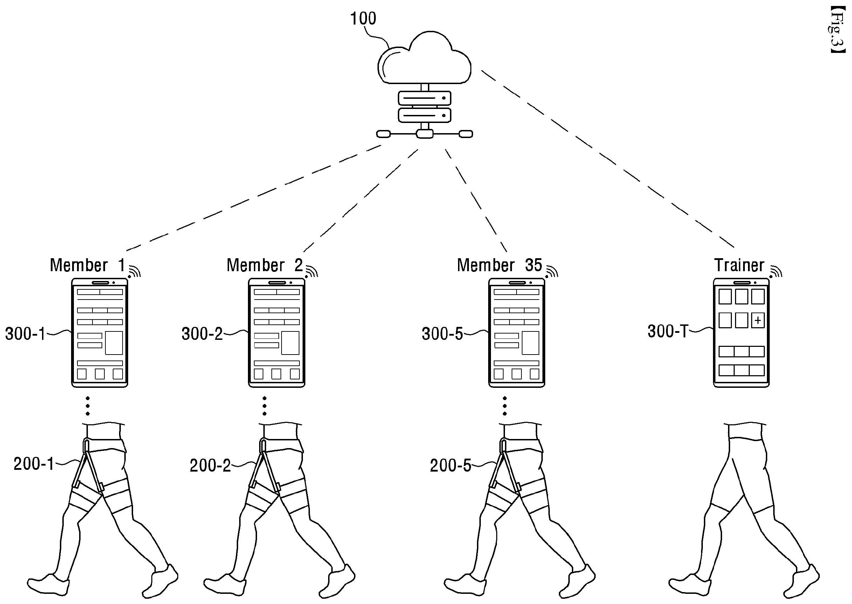

FIG. 3 is a diagram for explaining an embodiment in which a trainer collects device information of members during group exercise according to one embodiment of the present invention.

As shown in FIG. 3, the wearable robot of the present invention may have multi-party connection and real-time synchronization functions based on wireless connection and real-time data sharing through a cloud server (100) in order to improve the leg muscle force of the wearer and maximize the exercise effect.

According to the present invention, a system for managing the exercise of a wearer wearing the wearable robot (200) capable of estimating the physical ability of the wearer during walking exercise may be provided. The system for managing the exercise of a wearer wearing the wearable robot (200) capable of estimating the physical ability of the wearer during walking exercise may include a first user terminal that outputs analysis information on the exercise of the wearer, a server device (100) that receives the analysis information on the exercise of the wearer output by the first user terminal, and a second user terminal that receives the analysis information on the exercise of the wearer transmitted by the server device (100).

As a specific example, the first user terminal may be the smartphone (300-1) of member 1 shown in FIG. 3. The smartphone (300-1) of member 1 may receive information on the exercise of the wearer from the wearable robot (200-1) of member 1, and may output analysis information on the exercise of the wearer based on the received information. Accordingly, the server device (100) may receive the analysis information on the exercise of the wearer output by the smartphone (300-1) of member 1. Accordingly, the second user terminal may receive the analysis information on the exercise of the wearer from the server device (100), and the second user terminal may be the smartphone (300-T) of the trainer, but is not limited thereto.

According to one embodiment of the present invention, the server device (100) may receive, from the second wearer terminal, feedback information based on information on the exercise of the wearer transmitted to the second user terminal, and may transmit the received feedback information to the first wearer terminal. As a specific example, the second user terminal may be the smartphone (300-T) of the trainer, and the trainer may input feedback information based on information on the exercise of the wearer using the smartphone (300-T) of the trainer, and the input feedback information may be transmitted to the server device (100). The server device (100) may transmit the feedback information based on the information on the exercise of the wearer received to the first wearer terminal, and through this, member 1 may check the feedback information based on the information on the exercise of the wearer.

According to one embodiment of the present invention, the system for managing the exercise of a wearer wearing the wearable robot (200) capable of estimating the physical ability of the wearer during walking exercise may include a first user terminal that outputs analysis information on the exercise of the wearer, a server device (100) that receives the analysis information on the exercise of the wearer output by the first user terminal, and a second user terminal that receives the analysis information on the exercise of the wearer transmitted by the server device (100). At this time, the analysis information on the exercise of the wearer output by the first user terminal may be based on the ratio of power to torque calculated by the wearable robot (200). Specifically, the analysis information on the exercise of the wearer output by the first user terminal may be at least any one or more of the agility or the lower-body muscle force strength of the wearer.

According to one embodiment of the present invention, the physical ability of the wearer that may be estimated by the PTR (power torque ratio) value may include agility and lower-body muscle force strength. Specifically, the agility among the physical abilities of the wearer may be estimated using the mean positive power (Mean positive power/ RMS torque) in the walking assistance mode, and the lower-body muscle force strength may be estimated using the mean negative power (Mean negative power/ RMS torque) in the walking resistance mode.

According to one embodiment of the present invention, the server device (100) may receive, from the second wearer terminal, feedback information based on information on the exercise of the wearer transmitted to the second user terminal, and may transmit the received feedback information to the first wearer terminal. At this time, the feedback information received by the server device (100) may include an exercise intensity based on the walking information of the wearer. As a specific example, the trainer may input feedback information based on information on the exercise of the wearer using the smartphone (300-T) of the trainer, and in the case of inputting feedback information so that the wearer exercises at a stronger intensity, the input feedback information may be transmitted to the server device (100).

According to one embodiment of the present invention, the server device (100) for managing the exercise of the wearable robot wearer may receive, from the first user terminal, the walking information of the wearer wearing the wearable robot (200) that is linked with the first user terminal, and may transmit the received walking information of the wearer to the second user terminal. As a specific example, the server device (100) may receive, from the smartphone (300-1) of member 1, the walking information of the wearer wearing the wearable robot (200), and may transmit the received walking information of the wearer to the smartphone (300-T) of the trainer. Accordingly, the server device (100) may also receive, from the second user terminal, feedback information based on the walking information of the wearer received by the second user terminal. At this time, the feedback information based on the walking information of the wearer received by the second user terminal may include an exercise intensity based on the walking information of the wearer, and the feedback information based on the walking information of the wearer received by the second user terminal may include information that controls in real time the exercise intensity based on the walking information of the wearer. Therefore, in one embodiment of the present invention, the trainer may control in real time the exercise intensity of the wearable robot (200) worn by the member.

According to one embodiment of the present invention, the wearable robot (200-1) of member 1 and the smartphone (300-1) of member 1 may be connected wirelessly. The wireless connection between the wearable robot (200-1) of member 1 and the smartphone (300-1) of member 1 may be connected by Bluetooth, which is a short-range wireless communication, but not only this, may be connected by wireless communication technologies such as Wi-Fi, NFC, Zigbee, ANT plus, LTE-M, NB-IoT, Z-Wave, and LoRa, and is not limited thereto.

According to one embodiment of the present invention, the trainer may monitor in real time the exercise status of each of the members through his/her own smartphone, and may compare the performance among the members to provide individual feedback.

According to one embodiment of the present invention, the trainer may set in a customized manner the exercise intensity suitable for each individual member based on real-time data. For example, in the case of a member whose exercise ability is relatively low, it is possible to lower the exercise intensity by separately setting the strength of the device only for a specific member. That is, in the case of exercising in the walking resistance mode, the strength of the resistance may be lowered.

According to one embodiment of the present invention, the server device (100) may perform a step of accumulating the ratio of power to torque of the drive unit (230) calculated by the drive unit (230) for each walk. In addition, the server device (100) may perform a step of accumulating the ratio of power to torque of the drive unit (230) for each walk and calculating statistics on the accumulation. As a specific example, the server device (100) may receive, from the smartphone (300-1) of member 1, the ratio-of-power-to-torque information of the drive unit (230) calculated by the drive unit (230). Accordingly, the server device (100) may accumulate the ratio-of-power-to-torque information of the drive unit (230) received from the smartphone (300-1) for each walk. In addition, statistics on the accumulation may be calculated through the accumulated ratio-of-power-to-torque information of the drive unit (230).

According to still another embodiment of the present invention, the wearer terminal (300) may perform a step of receiving, from the server device (100), statistical information on the accumulation obtained by accumulating, for each walk, the ratio of power to torque of the drive unit (230) calculated by the drive unit (230). Accordingly, a step of displaying the statistics on the accumulation received from the server device (100) may be performed. As a specific example, the statistics on the accumulation received from the server device (100) may be provided in the form of a wearer interface, and the statistics on the accumulation received from the server device (100) may be provided in the form of a wearer interface in the form of a physical ability report of the wearer. In addition, the statistical information on the accumulation received from the server device (100) may include exercise program information provided to the wearer, and the exercise program information provided to the wearer may be provided in the form of a wearer interface.

### Method of Quantifying the Physical/Exercise Ability of the Wearable Robot Wearer

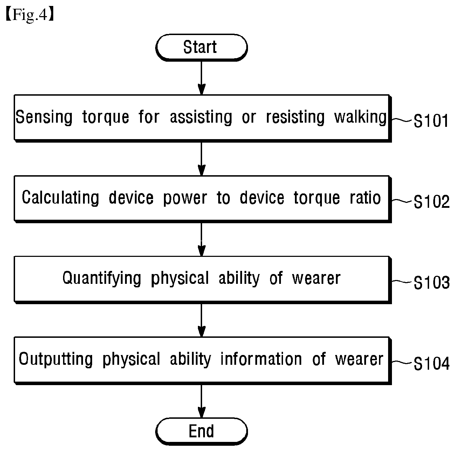

FIG. 4 is a diagram for explaining a method of quantifying the physical/exercise ability of the wearable robot wearer according to the present invention.

As shown in FIG. 4, in order to output physical ability information of the wearable wearer, the method of quantifying the physical/exercise ability of the wearable robot wearer may include a step (S103) of quantifying the physical ability of the wearable wearer. The physical ability information of the wearable wearer may include walking ability information of the wearer, balance ability information of the wearer, lower-body muscle force ability information of the wearer, and the like, but is not limited thereto. In addition, it may further include a step (S104) of outputting the physical ability information of the wearer.

According to the present invention, the wearable robot capable of estimating the physical ability of the wearer during walking exercise may include a fixing unit (210) that may be mounted on a body part and a drive unit (230) that may be mounted on the fixing unit (210). At this time, the drive unit (230) may estimate the physical ability of the wearer based on the power of the drive unit (230) due to the walking exercise of the wearer of the wearable robot (200).

Hereinafter, an embodiment of estimating the physical ability of the wearer based on the power of the drive unit (230) according to one embodiment of the present invention will be described.

### Torque Sensing for Assisting or Resisting Walking (S101)

According to the present invention, the wearable robot capable of estimating the physical ability of the wearer during walking exercise may include a fixing unit (210) that may be mounted on a body part and a drive unit (230) that may be mounted on the fixing unit (210). At this time, the drive unit (230) may estimate the physical ability of the wearer based on the power of the drive unit (230) due to the walking exercise of the wearer of the wearable robot (200). Specifically, the drive unit (230) may estimate the physical ability of the wearer based on a value obtained by normalizing the power of the drive unit (230) by torque. In addition, the drive unit (230) may calculate the ratio of power to torque of the drive unit (230) by dividing the power of the drive unit (230) by the RMS (Root Mean Square) torque of the drive unit (230).

As described above, since the torque must be sensed in order to estimate the physical ability of the wearer based on the power of the drive unit (230), hereinafter, the step (S101) of sensing the torque for assisting the walking of the wearable robot wearer or the torque for resisting will be described.

As shown in FIG. 4, in order to estimate the physical ability information of the wearable wearer, the step (S101) of sensing the torque for assisting the walking of the wearable robot wearer or the torque for resisting may be preceded.

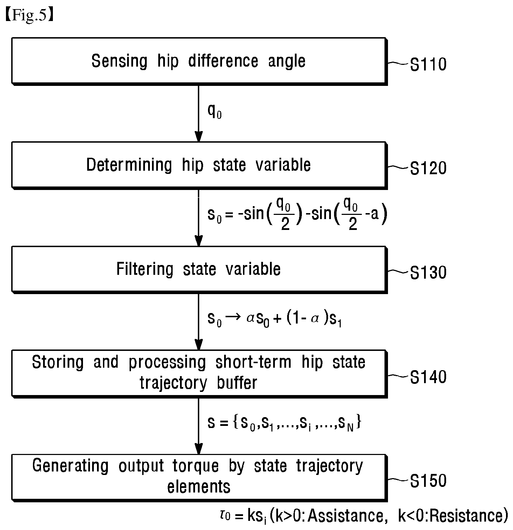

On the other hand, a step of outputting the torque for assisting the walking of the wearable robot wearer or the torque for resisting may be performed, and this step includes, as shown in FIG. 5, a step of sensing the hip angle difference value (q0). The torque output by performing the step of sensing the hip angle difference value (q0) may provide an assisting driving force to generate positive power in the case of the assistance mode, and may provide a resisting driving force to generate negative power in the case of the resistance mode (exercise mode).

That is, in other words, in order to estimate the physical ability information of the wearable wearer, the step (S101) of sensing the torque for assisting the walking of the wearable robot wearer or the torque for resisting may be preceded, and in order to calculate the device power calculated to estimate the physical ability information of the wearable wearer, the torque for assisting the walking of the wearable robot wearer or the torque for resisting is sensed, and in the case of providing a driving force to the wearer, the torque may be output by calculation. Hereinafter, a mechanism for obtaining the torque calculated using the state trajectory memory buffer (200') will be described.

### State Trajectory Memory Buffer and Motion State Value

FIG. 5 is a diagram showing a method of calculating torque to provide a driving force to the wearable robot wearer using the state trajectory memory buffer according to the present invention.

According to the present invention, the wearable robot capable of estimating the physical ability of the wearer during walking exercise may include a fixing unit (210) that may be mounted on a body part and a drive unit (230) that may be mounted on the fixing unit (210). At this time, the drive unit (230) may estimate the physical ability of the wearer based on the power of the drive unit (230) due to the walking exercise of the wearer of the wearable robot (200). Specifically, the drive unit (230) may estimate the physical ability of the wearer based on a value obtained by normalizing the power of the drive unit (230) by torque. In addition, the drive unit (230) may calculate the ratio of power to torque of the drive unit (230) by dividing the power of the drive unit (230) by the RMS (Root Mean Square) torque of the drive unit (230). At this time, the torque for calculating the power of the drive unit (230) may mean the torque value sensed by the drive unit (230) during the walking of the wearer. On the other hand, as described above, the torque output in the step of outputting the torque for assisting the walking of the wearable robot wearer or the torque for resisting may be used when providing a driving force to the wearer of the wearable robot (200).

According to one embodiment of the present invention, in order to provide a driving force to the wearer of the wearable robot (200), the movement detection sensor (247) of the drive unit (230) may perform a step (S110) of sensing the hip angle of the wearer of the wearable robot (200). The processor unit (not shown) of the drive unit (230) may calculate the angular velocity based on the sensed hip angle. Specifically, the movement detection sensor (247) located on the control board (237) of the drive unit (230) of FIG. 1B senses the hip angle of the wearer, and the processor unit (not shown) of the control board (237) may calculate the angular velocity by differencing and filtering the hip angle sensed by the movement detection sensor (247) at a speed of 1000 Hz or more. That is, according to the present invention, even without using a separate tachometer for sensing the angular velocity, the angular velocity may be calculated through hip angle sensing. More specifically, the processor unit (not shown) of the control board (237) performs real-time control, and the processor unit (not shown) may be divided into a low-level processor and a high-level processor, and the low-level processor, which has a relatively small amount of computation, may calculate the angular velocity by differencing and filtering the hip angle sensed by the movement detection sensor (247) at a speed of 1000 Hz or more.

According to one embodiment of the present invention, the torque output in the step of outputting the torque for assisting the walking of the wearable robot wearer or the torque for resisting may be a torque calculated by calculating a plurality of motion state values based on the angular velocity calculated by the drive unit (230), selecting at least one motion state value among them, and the weighted sum of the selected motion state value. At this time, the calculated plurality of motion state values may be stored by repeating a shift operation through the state trajectory memory buffer (200'). Hereinafter, the plurality of motion state values stored by repeating a shift operation through the state trajectory memory buffer (200') will be described.

As shown in FIG. 5, in order to provide a driving force to the wearer of the wearable robot (200), a step of outputting, in the processor unit (not shown) of the drive unit (230), the torque for assisting the walking of the wearable robot wearer or the torque for resisting may be performed. Specifically, the step of outputting the torque for assisting the walking of the wearable robot wearer or the torque for resisting may include a step (S120) of determining the motion state value. In addition, it may further include a step (S130) of filtering the motion state value. In addition, it may further include a step (S140) of deleting the previously stored oldest motion state value from the memory.

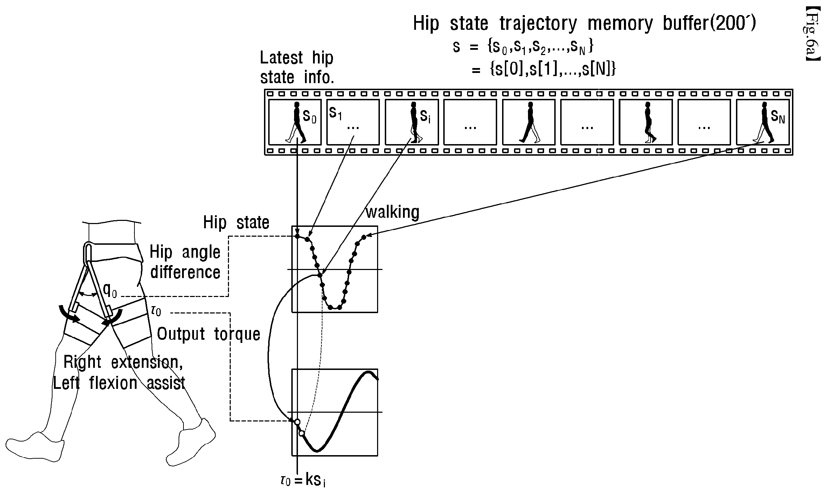

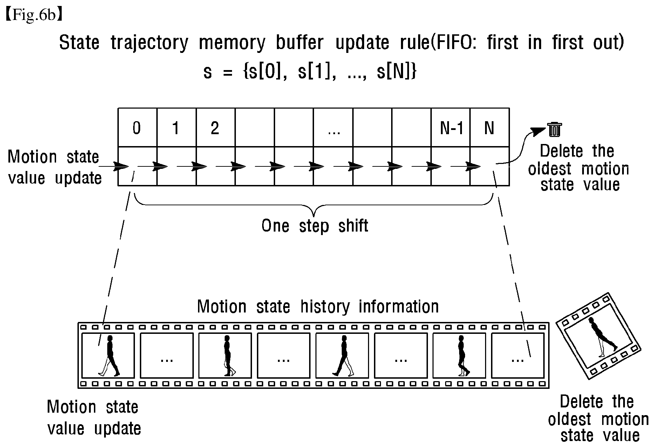

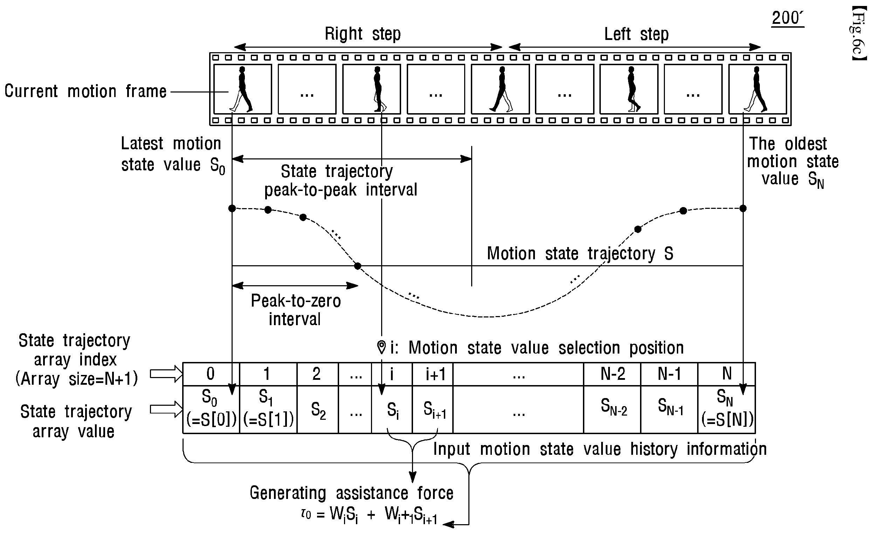

FIG. 6a is a diagram for showing the operation of sequentially storing motion state values based on the state trajectory memory buffer according to one embodiment of the present invention. FIG. 6b is a diagram showing an embodiment in which the motion state value stored at the last storage position (N) is deleted from the state trajectory memory buffer according to one embodiment of the present invention. FIG. 6c is a diagram showing an embodiment in which a motion state value reflecting the motion state of the wearer according to the motion of the wearer is stored in the state trajectory memory buffer according to one embodiment of the present invention.

FIG. 5 is a diagram showing a method of calculating torque to provide a driving force to the wearable robot wearer using the state trajectory memory buffer according to the present invention. Hereinafter, the method of calculating torque to provide a driving force will be described step by step based on FIG. 5.

As shown in FIG. 6a, the state trajectory memory buffer (200') is a storage device that sequentially stores the motion state values of the wearer. Specifically, as shown in FIG. 6b, when the first motion state value is input, it is stored at the first storage position (0) of the memory array. Then, when a new motion state value is input, the motion state value stored at the first storage position of the memory array is moved to the second storage position of the memory array, and the newly input motion state value is stored at the first storage position of the memory array.

Then, when a new motion state value is input, the motion state values respectively stored at the first storage position and the second storage position of the memory array are moved to the second storage position and the third storage position of the memory array, and the newly input motion state value is stored at the first storage position of the memory array.

When such a shift operation is repeated, N+1 motion state values are stored at all storage positions of the memory array. Accordingly, when a new motion state value is input, the motion state values stored in the memory array are moved to the next position, but the motion state value stored at the last storage position (N), which has no more place to move, is deleted from the state trajectory memory buffer (200').

By storing the motion state values in such a FIFO (First In First Out) manner, only a preset number of motion state values for the recent movement of the wearer may be stored in the state trajectory memory buffer (200').

In addition, the motion state value stored in the state trajectory memory buffer (103) may be a sensed value (q0) estimated by the sensor or a converted value of the sensed value that may show the motion state of the wearer. As a specific example, the hip angle difference value (q0) may be the angle value (q0) between the one-side target body part and the other-side target body part of the wearer estimated by the sensor of the wearable robot (200). That is, as shown in FIG. 5, the step (S110) of sensing the hip angle difference value (q0) may be performed first. The angle value (q0) between the one-side target body part and the other-side target body part of the wearer estimated by the sensor of the wearable robot (200) may itself be the motion state value (s0).

In addition, the converted value (s0) obtained by converting the estimated angle value (q0) between the one-side target body part and the other-side target body part by [Equation 1] below may be the motion state value. [Equation 1] ${s}_{o} = A sin \left(\frac{{q}_{o}}{2}\right)$

Here, A is an arbitrary constant value, such as, for example, -2.

The motion state value as above may be stored, as shown in FIG. 6c, in the state trajectory memory buffer (200') as a motion state value reflecting the motion state of the wearer according to the motion of the wearer.

In addition, in the original state variable, the following state variable, to which an asymmetry parameter a is added as in the following [Equation 2], may be used. Such a calculation may be performed, as shown in FIG. 5, in the step (S120) for determining the converted value (S0) by adding the asymmetry parameter a. [Equation 2] ${s}_{o} = - sin \left(\frac{{q}_{o}}{2}\right) - sin \left(\frac{{q}_{o}}{2 - a}\right)$

Here, if a=0, it means that there is no degree of asymmetry, and the same torque is symmetrically provided to the left-right motion. If the a value is negative, the assistance torque is increased for the motion of the left-side movement compared to the motion of the right-side movement. If the a value is positive, the assistance torque is increased for the motion of the right-side movement compared to the motion of the left-side movement. That is, the asymmetric assistance for the left-side or right-side movement may be adjusted with the single parameter a.

According to one embodiment of the present invention, the asymmetry of the left/right-side movement is determined by the sign of the a value, and the degree of asymmetry may be adjusted by the magnitude of the a value. For example, in the case where a=0.20 compared to when a=0.10, the right asymmetric assistance is further strengthened. The a value may be within the range of -1.0 to 1.0, and a value of -0.5 to 0.5 is preferably used.

By using the above method, asymmetric torque generation becomes possible even in a wearable robot using a single actuator. The asymmetric mode may be used for resolving the imbalance of the left/right-side range of motion and for posture improvement, and may be used for different purposes depending on the wearer or the use. For example, when assisting the motion of a person who performs an asymmetric motion due to a disease such as stroke, the asymmetric mode may be usefully used. In addition, for example, in the case where the wearable robot according to one embodiment of the present invention is used for the assistance of walking motion, the asymmetric mode may be utilized for walking navigation assistance.

In a complex environment, a wayfinding assistance function such as inducing a speed reduction by reducing the torque strength, direction guidance through asymmetric assistance at curves, and increasing the speed on straight distances would be possible. In addition, if a vision sensor is used by a method such as mounting a smartphone on an exoskeleton device, it could also be used for the walking movement of the visually impaired.

According to one embodiment of the present invention, after performing the step (S120) for determining the converted value (S0), a filtering step (S130) may be performed. The present invention may change a predetermined position (i) for selecting the motion state value according to the motion state of the wearer in order to adaptively respond to a sudden motion change of the wearable robot wearer. When the assistance force is determined by adaptively selecting the motion state value according to the motion change of the wearer, the motion change of the wearer may be responded to more quickly than in the case where the motion-state-value selection position is fixed.

Although the motion state values for one-cycle motion of the wearer are shown as being stored in FIG. 6c, the motion state values stored in the state trajectory memory buffer (200') need only represent the movement of the wearer during a recent short time (within several seconds, for example, within 1 to 2 seconds).

For example, the motion state value stored in the state trajectory memory buffer (103) may include only one motion information of the wearer (a half-cycle of the repetitive movement). The present invention may be carried out even if the motion state value stored in the state trajectory memory buffer (103) includes only one motion of the wearer, that is, a half-cycle of the repetitive movement.

Preferably, the motion state value stored in the state trajectory memory buffer (200') may store a motion state value for a movement including one cycle of the repetitive movement of the wearer. For example, in the case where the wearable robot according to one embodiment of the present invention is worn on a lower-body part such as the waist or the thigh, the one-cycle motion of the wearer may be a one-cycle walking motion of the wearer (for example, a step motion, etc.).

Hereinafter, the operation of storing the motion state value in the state trajectory memory buffer (200') will be described.

As shown in FIG. 6a, the state trajectory memory buffer (200') is a storage device that sequentially stores the motion state values of the wearer. Specifically, as shown in FIG. 6b, when the first motion state value is input, it is stored at the first storage position (0) of the memory array. Then, when a new motion state value is input, the motion state value stored at the first storage position of the memory array is moved to the second storage position of the memory array, and the newly input motion state value is stored at the first storage position of the memory array.

Then, when a new motion state value is input, the motion state values respectively stored at the first storage position and the second storage position of the memory array are moved to the second storage position and the third storage position of the memory array, and the newly input motion state value is stored at the first storage position of the memory array.

When such a shift operation is repeated, N+1 motion state values are stored at all storage positions of the memory array. Accordingly, when a new motion state value is input, the motion state values stored in the memory array are moved to the next position, but the motion state value stored at the last storage position (N), which has no more place to move, is deleted from the state trajectory memory buffer (200').

By storing the motion state values in such a FIFO (First In First Out) manner, in the state trajectory memory buffer (200'), a preset number of motion of the recent movement of the wearer; the asymmetric mode may be used for resolving the imbalance of the left/right-side range of motion and for posture improvement, and may be used for different purposes depending on the user or the use. For example, when assisting the movement of a person who performs asymmetric walking due to a stroke disease, the asymmetric mode may be usefully used. In addition, the asymmetric mode may be utilized for walking navigation assistance. In a complex environment, a wayfinding assistance function such as inducing a speed reduction by reducing the torque strength, direction guidance through asymmetric assistance at curves, and increasing the speed on straight distances would be possible. In addition, if a vision sensor is used by a method such as mounting a smartphone on an exoskeleton device, it could also be used for the walking movement of the visually impaired.

According to one embodiment of the present invention, the motion state value stored in the state trajectory memory buffer (200') need only represent the movement of the user during a recent short time (within several seconds, for example, within 1 to 2 seconds). For example, the motion state value stored in the state trajectory memory buffer (200') may include only one step information of the user (a walking half-cycle). The present invention may be carried out even if the motion state value stored in the state trajectory memory buffer (200') includes only one step of the user, that is, a walking half-cycle. Preferably, the motion state value stored in the state trajectory memory buffer (200') may store a motion state value for a movement including one cycle of the repetitive movement of the user.

Next, the drive unit (230) may include a determination unit (not shown). Here, the determination unit (not shown) is a processing device that determines the assistance force to be provided according to the movement of the user. The determination unit (not shown) uses the motion state values stored in the state trajectory memory buffer (200') to determine the assistance force. Specifically, the determination unit (not shown) selects at least one motion state value among the motion state values stored in the state trajectory memory buffer (200'), determines the assistance force (τ0) by the weighted sum of the selected motion state values, and outputs the determined assistance force, so that an appropriate assistance force is provided to the user. At this time, the determination unit (not shown) may be set to select the motion state value stored at a predetermined position (i) in the state trajectory memory buffer (200'). The predetermined position may be any position of the memory array. However, in order to improve the stability of the motion assistance device even in the case of a sudden motion change of the user and to provide a smooth assistance force to the user, it is preferable not to select the motion state value stored at the first storage position (0) of the memory array, which stores the current walking state value. For example, when the motion state value is a value generated every 0.01 seconds, the predetermined position i is preferably 20 to 40, and accordingly, it may be set so that the motion state value S[20] to the motion state value S[40] are selected. However, it is not necessarily limited to such a range.

In addition, the motion state value may have noise removed using a low-pass filter before being stored in the state trajectory memory buffer (200'), but the determination unit (not shown) may select two or more motion state values in consideration of the possibility that noise is included among the stored motion state values. At this time, the two or more motion state values are preferably continuous motion state values (for example, S[i], S[i+1], ...) rather than motion state values stored spaced apart in the memory array. In addition, the determination unit (not shown) may adaptively change the predetermined position (i) for selecting the motion state value according to the movement of the user. However, even in this case, in order to provide a stable assistance force, it is preferable to set a changeable range of the position for selecting the motion state value (for example, i is 10 to 50). At this time, if the determination unit (not shown) must recognize the user's motion speed, cadence, phase, and the like, as in the conventional technologies in order to grasp the movement of the user, walking state values corresponding to at least two or three steps must be analyzed, and due to the time required for the analysis, it cannot respond immediately to the change in the movement of the user. However, the determination unit (not shown) may provide an assistance force that responds immediately and adaptively to the change in the movement of the user merely by using the walking state values stored in the state trajectory memory buffer (200') to grasp the movement of the user and changing the position for selecting the motion state value according to the grasped result.

The motion assistance system according to one embodiment of the present invention has been described so far, and although such a motion assistance system has been described as a system included in the motion assistance device for control, the motion assistance system according to one embodiment of the present invention may also be the motion assistance device itself. In addition, although the assistance force has been described as being provided in the same direction as the movement of the user to assist the movement, it may also be provided in the opposite direction to the movement of the user for an exercise effect.

### Determination of Output Torque

Hereinafter, the step (S150) of generating the output torque based on the stored motion state value will be described.

According to the present invention, the wearable robot capable of estimating the physical ability of the wearer during walking exercise may include a fixing unit (210) that may be mounted on a body part and a drive unit (230) that may be mounted on the fixing unit (210). At this time, the drive unit (230) may estimate the physical ability of the wearer based on the power of the drive unit (230) due to the walking exercise of the wearer of the wearable robot (200). Specifically, the drive unit (230) may estimate the physical ability of the wearer based on a value obtained by normalizing the power of the drive unit (230) by the sensed torque. In addition, the drive unit (230) may calculate the ratio of power to torque of the drive unit (230) by dividing the power of the drive unit (230) by the RMS (Root Mean Square) torque of the drive unit (230).

At this time, the torque for calculating the power of the drive unit (230) may mean the torque value sensed by the drive unit (230) during the walking of the wearer. On the other hand, the torque output in the step (S101) of outputting the torque for assisting the walking of the wearable robot wearer or the torque for resisting may be used when providing the driving force provided to the wearer of the wearable robot (200). In this case, the torque may be calculated by calculating a plurality of motion state values based on the angular velocity calculated by the drive unit (230), selecting at least one motion state value among the calculated plurality of motion state values, and the sum of the weights of the selected motion state value.

According to the present invention, the step (S150) of outputting the assistance force to be provided according to the movement of the wearer may use the motion state values stored in the state trajectory memory buffer (103) to determine the assistance force. Specifically, in the step (S150) of outputting the assistance force to be provided according to the movement of the wearer, at least one motion state value among the motion state values stored in the state trajectory memory buffer (200') is selected, the assistance force (τ0) is determined by the weighted sum of the selected motion state values, and the determined assistance force is output, so that an appropriate assistance force is provided to the wearer.

At this time, it may be set to select the motion state value stored at a predetermined position (i) in the state trajectory memory buffer (200'). Specifically, the predetermined position may be any position of the memory array. However, in order to improve the stability of the motion assistance device even in the case of a sudden motion change of the wearer and to provide a smooth assistance force to the wearer, it is preferable not to select the motion state value stored at the first storage position (0) of the memory array, which stores the current walking state value. For example, when the motion state value is a value generated every 0.01 seconds, the predetermined position i is preferably 20 to 40, and accordingly, it may be set so that the motion state value S[20] to the motion state value S[40] are selected.

However, it is not necessarily limited to such a range.

In addition, the motion state value may have noise removed using a low-pass filter before being stored in the state trajectory memory buffer (200'). Two or more motion state values may be selected in consideration of the possibility that noise is included among the stored motion state values.

At this time, the two or more motion state values are preferably continuous motion state values (for example, S[i], S[i+1], ...) rather than motion state values stored spaced apart in the memory array.

In addition, the predetermined position (i) for selecting the motion state value according to the movement of the wearer may be adaptively changed. However, even in this case, in order to provide a stable assistance force, it is preferable to set a changeable range of the position for selecting the motion state value (for example, i is 10 to 50).

At this time, an assistance force that responds immediately and adaptively to the change in the movement of the wearer may be provided merely by using the walking state values stored in the state trajectory memory buffer (200') to grasp the movement of the wearer and changing the position for selecting the motion state value according to the grasped result.

### Calculation of the Device Power to Device Torque Ratio (S102)

FIG. 4 is a diagram for explaining a method of quantifying the physical/exercise ability of the wearable robot wearer according to the present invention.

As shown in FIG. 4, in order to output the physical ability information of the wearable wearer, after the step (S101) of sensing the torque for assisting the walking of the wearable robot wearer or the torque for resisting, a step (S102) of calculating the device power to device torque may be included.

According to the present invention, the wearable robot capable of estimating the physical ability of the wearer during walking exercise may include a fixing unit (210) that may be mounted on a body part and a drive unit (230) that may be mounted on the fixing unit (210). At this time, the drive unit (230) may estimate the physical ability of the wearer based on the power of the drive unit (230) due to the walking exercise of the wearer of the wearable robot (200). Specifically, the drive unit (230) may estimate the physical ability of the wearer based on a value obtained by normalizing the power of the drive unit (230) by torque.

In addition, the drive unit (230) may calculate the ratio of power to torque of the drive unit (230) by dividing the power of the drive unit (230) by the RMS (Root Mean Square) torque of the drive unit (230). At this time, the torque for calculating the power of the drive unit (230) may mean the torque value sensed by the drive unit (230) during the walking of the wearer. On the other hand, the torque output in the step (S101) of outputting the torque for assisting the walking of the wearable robot wearer or the torque for resisting may be used when providing the driving force provided to the wearer of the wearable robot (200).

According to one embodiment of the present invention, the drive unit (230) may sense the hip angle of the wearer of the wearable robot (200), and may calculate the angular velocity based on the sensed hip angle. Specifically, the movement detection sensor (247) located on the control board (237) of the drive unit (230) of FIG. 1b senses the hip angle of the wearer, and the processor unit (not shown) of the control board (237) may calculate the angular velocity by differencing and filtering the hip angle sensed by the movement detection sensor (247) at a speed of 1000 Hz or more. That is, according to the present invention, even without using a separate tachometer for sensing the angular velocity, the angular velocity may be calculated through hip angle sensing. More specifically, the processor unit (not shown) of the control board (237) performs real-time control, and the processor unit (not shown) may be divided into a low-level processor and a high-level processor, and the low-level processor, which has a relatively small amount of computation, may calculate the angular velocity by differencing and filtering the hip angle sensed by the movement detection sensor (247) at a speed of 1000 Hz or more.

According to one embodiment of the present invention, the drive unit (230) may sense the hip angle of the wearer of the wearable robot (200), and may calculate the angular velocity based on the sensed hip angle. Specifically, the calculated angular velocity may be an angular velocity calculated based on the walking of the wearer in the case where the wearer uses a backdrivable actuator. As a specific example, the backdrivable actuator may mean an actuator that moves with an external force of 0.5 Nm or less, that is, an external force equal to the rotational force generated by a force of 2.5 N at the position of a 20 cm moment arm, in a state where the device power is off, but is not limited thereto.

According to one embodiment of the present invention, the drive unit (230) may sense the hip angle of the wearer of the wearable robot (200), and may calculate the angular velocity based on the sensed hip angle. At this time, the calculated angular velocity may be an angular velocity calculated based on the walking of the wearer in the case where the wearer uses a backdrivable actuator, and through this, the actual angular velocity of the actual wearer of the wearable robot (200) may be reflected. In other words, because a backdrivable actuator is used, the hip swing speed due to the movement of the wearer may be reflected even in the case of the walking mode or the exercise mode. Because the drive unit (230) of the present invention uses force control rather than position control based on the backdrivable actuator, it may move adaptively to the movement of the actual wearer of the wearable robot (200).

In the step (S102) of calculating the device power to device torque, the device power torque ratio may be calculated by [Equation 3] below. [Equation 3] $Power _ Torque _ Ratio = \left(\begin{matrix}\frac{Mean _ Positive _ Power}{RMS _ Torque} , \left(for k>0;Assist\right) \\ \frac{Mean _ Negative _ Power}{RMS _ Torque} , \left(for k<0;Resist\right)\end{matrix}\right)$ [Equation 4] $\begin{matrix}Mean _ Positive _ Power = \frac{1}{n} {\sum }_{i = 1}^{{n}_{p}} {τ}_{i} {\dot{q}}_{i} , \left(for {τ}_{i}{\dot{q}}_{i}>0\right) \\ Mean _ Negative _ Power = \frac{1}{n} {\sum }_{i = 1}^{{n}_{n}} {τ}_{i} {\dot{q}}_{i} , \left(for {τ}_{i}{\dot{q}}_{i}<0\right) \\ RMS _ Torque = \sqrt{\frac{1}{n} {\sum }_{i = 1}^{n} {τ}_{i}^{2}} \\ n = {n}_{p} + {n}_{n}\end{matrix}$

According to the present invention, *n*(=*nₚ*+*nₙ*) may be equal to the number of cycle sample data at one walk of the wearable robot wearer, or may be a certain operation time or a certain number of steps. Preferably, *n*(=*nₚ+nₙ*) may be equal to the number of cycle sample data at one walk of the wearer. By [Equation 4], a positive power value and a negative power value may be calculated.

According to the present invention, *nₚ* is the number of positive power data, and *nₙ* may be the number of negative power data. Specifically, when the direction of the angular velocity of the hip angle difference value (q0) and the direction of the device torque are the same, that is, in the case of the walking assistance mode, power transmission from the wearable robot (200) to the wearer occurs, and at this time, since the motion state value is multiplied by a positive weight, the power value may be calculated as a positive number. Thus, in the walking assistance mode, positive power values may be mainly calculated.

According to still another embodiment of the present invention, when the direction of the angular velocity of the hip angle difference value (q0) and the direction of the device torque are different, that is, in the case of the walking resistance mode, power transmission from the wearer of the wearable robot (200) to the wearable robot (200) occurs, and since the motion state value is multiplied by a negative weight, the power value may be calculated as a negative number.

### One Embodiment of the Method Invention

According to one embodiment of the present invention, the drive unit (230) may accumulate the calculated ratio of power to torque of the drive unit (230) for each walk. The processor unit (not shown) of the control board (237) of the drive unit (230) may accumulate the ratio of power to torque of a specific wearer of the wearable robot (200) for each walk. In addition, statistics for estimating the physical ability of the specific wearer of the wearable robot (200) may be calculated through the accumulated ratio of power to torque of the specific wearer of the wearable robot (200).

According to the present invention, a method of estimating the physical ability of the wearer during walking using a wearable robot including a drive unit may be provided. The method of estimating the physical ability of the wearer during walking using a wearable robot including a drive unit may include a step of accumulating, for each walk, the ratio of power to torque of the specific wearer of the wearable robot (200) of the drive unit (230) calculated by the drive unit (230), and a step of calculating statistics on the accumulation. As a specific example, through this, it is possible to numerically quantify the physical ability of the wearer or the exercise ability of the wearer, and a wearer interface that may visualize the change in the exercise ability of the wearer may also be provided in the case where an exercise program is performed. As another example, it is possible to quantify and visualize the level of the physical ability of the wearer or the exercise ability of the wearer at the end of each exercise of the wearer, and it is also possible to visually represent the change in the physical ability of the wearer or the exercise ability of the wearer after the end of an exercise program for a specific period, and it is also possible to provide a wearer interface in the form of a report analyzing the physical ability or the exercise ability.

According to one embodiment of the present invention, in the case of providing the step of accumulating the ratio of power to torque of the specific wearer of the wearable robot (200) for each walk and the step of calculating statistics on the accumulation, the wearer of the wearable robot (200) may be motivated to complete the exercise program, and the trainer may effectively carry out the individually customized response of the exercise program using the wearable robot (200).

According to still another embodiment of the present invention, in the case of providing a method of estimating the physical ability of the wearer during walking using a wearable robot including a drive unit, a step of receiving, from the server device (100) shown in FIG. 3, statistics on the accumulation obtained by accumulating, for each walk, the ratio of power to torque of the drive unit (230) calculated by the drive unit (230), and a step of displaying the calculated statistics on the accumulation may be provided. Specifically, the statistics on the accumulation obtained by accumulating, for each walk, the ratio of power to torque of the drive unit (230) calculated by the drive unit (230) may be output in the form of a wearer interface such as a walking analysis report. In this case, the wearer of the wearable robot (200) may easily recognize the change in physical ability during a specific period or the strengths and points to be supplemented among the physical abilities of the wearer of the wearable robot (200) through a wearer interface such as a visualized walking analysis report.

### Embodiment Utilizing Artificial Intelligence

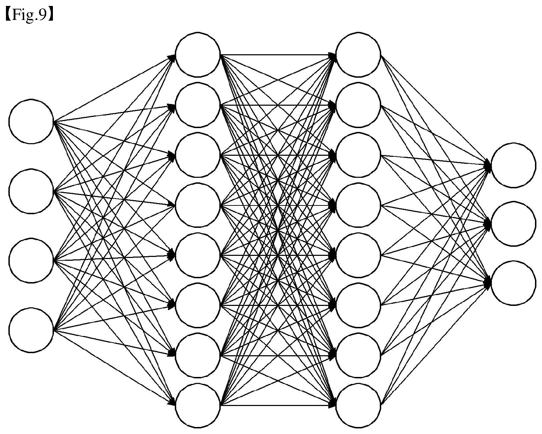

FIG. 9 is a schematic diagram showing one or more network functions for performing a method of proposing exercise information based on a database according to the correlation between the ratio of power to torque of the drive unit (230) according to the present invention and the wearer's physical condition.

According to one embodiment of the present invention, a method of estimating the physical ability of the wearer during walking using a wearable robot including a drive unit may be provided, and the method of estimating the physical ability of the wearer during walking using a wearable robot including a drive unit may include a step of proposing exercise information based on a database according to the correlation between the ratio of power to torque of the drive unit (230) calculated by the drive unit (230) and the wearer's physical condition. As a specific example, the step of proposing exercise information based on a database according to the correlation between the ratio of power to torque of the drive unit (230) calculated by the drive unit (230) and the wearer's physical condition may be performed in the server device (100), but may also be performed in the processor unit (not shown) of the drive unit (230).

In addition, according to one embodiment of the present invention, the method of estimating the physical ability of the wearer during walking using a wearable robot including a drive unit (230) may include a step of proposing exercise information based on a deep learning model trained using, as training data, the ratio of power to torque of the drive unit (230) calculated by the drive unit (230) and the wearer's physical condition. As a specific example, the step of proposing exercise information based on a deep learning model trained using, as training data, the ratio of power to torque of the drive unit (230) calculated by the drive unit (230) and the wearer's physical condition may be performed in the server device (100), but may also be performed in the processor unit (not shown) of the drive unit (230).

In addition, according to one embodiment of the present invention, the method of estimating the physical ability of the wearer during walking using a wearable robot including a drive unit may include a step of predicting an exercise effect based on a deep learning model trained using, as training data, at least any one or more of the ratio of power to torque of the drive unit (230) calculated by the drive unit (230) and the wearer's physical condition, wearable robot usage information, and exercise effect. As a specific example, the step of predicting an exercise effect based on a deep learning model trained using, as training data, at least any one or more of the ratio of power to torque of the drive unit (230) calculated by the drive unit (230) and the wearer's physical condition, wearable robot usage information, and exercise effect may be performed in the server device (100), but may also be performed in the processor unit (not shown) of the drive unit (230).

Hereinafter, the operation of the server device (100) or the processor unit (not shown) of the drive unit (230) will be described.

The server device (100) or the processor unit (not shown) of the drive unit (230) may perform calculations for the learning of a neural network, such as processing of input data for learning in deep learning (DL), feature extraction from input data, error calculation, and weight update of the neural network using backpropagation. In addition, at least one of the CPU, GPGPU, and TPU of the server device (100) or the processor unit (not shown) of the drive unit (230) may process the learning of the network function. For example, the CPU and the GPGPU together may process the learning of the network function and the data classification using the network function. In addition, in one embodiment of the present invention, the processors of a plurality of computing devices may be used together to process the learning of the network function and the data classification using the network function. In addition, the computer program executed in the computing device according to one embodiment of the present invention may be a CPU, GPGPU, or TPU executable program.

In this specification, the network function may be used interchangeably with an artificial neural network and a neural network. In this specification, the network function may include one or more neural networks, and in this case, the output of the network function may be an ensemble of the outputs of one or more neural networks. In this specification, a model may include a network function. A model may include one or more network functions, and in this case, the output of the model may be an ensemble of the outputs of one or more network functions.

According to the present invention, a deep neural network (DNN) may mean, as shown in FIG. 9, a neural network including a plurality of hidden layers in addition to an input layer and an output layer. The deep neural network may include a convolutional neural network (CNN), a recurrent neural network (RNN), an auto encoder, GAN (Generative Adversarial Networks), a restricted boltzmann machine (RBM), a deep belief network (DBN), a Q network, a U network, a Siamese network, a Transformer, ViT (Vision Transformer), Mobile ViT (Mobile Vision Transformer), and the like. The description of the aforementioned deep neural network is merely an example, and the present invention is not limited thereto.

In the present invention, the network function may also include an auto encoder. The auto encoder may be a kind of artificial neural network for outputting output data similar to input data. The auto encoder may include at least one hidden layer, and an odd number of hidden layers may be disposed between the input and output layers. The number of nodes of each layer may be reduced from the number of nodes of the input layer to an intermediate layer called the bottleneck layer (encoding), and then expanded symmetrically with the reduction from the bottleneck layer to the output layer (symmetrical to the input layer). The nodes of the dimension reduction layer and the dimension restoration layer may or may not be symmetrical.

According to the present invention, the auto encoder may perform nonlinear dimension reduction. The number of input layers and output layers may correspond to the number of sensors remaining after the preprocessing of the input data. In the auto encoder structure, the number of nodes of the hidden layer included in the encoder may have a structure that decreases as it moves away from the input layer. Since a sufficient amount of information may not be transmitted if the number of nodes of the bottleneck layer (the layer with the fewest nodes located between the encoder and the decoder) is too small, it may be maintained at a certain number or more (for example, half or more of the input layer, etc.).

According to the present invention, the neural network may be trained by at least one of supervised learning, unsupervised learning, and semi-supervised learning. The learning of the neural network is for minimizing the error of the output. In the learning of the neural network, it is a process of repeatedly inputting training data into the neural network, calculating the error of the output of the neural network and the target for the training data, and backpropagating the error of the neural network from the output layer of the neural network toward the input layer in a direction to reduce the error, thereby updating the weight of each node of the neural network. In the case of supervised learning, training data in which the correct answer is labeled on each piece of training data is used (that is, labeled training data), and in the case of unsupervised learning, the correct answer may not be labeled on each piece of training data. That is, for example, in the case of supervised learning regarding data classification, the training data may be data in which a category is labeled on each piece of training data. The labeled training data is input into the neural network, and the error may be calculated by comparing the output (category) of the neural network with the label of the training data.

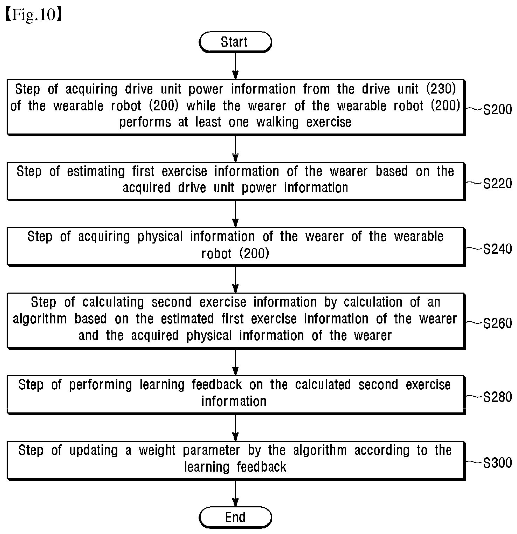

FIG. 10 is a diagram for explaining a method of calculating an importance score of a category of physical information or exercise information of the wearer of the wearable robot (200) based on a deep neural network, according to one embodiment of the present invention.

According to one embodiment of the present invention, an importance parameter may be trained based on Reinforcement Learning with Human Feedback (RLHF). Reinforcement Learning with Human Feedback (RLHF) is a combination of reinforcement learning and human feedback, and is a learning method that improves the performance of artificial intelligence by training the information output through an artificial intelligence model based on human feedback. The importance score of the category of the physical information or exercise information of the wearer of the wearable robot (200) may be calculated based on the importance parameter trained according to one embodiment of the present invention.

The reinforcement learning method based on human feedback according to one embodiment of the present invention may include a step (S200) of acquiring drive unit power information from the drive unit (230) of the wearable robot (200) while the wearer of the wearable robot (200) performs at least one walking exercise, a step (S220) of estimating first exercise information of the wearer based on the acquired drive unit power information, a step (S240) of acquiring physical information of the wearer of the wearable robot (200), a step (S260) of calculating second exercise information by calculation of an algorithm based on the estimated first exercise information of the wearer and the acquired physical information of the wearer, a step (S280) of performing learning feedback with respect to the calculated second exercise information, and a step (S300) in which the algorithm updates the weight parameter according to the learning feedback.

According to one embodiment of the present invention, a step (S200) of acquiring drive unit power information from the drive unit (230) of the wearable robot (200) while the wearer of the wearable robot (200) performs at least one walking exercise may be performed. As a specific example, in the case where the wearer of the wearable robot (200) performs one walking exercise for the first time or in the case of starting a walking exercise to estimate the physical ability of the wearer, it may be performed to acquire the drive unit power information.

According to one embodiment of the present invention, next, a step (S220) of estimating first exercise information of the wearer based on the acquired drive unit power information may be performed. As a specific example, in the case where the drive unit power information acquired from the drive unit (230) has a value at or below a threshold, the first exercise information of the corresponding wearer may be primarily estimated as having a physical ability at or below the threshold. As another example, in the case where the drive unit power information acquired from the drive unit (230) has a value at or above a threshold, the first exercise information of the corresponding wearer may be primarily estimated as having a physical ability at or above the threshold.

According to one embodiment of the present invention, next, a step (S240) of acquiring physical information of the wearer of the wearable robot (200) and a step (S260) of calculating second exercise information by calculation of an algorithm based on the estimated first exercise information of the wearer and the acquired physical information of the wearer may be performed. Specifically, the physical information of the wearer may correspond to age, sex, weight, height, and the like, but is not limited thereto. As a specific example, in the case where the drive unit power information acquired from the drive unit (230) has a value at or below a threshold, the first exercise information of the corresponding wearer may be primarily estimated as having a physical ability at or below the threshold. However, in the case where the age of the corresponding wearer is at or above 80 years old, in the case where the physical information of the corresponding wearer and the estimated first exercise information of the wearer are judged together, the second exercise information may be calculated as having a physical ability at or above the threshold by the calculation of the algorithm.

According to one embodiment of the present invention, next, a step (S280) of performing learning feedback with respect to the calculated second exercise information may be performed. According to the present invention, it may further include a step of setting the ranking of the categories judged to have high importance among the physical information of the wearer in order to perform the learning feedback. In addition, according to one embodiment of the present invention, the importance of the categories of the wearer's physical information may be judged, and the ranking of the three judged to have high importance may be determined. In this case, the sample set for performing the learning feedback may correspond to a data set consisting of ordered pairs of the rankings from 1st place to 3rd place and the names of the categories of the wearer's physical information corresponding to each ranking, but the specific number of 3rd place here is merely an example and is not limited thereto. According to the present invention, the step (S280) of performing learning feedback with respect to the calculated second exercise information performs learning feedback that compares the calculated importance score with the sample set for performing the learning feedback.

According to one embodiment of the present invention, next, a step (S300) in which the algorithm updates the weight parameter according to the learning feedback may be performed. As a specific example, the step (S300) in which the algorithm updates the weight parameter according to the learning feedback may, when learning feedback that compares the importance score calculated in the step (S280) of performing learning feedback with respect to the calculated second exercise information with the sample set for performing the learning feedback is performed, update the importance parameter so that the ranking of the categories of the wearer's physical information according to the calculated importance score matches the generated sample set for performing the learning feedback. In addition, by repeating the process of calculating the importance score by calculation of an algorithm that determines the importance among the categories of the wearer's physical information based on the trained importance parameter, and comparing the calculated importance score again with the sample set for performing the learning feedback, the importance parameter may be re-updated.

According to one embodiment of the present invention, a method of estimating the physical ability of the wearer during walking using a wearable robot including a drive unit may be provided, and the method of estimating the physical ability of the wearer during walking using a wearable robot including a drive unit may include a step of proposing exercise information based on a database according to the correlation between the ratio of power to torque of the drive unit (230) calculated by the drive unit (230) and the wearer's physical condition. As a specific example, the step of proposing exercise information based on a database according to the correlation between the ratio of power to torque of the drive unit (230) calculated by the drive unit (230) and the wearer's physical condition may be performed in the server device (100), but may also be performed in the processor unit (not shown) of the drive unit (230).

According to one embodiment of the present invention, the device power torque ratio calculated by the drive unit (230) may be used as an index that may judge the degree of the physical ability of the wearer. As a specific example, it may be used as an index that may judge the walking ability, balance ability, lower-body muscle force degree, and the like, of the wearable robot wearer, but is not limited thereto.

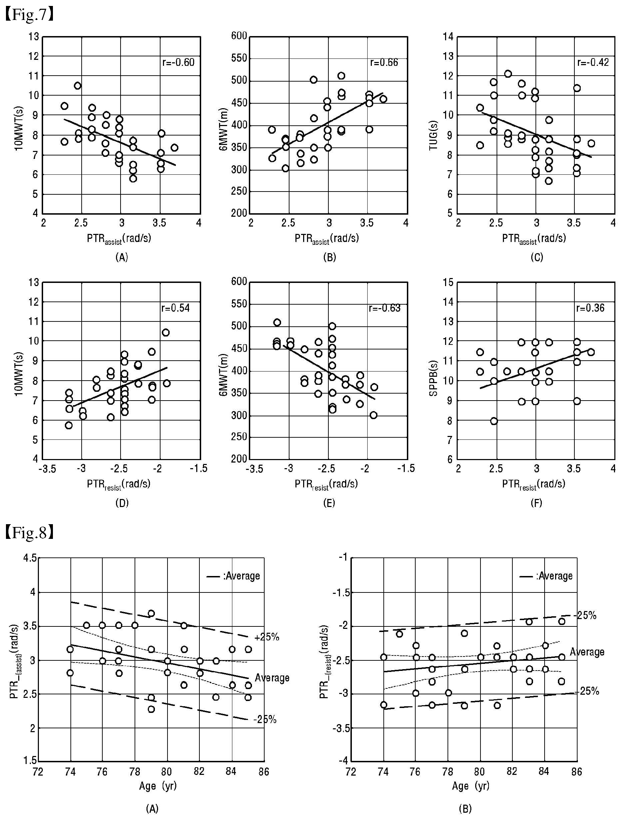

FIG. 7 is a diagram showing the result of analyzing the Pearson correlation between the walking, balance, and physical function indices of the wearable robot wearer and the device power torque ratio. FIG. 7 shows that the device power torque ratio may show a statistically significant linear correlation with the existing verified walking/physical ability indices, and as a result of the analysis, since the p-value is at or below 0.05, it may be judged to be statistically significant.

(A) of FIG. 7 is a diagram showing the result of analyzing the Pearson correlation between the 10MWT (10-meter walking test), which is a balance and walking evaluation index, and the PTR (power torque ratio) value in the case of the walking assistance mode.

According to one embodiment of the present invention, the 10MWT (10-meter walking test), which is a balance and walking evaluation index, is an evaluation tool that may test short-distance walking speed and short-distance walking ability, and is an index that may be used in subject groups such as the elderly, stroke, spinal cord injury, Parkinson's disease, and other central nervous system injury patient groups. At this time, indoor walking and outdoor walking ability may be distinguished according to the measured walking speed, and as shown in (A) of FIG. 7, as the value of 10MWT(s) on the y-axis increases, it means that the measured walking time value is greater, so it may mean that the short-distance walking speed and ability are more lacking.

In addition, since the case where the PTR (power torque ratio) value on the x-axis (in the case of the walking assistance mode) increases may mean that stronger power data is being measured from the wearer, it can be seen that there is a correlation inversely proportional to the value of 10MWT(s) on the y-axis, and accordingly, a graph inversely proportional to the value of 10MWT(s) on the y-axis may be shown. Through this, a database according to the correlation between the ratio of power to torque of the drive unit (230) calculated by the drive unit (230) and the wearer's physical condition may be constructed, and the short-distance walking speed and ability of the wearer may be estimated through the PTR (power torque ratio) value in the case of the walking assistance mode.

(B) of FIG. 7 is a diagram showing the result of analyzing the Pearson correlation between the 6MWT (6-minute walking test), which is a walking endurance evaluation index, and the PTR (power torque ratio) value in the case of the walking assistance mode.

According to one embodiment of the present invention, the 6MWT (6-minute walking test), which is a walking endurance evaluation index, measures the total distance that a walker may walk during 6 minutes, and may evaluate the walking endurance by a method of checking vital signs (mild headache, dizziness, paleness, dyspnea, fatigue, pain, etc.) after sitting on a chair at the time point of 6 minutes. As shown in FIG. 7(B), as the value of 6MWT (6-minute walking test) on the y-axis increases, it means that the distance walked by the walker during 6 minutes is greater, so it may mean that the walking endurance of the walker is more excellent.

In addition, since the case where the PTR (power torque ratio) value on the x-axis (in the case of the walking assistance mode) increases may mean that stronger power data is being measured from the wearer, it can be seen that there is a correlation proportional to the value of 6MWT (6-minute walking test) on the y-axis, and accordingly, a graph proportional to the value of 6MWT (6-minute walking test) on the y-axis may be shown. Through this, a database according to the correlation between the ratio of power to torque of the drive unit (230) calculated by the drive unit (230) and the wearer's physical condition may be constructed, and the walking endurance ability of the wearer may be estimated through the PTR (power torque ratio) value in the case of the walking assistance mode.

(C) of FIG. 7 is a diagram showing the result of analyzing the Pearson correlation between the TUG (timed up and go test), which is a dynamic balance evaluation index, and the PTR (power torque ratio) value in the case of the walking assistance mode.

According to one embodiment of the present invention, the TUG (timed up and go test), which is a dynamic balance evaluation index, is an index that may evaluate the ability to perform activities of daily living, and may be evaluated by a method of measuring that a wearer sitting on a chair stands up, walks to a point located at a certain distance upon receiving a start signal, rotates 180 degrees at the corresponding point, and sits on the chair again. In the case where time is taken at or above a threshold time, it may be estimated that the walking ability, balance, ability to perform activities of daily living, and the like, of the walker are poor. As shown in (C) of FIG. 7, as the value of TUG (timed up and go test) on the y-axis increases, it means that the time walked by the walker while a wearer sitting on a chair stands up, walks to a point located at a certain distance upon receiving a start signal, rotates 180 degrees at the corresponding point, and sits on the chair again is longer, so it may mean that the dynamic balance ability or the ability to perform activities of daily living of the walker is more lacking.

In addition, since the case where the PTR (power torque ratio) value on the x-axis (in the case of the walking assistance mode) increases may mean that stronger power data is being measured from the wearer, it can be seen that there is a correlation inversely proportional to the value of TUG (timed up and go test) on the y-axis, and accordingly, a graph inversely proportional to the value of TUG (timed up and go test) on the y-axis may be shown. Through this, a database according to the correlation between the ratio of power to torque of the drive unit (230) calculated by the drive unit (230) and the wearer's physical condition may be constructed, and the dynamic balance ability of the wearer may be estimated through the PTR (power torque ratio) value in the case of the walking assistance mode.

(D) of FIG. 7 is a diagram showing the result of analyzing the Pearson correlation between the 10MWT (10-meter walking test), which is a balance and walking evaluation index, and the PTR (power torque ratio) value in the case of the walking resistance mode. According to the present invention, in the case of the walking resistance mode, since a negative power value is used, as shown in (D) of FIG. 7, the PTR (power torque ratio) value may have a negative value.

According to one embodiment of the present invention, the 10MWT (10-meter walking test), which is a balance and walking evaluation index, is an evaluation tool that may test short-distance walking speed and short-distance walking ability, and is an index that may be used in subject groups such as the elderly, stroke, spinal cord injury, Parkinson's disease, and other central nervous system injury patient groups. At this time, indoor walking and outdoor walking ability may be distinguished according to the measured walking speed, and as shown in (D) of FIG. 7, as the value of 10MWT(s) on the y-axis increases, it means that the measured walking time value is greater, so it may mean that the short-distance walking speed and ability are more lacking.

In addition, in the case where the PTR (power torque ratio) value on the x-axis (in the case of the walking resistance mode) increases, since in the walking resistance mode the device angular velocity direction and the torque direction are opposite and the power value is calculated as a negative number, it may mean that weaker power data is being measured from the wearer. Therefore, it can be seen that there is a correlation proportional to the value of 10MWT(s) on the y-axis, and accordingly, a graph proportional to the value of 10MWT(s) on the y-axis may be shown. Through this, a database according to the correlation between the ratio of power to torque of the drive unit (230) calculated by the drive unit (230) and the wearer's physical condition may be constructed, and the short-distance walking speed and ability of the wearer may be estimated through the PTR (power torque ratio) value in the case of the walking resistance mode.

(E) of FIG. 7 is a diagram showing the result of analyzing the Pearson correlation between the 6MWT (6-minute walking test), which is a walking endurance evaluation index, and the PTR (power torque ratio) value in the case of the walking resistance mode. According to the present invention, in the case of the walking resistance mode, since a negative power value is used, as shown in (E) of FIG. 7, the PTR (power torque ratio) value may have a negative value.

According to one embodiment of the present invention, the 6MWT (6-minute walking test), which is a walking endurance evaluation index, measures the total distance that a walker may walk during 6 minutes, and may evaluate the walking endurance by a method of checking vital signs (mild headache, dizziness, paleness, dyspnea, fatigue, pain, etc.) after sitting on a chair at the time point of 6 minutes. As shown in FIG. 7(E), as the value of 6MWT (6-minute walking test) on the y-axis increases, it means that the distance walked by the walker during 6 minutes is greater, so it may mean that the walking endurance of the walker is more excellent.

In addition, in the case where the PTR (power torque ratio) value on the x-axis (in the case of the walking resistance mode) increases, since in the walking resistance mode the device angular velocity direction and the torque direction are opposite and the power value is calculated as a negative number, it may mean that weaker power data is being measured from the wearer, so it can be seen that there is a correlation inversely proportional to the value of 6MWT (6-minute walking test) on the y-axis, and accordingly, a graph inversely proportional to the value of 6MWT (6-minute walking test) on the y-axis may be shown. Through this, a database according to the correlation between the ratio of power to torque of the drive unit (230) calculated by the drive unit (230) and the wearer's physical condition may be constructed, and the walking endurance ability of the wearer may be estimated through the PTR (power torque ratio) value in the case of the walking resistance mode.

(F) of FIG. 7 is a diagram showing the result of analyzing the Pearson correlation between the SPPB (short physical performance battery), which is a physical function test index, and the PTR (power torque ratio) value in the case of the walking assistance mode. According to the present invention, in the case of the walking resistance mode, since a negative power value is used, as shown in (F) of FIG. 7, the PTR (power torque ratio) value may have a negative value.

According to one embodiment of the present invention, the SPPB (short physical performance battery), which is a physical function test index, is an index that comprehensively combines a static balance test, a walking speed test, and a stand-up test to evaluate the overall physical function, and is an index that may evaluate physical function, the degree of frailty, the presence of sarcopenia, the risk of falls, and the like, and may predict the health risk. As shown in (F) of FIG. 7, as the value of SPPB (short physical performance battery) on the y-axis increases, it may mean that the physical function is more excellent. As a specific example, in the case of the static balance test, the difficulty increases in the order of the general posture, the semi-tandem posture, and the tandem posture, and since the time of maintaining balance without losing it in each posture may be measured, as the value of SPPB (short physical performance battery) on the y-axis increases, it may mean that the physical function is more excellent.

In addition, since the case where the PTR (power torque ratio) value on the x-axis (in the case of the walking assistance mode) increases may mean that stronger power data is being measured from the wearer, it can be seen that there is a correlation proportional to the value of SPPB (short physical performance battery) on the y-axis, and accordingly, a graph proportional to the value of SPPB (short physical performance battery) on the y-axis may be shown. Through this, a database according to the correlation between the ratio of power to torque of the drive unit (230) calculated by the drive unit (230) and the wearer's physical condition may be constructed, and the physical function ability of the wearer may be estimated through the PTR (power torque ratio) value in the case of the walking assistance mode.

FIG. 8 is a diagram showing the correlation between the age of the wearer and the device power torque ratio in the case of the walking assistance mode of the wearable robot according to the present invention, and the correlation between the age of the wearer and the device power torque ratio in the case of the walking resistance mode of the wearable robot according to the present invention.

(A) of FIG. 8 is a diagram showing the correlation between the age of the wearer and the device power torque ratio in the case of the walking assistance mode of the wearable robot according to the present invention. (B) of FIG. 8 is a diagram showing the correlation between the age of the wearer and the device power torque ratio in the case of the walking resistance mode of the wearable robot according to the present invention.

As shown, according to one embodiment of the present invention, based on the power torque ratio of the drive unit (230), it is possible to estimate at which point the walking, balance, lower-body muscle force ability, and the like, of the wearer are located from the average with respect to age. As a specific example, in the case of setting the average score to 75 points, through [Equation 5] and [Equation 6] below, the PTR value in the case of the walking assistance mode may be input to be scored from 50 points (lowest score) to 100 points (highest score). [Equation 5] $\begin{matrix}PTR _ assist _ score = 75 + \left(\frac{PTR _ assist}{PTR _ assist _ avg}-1\right) \times 100 \\ 0 \leq PTR _ assist _ score \leq 100\end{matrix}$ [Equation 6] $PTR _ assist _ avg = - 0.4554 \times avg + 6.598$

According to one embodiment of the present invention, if it is at or above 25% of the average for the age, it may be measured as a full score of 100 points, and if it is at or below -25% of the age average, it may be matched to 50 points. In addition to this, if the age, sex, and physical condition (height, weight, BMI) of the wearer are even used, a more sophisticated model may be constructed.

In addition, according to one embodiment of the present invention, the method of estimating the physical ability of the wearer during walking using a wearable robot including a drive unit (230) may include a step of proposing exercise information based on a deep learning model trained using, as training data, the ratio of power to torque of the drive unit (230) calculated by the drive unit (230) and the wearer's physical condition. As a specific example, the step of proposing exercise information based on a deep learning model trained using, as training data, the ratio of power to torque of the drive unit (230) calculated by the drive unit (230) and the wearer's physical condition may be performed in the server device (100), but may also be performed in the processor unit (not shown) of the drive unit (230).

In addition, according to one embodiment of the present invention, the method of estimating the physical ability of the wearer during walking using a wearable robot including a drive unit may include a step of predicting an exercise effect based on a deep learning model trained using, as training data, at least any one or more of the ratio of power to torque of the drive unit (230) calculated by the drive unit (230) and the wearer's physical condition, wearable robot usage information, and exercise effect. As a specific example, the step of predicting an exercise effect based on a deep learning model trained using, as training data, at least any one or more of the ratio of power to torque of the drive unit (230) calculated by the drive unit (230) and the wearer's physical condition, wearable robot usage information, and exercise effect may be performed in the server device (100), but may also be performed in the processor unit (not shown) of the drive unit (230). More specifically, the wearer's physical condition that may be used as training data may be a condition that considers at least any one or at least two or more of the wearer's age, sex, height and weight, and exercise ability, but is not limited thereto.

According to one embodiment of the present invention, the method of estimating the physical ability of the wearer during walking using a wearable robot including a drive unit may include a step of predicting an exercise effect based on a deep learning model trained using, as training data, at least any one or more of the ratio of power to torque of the drive unit (230) calculated by the drive unit (230) and the wearer's physical condition, wearable robot usage information, and exercise effect, and the wearable robot usage information may include the PTR (power torque ratio) value accumulated by the wearer's past wearing of the wearable robot (200) and walking. At this time, the exercise effect that may be used as training data may include the PTR (power torque ratio) value that has changed over time by the wearer's past wearing of the wearable robot (200) and walking. Since the exercise effect of the wearer of the wearable robot (200) may be estimated through the PTR (power torque ratio) value that has changed over time, a step of again predicting the exercise effect based on a deep learning model trained using this again as training data may be performed.

According to one embodiment of the present invention, the user terminal (300) may perform a step of receiving, from the server device (100), exercise information based on a database according to the correlation between the ratio of power to torque of the drive unit (230) calculated by the drive unit (230) and the wearer's physical condition, and a step of displaying the received exercise information. As a specific example, the exercise information based on a database according to the correlation between the ratio of power to torque of the drive unit (230) calculated by the drive unit (230) and the wearer's physical condition from the server device (100) may be provided as a user interface in the form of a report regarding the physical ability related to the walking of the wearer, but is not limited thereto.

### System Configuration

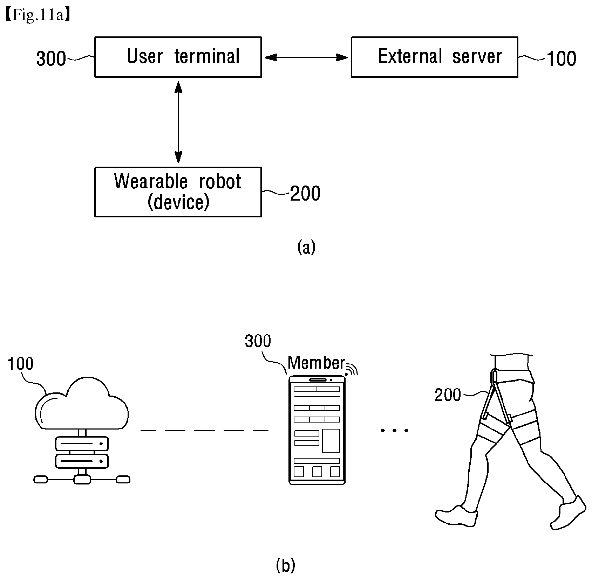

FIG. 11a to FIG. 11d are block diagrams of a system for generating a graphical user interface for providing information regarding the walking of the wearable robot wearer using a wearable robot capable of estimating the walking ability of the wearer during walking exercise according to one embodiment of the present invention.

As shown in FIG. 11a, the system for implementing a method of generating/providing a graphical user interface for providing information regarding the walking of the wearable robot wearer using the wearable robot (200) capable of estimating the walking ability of the wearer during walking exercise may include a user terminal (300), a wearable robot (200), and an external server (100), and (a) of FIG. 11a is a diagram illustrating each configuration that may be configured in the system as blocks, and FIG. 11a(b) is a diagram illustrating it as an image.

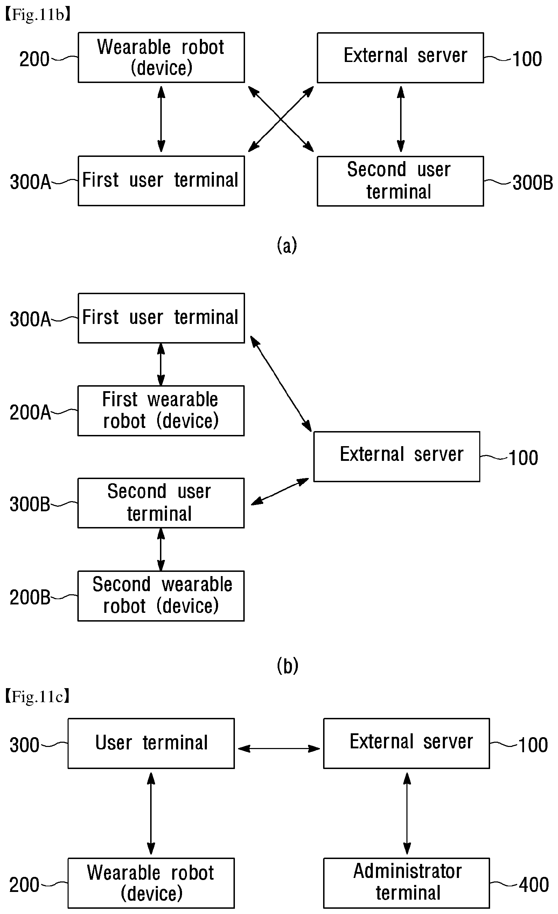

As shown in FIG. 11b, the system according to one embodiment of the present invention may include a plurality of user terminals (300A, B), a plurality of wearable robots (200A, B), and an external server (100). For example, a plurality of user terminals (300A, B) may be connected to one wearable robot (200), and a plurality of user terminals (300A, B) may be connected corresponding to each of the plurality of wearable robots (200A, B). Such a system configuration may be configured differently according to the business model. For example, (a) of FIG. 11b is a diagram illustrating, as one embodiment, a system configured for the purpose of a B2B (Business to Business) business model, in which a plurality of user terminals (300A, B) including a first user terminal (300A) and a second user terminal (300B), and the like, are connected to one wearable robot (200), and (b) of FIG. 11b is a diagram illustrating, as one embodiment, a system configured for the purpose of a B2C (Business to customer) business model, in which a plurality of user terminals (300A, B) are connected to a plurality of wearable robots (200A, B) including a first wearable robot (200A) and a second wearable robot (200B), and the like.

Referring to FIG. 11c, unlike FIG. 11a, an administrator terminal (400) may be further included in a system that may include a user terminal (300), a wearable robot (200), and an external server (100).

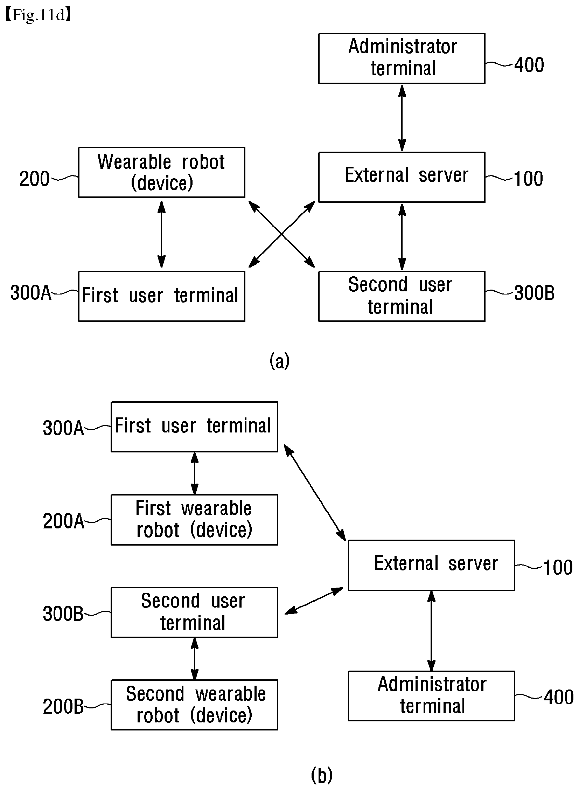

Referring to FIG. 11d, unlike FIG. 11d, an administrator terminal (400) may be further included in a system that may include a plurality of user terminals (300A, B), a plurality of wearable robots (200A, B), and an external server (100), and such a system configuration may be configured differently according to the business model. (a) of FIG. 11d is a diagram illustrating, as one embodiment, a system configured for the purpose of a B2B (Business to Business) business model in which an administrator terminal (400) may be included in the system configuration illustrated in (a) of FIG. 11b, and (b) of FIG. 11d is a diagram illustrating, as one embodiment, a system configured for the purpose of a B2C (Business to Customer) business model in which an administrator terminal (400) may be included in the system configuration illustrated in (b) of FIG. 11b.

As shown in FIG. 11a to FIG. 11d, the system according to one embodiment of the present invention may be a terminal or a server, and the system may be a web server that processes services.

Here, the system in which the method, device, and program for generating/providing a graphical user interface for providing information regarding the walking of the wearer using the wearable robot (200) capable of estimating the walking ability of the wearer during walking exercise shown in FIG. 11a to FIG. 11d are implemented is according to one embodiment, and its components are not limited to those shown in FIG. 11a to FIG. 11d, and may be added, changed, or deleted as necessary.

The external server (100) according to one embodiment of the present invention may be a web server, a database server, an application server, a cloud server, a DNS server, a backup server, and the like. However, the aforementioned external server (100) is merely an embodiment of a server capable of data communication through a network from the outside, and is not limited thereto.

The network according to the embodiments of the present invention may use various wired communication systems such as a public switched telephone network (PSTN), x Digital Subscriber Line (xDSL), Rate Adaptive DSL (RADSL), Multi Rate DSL (MDSL), Very High Speed DSL (VDSL), Universal Asymmetric DSL (UADSL), High Bit Rate DSL (HDSL), and a local area network (LAN).

In addition, the network presented here may use various wireless communication systems such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

The network according to the embodiments of the present invention may be configured regardless of its communication mode, such as wired and wireless, and may be configured of various communication networks such as a personal area network (PAN) and a wide area network (WAN). In addition, the network may be the well-known World Wide Web (WWW), and may use a wireless transmission technology used for short-range communication, such as Infrared Data Association (IrDA) or Bluetooth. The technologies described in this specification may be used not only in the networks mentioned above, but also in other networks.

In addition, in order to achieve the object of the invention according to one embodiment of the present invention, the user terminal (300), the wearable robot (200), the external server (100), and the administrator terminal (400) are interchangeable and do not necessarily have to be distinguished. That is, it should be understood that the operations necessary for generating/providing the graphical user interface may occur in at least one of the user terminal (300), the wearable robot (200), the external server (100), and the administrator terminal (400) in one device, and may be appropriately selected to achieve the object of the present invention.

### Terminal Configuration

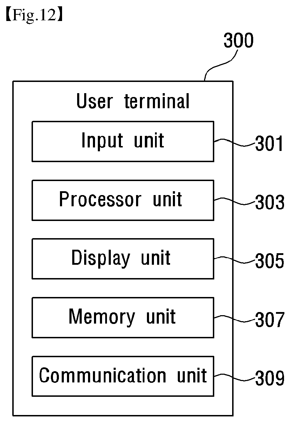

FIG. 12 is a block diagram of a user terminal of a system for generating a graphical user interface displaying information regarding the walking of the wearable robot wearer according to one embodiment of the present invention.

Referring to FIG. 12, the user terminal (300) according to one embodiment of the present invention may include an input unit (301), a processor unit (303), a display unit (305), a memory unit (307), and a communication unit (309). These components each perform independent functions, and at the same time interwork with each other to implement the overall function of the user terminal (300). However, these components do not necessarily have to be physically separated and exist individually. In addition, the administrator terminal (400) according to one embodiment of the present invention may also include the components of the user terminal (300).

The user terminal (300) according to one embodiment of the present invention may include a portable terminal such as a smartphone, a computer, a notebook equipped with a web browser (WEB Browser), a desktop, a laptop, a tablet PC, a slate PC, a smart watch, smart glasses, a head mount device, and the like. However, the aforementioned user terminal (300) is merely an embodiment for explaining the means that the user may use, and is not limited thereto. A person skilled in the art should understand that, for the object of the present invention, the user terminal used by the user may be selected as an appropriate means.

According to one embodiment of the present invention, the input unit (301) may include a hardware-type physical key and a software-type touch key. Specifically, the software-type touch key may be composed of a virtual key, a soft key, or a visual key displayed on a touch-screen-type display unit through software processing.

The virtual key or the visual key may be displayed on the touch screen while having various forms. For example, it may be composed of a graphic, text, an icon, a video, or a combination thereof.

According to one embodiment of the present invention, the processor unit (303) may be implemented as at least one of a memory unit (307) that stores data on an algorithm for controlling the operations of the components within the user terminal (300) or a program reproducing the algorithm, and at least one or more processors that perform overall operations using the data stored in the memory unit (307). At this time, the memory unit (307) and the processor unit (303) may each be implemented as a separate chip. Alternatively, the memory unit (307) and the processor unit (303) may be implemented as a single chip.

According to one embodiment of the present invention, the display unit (305) is a component that receives and displays information processed in the user terminal (300), and may display execution screen information of an application program (e.g., an application) driven in the user terminal (300), or user interface (UI) and graphical user interface (GUI) information according to such execution screen information.

According to one embodiment of the present invention, the memory unit (307) may store one or more programs, and the memory unit (307) capable of storing one or more programs may store data input and output through the communication unit (309), and may store a plurality of application programs (application programs or applications) driven in the user terminal (300), data for the operation of the user terminal (300), and commands.

In addition, the memory unit (307) capable of storing one or more programs includes a high-speed random access memory such as DRAM, SRAM, DDR RAM, or other random access solid state memory devices, and may include a non-volatile memory such as one or more magnetic disk storage devices, optical disk storage devices, flash memory devices, or other non-volatile solid state storage devices.

In addition, the memory unit (307) capable of storing one or more programs may store real-time walking information of the wearer regarding the walking of the wearer generated by receiving input from the user through a graphical user interface for providing information regarding the walking of the wearable robot wearer on the user terminal (300) or by receiving the walking information of the wearable robot wearer, statistical information according to accumulated walking information, a walking analysis report, a walking ability estimation report based on a deep learning model, and customized program information.

According to the present invention, the communication unit (309) may include one or more components that enable network communication with an external device, and may include, for example, at least one of a wired communication module, a wireless communication module, and a short-range communication module.

### Login Process

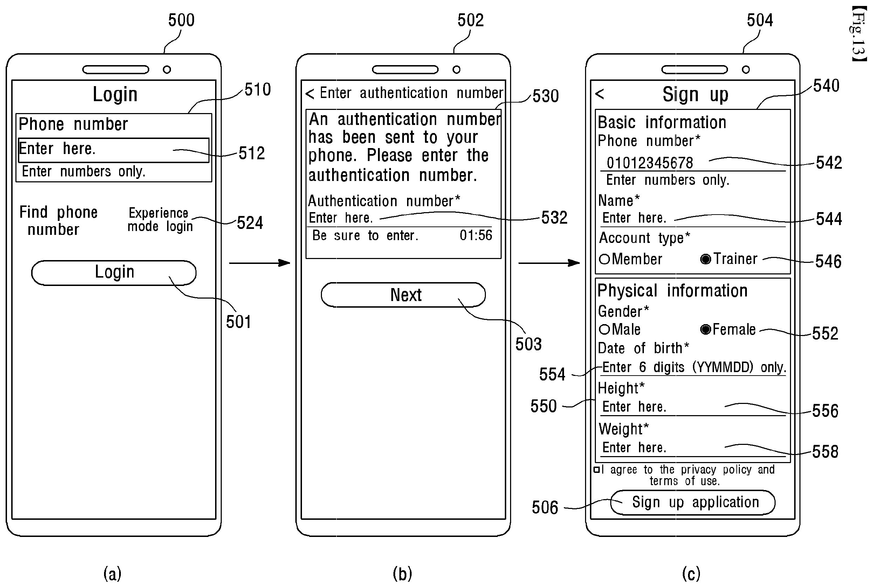

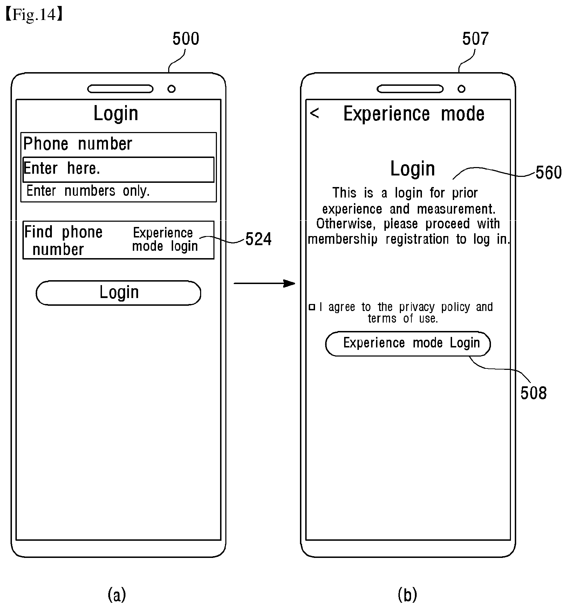

FIG. 13 and FIG. 14 are diagrams showing graphical user interfaces representing the user/administrator and experience mode login processes for accessing the system displaying information regarding the walking of the wearable robot wearer according to one embodiment of the present invention.

A login process may be preceded in order to transmit and receive data with the external server (100) or to connect with the wearable robot (200) in the user terminal (300) or the administrator terminal (400). The login process according to one embodiment of the present invention may be composed of a user/administrator login that inputs an authentication number and performs membership registration, and an experience mode login for experience without information input.

As shown in (a) to (c) of FIG. 13, the user/administrator login process may include a step of inputting a phone number in the phone number input area (512) of the phone number input display area (510) in the user/administrator login graphical user interface (500) and selecting login (501), a step of inputting an authentication number in the authentication number input area (532) according to the guidance phrase of the authentication number input guidance area (530) in the authentication number input graphical user interface (502) and selecting next (503), and a step of inputting relevant information in the basic information display area (540) and the physical information display area (550) in the membership registration graphical user interface (504) and selecting registration application (506). At this time, the basic information display area (540) may include a phone number input area (542), a name input area (544), and an account type selection area (546), and the physical information display area (550) may include a sex selection area (552), a date-of-birth input area (554), a height (cm) input area (556), and a weight (kg) input area (558), and the information input in each area may be added, changed, or deleted as necessary.

As shown in FIG. 14, the experience mode login process may include a step of selecting experience mode login (524) in the user/administrator login graphical user interface (500), and a step of selecting experience mode login (508) in the experience mode login graphical user interface (507) without the process of inputting an authentication number or inputting information for membership registration.

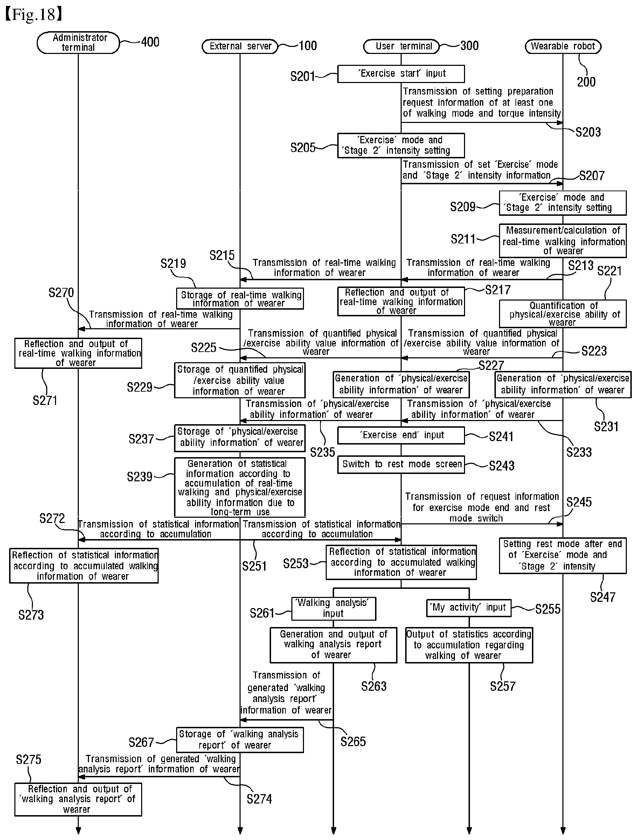

In addition, unlike the experience mode login, in the case where the user, that is, the wearable robot wearer, logs in through the process of inputting a phone number, then authenticating the authentication number, and inputting information for membership registration, the login information may be stored in the user terminal (100). In the case where the login information is stored in the user terminal (100), there is no need to perform the login process again unless logout (1802) is selected in the user account management graphical user interface (1800) shown in FIG. 18 after selecting the account management (118) tab of the category selection area (110) in the user terminal (100). This may be the same in the case of performing login in the administrator terminal (400).

However, the login process according to one embodiment of the present invention is merely exemplary, and various types of login processes may be adopted. For example, a method in which user information is stored in a memory within the user terminal and a login is performed through user authentication based on the corresponding information, or a method in which account information of a general social media platform is input and the user logs in through the account of the social media platform by the user approving a request for data access authority on the platform, may be adopted.

### Bluetooth Connection

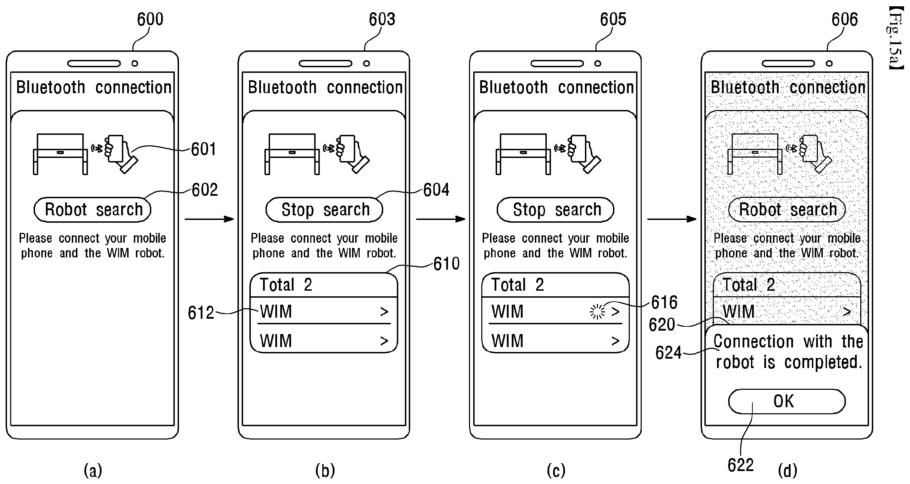

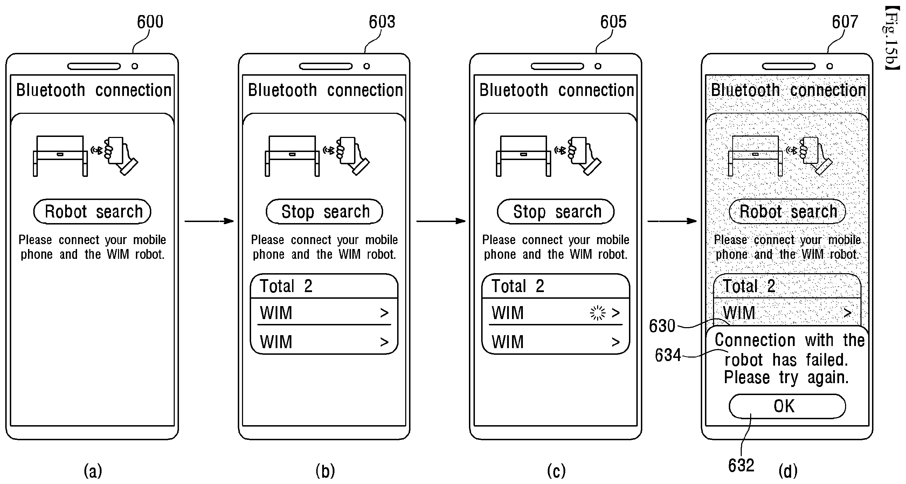

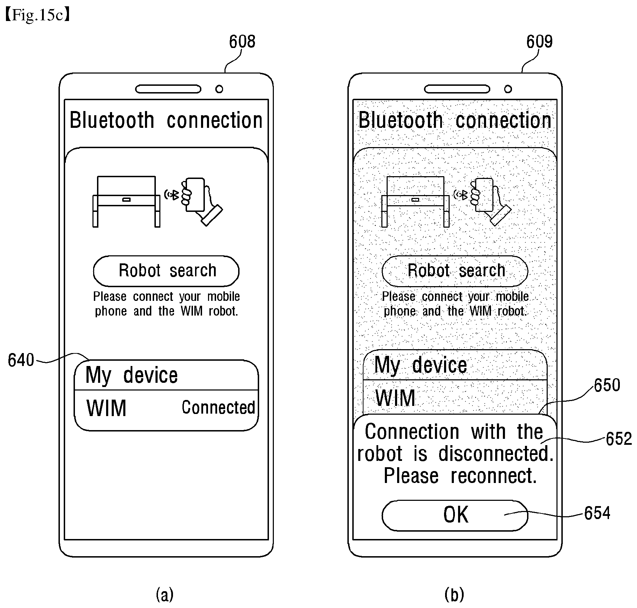

FIG. 15a is a diagram showing a graphical user interface representing the process in which a Bluetooth connection has been completed for the interworking of the user terminal and the wearable robot according to one embodiment of the present invention. FIG. 15b is a diagram showing a graphical user interface representing the process in which the Bluetooth connection of the user terminal and the wearable robot has failed according to one embodiment of the present invention. FIG. 15c is a diagram showing a graphical user interface representing the process in which the Bluetooth connection of the user terminal and the wearable robot has been disconnected according to one embodiment of the present invention.

According to one embodiment of the present invention, as described above, after going through the login process, a Bluetooth connection may proceed between the wearable robot (200) and the user terminal (300). At this time, the login process may include both the user/administrator login and the experience mode login.

As shown in FIG. 15a, the process in which the Bluetooth connection between the user terminal (300) and the wearable robot (200) according to one embodiment of the present invention is completed may include a step of selecting robot search (602) in the Bluetooth connection start graphical user interface (600) to search for a wearable robot (200) that may be connected with the user terminal (300), a step of selecting the searched wearable robot selection area (612) in the searched wearable robot display area (610) of the Bluetooth searching graphical user interface (603), and a step in which, through a loading process representing Bluetooth connection in progress (616) in the Bluetooth connection in progress graphical user interface (605), the Bluetooth connection complete display area (620) appears together with the Bluetooth connection complete guidance phrase (624) in the Bluetooth connection complete graphical user interface (606), and the connection is completed when confirm (622) within that area is selected. At this time, the searched wearable robot display area (610) may include the searched wearable robot selection area (612) and the searched wearable robot number information display area (614).

As shown in FIG. 15b, the process in which the Bluetooth connection between the user terminal (300) and the wearable robot (200) according to one embodiment of the present invention fails may include a step of selecting robot search (602) in the Bluetooth connection start graphical user interface (600) to search for a wearable robot (200) that may be connected with the user terminal (300), a step of selecting the searched wearable robot selection area (612) in the searched wearable robot display area (610) of the Bluetooth searching graphical user interface (603), and a step in which, although having gone through a loading process representing Bluetooth connection in progress (616) in the Bluetooth connection in progress graphical user interface (605), the Bluetooth connection failure display area (630) appears together with the Bluetooth connection failure guidance phrase (634) in the Bluetooth connection failure graphical user interface (607), and the connection fails when confirm (632) within that area is selected.

As shown in FIG. 15c, the process in which the Bluetooth connection between the user terminal (300) and the wearable robot (200) according to one embodiment of the present invention is disconnected may be such that, from the Bluetooth-connected graphical user interface (608) through which it can be confirmed that the connection with the wearable robot (200) has been made via the Bluetooth-connected wearable robot display area (640), it switches to the Bluetooth disconnected graphical user interface (609) including the Bluetooth disconnection display area (650) together with the Bluetooth disconnection guidance phrase (652), and the existing connection between the user terminal (300) and the wearable robot (200) may be disconnected.

FIG. 15a to FIG. 15c show a general embodiment of the Bluetooth connection process between the user terminal (300) and the wearable robot (200), and various Bluetooth connection situations may be assumed without being limited thereto. In addition, these examples are not limited only to the connection between the user terminal (300) and the wearable robot (200), and may likewise be applied between the administrator terminal (400) and the wearable robot (200).

In addition, although the connection between the user terminal (300) and the wearable robot (200) is assumed to be a 'Bluetooth connection' as one embodiment of the present invention, various technologies may be used for the wireless connection between the wearable robot (200) and a user terminal (300) such as a smartphone. Specifically, there may be BLE (Bluetooth Low Energy), UWB (Ultra-Wide-Band), Wi-Fi Direct, NFC (Near Field Communication), Zigbee, and the like.

According to the present invention, to describe in detail BLE (Bluetooth Low Energy) and UWB (Ultra-Wide-Band), BLE (Bluetooth Low Energy) is a wireless communication technology that maximizes energy efficiency, is mainly widely used in small, battery-driven devices, and this enables long-time communication even with low power, so that it may play a key role in various fields such as wearable devices, healthcare monitors, and smart home devices. UWB (Ultra-Wide-Band) is a technology that shows excellent performance in high-precision position tracking and data transmission, and uses ultra-wideband frequencies to enable precise distance measurement and position tracking along with a high data transmission rate. In addition, this technology is used in smartphones, automobiles, industrial automation, and the like, so that the interaction within a physical space may be innovatively improved.

Therefore, the connection between the user terminal (300) and the wearable robot (200) is such that the Bluetooth connection is merely exemplary and is not limited thereto, and various wireless connection technologies may be applied between the two devices.

In addition, there may be cases where a wireless connection is not made, such as a Bluetooth connection not being made, between the user terminal (300) and the wearable robot (200) according to one embodiment of the present invention. In such a case, this may be resolved through a wired connection between the user terminal (300) and the wearable robot (200).

### Example of the Main Screen (First Screen) After the Connection of the Wearable Robot and the User Terminal

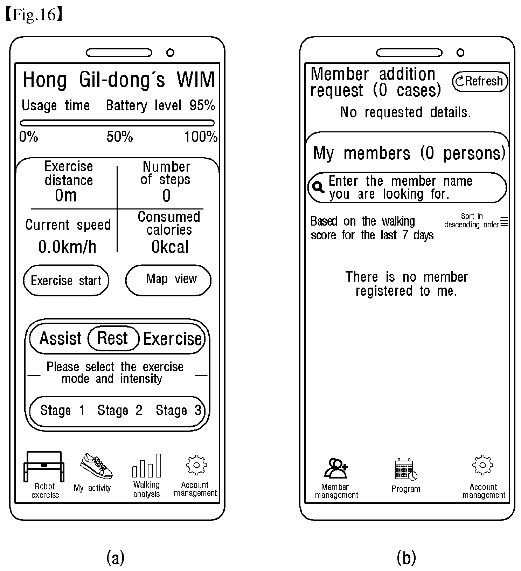

FIG. 16 is a diagram showing a graphical user interface representing the main screen on the user/administrator terminal after the login and Bluetooth connection are completed according to one embodiment of the present invention.

When the login and Bluetooth connection are sequentially completed according to one embodiment of the present invention, a graphical user interface displaying the main screen may be displayed on the terminal. (a) of FIG. 16 is a diagram showing the graphical user interface of the user terminal (300) after the login and Bluetooth connection are completed according to one embodiment of the present invention, and (b) of FIG. 16 is a diagram showing the graphical user interface of the administrator terminal (400), and the detailed contents of each graphical user interface will be described later together with the drawings.

### Real-time Walking Information Graphical User Interface of the Wearable Robot Wearer

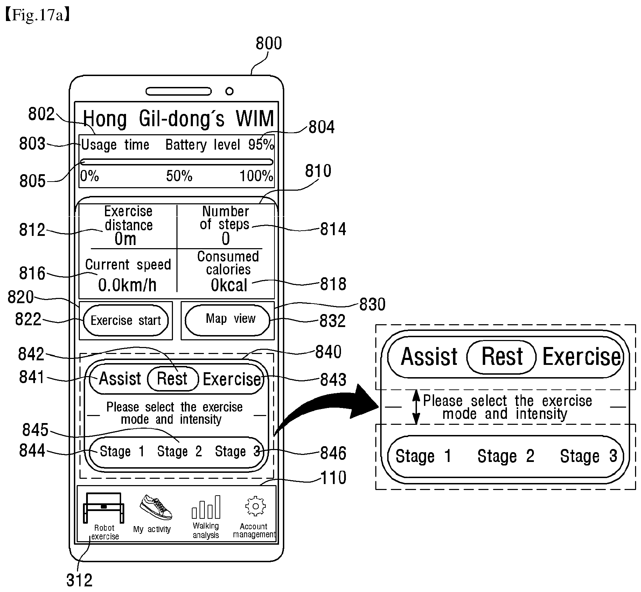

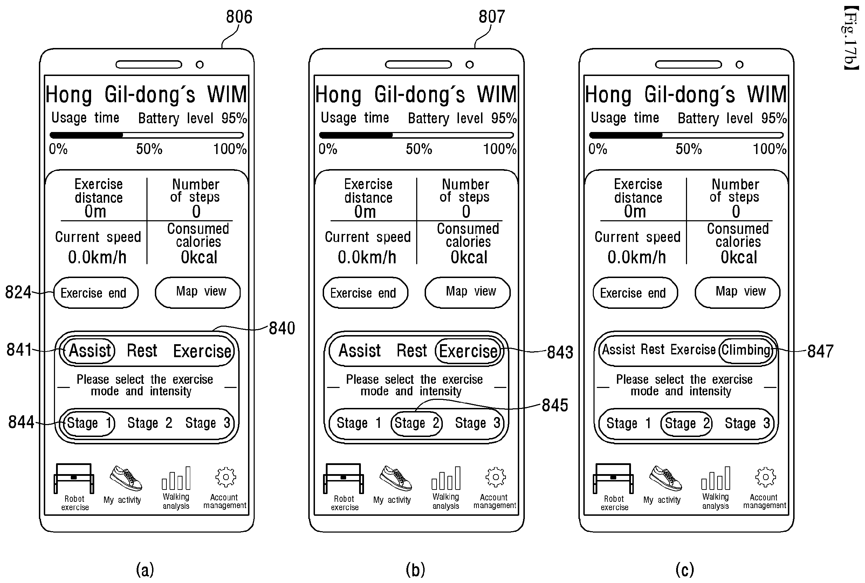

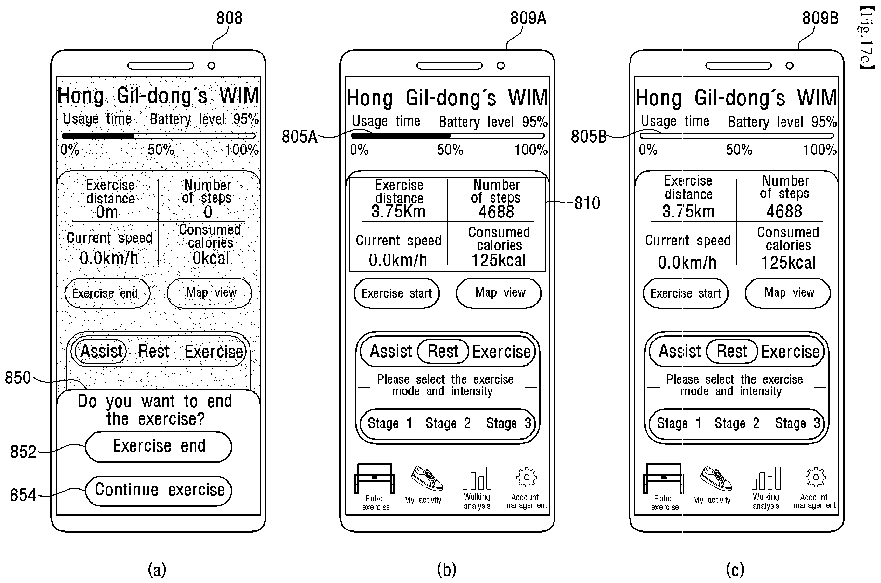

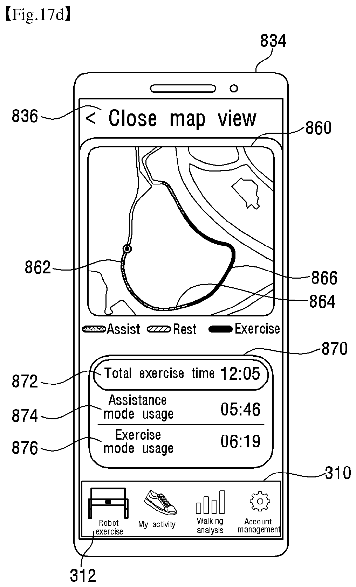

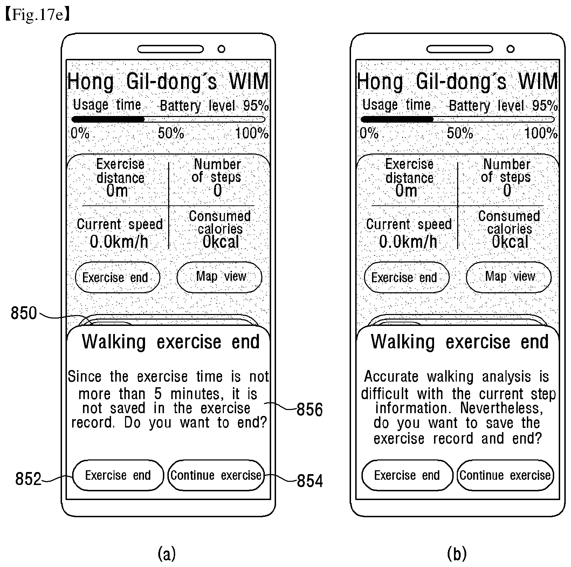

FIG. 17a is a diagram showing the graphical user interface of the user terminal representing the rest mode before robot exercise according to one embodiment of the present invention. FIG. 17b is a diagram showing the graphical user interface of the user terminal in which at least one of the assistance/exercise mode and the torque intensity is set at the start of robot exercise according to one embodiment of the present invention. FIG. 17c is a diagram showing the graphical user interface of the user terminal displaying information regarding the walking of the wearer measured during the robot exercise process at the end of the robot exercise according to one embodiment of the present invention. FIG. 17d is a diagram showing the graphical user interface displaying the walking map of the user terminal, which displays the current position information and distance information of the wearer and the walking time information of the wearer during the robot exercise process according to one embodiment of the present invention. FIG. 17e is a diagram showing the graphical user interface displaying a notification that walking analysis information has not been generated when the robot exercise is to be ended according to one embodiment of the present invention.

As shown in FIG. 17a, when the robot exercise (312) tab is selected in the category selection area (310) of the user terminal (300) according to one embodiment of the present invention, the robot exercise graphical user interface (800) displaying the real-time walking information of the wearable robot wearer appears. The robot exercise graphical user interface (800) may include at least one of a status information display area (802) of the wearable robot, a real-time walking information display area (810) of the wearer, an exercise start/end selection area (820), a walking map selection area (830), and a robot exercise setting area (840).

To describe in detail the areas displayed in the robot exercise graphical user interface (800) according to one embodiment of the present invention, the status information display area (802) of the wearable robot may include at least one of a usage time information display area (803), a battery remaining information display area (804), and a torque gauge bar information display area (805), the real-time walking information display area (810) of the wearer may include at least one of a distance information display area (812), a number of steps information display area (814), a speed information display area (816), and a consumed calorie information display area (818), the exercise start/end selection area (820) is an area in which exercise start (822) or exercise end (824) may be selected, the walking map selection area (830) is an area in which, when map view (832) is selected, it may be switched to the graphical user interface (834) displaying the walking map, and the robot exercise setting area (840) may be an area in which at least one of the walking mode and the torque intensity corresponding to the walking purpose of the wearer is set.

In the status information display area (802) of the wearable robot according to one embodiment of the present invention, the usage time information display area (803) may display information regarding the usage time of the wearable robot based on the operation information of the wearable robot worn by the user. The battery remaining information display area (804) may display information regarding the battery remaining of the wearable robot based on the operation information of the wearable robot worn by the user. The torque gauge bar information display area (805) may display, as a bar gauge, information regarding the torque value that the wearable robot applies to the wearable robot wearer based on the operation information of the wearable robot worn by the user.

In the torque gauge bar information display area (805) according to one embodiment of the present invention, the torque gauge bar is an index for confirming the torque value actually applied to the wearer during walking assistance or exercise, and may be displayed in real time in the form of a gauge in proportion to the torque value determined in the drive unit (230) of the wearable robot (200) based on the hip angle of the wearer.

According to the present invention, the torque gauge bar may be calculated through [Equation 7] below. [Equation 7] $Torque Gage \left(%\right) = \left(\frac{\left|{Torque}_{max}-{Torque}_{min}\right|}{12 Nm}\right) \times 100$

The above [Equation 7] is an equation that calculates the torque ratio based on the difference between the maximum torque value (max_torque) and the minimum torque value (min_torque) that occurred during the most recent 2 steps (1 stride) in the drive unit (230) of the wearable robot (200). At this time, [Equation 7] was set so that the difference between the maximum torque value and the minimum torque value becomes a maximum of 12 Nm (|*Torqueₘₐₓ-Torqueₘᵢₙ*|<12*Nm*), and this is because the range of the ideal torque value that the wearable robot (200) applies to the wearer was set to a range from - 6 Nm to +6 Nm. This range may not necessarily accurately reflect the range of torque values that occur during the general walking exercise process of all people. However, since too low a torque may not provide sufficient support or resistance in the walking process, and too high a torque may interfere with the walking pattern of the user or increase the risk of injury, the range of -6 Nm to +6 Nm may be a range that may satisfy both the purpose of using the wearable robot (200) and the safety of the user. In addition, the reason that the absolute value of the difference between the maximum torque value and the minimum torque value is used in [Equation 7] is that, since the process of a person walking includes both the motion of the leg moving forward and the motion of being pulled backward, the torque occurs in both directions (positive torque and negative torque) so that the torque value may have both positive and negative values.

Specifically, an example of the torque gauge value is given as follows.

In the case where Max_torque = +6 Nm, Min_torque = -6 Nm,
it is $Torque Gage \left(%\right) = \left(\frac{\left|6Nm-\left(-6Nm\right)\right|}{12 Nm}\right) \times 100 = \frac{12 Nm}{12 Nm} \times 100 = 100 %$, and
in the case where Max_torque = +3 Nm, Min_torque = -3 Nm,
it becomes $Torque Gage \left(%\right) = \left(\frac{\left|3Nm-\left(-3Nm\right)\right|}{12 Nm}\right) \times 100 = \frac{6 Nm}{12 Nm} \times 100 = 50 %$, and
when the stride of the wearable robot wearer is small or stopped, the torque value becomes 0 Nm, so that the torque gauge becomes 0%. In (b) and (c) of FIG. 17c, graphical user interfaces respectively displaying the cases where the torque gauge bar is 50% and 0% are shown. For example, the graphical user interface in which the torque gauge bar is 50% may be a graphical user interface in the case where the torque gauge bar is 50% during the robot exercise or in the case immediately before the end of the robot exercise, and the graphical user interface in which the torque gauge bar is 0% may be a graphical user interface in the case where the stride of the wearer is small or in a stopped state, or in the case where 2 to 3 seconds have passed after the end of the robot exercise.

In addition, since the torque gauge may be determined depending on the torque value determined in proportion to the hip stride (Hip Range of Motion) of the wearable robot wearer in the current walking process, in other words, the range of motion of the hip joint, regardless of the setting of the torque intensity including at least one of a plurality of stages that provide differential torque in the robot exercise setting area (840), the wearable robot wearer may anticipate the torque value applied from the wearable robot (200) to the wearer through the torque gauge displayed in the robot exercise graphical user interface (800), and based on this, may adjust the torque intensity to perform walking assistance or exercise suitable for the current state of the wearer.

For example, such a system may be likened to the accelerator pedal and the speedometer of an automobile. Pressing the accelerator pedal is similar to adjusting the strength (torque intensity) of the walking assistance or exercise device, and the actual speed (torque in the case of the wearable robot) may be confirmed through the instrument panel (torque measurement and ratio calculation).

Therefore, the wearable robot wearer may control the wearable robot in accordance with the status of the wearable robot in the process of performing robot exercise such as walking assistance or exercise, based on the information that can be known in the status information display area (802) of the wearable robot according to one embodiment of the present invention.

In the real-time walking information display area (810) of the wearer according to one embodiment of the present invention, the distance information display area (812) may display information regarding the exercise distance of the user based on the operation information of the wearable robot worn by the user. The number of steps information display area (814) may display information regarding the number of steps of the user based on the operation information of the wearable robot worn by the user. The speed information display area (816) may display information regarding the current walking speed of the user based on the operation information of the wearable robot worn by the user. The consumed calorie information display area (818) may display information regarding the consumed calories of the user based on the operation information of the wearable robot worn by the user. However, the information included in the real-time walking information display area (810) of the wearer is exemplary, and may be added, changed, or deleted as necessary. For example, information such as step length, step pattern, continuous walking time, walking rhythm, slope, and number of steps and number of floors when ascending or descending stairs, based on the operation information of the wearable robot worn by the user, may be additionally included.

In the exercise start/end selection area (820) according to one embodiment of the present invention, exercise start (822) and exercise end (824) may be selected. Specifically, when the user selects exercise start (822), the wearable robot in the state of the rest mode (842) may start the robot exercise according to at least one of the set walking mode and torque intensity, and when exercise end (824) is selected, as shown in (a) of FIG. 17c, the exercise end display area (850) is displayed in the robot exercise end graphical user interface (808), and when exercise end (852) within that area is selected, the robot exercise of the wearable robot may be ended. However, when continue exercise (854) within the exercise end display area (850) is selected, the robot exercise is not ended and may continue to proceed.

As shown in FIG. 17e, in the case where the necessary condition for generating the walking analysis information at the end of the robot exercise according to the present invention is not satisfied, a graphical user interface displaying a notification (856) that the walking analysis information has not been generated in the exercise end display area (850) may appear. Specifically, in the case where the wearable robot wearer selects exercise end (824) in the exercise start/end selection area (820), if there is insufficient meaningful information to perform the walking analysis of the wearer, the exercise records measured and calculated during the robot exercise process may not be stored and the walking analysis may not be performed. At this time, the meaningful information corresponds to the necessary condition (or walking analysis condition) for generating the walking analysis information, and the information may include the walking information of the wearer measured during the robot exercise process, such as valid step information and robot exercise time.

For example, in the case where the 'robot exercise time' is not at or above 5 minutes, as shown in (a) of FIG. 17e, the notification (856) that the walking analysis information has not been generated, stating "The exercise time is not at or above 5 minutes, so the exercise record is not stored. Would you like to end?" may be displayed on the graphical interface. Since storing the exercise record must be preceded before performing the walking analysis, the phrase that the exercise record has not been stored may imply that the walking analysis information has not been generated.

As another example, in the case where the 'valid step information' collected from the wearable robot wearer is insufficient, as shown in (b) of FIG. 17e, the notification (856) that the walking analysis information has not been generated, stating "Accurate walking analysis is difficult with the current step information. Would you nevertheless like to store the exercise record and then end?" may be displayed on the graphical interface. The step information required for the walking analysis may be exemplified as about 500 steps. At this time, the step information may be step information recognized using various conditions for filtering out low-speed and slow hip swing, steps during resting motions, and the like, in order to reflect, in the walking analysis data, the number of steps measured in straight-line walking on flat ground. However, the example that the step information required for the walking analysis is 500 steps may be a normal walking analysis condition required on the basis of a healthy walker, and in the case of low-speed walkers (the walking-disadvantaged) such as the elderly and pregnant women, a more relaxed walking analysis condition may be applied. For example, if the normal walking analysis condition requires 500 steps, the relaxed walking analysis condition may require 50 steps.

In the walking map selection area (830) according to one embodiment of the present invention, when map view (832) is selected, it may be switched to the graphical user interface (832) displaying the walking map. As shown in FIG. 17d, the graphical user interface (832) displaying the walking map may include at least one of a walking map display area (860) displaying the current position information and distance information of the wearer and a walking time information display area (870) of the wearer. Specifically, the walking map display area (860) displaying the current position information and distance information of the wearer may include a walking map generated by displaying on a map at least one of an assistance mode usage display area (862), a rest mode usage display area (864), and an exercise mode usage display area (866) based on the current position information of the wearable wearer, and each walking mode may be distinguished by being displayed in a different color on the walking map. The walking time information display area (870) of the wearer may include at least one of a total exercise time display area (872), an assistance mode usage time display area (874), and an exercise mode usage time display area (876).

The method of generating the graphical user interface (834) displaying the walking map according to one embodiment of the present invention may include a step of receiving input information based on the walking map selection area (830) from the user terminal (300), a step of, after receiving the input information, collecting the current position information of the wearer, a step of generating a walking map based on the collected current position information of the wearer and a map development tool, and a step of generating the graphical user interface (834) displaying the walking map based on the generated walking map and the received real-time walking information of the wearer. To describe the generation process in detail, when the wearable robot wearer selects map view (832) of the walking map selection area (830) on the interface of the user terminal (300) and the input information is transmitted, after receiving the input information, a walking map reflecting the position information of the wearer and the real-time walking information of the wearer may be generated using a publicly available API (Application Programming Interface) or SDK (Software Development Kit) that provides a map service, and the generated walking map may be located in the walking map display area displaying the current position information and distance information of the wearer of the graphical user interface (832) displaying the walking map as shown in FIG. 17d.

In the robot exercise setting area (840) according to one embodiment of the present invention, at least one of the walking mode and the torque intensity may be set. When exercise start (822) is selected in the exercise start/end selection area (820), at least one of the walking mode and the torque intensity may be set in the rest mode (842), the walking mode may include at least one torque transmission mode, and each torque transmission mode may be a mode that transmits torque corresponding to the walking purpose. For example, in the case where the wearable robot (200) is used for the purpose of assisting the walking of the wearer, the wearable robot may provide torque that plays the role of an assistance force to the wearer, and in the case where it is used for the purpose of strengthening the lower-body muscle force of the wearer, the wearable robot may provide torque that plays the role of a resistance force to the wearer.

As a specific example, the plurality of walking modes according to one embodiment of the present invention may be an assistance mode (841), a rest mode (842), an exercise mode (843), and a climbing mode (847). The assistance mode (841) may be a mode in which the wearable robot (200) applies an assistance force to the wearer in order to assist the walking of the wearer, and the rest mode (842) may be a mode in which the wearable robot (200) does not apply any torque to the wearer. The exercise mode (843) may be a mode in which the wearable robot (200) applies a resistance force to the wearer in order to strengthen the lower-body muscle force of the wearer. The climbing mode (847) may be a mode for assisting the walking of the wearable robot wearer who moves on an inclined uphill or downhill that is not flat ground. At this time, the climbing mode (847) may, in the process of climbing uphill during climbing, provide an assistance force in order for the wearable robot (200) to assist the walking of the wearer or provide a resistance force in order to strengthen the lower-body muscle force, to the wearer. Likewise, the climbing mode (847) may, even in the process of going down a downhill during climbing, provide an assistance force or a resistance force according to the walking purpose of the wearer. In addition, the climbing mode (847) may be divided into an uphill mode or a downhill mode, and may be replaced by an uphill mode or a downhill mode.

In addition, the plurality of walking modes according to one embodiment of the present invention may be distinguished by different colors. By distinguishing the plurality of walking modes by different colors, it is possible to prevent in advance the wrong control of the walking mode, and in the case where a mode change has been made, the fact that the mode has been changed and the set mode information may be clearly recognized. For example, the rest mode, the assistance mode, the exercise mode, and the climbing mode may each be displayed in green, blue, magenta, and orange. Alternatively, in the case where the climbing mode is divided into an uphill mode and a downhill mode, the uphill mode may be displayed in sky blue, and the downhill mode may be displayed in orange. As such, when the plurality of modes are distinguished by different colors and displayed on the graphical user interface, the LED display lights or the like built into the wearable robot (200) may also be expressed in the same color.

The torque intensity according to one embodiment of the present invention may include at least one of a plurality of stages that provide differential torque. As a specific example, the torque intensity may be represented by stage 1 (844), stage 2 (845), and stage 3 (846), and as it goes from stage 1 to stage 3, the strength of the assistance force in the assistance mode (841) and the resistance force in the exercise mode (843) may increase. However, it is not limited thereto, and the plurality of stages included in the torque intensity may be further subdivided as necessary.

FIG. 17b is a diagram showing the graphical user interface screen in which the walking mode and the torque intensity are set according to one embodiment of the present invention. (a) of FIG. 17b is a diagram illustrating the assistance mode (841) and stage 1 (844), (b) of FIG. 17b is a diagram illustrating the exercise mode (843) and stage 2 (845), and (c) of FIG. 17b is a diagram illustrating the climbing mode (847) and stage 2 (845). The walking mode and torque intensity in the drawings are exemplary as described above, and are not limited thereto.

The robot exercise setting area (840) according to one embodiment of the present invention may have its entire area exposed and its position fixed in the robot exercise graphical user interface (800) displaying the real-time walking information of the wearable robot wearer. This prevents the robot exercise setting area (840) from being moved by scrolling or the like within one graphical user interface or from being partially not displayed, so that at least one of the desired walking mode and torque intensity may be set for the wearable robot at any time.

In the robot exercise setting area (840) according to one embodiment of the present invention, the area for setting the walking mode and the torque intensity may be separated. For example, referring to the enlarged portion of the robot exercise setting area (840) in FIG. 17a, it may be a method of disposing the area for setting the walking mode and the area for setting the torque intensity at a certain interval between them. In the case where the areas for setting the walking mode and the torque intensity are not separated, a situation dangerous to the safety of the wearer may occur. For example, in the case where the wearable robot wearer tried to change the torque intensity but changed the walking mode from the assistance mode (841) to the exercise mode (843), the torque generated in the wearable robot acts on the wearer as a resistance force instead of an assistance force in an unexpected situation for the wearer, so that an unforeseen dangerous accident due to cognitive mismatch may occur. However, it is not limited to the areas for setting the walking mode and the torque intensity being separated in the robot exercise setting area (840), and there may also be a method such as separately adopting a lock function so as not to change the mode by mistake after setting the walking mode.

In addition, as shown in FIG. 17c, in the case where the wearable robot wearer selects exercise end (854) in the robot exercise end graphical user interface (808) after completing walking or exercise, the record of the walking process after the end of walking or exercise may be confirmed. (b) and (c) of FIG. 17c are diagrams showing examples of the information recorded during the robot exercise process after the end of exercise, and respectively show the graphical user interface immediately before the end of the robot exercise (809A) and the graphical user interface 2 to 3 seconds after the end of the robot exercise (809B). Looking at the torque gauge bar information display area (805) in the status information display area (802) of the wearable robot, in the case of the graphical user interface immediately before the end of the robot exercise (809A), since the wearable robot (200) is applying torque to the wearer immediately before the end of the exercise, it can be confirmed that the torque gauge is 50% (805A), and in the case of the graphical user interface 2 to 3 seconds after the end of the robot exercise (809B), since there was no movement of the wearer for 2 to 3 seconds after the end of the exercise, the hip angle of the wearer becomes 0, so that the torque value also becomes 0, and it can be confirmed that the torque gauge bar information display area (805) represents a torque gauge of 0% (805B).

Therefore, in the robot exercise graphical user interface of the robot exercise (312) tab of the user terminal (300), the wearable robot wearer may confirm information regarding walking in real time in the real-time walking information display area (810) of the wearer during the robot exercise, and may confirm the information regarding walking recorded during the robot exercise process at the end of the robot exercise.

The method of generating the graphical user interface displaying the real-time walking information of the wearable robot wearer according to one embodiment of the present invention may include a step of receiving the real-time walking information of the wearer from the wearable robot (200) and a step of generating the graphical user interface based on the received real-time walking information of the wearer. Through this, the graphical user interface displaying the real-time walking information of the wearable robot wearer according to one embodiment of the present invention may be generated, and the detailed process will be described below.

### Process of Generating Walking Analysis Information of the Wearable Robot Wearer and Generating a Graphical User Interface Based on the Walking Analysis Information

FIG. 18 is a flowchart for explaining a method of generating a graphical user interface of the user terminal representing information regarding the walking of the wearable robot wearer transmitted and received among the wearable robot, the user terminal, and the external server according to one embodiment of the present invention.

As shown in FIG. 18, through the flowchart, the roles performed by the user terminal (300), the wearable robot (200), the external server (100), and the administrator terminal (400) and the data transmission/reception process in the series of processes from inputting the exercise start in the user terminal (300), going through the robot exercise process of the wearable robot wearer, to inputting the exercise end, may be represented according to the flow of time.

The information regarding the walking of the wearable robot wearer according to the present invention may be classified into walking information of the wearer and walking analysis information of the wearer. The walking information of the wearer may include at least one of the real-time walking information of the wearer obtained during the robot exercise process and the quantified physical/exercise ability values of the wearer, and the walking analysis information of the wearer may include at least one of the physical/exercise ability information of the wearer and the statistical information according to the accumulated walking information of the wearer stored in the external server (100) or the user terminal (300). In addition, the walking information of the wearer may include the basic information of the wearer input to the user terminal.

The graphical user interface displaying the information regarding walking based on the robot exercise of the wearable robot wearer according to one embodiment of the present invention may include a graphical user interface displaying the real-time walking information of the wearer, a graphical user interface displaying the statistical information according to the accumulated walking information of the wearer, and a graphical user interface displaying the walking analysis report of the wearer. The graphical user interface displaying the real-time walking information of the wearer may be generated based on the real-time walking information of the wearer from the wearable robot, and the graphical user interface displaying the statistical information according to the accumulated walking information of the wearer or the walking analysis report of the wearer may be generated based on the walking analysis information of the wearer generated based on the walking information of the wearer.

To describe in detail the step in which such a graphical user interface is generated as a process from the start of exercise to the end thereof, a method of generating a graphical user interface displaying real-time walking information of a wearable robot wearer according to an embodiment of the present invention may include a 'exercise start' input step (S201) in which the wearer inputs exercise start (822) in the exercise start/end selection area (820) on the robot exercise graphical user interface (800) shown in FIG. 17a of the user terminal (300), a step (S203) in which setting preparation request information of at least one of a walking mode and a torque intensity is transmitted from the user terminal (300) to the wearable robot (200), a step (S205) in which the wearer sets the 'exercise' mode and the '2nd-level' intensity in the robot exercise setting area (840) of the robot exercise graphical user interface (800) displayed on the user terminal (300), a step (S207) in which the set 'exercise' mode and '2nd-level' intensity information is transmitted from the user terminal (300) to the wearable robot, a step (S209) in which the 'exercise' mode and the '2nd-level' intensity are set in the wearable robot (200), a step (S211) in which the drive unit (230) of the wearable robot (200) measures or calculates the real-time walking information of the wearer, a step (S213) in which the real-time walking information of the wearer is transmitted from the wearable robot (200) to the user terminal (300), and a step (S217) in which the user terminal (300) reflects and outputs the real-time walking information of the wearer. In addition, the external server (100) may include a step (S215) in which the real-time walking information of the wearer is transmitted from the user terminal (300) to the external server (100), and a step (S219) in which the real-time walking information of the wearer is stored in the external server (100).

A method of generating a graphical user interface displaying statistical information according to accumulated walking information or a walking analysis report of a wearable robot wearer according to an embodiment of the present invention may include a step of receiving the walking information of the wearer, a step of generating walking analysis information of the wearer based on the walking information of the wearer, and a step of generating a graphical user interface displaying statistical information according to the accumulated walking information or a walking analysis report based on the generated walking analysis information of the wearer.

A step of generating walking analysis information of the wearer according to an embodiment of the present invention may include a step of receiving the walking information of the wearer from the wearable robot, a step of generating physical/exercise ability information of the wearer based on the received walking information of the wearer, and a step of generating walking analysis information of the wearer based on at least one of the generated physical/exercise ability information of the wearer and statistical information according to the accumulated walking information of the wearer stored in the external server.

A step of receiving the real-time walking information of the wearer from the wearable robot (200) according to an embodiment of the present invention may include a step of transmitting at least one of walking mode and torque intensity information set in the user terminal (300) to the wearable robot (200), and a step of, after transmitting, receiving the real-time walking information of the wearer generated in the wearable robot (200) based on at least one of the set walking mode and torque intensity information.

Transmitting at least one of the set walking mode and torque intensity information according to an embodiment of the present invention may include a step of displaying a graphical user interface displaying the robot exercise setting area (840) on the user terminal (300), and a step of receiving an input of a setting of at least one of a walking mode and a torque intensity from the user terminal (300) and transmitting it to the wearable robot.

Specifically, a method of generating physical/exercise ability information of the wearer according to an embodiment of the present invention may include a step (S221) of quantifying the physical/exercise ability of the wearer in the wearable robot (200), a step (S223) of transmitting the quantified physical/exercise ability numerical value information of the wearer from the wearable robot (200) to the user terminal (300), a step (S225, S229) of transmitting the quantified physical/exercise ability numerical value information of the wearer from the user terminal (300) to the external server (100) and storing it, a step (S227, S231) of generating 'exercise/physical ability information' of the wearer in the wearable robot (200) and the user terminal (300), a step (S233) of transmitting the physical/exercise ability information of the wearer from the wearable robot (200) to the user terminal (300), and a step (S235) of transmitting the physical/exercise ability information of the wearer from the user terminal (300) to the external server (100).

After the physical/exercise ability information of the wearer according to an embodiment of the present invention is generated, the external server (100) may include a step (S237) of storing the physical/exercise ability information of the wearer, a step (S239) of generating statistical information according to the accumulation of real-time walking information and physical/exercise ability information according to long-term use, a step of transmitting (S251) the generated statistical information according to the accumulation to the user terminal (300), and a step (S253) of reflecting statistical information according to the accumulated walking information of the wearer within the user terminal (300). The user terminal (300) may include a 'exercise end' input step (S241) in which the exercise end (854) is input by the wearable robot wearer in the exercise end display area (820) of the robot exercise end graphical user interface (808) shown in (a) of FIG. 17c, a step (S243) in which the screen is switched to a rest mode based on the exercise end input information, and a step (S247) of transmitting (S245) the exercise mode end and rest mode switch request information to the wearable robot (200) and setting the rest mode after ending the exercise mode and the 2nd-level intensity in the wearable robot (200).

On the basis of such a process, a graphical user interface displaying statistical information according to accumulated walking information of a wearable robot wearer according to an embodiment of the present invention may be generated through a step (S255) of inputting My Activity (314) in the graphical user interface of the user terminal (300) and a step (S257) of outputting statistical information according to the accumulated walking information regarding the walking of the wearer.

A graphical user interface displaying a walking analysis report of the wearer according to an embodiment of the present invention may be generated through a step (S261) of inputting Walking Analysis (316) in the graphical user interface of the user terminal (300) and a step (S263) of generating and outputting the walking analysis report of the wearer. At this time, the generated walking analysis report information of the wearer is transmitted (S265) from the user terminal (300) to the external server (100), and the walking analysis report of the wearer may be stored (S267) in the external server (100).

On the basis of the content described in detail above, statistical information according to accumulated walking of the wearer or a walking analysis report of the wearer, which is generated based on the walking analysis information of a wearable robot wearer according to an embodiment of the present invention, may be generated when the robot exercise is ended. Therefore, the step of generating a graphical user interface based on the walking analysis information of the wearer may include a step that is preceded by a step of receiving an input of exercise end in the user terminal and transmitting the received exercise end information to the wearable robot.

As shown in FIG. 18, the administrator terminal (400) according to an embodiment of the present invention may transmit and receive information regarding walking through the external server (100); specifically, it may receive a transmission (S270) of the real-time walking information of the wearer stored in the external server (100) and reflect and output (S271) the real-time walking information of the wearer on the administrator terminal (400), and likewise, it may receive a transmission (S272) of statistical information according to the accumulated walking information of the wearer stored in the external server (100) and reflect and output (S273) the statistical information according to the accumulated walking information of the wearer on the administrator terminal (400), and it may receive a transmission (S274) of the walking analysis report information of the wearer stored in the external server (100) and reflect and output (S275) the walking analysis report of the wearer on the administrator terminal (400). However, the administrator terminal (400) is not limited to being able to transmit and receive data only through the external server (100), and may transmit and receive data directly through a wired or wireless connection with the wearable robot (200).

### Graphical user interface displaying statistical information according to accumulated walking information of a wearable robot wearer

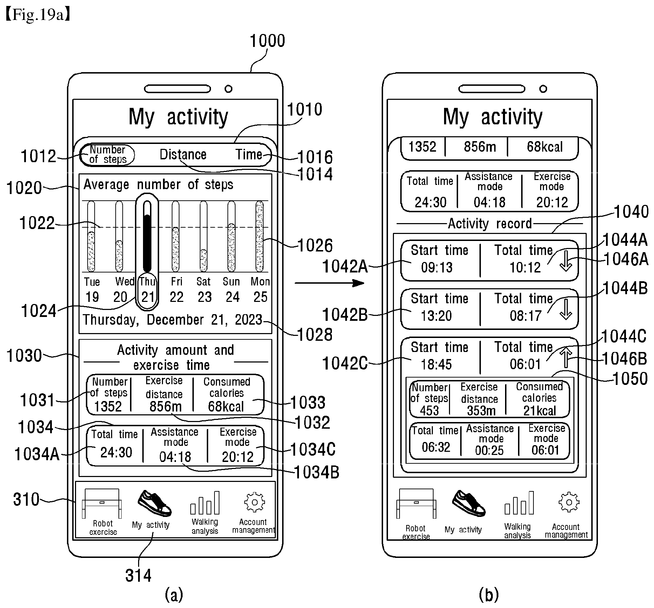

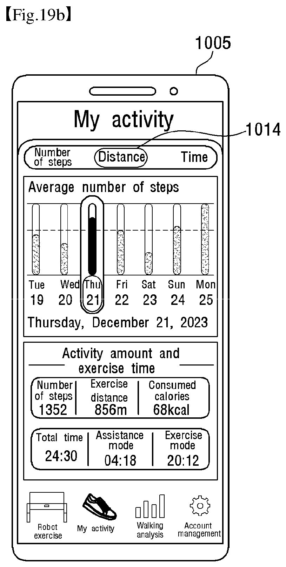

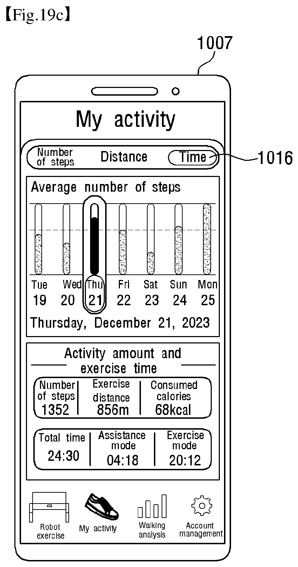

FIG. 19a is a diagram showing a graphical user interface of a user terminal displaying statistical information according to accumulated walking information of the wearer based on the 'number of steps' by recording robot exercise processes according to an embodiment of the present invention. FIG. 19b is a diagram showing a graphical user interface of a user terminal displaying statistical information according to accumulated walking information of the wearer based on 'distance' by recording robot exercise processes according to an embodiment of the present invention. FIG. 19c is a diagram showing a graphical user interface of a user terminal displaying statistical information according to accumulated walking information of the wearer based on 'time' by recording robot exercise processes according to an embodiment of the present invention.

When the My Activity (314) tab is selected in the category selection area (310) of the user terminal (300) according to an embodiment of the present invention, a graphical user interface in which statistical information according to accumulated walking information of the wearable robot wearer is displayed appears. As shown in FIG. 19a, the graphical user interface (1000) displaying statistical information according to accumulated walking information of the wearer based on the number of steps in which the number of steps (1012) is selected in the category selection area (1010) may include at least one of a category selection area (1010), a statistical information display area according to accumulated walking information (1020), an entire walking information display area of the selected date (1030), and a per-walking record information display area of the selected date (1040).

To describe in detail the areas displayed in the graphical user interface displaying statistical information according to accumulated walking information of the wearer according to an embodiment of the present invention, the category selection area (1010) is an area for selecting a category of statistical information according to accumulated walking information, the statistical information display area according to accumulated walking information (1020) is an area for displaying statistical information according to accumulated walking information based on the category selected in the category selection area (1010), the statistical information being generated by accumulating information regarding the walking of the wearer in the external server (100), the entire walking information display area of the selected date (1030) is an area for displaying the entire walking information accumulated in one or more robot exercise processes performed on the date selected in the statistical information display area according to accumulated walking information (1020), and the per-walking record information display area of the selected date (1040) is an area for displaying the walking information recorded in each robot exercise process.

The category selection area (1010) according to an embodiment of the present invention may select the number of steps (1012), distance (1014), or time (1016), and although there are three categories shown in (a) of FIG. 19a, the category of statistical information according to accumulated walking information may be added, changed, and deleted as needed.

The statistical information display area according to accumulated walking information (1020) according to an embodiment of the present invention may be represented by a bar or chart so that the statistical information according to accumulated walking information can be easily known, the bar or chart may include an average trend line (1022) representing the average of the statistical information according to accumulated walking information, and may have a plurality of areas according to dates (1026). The plurality of areas according to dates (1026) appear in proportion to the statistical information according to accumulated walking information, and may have a relative size compared with other dates. The relative size may be displayed by a bar length, a gauge bar, a ratio, or a graph, etc., and is not limited to what is shown in FIG. 19a, and may be replaced as long as it can be displayed so that the information displayed in the plurality of areas according to dates (1026) can be easily identified. In addition, when one area (1024) indicating a certain date on the plurality of areas according to dates (1026) is selected, the selected area may be distinguished from the other plurality of areas (1026) by having a different color or being highlighted, etc., and information regarding walking according to the robot exercise process of the selected date may be displayed in another area on the graphical user interface.

The entire walking information display area of the selected date (1030) according to an embodiment of the present invention may include at least one of a number-of-steps information display area (1031), a distance information display area (1032), a consumed-calorie information display area (1033), an entire-time information display area (1034), and a walking-time information display area (1034). The number-of-steps information display area (1031) may display information related to the number of steps of the user based on the entire operation information of the wearable robot worn by the user on the selected date. The distance information display area (1032) may display information related to the exercise distance of the user based on the entire operation information of the wearable robot worn by the user on the selected date. The consumed-calorie information display area (1033) may display information related to the consumed calories of the user based on the entire operation information of the wearable robot worn by the user on the selected date. The walking-time information display area (1034) may include an entire-time information display area (1034A), an assistance-mode time information display area (1034B), and an exercise-mode time information display area (1034C).

In the walking-time information display area (1034) according to an embodiment of the present invention, the entire-time information display area (1034A) may display information related to the operating time of the user based on the entire operation information of the wearable robot worn by the user on the selected date, and the operating time may be the sum of the use times in the assistance mode, the rest mode, and the exercise mode. The assistance-mode time information display area (1034B) is an area for displaying the operating time of the wearable robot (200) in the assistance mode state out of the entire-time information, and the exercise-mode time information display area (1034C) is an area for displaying the operating time of the wearable robot (200) in the exercise mode state out of the entire-time information.

However, the information included in the entire walking information display area of the selected date (1030) is exemplary, and it may also include information such as step length, step pattern, continuous walking time, walking rhythm, slope, number of steps when ascending or descending stairs, and number of floors, etc., based on the operation information of the wearable robot worn by the user.

The per-walking record information display area of the selected date (1040) according to an embodiment of the present invention may include a plurality of areas (1042, 1044) in which the per-walking records of the selected date are displayed. The number of the plurality of areas (1042, 1044) is determined by the number of walkings performed on the selected date, and the plurality of areas may include a start-time information display area (1042A,B,C) and an entire-time information display area (1044A,B,C). However, this is exemplary, and information regarding walking such as the number of steps, exercise distance, etc., may be added, changed, or deleted as needed. In addition, in the per-walking record information display area of the selected date (1040), when a downward arrow (1046A) disposed for each area among the plurality of areas (1042, 1044) is selected, a per-walking detailed record information display area (1050) appears based on the arrow input information of one area, and each of the per-walking records including at least one of the number of steps, exercise distance, consumed calories, entire time, assistance-mode time, and exercise-mode time may be disposed in the per-walking detailed record information display area (1050) within the plurality of areas (1042, 1044). When the per-walking detailed record information display area (1050) is activated on the graphical user interface, when an upward arrow (1046B) disposed in the plurality of areas (1042, 1044) is selected, the per-walking detailed record information display area (1050) is deactivated.

As shown in FIG. 19b, when the distance (1014) is selected in the category selection area (1010), a graphical user interface (1005) displaying statistical information according to accumulated walking information of the wearer based on distance appears, and as shown in FIG. 19c, a graphical user interface (1007) displaying statistical information according to accumulated walking information of the wearer based on the time in which the time (1016) is selected in the category selection area (1010) appears. However, the detailed configuration displayed within the graphical user interface is exemplary, and may be added, changed, or deleted as needed.

Therefore, the wearable robot wearer can check, in the graphical user interface displaying statistical information according to accumulated walking information of the wearer of the My Activity (314) tab of the user terminal (100), statistical information according to accumulated walking information based on the category selected from among the number of steps, distance, or time, and can check at least one of the entire walking information and the per-walking record information of the selected date.

### Graphical user interface displaying a walking analysis report of a wearable robot wearer

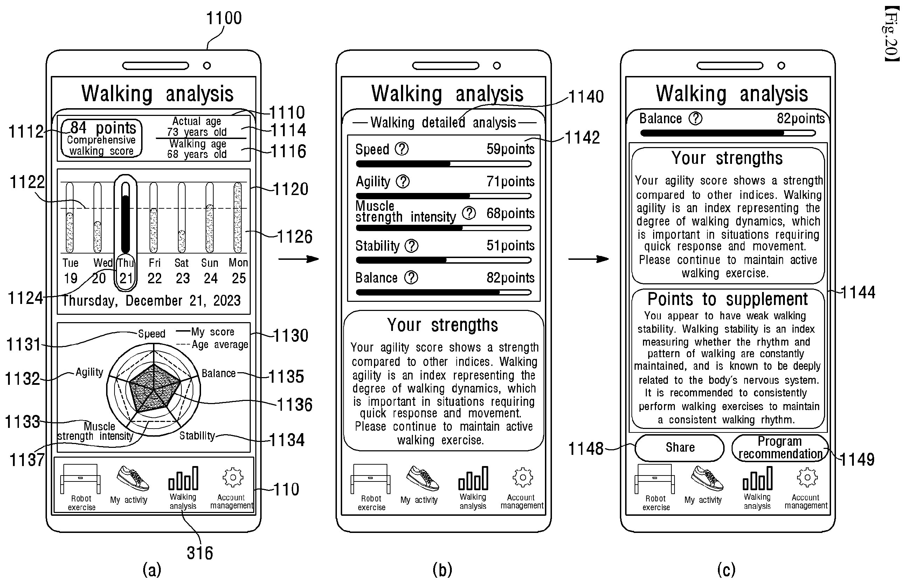

FIG. 20 is a diagram showing a graphical user interface of a user terminal displaying a walking analysis report based on walking analysis information of the wearer based on at least one of information regarding the walking of the wearer obtained in a past robot exercise process and accumulated data stored in an external server according to an embodiment of the present invention.

As shown in FIG. 20, when the Walking Analysis (316) tab is selected in the category selection area (310) of the user terminal (300) according to an embodiment of the present invention, a graphical user interface (1100) displaying a walking analysis report of the wearable robot wearer appears. The graphical user interface (1100) displaying a walking analysis report of the wearable robot wearer may include at least one of a comprehensive walking score and age information display area (1110), a statistical information display area according to accumulated walking information (1120), a walking index schematization area (1130), and a walking detailed analysis information display area (1140).

To describe in detail the areas displayed in the graphical user interface (1100) displaying a walking analysis report of a wearable robot wearer according to an embodiment of the present invention, the comprehensive walking score and age information display area (1110) may display a comprehensive walking score and walking age information based on basic information such as the wearer's body, age, etc., and the walking analysis information of the wearer. The statistical information display area according to accumulated walking information (1120) may display statistical information about the accumulation regarding the per-date walking score of the wearer based on at least one of information regarding the walking of the wearer and accumulated data stored in the external server. The walking index schematization area (1130) may represent the walking ability of the wearer through schematization so that the wearer's score for each walking index and the average score of the age can be easily compared and checked. The walking detailed analysis information display area (1140) may display the score for each walking index calculated based on the walking analysis information of the wearer so that it can be checked, and may display feedback on the current walking ability of the wearer according to the score for each walking index so that it can be checked.

The comprehensive walking score and age information display area (1110) according to an embodiment of the present invention may include a comprehensive walking score display area of the wearer (1112), an actual age information display area (1114), and a walking age information display area (1116), and the information displayed in the comprehensive walking score display area of the wearer (1112) and the walking age information display area (1116) may be information generated based on statistical information according to the accumulation of real-time walking information and physical/exercise ability information according to the long-term use of the wearer.

The statistical information display area according to accumulated walking information (1120) according to an embodiment of the present invention may be represented by a bar or chart so that the statistical information according to accumulated walking information can be easily known, the bar or chart may include an average trend line (1122) representing the average of the statistical information according to accumulated walking information, and may have a plurality of areas according to dates (1126). The plurality of areas according to dates (1126) appear in proportion to the statistical information according to accumulated walking information, and may have a relative size compared with other dates. The relative size may be anything that can be displayed so that the numerical values displayed in the plurality of areas according to dates (1126), such as a bar length, a gauge bar, a ratio, or a graph, etc., can be easily identified.

In addition, when one area (1124) indicating a certain date on the plurality of areas according to dates (1126) is selected, the selected area may be distinguished from the other plurality of areas (1126) by having a different color or being highlighted, etc., and information regarding walking according to the robot exercise process of the selected date may be displayed in another area on the graphical user interface. In addition, the statistical information according to accumulated walking information may represent statistical information about the accumulation regarding the per-date walking score, but it may also be about the per-week or per-month walking score rather than about the per-date walking score, and is not limited thereto.

The walking index schematization area (1130) according to an embodiment of the present invention may include a score for each walking index, a walking index graph of the user (1136) connecting the scores for each walking index, and an age-average walking index graph (1137) connecting the scores for each walking index according to the age average. The above area is one that schematizes the score for each walking index of the wearer, and may represent the walking ability of the wearer with a figure so that the score for each walking index of the wearer and the score for each walking index according to the age average can be compared.

Specifically, methods of schematizing the score for each walking index of the wearer may include a bar graph, a line graph, a pie graph, a scatter plot, a histogram, a box plot, a heatmap, etc., and such methods are not limited as long as the score for each walking index of the wearer and the score for each walking index according to the age average can be easily compared. In addition, the score for each walking index may be a score of speed (1131), agility (1132), muscle strength intensity (1133), stability (1134), and balance (1135). However, if there is another factor that is important in measuring and analyzing the physical/exercise ability regarding the walking of the wearer, it may be added, changed, or deleted in addition to the five walking indices mentioned above.

The walking detailed analysis information display area (1140) according to an embodiment of the present invention may include at least one of a score-per-walking-index display area (1142) and a feedback display area according to the score for each walking index (1144). As mentioned above, the score for each walking index displayed in the score-per-walking-index display area (1142) is not limited to what is shown in FIG. 11, and the score for each index may be displayed only with numbers, or may be displayed in various ways, such as a bar graph or a linear graph, so that the wearable robot wearer can easily check his or her own score for each walking index. The feedback display area according to the score for each walking index (1144) may analyze the score for each walking index of the wearer to include feedback appropriate for the wearer, and specifically, the feedback of the wearer may include the strengths and points to be supplemented of the walking ability of the wearer, problems that may occur on the body if the walking ability of the wearer is maintained, or an introduction of a method or program for acquiring a walking ability suited to the age of the wearer, etc.

In the score for each walking index according to an embodiment of the present invention, the speed may be measured through the moving distance versus the time measured in the robot exercise process, and the agility may be measured through the time taken to quickly and accurately complete an appropriate course for measuring agility, such as the TUG (Test Up and Go). The muscle strength intensity may be measured by measuring the maximum force on a specific muscle group or by evaluating endurance through a repetition test, and for example, the lower-body muscle strength may be evaluated by measuring the time taken while repeatedly standing up from a chair. The safety may be measured through how long a specific posture can be maintained while keeping balance, or through measurement of the reaction to the ground during the walking process. The balance may be measured by simply measuring the time of standing on one foot, or by performing a more complex balance test to measure the balance-maintaining ability. For the numerical value for each walking index measured in this way, an appropriate evaluation criterion may be set, and the numerical value for each walking index may be scored according to the set criterion. As the evaluation criterion, there may be various evaluation criteria, for example, an absolute score, a standardized score, a grade score, or a percentile score, etc.

Therefore, the graphical user interface displaying a walking analysis report of the wearer of the Walking Analysis (316) tab of the user terminal (300) may serve to measure the numerical value for each walking index based on the generated walking analysis information of the wearer, score such numerical values according to the evaluation criterion, then synthesize all the indices to evaluate the overall physical ability, and provide such analysis results so that the wearable robot wearer can easily check them. In addition, based on such analysis results, the wearer can receive a recommendation of customized program information through Program Recommendation (1149), and can share the analysis results with others or store them in another device, etc., through Share (1148).

### Graphical user interface displaying a walking analysis estimation report based on a deep learning model and customized program information

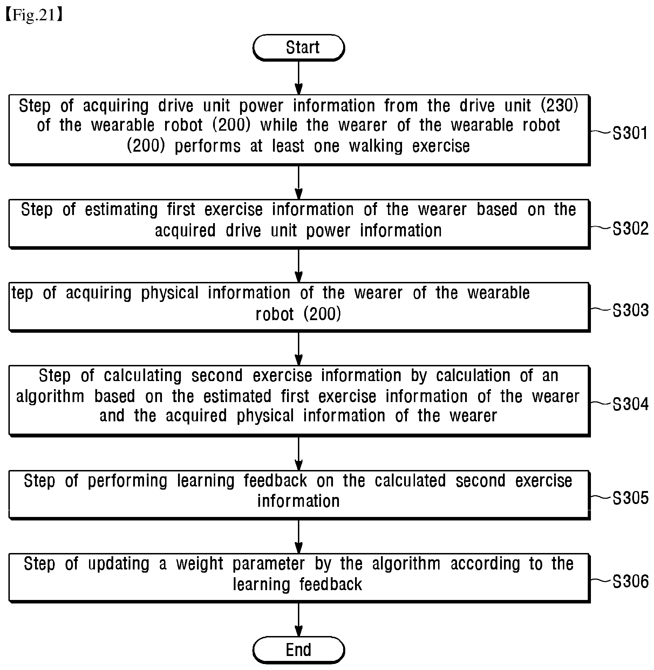

FIG. 21 is a diagram for explaining a method of estimating walking analysis information of a wearable robot wearer based on a deep learning model according to an embodiment of the present invention.

According to an embodiment of the present invention, an importance parameter may be learned based on Reinforcement Learning with Human Feedback (RLHF). Reinforcement Learning with Human Feedback (RLHF) is a combination of reinforcement learning and human feedback, and is a learning method that improves the performance of an artificial intelligence by training, based on human feedback, the information output through an artificial intelligence model. The walking analysis information of the wearable robot wearer may be estimated based on the importance parameter learned according to an embodiment of the present invention.

A reinforcement learning method based on human feedback according to an embodiment of the present invention may include a step (S301) of acquiring drive-unit power information from the drive unit of the wearable robot while the wearable robot wearer performs at least one walking exercise, a step (S302) of estimating first exercise information of the wearer based on the acquired drive-unit power information, a step (S303) of acquiring physical information of the wearable robot wearer, a step (S304) of calculating second exercise information by computation of an algorithm based on the estimated first exercise information of the wearer and the obtained physical information of the wearer, a step (S305) of performing learning feedback on the calculated second exercise information, and a step (S306) in which the algorithm updates a weight parameter according to the learning feedback.

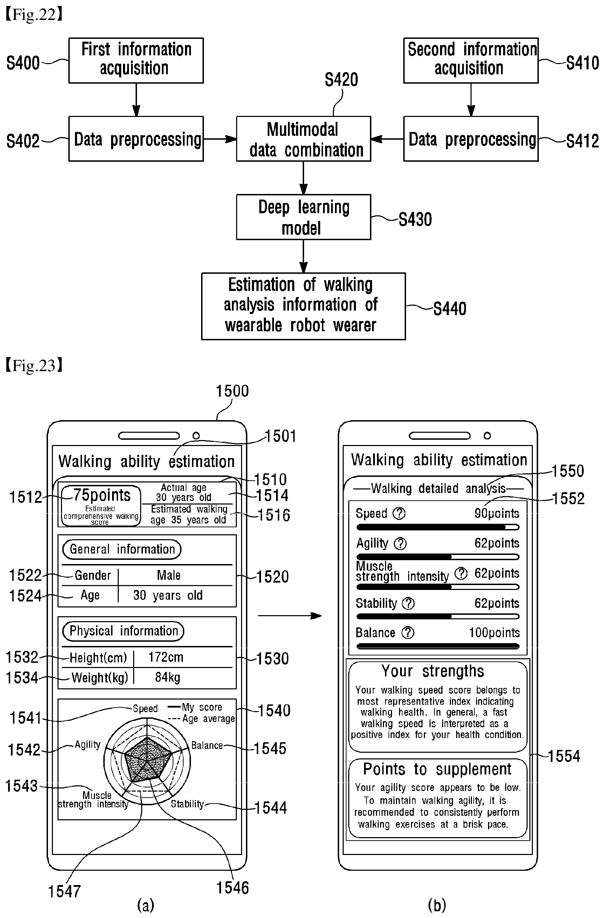

FIG. 22 is a flowchart for explaining a process of combining information regarding different types of walking of the wearer into multimodal data and estimating walking analysis information based on a deep learning model according to an embodiment of the present invention.

According to an embodiment for achieving the object of the present invention, a method for combining information regarding different types of walking of the wearable wearer into multimodal data and estimating walking analysis information through a deep learning model may include, when there are two pieces of information regarding different types of walking, a first information obtaining step (S400), a step (S402) of performing data preprocessing of the obtained first information, a second information obtaining step (S410), a step (S412) of performing data preprocessing of the obtained second information, a step (S420) of combining into multimodal data, a step (S430) of training the multimodal data on the deep learning model, and a step (S440) of estimating and obtaining the walking analysis information of the wearable robot wearer as an output of the deep learning model.

The first information obtaining step (S400) according to an embodiment of the present invention may obtain the basic information of the wearable robot wearer in the user terminal (300). The basic information of the wearable robot wearer may include not only information in the user terminal but also information stored in the external server, and may include general information such as the age, gender, etc., of the wearer, and physical information such as the body, weight, etc., of the wearer. However, the first information is not limited to the basic information of the wearable robot wearer, and may include various types of information.

The step (S402) of performing data preprocessing of the obtained first information according to an embodiment of the present invention may convert the obtained first information into a form suitable for combining into multimodal data. For example, the obtained first information may be text information, which is generally an example of natural language information. The data preprocessing process for text information is a process of natural language processing (NLP), and may generally include a step of dividing the text into tokens, a step of removing unnecessary tokens, and a step of converting the text into numbers.

The step of dividing the text into tokens according to an embodiment of the present invention is tokenizing the text, and may split a long text string into tokens, which are smaller and more manageable units. Methods of dividing the text into tokens may include word tokenization, sentence tokenization, phrase tokenization, subword tokenization, etc.

In addition, after the tokenization of the text information proceeds, a cleaning operation and a normalization operation may proceed. The cleaning operation is for excluding parts that interfere with the tokenization operation, and may be performed prior to the tokenization operation. The normalization operation may standardize the text information and convert it into a standard form. Methods for performing the cleaning and normalization operations may include normalization, stemming, lemmatization, case unification, number processing, synonym processing, etc. The cleaning and normalization operations may include parts that overlap with the step of removing unnecessary tokens below, in the process of excluding and standardizing unnecessary text information.

The step of removing unnecessary tokens according to an embodiment of the present invention may remove unnecessary information that interferes with converting the text information into a form suitable for combining into multimodal data. Methods of removing unnecessary tokens may include a method of removing unnecessary tokens by setting a removal list such as stopword removal, special character and number removal, short word removal, etc.

The step of converting the text into numbers according to an embodiment of the present invention may convert the text information into numerical information. This is because a machine learning model such as a deep learning model processes numerical data, and methods of converting the text into numbers may include integer encoding, one-hot encoding, byte pair encoding (BFE), Bag of Words (BoW), TF-IDF, word embeddings, etc.

Meanwhile, the specific description of the data preprocessing process of the text information described above is merely a simple example for explaining the step of performing data preprocessing of the obtained first information according to an embodiment of the present invention, and the present invention is not limited thereto.

According to an embodiment of the present invention, the step (S402) of performing data preprocessing of the first information may include a data augmentation process for obtaining a sufficient amount of meaningful data to input the obtained information into the deep learning model. Data augmentation techniques may include pitch shifting augmentation, TUT (TileUnTile) augmentation, and noise addition augmentation. The augmentation techniques described above are merely simple examples, and the present invention is not limited thereto.

According to an embodiment of the present invention, when there are two pieces of information regarding different types of walking, the second information obtaining step (S410) may obtain information regarding walking through at least one of the user terminal (300), the wearable robot (200), and the external server (100) in the robot exercise process of the wearable robot wearer. The information regarding the walking of the wearer according to the present invention may be classified into walking information of the wearer and walking analysis information of the wearer. The walking information of the wearer may include at least one of real-time walking information of the wearer in the robot exercise process and the quantified physical/exercise ability numerical value of the wearer, and the walking analysis information of the wearer may include at least one of physical/exercise ability information of the wearer and statistical information according to accumulated walking information.

According to an embodiment of the present invention, the step (412) of performing data preprocessing of the second information may include a data augmentation process for obtaining a sufficient amount of meaningful data to input the obtained information into the deep learning model.

The step (S412) of performing data preprocessing of the obtained second information according to an embodiment of the present invention may convert the obtained second information into a form suitable for combining into multimodal data. For example, the obtained second information may be sensed data obtained from a plurality of sensors. The data preprocessing process for the sensed data may generally include a step of cleaning and normalizing the sensed data, a step of extracting and transforming the characteristics of the sensed data, and a step of integrating the sensed data and adjusting the format.

The step of cleaning and normalizing the sensed data according to an embodiment of the present invention is for ensuring the consistency of the data and minimizing errors in the analysis or modeling process, and may be an initial data processing process for removing noise from the sensed data and improving the quality of the data. Such methods may include outlier removal, missing value processing, normalization and standardization, etc.

The step of extracting and transforming the characteristics of the sensed data according to an embodiment of the present invention may extract useful information of the sensor data and transform the data into a form suitable for analysis or modeling. Such methods may include extracting important characteristics of the data using the mean, standard deviation, FFT (Fast Fourier Transform), etc., and transforming the data using PCA (Principal Component Analysis), MDS (Multidimensional Scaling), T-SNE (t-distributed Stochastic Neighbor Embedding), ICA (Independent Component Analysis), LDA (Linear Discriminant Analysis), etc.

The step of integrating the sensed data and adjusting the format according to an embodiment of the present invention may integrate the data obtained from a plurality of sensors and adjust it into a final form suitable for analysis or modeling. Such methods may include sequence padding, splitting of the data set into training, validation, and test, final data format conversion, etc.

Meanwhile, the specific description of the data preprocessing process of the sensed data described above is merely a simple example for explaining the step of performing data preprocessing of the obtained second information according to an embodiment of the present invention, and the present invention is not limited thereto.

The step (S420) of combining into multimodal data according to an embodiment of the present invention combines a plurality of pieces of information of different types that have gone through the data preprocessing process into multimodal data, and combines the data in order to input the multimodal data into the deep learning model.

The step (S420) of combining into multimodal data according to an embodiment of the present invention may combine the preprocessed first information and the preprocessed second information into data of the same format. Specifically, the first information may be text information obtained in the user terminal (300), and the second information may be sensed data obtained in the user terminal (300) or the external server (100). At this time, in order to be used as an input of the deep learning model, since the first information and the second information may not have the same domain, each piece of data may be labeled as being about the first information and the second information.

The step (S420) of combining into multimodal data according to an embodiment of the present invention may combine each piece of information after performing augmentation on at least one of the first information and the second information.

However, the specific description of the step (S420) of combining into multimodal data according to an embodiment of the present invention is merely a simple example of combining the preprocessed first information and second information into multimodal data when there are two pieces of information of different types, and the present invention is not limited thereto.

The step (S430) of training the multimodal data on the deep learning model according to an embodiment of the present invention may process a plurality of pieces of data into a form suitable for the training of the deep learning model described above and input the combined multimodal data into the deep learning model to train it. In addition, if the form of the multimodal data is not the form required by the deep learning model, it may be processed.

The step (S440) of estimating and obtaining the walking analysis information of the wearable robot wearer as an output of the deep learning model according to an embodiment of the present invention may estimate the walking analysis information of the wearer using the deep learning model trained by receiving the multimodal data as input, and may output it. At this time, the estimated walking analysis information of the wearer is not limited to one form, and may have various forms.

According to another embodiment for achieving the object of the present invention, it may include a step of combining, into multimodal data, the inference of each piece of information regarding different types of walking of the wearable wearer. Specifically, when there are two pieces of information regarding different types of walking, it may include a first information obtaining step, a step of performing preprocessing of the obtained first information, a step of inferring information regarding walking by using the preprocessed first information as an input of the deep learning model, a second information obtaining step, a step of performing preprocessing of the obtained second information, a step of inferring information regarding walking by using the preprocessed second information as an input of an inference model, a step of combining the data in a multimodal manner, and a step of estimating and obtaining the walking analysis information of the wearable robot wearer by the combination of the multimodal data.

According to an embodiment of the present invention, the step of inferring information regarding walking by using the preprocessed second information as an input of an inference model may infer information regarding walking by using it as an input of a pre-trained inference model. The inference model in the deep learning process is a model that derives a prediction or conclusion for new information using a pre-trained model, and the pre-trained inference model may be the walking analysis model described above but is not limited thereto, and the pre-trained inference model may be an inference model of various types of methods for achieving the object. Various methods may be adopted for the pre-trained inference model.

However, the specific description explaining the flowchart shown in FIG. 22 is merely a simple example explaining the case where there are two pieces of information of different types, and the number of pieces of information or the type of information is not limited.

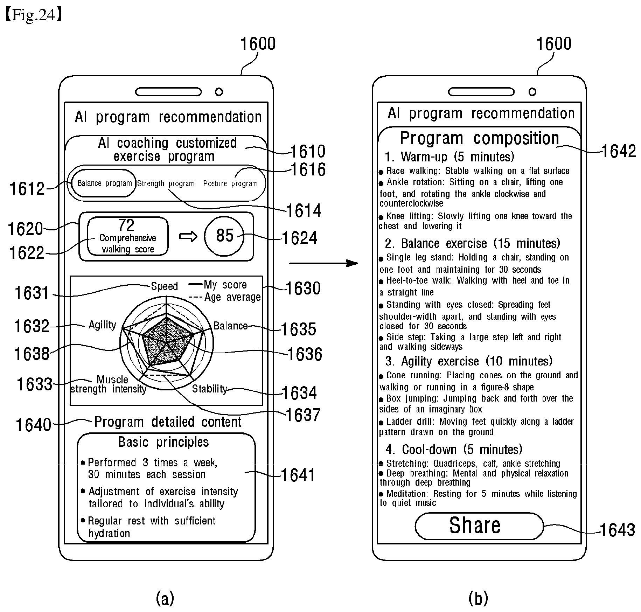

FIG. 23 is a diagram showing a graphical user interface displaying a walking ability estimation report based on the walking analysis information of the wearer estimated based on a deep learning model according to an embodiment of the present invention. FIG. 24 is a diagram showing a graphical user interface displaying customized exercise program information suitable for the walking ability of a wearable robot wearer based on a deep learning model according to an embodiment of the present invention.

A graphical user interface for providing information regarding walking generated based on a deep learning model according to an embodiment of the present invention may include a graphical user interface displaying a walking ability estimation report of the wearable robot wearer and a graphical user interface displaying customized program information of the wearer.

At this time, a method of generating a graphical user interface displaying a walking ability estimation report or customized program information of a wearable robot wearer based on a deep learning model according to an embodiment of the present invention may include a step of obtaining information regarding the walking of the wearer, a step of training a deep learning model based on the obtained information regarding the walking of the wearer, a step of estimating walking analysis information of the wearer based on the trained deep learning model, and a step of generating a graphical user interface based on the estimated walking analysis information of the wearer.

The step of obtaining information regarding the walking of the wearer according to an embodiment of the present invention may include a step of receiving walking information of the wearer from the wearable robot, a step of generating walking analysis information of the wearer based on the received walking information of the wearer, and a step of obtaining information regarding the walking of the wearer based on the generated walking analysis information of the wearer. At this time, the information regarding the walking of the wearer may be walking information measured in the robot exercise process of the wearer, and walking analysis information of the wearer stored in the user terminal (300) or the external server (100). In addition, in addition to the information regarding the walking of the wearer, it may include various information regarding walking and various program information that can improve the physical/exercise ability in the walking or exercise process of the wearer.

The step of training a deep learning model according to an embodiment of the present invention may be a step of, when the obtained information regarding the walking of the wearer is of a plurality of different forms, combining the obtained information regarding the walking of the wearer into multimodal data, and training the deep learning model based on the combined multimodal data. However, if the plurality of pieces of information regarding walking are of the same form, that is, the same domain, they are regarded as single-modal data, so the step of combining into multimodal data may be omitted.

The step of generating the walking analysis information of the wearable robot wearer, the step of receiving the walking information of the wearer in order to generate the walking analysis information of the wearer, and the step of transmitting one of the walking mode and torque intensity information set in order to receive the walking information of the wearer, according to an embodiment of the present invention, may be the same as the content described above, as shown in FIG. 18.

As shown in FIG. 23, a graphical user interface (1500) displaying a walking ability estimation report of the wearable robot wearer based on the walking analysis information of the wearer estimated based on a deep learning model according to an embodiment of the present invention appears in the user terminal (300). The graphical user interface (1500) displaying a walking ability estimation report of the wearable robot wearer may include at least one of an estimated comprehensive walking score display area (1510), basic information display areas of the wearer (1520, 1530), an estimated walking index schematization area (1540), and an estimated walking detailed analysis information display area (1550).

To describe in detail the areas displayed in the graphical user interface (1500) displaying a walking ability estimation report of the wearable wearer according to an embodiment of the present invention, the estimated comprehensive walking score display area (1510) may display information regarding the estimated walking comprehensive score and the estimated walking age of the wearer based on the walking analysis information of the wearer estimated based on a deep learning model. The basic information display areas of the wearer (1520, 1530) may display basic information that the wearer entered in the user terminal (300) or basic information stored in the external server (100).

According to the present invention, the estimated walking index schematization area (1540) may display, through schematization, information regarding the estimated walking ability of the wearer so that the estimated score of the wearer for each walking index and the average score of the age, based on the walking analysis information of the wearer estimated based on a deep learning model, can be easily compared and checked. The walking detailed analysis information display area (1550) may display information by which the estimated score for each walking index calculated based on the walking analysis information of the wearer estimated based on a deep learning model can be checked, and information regarding feedback on the estimated walking ability of the wearer according to the estimated score for each walking index.

The estimated comprehensive walking score display area (1510) according to an embodiment of the present invention may include an estimated comprehensive walking score display area of the wearer (1512), an actual age information display area (1514), and an estimated walking age information display area (1516), and the information displayed in the estimated comprehensive walking score display area of the wearer (1512) and the estimated walking age information display area (1516) may be information based on the walking analysis information of the wearer estimated based on a deep learning model. At this time, the information trained on the deep learning model may be information regarding the walking of the wearer. Specifically, the information regarding the walking of the wearer may be at least one of the basic information of the wearer, the real-time walking information of the wearer, and information generated based on statistical information according to the accumulation of real-time walking information and physical/exercise ability information according to the long-term use of the wearer stored in the external server (100).

The basic information display areas of the wearer (1520, 1530) according to an embodiment of the present invention may include a general information display area of the wearer (1520) and a physical information display area of the wearer (1530), and other than that, if it is basic information regarding the wearable wearer, it may be added as needed. The general information display area of the wearer (1520) may include a general information display area such as a gender information display area of the wearer (1522) and an age information display area (1524), and the physical information display area of the wearer (1530) may include an information display area regarding the body of the wearer such as a height (cm) information display area of the wearer (1532) and a weight (kg) information display area (1534).

The estimated walking index schematization area (1540) according to an embodiment of the present invention may include an estimated score for each walking index, a walking index graph of the user (1546) connecting the estimated scores for each walking index, and an age-average walking index graph (1547) connecting the scores for each walking index according to the age average. The above area is one that schematizes the estimated score for each walking index of the wearer, and may represent the estimated walking ability of the wearer with a figure so that the estimated score for each walking index of the wearer and the score for each walking index according to the age average can be compared.

Specifically, methods of schematizing the estimated score for each walking index of the wearer may include a bar graph, a line graph, a pie graph, a scatter plot, a histogram, a box plot, a heatmap, etc., and such methods are not limited as long as the estimated score for each walking index of the wearer and the score for each walking index according to the age average can be easily compared. In addition, the estimated score for each walking index may be a score of speed (1541), agility (1542), muscle strength intensity (1543), stability (1544), and balance (1545). However, if there is another factor that is important in estimating the walking ability of the wearer and analyzing the estimated walking ability of the wearer, it may be added, changed, or deleted in addition to the five walking indices mentioned above.

The estimated walking detailed analysis information display area (1550) according to an embodiment of the present invention may include at least one of an estimated-score-per-walking-index display area (1552) and a feedback display area according to the estimated score for each walking index (1554). As mentioned above, the estimated score for each walking index displayed in the estimated-score-per-walking-index display area (1552) is not limited to what is shown in FIG. 15, and the estimated score for each index may be displayed only with numbers, or may be displayed in various ways, such as a bar graph or a linear graph, so that the wearable robot wearer can easily check his or her own estimated score for each walking index. The feedback display area according to the estimated score for each walking index (1554) may analyze the estimated score for each walking index of the wearer to include feedback appropriate for the wearer, and specifically, the feedback of the wearer may include the strengths and points to be supplemented according to the estimated walking ability of the wearer, problems that may occur on the body if the estimated walking ability of the wearer is maintained, or an introduction of a method or program for acquiring an appropriate walking ability suited to the age of the wearer, etc.

As shown in FIG. 24, a graphical user interface (1500) displaying customized exercise program information suitable for the walking ability of the wearable robot wearer based on a deep learning model according to an embodiment of the present invention appears in the user terminal (300). The graphical user interface (1600) displaying customized program information of the wearable robot wearer may include at least one of a proposed customized program display area (1610), a comprehensive walking score display area (1620), a walking index schematization area (1630), and a detailed content display area of the proposed program (1640).

To describe in detail the areas displayed in the graphical user interface (1500) displaying customized program information of the wearable wearer according to an embodiment of the present invention, the proposed customized program display area (1610) may display a customized program suitable for the walking analysis information of the wearable robot wearer among a plurality of programs. The comprehensive walking score display area (1620) may display the comprehensive walking score that is expected to be improved when a customized program based on a deep learning model is performed. The walking index schematization area (1630) may represent the walking ability of the wearer through schematization so that the wearer's score for each walking index, the average score of the age, and the wearer's score that is expected to be improved when a customized program based on a deep learning model is performed can be easily compared and checked. The detailed content display area of the proposed program (1640) may display the customized program information based on a deep learning model in detail by basic principles, program composition, etc.

The proposed customized program display area (1610) according to an embodiment of the present invention is an area that proposes a program suitable for the walking ability of the wearer from among a balance program (1612), a strength program (1614), and a posture correction program (1616) based on a deep learning model, and may emphasize and display the proposed program by highlighting, underlining, bolding, or utilizing a figure, etc. In addition, as will be described later, the program is not limited to the types described above.

The comprehensive walking score display area (1620) according to an embodiment of the present invention may include comprehensive walking score information (1622) according to the walking ability of the wearer based on the walking analysis information of the wearer, and comprehensive walking score information (1624) that is expected to be improved when a customized program based on a deep learning model is performed. This is merely exemplary, and additional matters may be added as long as it represents the comprehensive walking score expected by executing a customized program based on a deep learning model.

The walking index schematization area (1630) according to an embodiment of the present invention may include a score for each walking index, a walking index graph of the user (1636) connecting the scores for each walking index, and an age-average walking index graph (1637) connecting the scores for each walking index according to the age average. In addition, it may include a walking index expected graph (1638) connecting the scores for each walking index that are expected to be improved when a customized program based on a deep learning model is performed. The above area is one that schematizes the score for each walking index of the wearer, and may represent the walking ability of the wearer with a figure so that the score for each walking index of the wearer, the score for each walking index according to the age average, and the score for each walking index that is expected to be improved when a customized program based on a deep learning model is performed can be compared.

Specifically, methods of schematizing the score for each walking index of the wearer may include a bar graph, a line graph, a pie graph, a scatter plot, a histogram, a box plot, a heatmap, etc., and such methods are not limited as long as the score for each walking index of the wearer and the score for each walking index according to the age average can be easily compared. In addition, the score for each walking index may be a score of speed (1631), agility (1632), muscle strength intensity (1633), stability (1634), and balance (1635). However, if there is another factor that is important in measuring and analyzing the physical/exercise ability regarding the walking of the wearer, it may be added, changed, or deleted in addition to the five walking indices mentioned above.

In the walking index schematization area (1630) according to an embodiment of the present invention, the score for each walking index of the wearer may include the estimated score for each walking index of the wearer estimated based on a deep learning model.

The detailed content display area of the proposed program (1640) according to an embodiment of the present invention represents the customized program information proposed based on a deep learning model, and may include a basic principle information display area (1641) for executing the program and a program composition information display area (1642). Specifically, it may include points of caution before proceeding with the program, the detailed content of each step constituting the program, or safety rules, etc., and is not limited to merely what is displayed on the graphical user interface.

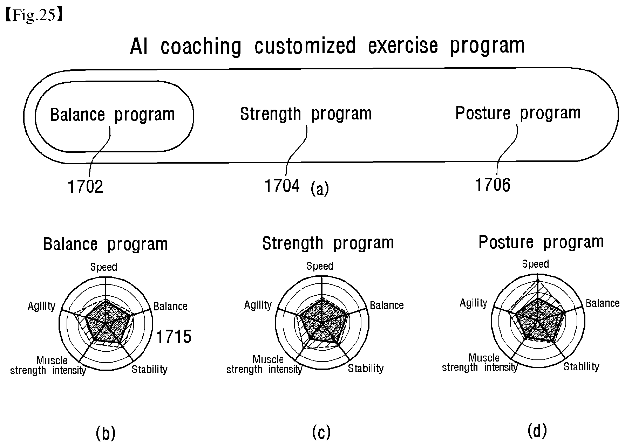

FIG. 25 is a diagram showing customized exercise program information suitable for the walking ability of a wearable robot wearer and the expected effects based on a deep learning model according to an embodiment of the present invention.

As shown in FIG. 25, customized program information that can be proposed to the wearable robot wearer based on a deep learning model according to an embodiment of the present invention and the expected effects when the program is performed may be illustrated. Specifically, the customized program information that can be proposed may include the balance program (1702), the strength program (1704), and the posture correction program (1706) illustrated in FIG. 25(a), and the expected effects when each program is performed may be displayed as a walking index schematization area as shown in FIG. 25(b) to FIG. 25(d). However, the expected effects are not limited in the form or manner in which they are expressed, as long as the wearable robot wearer can intuitively recognize the expected effects in the graphical user interface on the user terminal (300). In addition, the customized program information that can be proposed to the wearable robot wearer based on a deep learning model according to an embodiment of the present invention may be customized program information having different characteristics depending on the score for each walking index that is improved.

### Addition and registration of a trainer

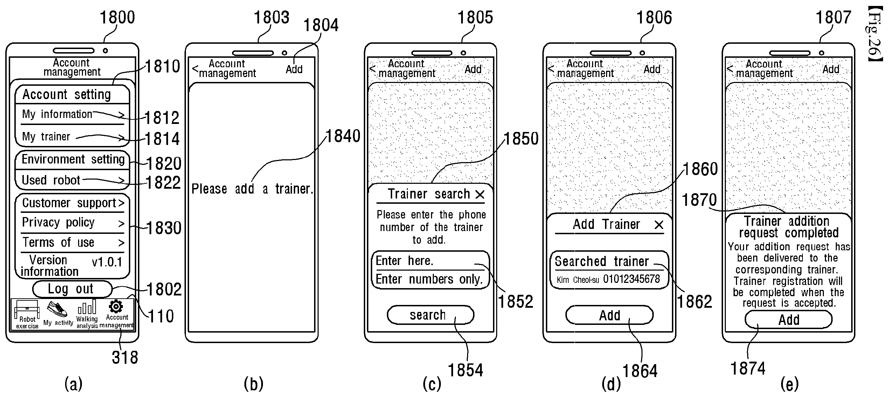

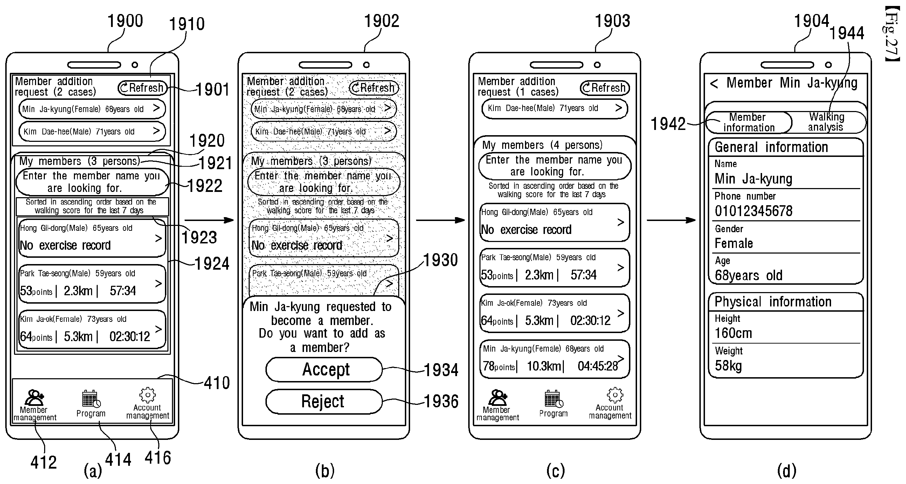

FIG. 26 is a diagram showing a graphical user interface representing a process for registering administrator information in a user terminal according to an embodiment of the present invention. FIG. 27 is a diagram showing a graphical user interface representing a process for registering user information in an administrator terminal according to an embodiment of the present invention.

As shown in (a) to (e) of FIG. 26, the process for a wearable robot wearer to register administrator information in the user terminal (300) according to an embodiment of the present invention may include a step of selecting the Account Management (318) tab in the category selection area (310) of the user terminal (300) so that a user account management graphical user interface (1800) is displayed, a step of selecting 'My Trainer (1814)' in the account setting area (1810) on the user account management graphical user interface (1800), a step of selecting 'Add (1804)' in the graphical user interface (1803) displaying trainer information, a step of entering the administrator's phone number in the trainer phone number input area (1852) and selecting Search (1854) in the trainer search graphical user interface (1805), a step of checking the searched administrator in the search information check area (1862) and selecting Add (1864) in the trainer addition graphical user interface (1806), and a step of checking that the trainer addition request completion display area (1870) is displayed and selecting Add (1874) in the trainer addition request completion graphical user interface (1807). At this time, the trainer may be a term referring to the administrator.

As shown in (a) and (b) of FIG. 27, the process for an administrator to register user information in the administrator terminal according to an embodiment of the present invention may include a step of selecting the Account Management (416) tab in the category selection area (410) of the administrator terminal (400) so that a member management graphical user interface (1900) is displayed, a step of selecting a member displayed in the member addition request display area (1910) in the member management graphical user interface (1900), and a step of selecting one of Accept (1934) and Reject (1936) in the response display area to the member addition request (1930) of the member addition request response graphical user interface (1902) after selecting the member.

As shown in FIG. 27(c), when Accept (1934) is selected in the response display area to the member addition request (1930), a graphical user interface (1903) reflecting the added member appears, and as shown in FIG. 27(d), when the added member is selected, the member information (1942) or walking analysis information (1944) for the selected member can be checked in the graphical user interface (1904) displaying information of the member being managed.

In addition, in the member management graphical user interface (1900) according to an embodiment of the present invention, when member addition request information is not received in the member addition request display area (1910), the corresponding information can be received by selecting the Refresh (1901) button.

In addition, in the member management graphical user interface (1900) according to an embodiment of the present invention, the member information display area being managed by the administrator (1920) may display a list of members for whom the administrator received member addition request information and accepted it. At this time, the member information display area being managed (1920) may include a member count information display area (1921), a member name search area (1922), a classification criterion display area (1923), and a registered member information display area (1924).

The member count information display area (1921) according to an embodiment of the present invention may display the number of members that the administrator registered in the administrator terminal (400), the member name search area (1922) is an area in which the member name to be found can be entered in a search window, the classification criterion display area (1923) is an area for setting so that the members displayed in the registered member information display area (1924) can be displayed according to a classification criterion, and the registered member information display area (1924) is an area that can display at least one of the basic information and walking information of the member together with the name of the member.

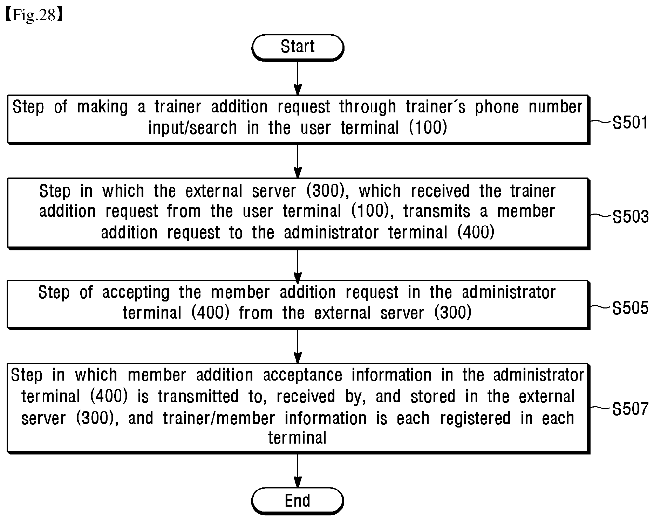

FIG. 28 is a flowchart for explaining a process in which each piece of information of the user and the administrator is registered in each of the user terminal and the administrator terminal according to an embodiment of the present invention.

As shown in FIG. 28, the process in which each piece of information of the user and the administrator is registered in each of the user terminal (300) and the administrator terminal (400) may include a step (S501) of making a trainer addition request through phone number input/search of the trainer in the user terminal (300), a step (S503) in which the external server (100), which received the trainer addition request from the user terminal (300), transmits a member addition request to the administrator terminal (400), a step (S505) in which the administrator terminal (400) accepts the member addition request from the external server (100), and a step (S507) in which the member addition acceptance information at the administrator terminal (400) is transmitted to and stored in the external server (100), and the trainer/member information is each registered in each terminal.

The process in which each piece of information of the user and the administrator is registered in each of the user terminal and the administrator terminal according to an embodiment of the present invention is merely exemplary, and rather than the user making a trainer addition request and the administrator accepting it so that each piece of information is registered in each terminal, it is possible to register administrator information in the user terminal (300) or register user information in the administrator terminal (400) independently using another authentication means.

In addition, the trainer or administrator described in detail above refers to a person who is in charge of managing and supervising the robot exercise process of the wearable robot wearer, and the administrator may be a doctor, a rehabilitation therapist, a nurse, a fitness trainer at a gym, an exercise program specialist, etc.

### User account management

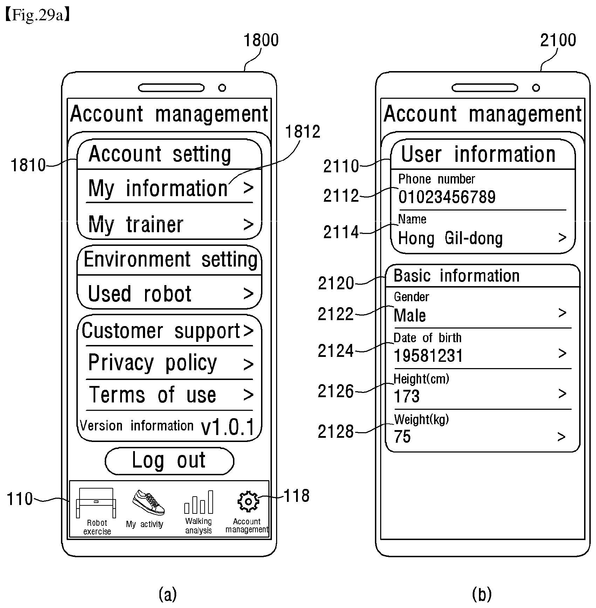

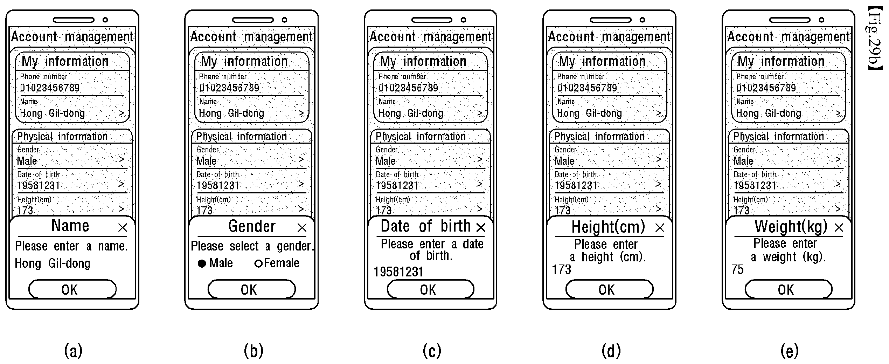

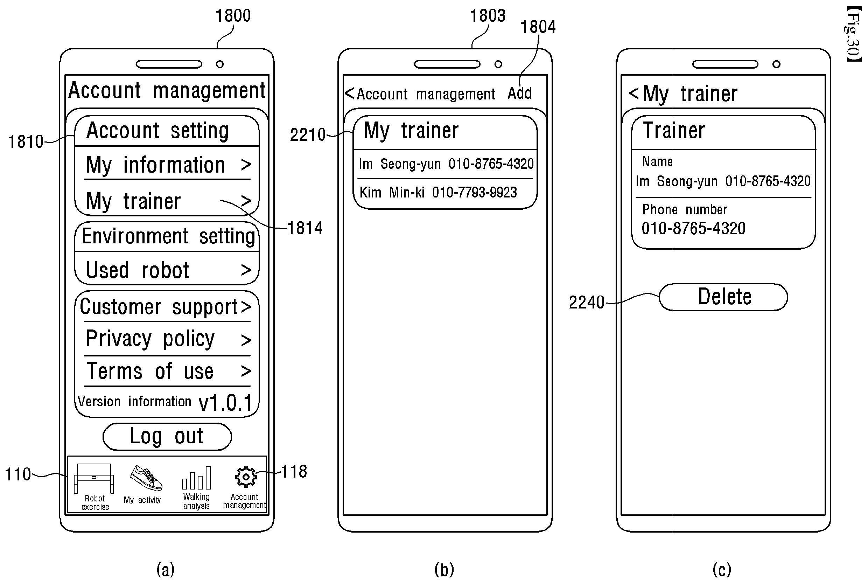

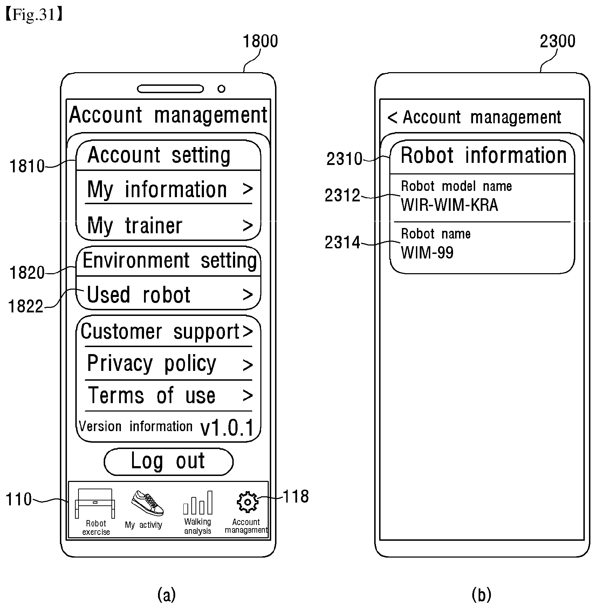

FIG. 29a and FIG. 29b are diagrams showing a graphical user interface of a user terminal that can check and modify the account information of the user according to an embodiment of the present invention. FIG. 30 is a diagram showing a graphical user interface of a user terminal that can check the information of the administrator and delete the registration according to an embodiment of the present invention. FIG. 31 is a diagram showing a graphical user interface of a user terminal that can check the information of the robot used by the user according to an embodiment of the present invention.

The user account management graphical user interface (1800) displayed by selecting the Account Management (318) tab in the category selection area (310) of the user terminal (300) according to an embodiment of the present invention may include an account setting area (1810), an environment setting area (1820), an other area (1830), and Log Out (1802).

The account setting area (1810) on the user account management graphical user interface (1800) according to an embodiment of the present invention can check user information and administrator information. When 'My Information (1812)' is selected in the account setting area (1810), as shown in (b) of FIG. 29a, a graphical user interface (2100) displaying user information appears, and the graphical user interface (2100) displaying user information may include a user information display area (2110) and a basic information display area (2120). The user information display area (2110) may include a phone number information display area (2112) and a name information display area (2114), and the basic information display area (2120) may include a gender information display area (2122), a date-of-birth information display area (2124), a height (cm) information display area (2126), and a weight (kg) information display area (2128). However, the detailed content included in the user information display area (2110) and the basic information display area (2120) may be changed.

In addition, when 'My Information (1812)' is selected in the account setting area (1810) according to an embodiment of the present invention and then an area displaying each piece of information is selected, as shown in (a) to (d) of FIG. 29b, the user information and basic information regarding the user stored within the user terminal (300) can be deleted and modified with new information. At this time, the modified information may be transmitted from the user terminal (300) to the external server (100), and may be stored within the external server (100) that received it.

When 'My Trainer (1814)' is selected in the account setting area (1810) according to an embodiment of the present invention, as shown in (b) of FIG. 30, a graphical user interface (1803) displaying administrator information appears, and the graphical user interface (1803) displaying administrator information includes a registered administrator information display area (2210), and may include an Add (1804) button by which the user described above can make an addition request to add a new administrator.

The registered administrator information display area (2210) according to an embodiment of the present invention may display the information of the administrator registered in the user terminal. For example, the information of the administrator may include the administrator's name, gender, phone number, other information, etc. When one of the registered administrators is selected in the registered administrator information display area (2210), as shown in (c) of FIG. 30, the information about the selected administrator can be checked in detail, and the information of the registered administrator can be deleted through the Delete (2240) button. In addition, when the information of the registered administrator is deleted within the user terminal, information that it has been deleted is transmitted to the external server (100), and the external server (100) that received it can delete the stored information of the administrator. However, the log records that are stored and deleted may still remain in the external server (100).

The environment setting area (1820) on the user account management graphical user interface (1800) according to an embodiment of the present invention may include a used-robot information display area (1822). The used-robot information display area (1822) is wirelessly connected to the user terminal, and may display information regarding the wearable robot worn by the user.

When 'Used Robot (1822)' is selected in the environment setting area (1820) according to an embodiment of the present invention, as shown in (b) of FIG. 31, a graphical user interface (2300) in which wearable robot information including a wearable robot information display area (2310) is displayed appears. The wearable robot information display area (2310) may include a robot model name information display area (2312) that can display the unique model name of the wearable robot and a robot name information display area (2314) that displays the name (management number) set for the wearable robot. At this time, the robot name (management number) displayed in the robot name information display area (2314) can be set by the user or the administrator.

The other area (1830) on the user account management graphical user interface (1800) according to an embodiment of the present invention may include an area that can display information regarding customer support, privacy policy, terms of use, and version information of the application stored in the user terminal. When each area is clicked, the detailed content regarding customer support, privacy policy, terms of use, and version information of the application stored in the user terminal can be checked.

The plurality of areas on the user account management graphical user interface (1800) according to an embodiment of the present invention are exemplary, and may additionally include an area that displays information necessary for managing the user's account, other than that.

### Administrator account management

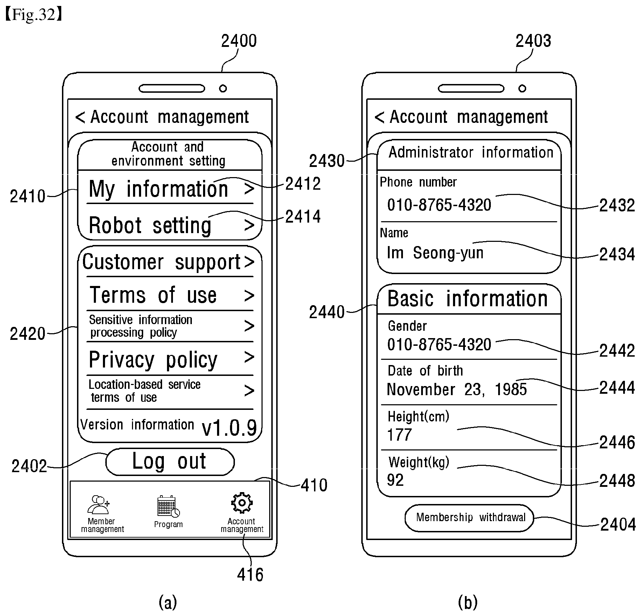

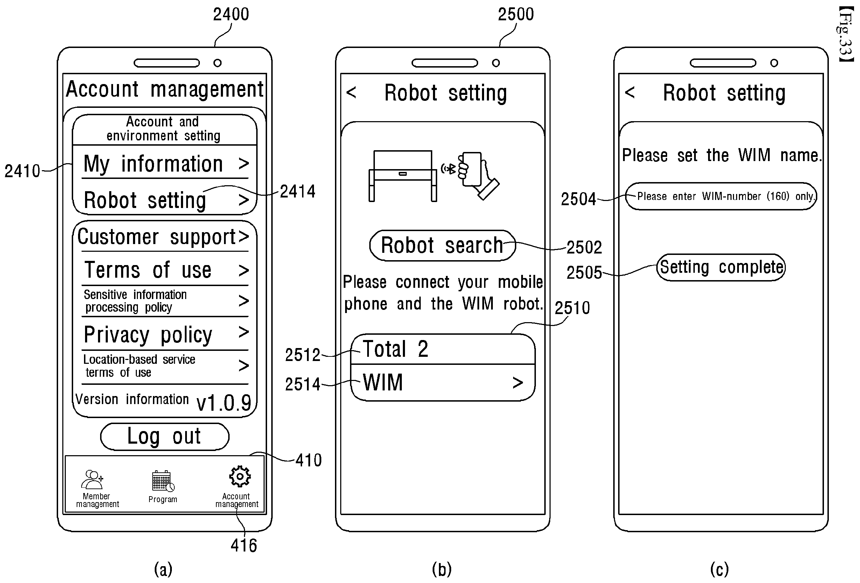

FIG. 32 is a diagram showing a graphical user interface of an administrator terminal that can check and modify the account information of the administrator according to an embodiment of the present invention. FIG. 33 is a diagram showing a graphical user interface of an administrator terminal that sets the robot name (management number) of the wearable robot connected to the user terminal according to an embodiment of the present invention.

The administrator account management graphical user interface (2400) displayed by selecting the Account Management (416) tab in the category selection area (410) of the administrator terminal (400) according to an embodiment of the present invention may include an account and environment setting area (1810), an other area (2420), and Log Out (2402).

The account and environment setting area (2410) on the administrator account management graphical user interface (2400) according to an embodiment of the present invention can check administrator information and information of the wearable robot connected with the user terminal (300). When 'My Information (2412)' is selected in the account and environment setting area (2410), as shown in (b) of FIG. 32, a graphical user interface (2403) displaying administrator information appears, and the graphical user interface (2403) displaying administrator information may include an administrator information display area (2430), a basic information display area (2440), and Membership Withdrawal (2404). The administrator information display area (2430) may include a phone number information display area (2432) and a name information display area (2434), and the basic information display area (2440) may include a gender information display area (2442), a date-of-birth information display area (2444), a height (cm) information display area (2446), and a weight (kg) information display area (2448). However, the detailed content included in the administrator information display area (2430) and the basic information display area (2440) may be changed.

In addition, when 'My Information (2412)' is selected in the account and environment setting area (2410) according to an embodiment of the present invention and then an area displaying each piece of information is selected, as in (a) to (d) of FIG. 29b regarding the user terminal (300), the administrator information and basic information regarding the administrator stored within the administrator terminal (400) can be deleted and modified with new information. At this time, the modified information may be transmitted from the administrator terminal (400) to the external server (100), and may be stored within the external server (100) that received it.

When 'Robot Setting (2414)' is selected in the account and environment setting area (2410) on the administrator account management graphical user interface (2400) according to an embodiment of the present invention, as shown in (b) and (c) of FIG. 33, a wearable robot name setting graphical user interface (2500) appears. The process of setting the name of the wearable robot may include a step of selecting the Robot Search (2502) button on the wearable robot name setting graphical user interface (2500), a step of, after selecting the Robot Search (2502) button, displaying a wearable robot that can be connected with the user terminal (300) or the administrator terminal (400) in the searched wearable robot display area (2510), a step of selecting the searched wearable robot (2514), and a step of entering the name (management number) in the name (management number) input area of the wearable robot (2504) and selecting the Setting Complete (2505) button.

In the case where such a process is gone through, the set wearable robot name (management number) information is stored in the administrator terminal, and may be transmitted from the administrator terminal (400) to the external server (100). The wearable robot name (management number) information transmitted to and stored in the external server (100) may be transmitted to the user terminal (300), and the wearable robot wearer can check the wearable robot name (management number) information set in the administrator terminal (400) by selecting 'Used Robot (1822)' in the environment setting area (1820) of the user account management graphical user interface (1800) of the user terminal (300).

The searched wearable robot display area (2510) according to an embodiment of the present invention may include a searched wearable robot count display area (2512) and the searched wearable robot (2514).

The other area (2420) on the administrator account management graphical user interface (2400) according to an embodiment of the present invention may include an area that can display information regarding customer support, privacy policy, terms of use, and version information of the application stored in the user terminal. In addition, the other area (2420) may include an area that can display information regarding the sensitive information processing policy and the location-based service terms of use. When each area is clicked, the detailed content regarding customer support, privacy policy, terms of use, version information of the application stored in the user terminal, the sensitive information processing policy, and the location-based service terms of use can be checked.

The plurality of areas on the administrator account management graphical user interface (2400) according to an embodiment of the present invention are exemplary, and may additionally include an area that displays information necessary for managing the user's account, other than that.

Graphical user interface providing program information generated for the administrator's 1:N group exercise and information regarding the walking of a plurality of members participating in the program

According to an embodiment of the present invention, the wearable robot (200), as described above, may adjust the assistance force or resistance force provided for each purpose of the walking or exercise of the wearable robot user through the user terminal (300) or the administrator terminal (400). At this time, the wearable robot wearer can directly adjust at least one of the walking mode and torque intensity through the user terminal (300), but the administrator can control, through the administrator terminal (400), at least one of the walking mode and torque intensity of the wearable robot of each of a plurality of wearable robot wearers participating in a program generated for 1:N group exercise, and can run the program and check the walking analysis information measured and calculated in the user terminal (300) or the wearable robot (200).

According to the present invention, a method for the administrator to control at least one of the walking mode and torque intensity of the wearable robot (200) of each of a plurality of wearable robot wearers participating in a program through the administrator terminal (400) according to an embodiment of the present invention may be a method of controlling while setting at least one of the walking mode and torque intensity of the wearable robot through a wireless connection between the administrator terminal and the wearable robots of the participating members. In addition, a method by which the administrator can check, through the administrator terminal (400), the general information or information regarding the walking of each of a plurality of wearable robot wearers participating in a program may be a method of receiving, at the administrator terminal (400), information regarding the walking of a plurality of wearers participating in a program stored in the external server (100) or in the user terminals (300) of the plurality of wearable robot wearers, and checking the received information regarding the walking of the plurality of wearers through a graphical user interface.

At this time, in the case where the administrator is controlling at least one of the walking mode and torque intensity of the wearable robot (200) of each of a plurality of wearable robot wearers participating in a program through the administrator terminal (400) according to an embodiment of the present invention, the administrator may selectively enable the wearer to control at least one of the walking mode and exercise intensity of the wearable robot by himself or herself through the user terminal (300), or may restrict the controlling.

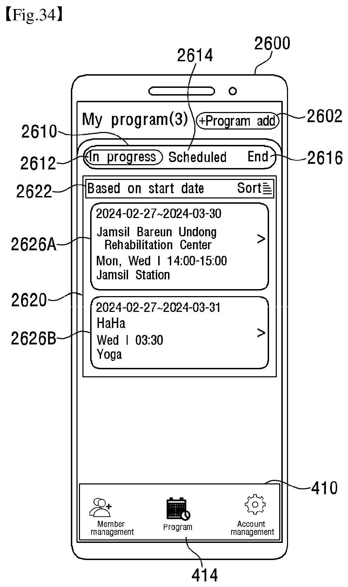

FIG. 34 is a diagram showing a graphical user interface of an administrator terminal representing the basic screen when the program tab for 1:N group exercise is clicked according to an embodiment of the present invention.

The graphical user interface of the administrator terminal (400) according to an embodiment of the present invention may include a category selection area, and the category selection area (410) may include Member Management (412), Program (414), and Account Management (416). At this time, the administrator can group people having similar exercise abilities and proceed with an exercise program through the Program (414) of the category selection area (410).

As shown in FIG. 34, the graphical user interface (2600) displaying program information generated for group exercise of the administrator terminal (400) according to an embodiment of the present invention may include a program progress status selection area (2610) and a program information display area according to the progress status (2620).

The program progress status selection area (2610) according to an embodiment of the present invention may include In Progress (2612), Scheduled (2614), and Ended (2616). When one status among such a plurality of progress statuses is selected, program information matching the progress status may be displayed in the generated program information display area.

The generated program information display area (2620) according to an embodiment of the present invention may, when one status among a plurality of progress statuses is selected in the program progress status selection area (2610), display the information of the program that is in the selected progress status among a plurality of programs generated for group exercise. The generated program information display area (2620) is an area that can display a plurality of pieces of generated program information, and may include a sort criterion display and setting area of the generated programs (2622) and a per-program information display area (2626A,B). The sort criterion display and setting area of the generated programs is an area that sets and displays the sort criterion for listing the generated programs, and the sort criterion may be by start date, by end date, by day of the week, etc. The information included in the program information display area (2626A,B) may include information such as the program progress period, the program progress location, the progress day of the week and time, etc.

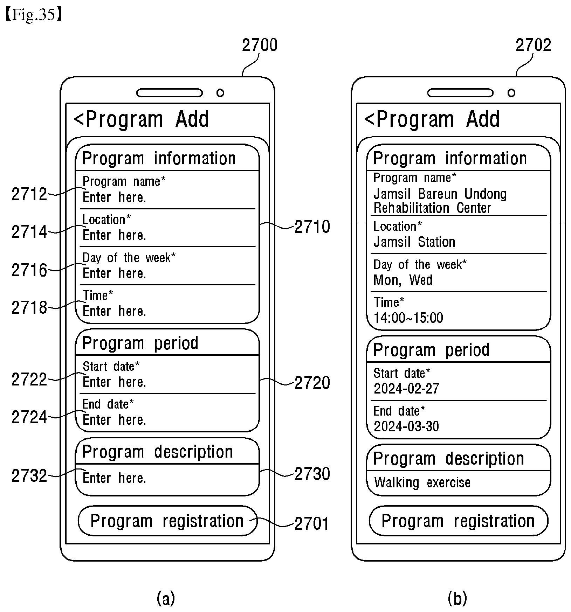

FIG. 35 is a diagram showing a graphical user interface of an administrator terminal that generates a program for 1:N group exercise according to an embodiment of the present invention.

When the Program Add (2602) button on the graphical user interface (2600) displaying program information generated for group exercise according to an embodiment of the present invention is selected, as shown in FIG. 35, a program addition graphical user interface (2700) appears.

As shown in (a) of FIG. 35, the program addition graphical user interface (2700) may include a program information input area (2710), a program period information input area (2720), and a program description information input area (2730). The program information input area (2710) may include a program name information input area (2712), a location information input area (2714), a day-of-the-week information input area (2716), and a time information input area (2718). The program period information input area (2720) may include a start date information input area (2722) and an end date information input area (2724). The program description information input area (2730) may include a description information input area (2732) for entering an introduction regarding the program, etc. However, such a plurality of input areas are exemplary, and a plurality of input areas may be added or changed.

(b) of FIG. 35 is a diagram regarding a graphical user interface (2702) in which the administrator entered information regarding the program in a plurality of input areas in order to generate a program in the program addition graphical user interface (2700), and in the case where information is entered in the plurality of input areas, the program can be generated by selecting the Program Register (2701) button.

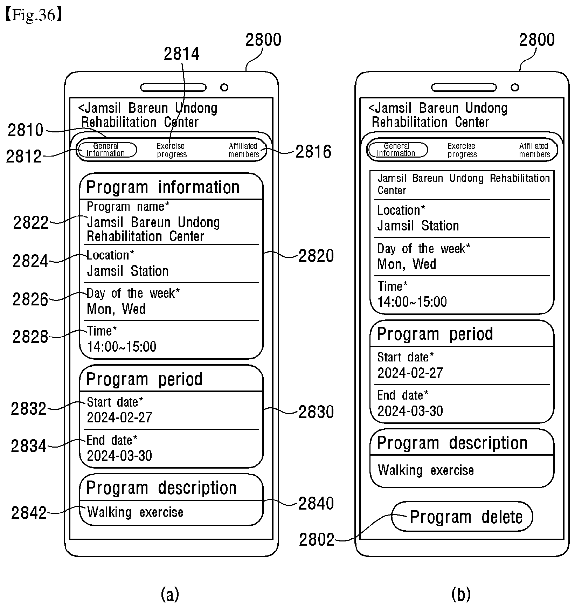

FIG. 36 is a diagram showing a graphical user interface of an administrator terminal representing the general information of a program generated for 1:N group exercise according to an embodiment of the present invention.

When one program among a plurality of programs displayed in the program information display area according to the progress status (2620) on the graphical user interface (2600) displaying program information generated for group exercise according to an embodiment of the present invention is selected, as shown in FIG. 28, a graphical user interface (2800) displaying the general information of the program appears, and may be the basic screen in the case where a program is selected.

The graphical user interface (2800) displaying the general information of a program according to an embodiment of the present invention is one that appears in the case where General Information (2812) is selected in the category selection area (2810), and may include a program information display area (2820), a program period information display area (2830), a program description information display area (2840), and Program Delete (2808).

The category selection area (2810) according to an embodiment of the present invention may include General Information (2812), Exercise Progress (2814), and Affiliated Members (2816). General Information (2812) may display information regarding the program, Exercise Progress (2814) may display information in the process of executing the generated program, and Affiliated Members (2816) may display the information of members participating in the program or members affiliated with an institution such as a hospital or athletic facility that runs the program.

The program information display area (2820) according to an embodiment of the present invention-the program information display area (2820) may include a program name information display area (2822), a location information display area (2824), a day-of-the-week information display area (2826), and a time information display area (2828).

The program period information display area (2830) according to an embodiment of the present invention may include a start date information display area (2832) and an end date information display area (2834).

The program description information display area (2840) and Program Delete (2802) according to an embodiment of the present invention-the program description information display area (2840) may include a description information display area (2842) for entering an introduction regarding the program, etc., and Program Delete (2802) can delete the generated program.

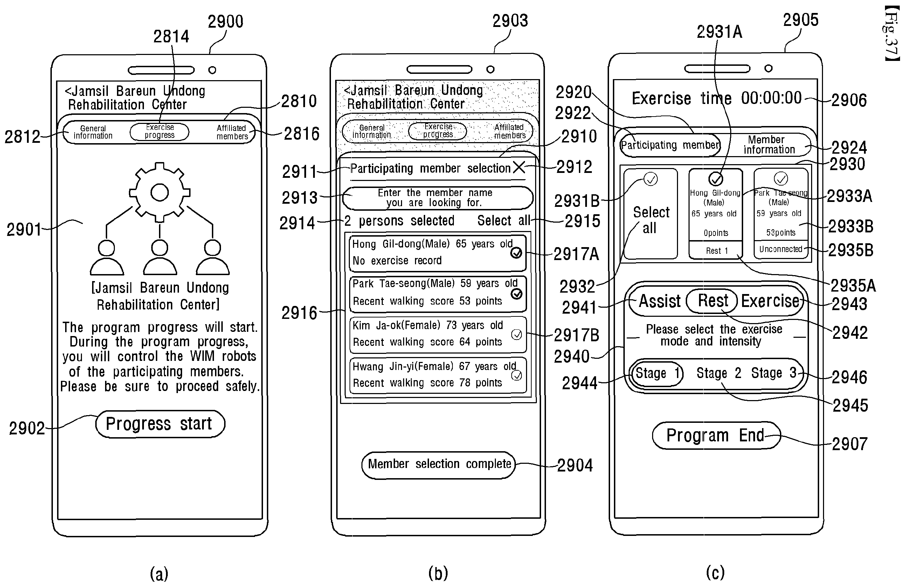

FIG. 37 is a diagram showing a graphical user interface of an administrator terminal representing the process of selecting members to participate in a program generated for 1:N group exercise according to an embodiment of the present invention.

As shown in (a) of FIG. 37, in the case where Exercise Progress (2814) is selected in the category selection area (2810), a graphical user interface (2900) representing before the start of the progress of the program appears. The graphical user interface (2900) representing before the start of the progress of the program may include a guidance picture and phrase display area regarding the program progress (2901) and a Progress Start (2902) button. At this time, the guidance picture and phrase regarding the program progress are exemplary and the picture and phrase may be replaced, and in order to proceed with the program generated by the administrator, the program can be run by selecting the Progress Start (2902) button on the graphical user interface (2900) representing before the start of the progress of the program and then selecting the members who will participate in the program.

As shown in (b) of FIG. 37, when the Progress Start (2902) button is pressed, a graphical user interface (2903) for selecting program participating members appears. The graphical user interface (2903) for selecting program participating members according to an embodiment of the present invention may include a participating member selection area (2910) and a Member Selection Complete (2904) button.

The participating member selection area (2910) according to an embodiment of the present invention may include a participating member selection phrase (2911) and Close (2912), a member name search area (2913), a selected member count display area (2914), Select All (2915), and a selectable member information display area (2916). At this time, the member name search area (2913) may display the information of the member to be selected quickly through a name search, the selected member count display area (2914) may display the number of members selected to participate in the program, and the selectable member information display area (2916) may display basic information such as the name, age, etc., of the selectable member and information regarding walking such as the comprehensive walking score, etc.

For example, it may display basic information such as the gender (male), age (65 years old), etc., of the member 'Hong Gil-dong' and information regarding walking such as the comprehensive walking score (0 points), etc. In addition, when a member is selected, it may change from the selection inactive state (2917B) to the selection active state (2917A), and when the member is selected again, it may reverse from the selection active state (2917A) to the selection inactive state (2917B). At this time, the active and inactive states can be distinguished by a change of color or highlighting, etc. In addition, Close (2912) can cancel selecting the members to participate in the program, and Select All (2915) can change all selectable members to the selection active state (2917A).

When the Member Selection Complete (2904) button on the graphical user interface (2903) for selecting program participating members according to an embodiment of the present invention is selected, the program proceeds targeting the selected members.

As shown in (c) of FIG. 37, in the case where the Member Selection Complete (2904) button is selected, a graphical user interface (2905) displaying the progress status information of the program appears. The graphical user interface (2905) displaying the progress status information of the program may include an exercise time display area (2906), a category selection area (2920), a program participating member information display area (2930), a robot exercise setting area (2940), and a Program End button (2907).

The exercise time display area (2906) according to an embodiment of the present invention may display the exercise time taken in the program progress process.

The category selection area (2920) according to an embodiment of the present invention may include Participating Members (2922) and Member Information (2924). Participating Members (2922) may display the information of the members participating in the program, and Member Information (2924) may display the information of the members participating in the program in detail, and when one of a plurality of members is selected, may display the information about that selected member in detail.

The program participating member information display area (2930) according to an embodiment of the present invention may display the information of the members participating in the program. Specifically, the program participating member information display area (2930) may include Select All (2932), the selection active and inactive states (2931A, B), participating member information (2933), and the status information of the wearable robot of the participating member (2935). The participating member information (2933) may display, for example, basic information such as the gender (male), age (65 years old), etc., of the member 'Hong Gil-dong' and information regarding walking such as the comprehensive walking score (0 points), etc. The status information of the wearable robot of the participating member (2935) may display, for example, the current status information in the program progress process, such as the rest state, the robot exercise state, the unconnected state with the wearable robot, etc.

The robot exercise setting area (2940) according to an embodiment of the present invention includes a walking mode and torque intensity setting area, and can set at least one of a walking mode and a torque intensity. The walking mode may include at least one torque transmission mode, and each torque transmission mode may be a mode that transmits a torque corresponding to the walking purpose. For example, when the wearable robot (200) is used for the purpose of assisting the walking of the wearer, the wearable robot may provide a torque that serves as an assistance force to the wearer, and when it is used for the purpose of strengthening the lower-body muscle strength of the wearer, the wearable robot may provide a torque that serves as a resistance force to the wearer. The torque intensity may include at least one of a plurality of levels that provide differential torque. A specific example regarding the walking mode and torque intensity displayed in the robot exercise setting area (2940) on the graphical user interface is the same as described in detail above in relation to the user terminal (300).

In addition, the plurality of walking modes according to an embodiment of the present invention may be distinguished by mutually different colors. By distinguishing the plurality of walking modes by mutually different colors, it is possible to prevent in advance the walking mode from being controlled incorrectly, and in the case where a mode change is made, the fact that the mode has been changed and the set mode information can be clearly recognized. A specific example is the same as described in detail above.

In addition, the robot exercise setting area (2940) according to an embodiment of the present invention may have its position fixed on the graphical user interface, and the walking mode and torque intensity setting areas included in the robot exercise setting area (2940) may be separated. The specific content is the same as described in detail above.

The Program End button (2907) according to an embodiment of the present invention can end the program in progress.

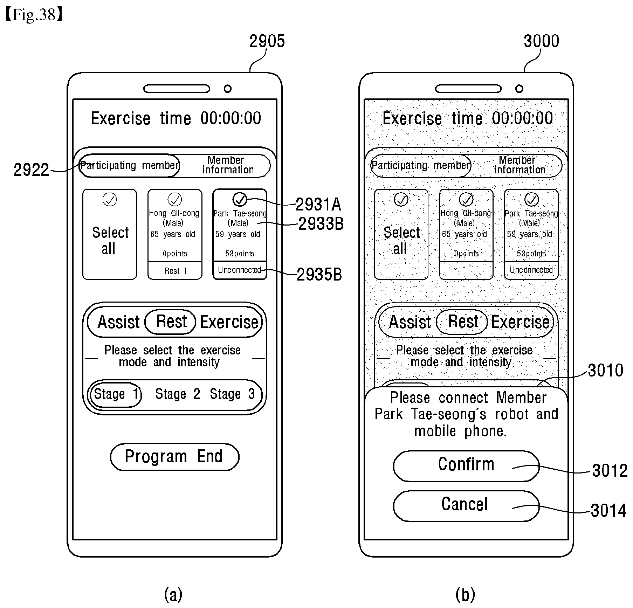

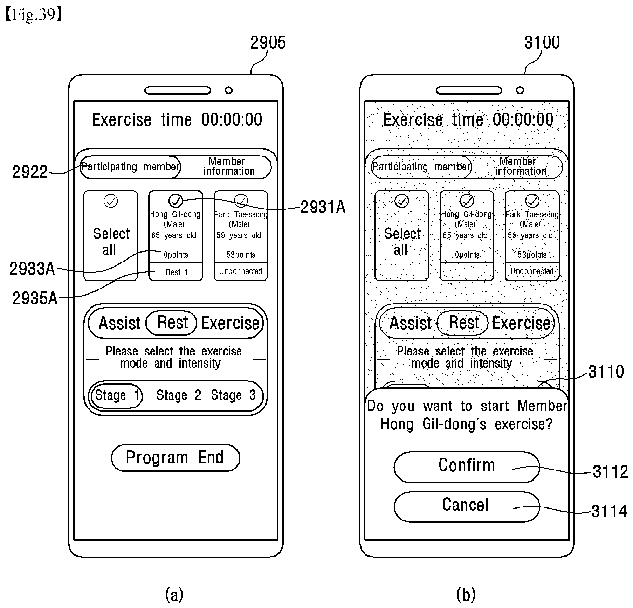

FIG. 38 is a diagram showing a graphical user interface of an administrator terminal representing the process of connecting in the case where, among the members participating in a program for 1:N group exercise, there is no connection with the wearable robot according to an embodiment of the present invention. FIG. 39 is a diagram showing a graphical user interface of an administrator terminal representing the process of starting robot exercise for at least one or more of the members participating in a program for 1:N group exercise according to an embodiment of the present invention.

As shown in FIG. 38, in the case where the wearable robot (200) of a member participating in the program and the administrator terminal (400) are not connected, connection with the wearable robot can be attempted. For example, the process of attempting connection with the wearable robot may include a step of checking the unconnected state between the wearable robot (200) of the member 'Park Tae-seong' and the administrator terminal (400) in the program participating member information display area (2930) on the graphical user interface (2905) displaying the progress status information of the program, a step of selecting the area including the information (2933B) of the member 'Park Tae-seong' and the unconnected state information (2935B) in order to change the unconnected state to the connected state. After selecting the area displaying the information of the member 'Park Tae-seong' to be changed to the connected state, a step in which a graphical user interface (3000) for attempting wearable robot connection appears, and a step of selecting Confirm (3012) in the wearable robot connection attempt display area (3010), which may include Confirm (3012) and Cancel (3014), on the graphical user interface (3000) for attempting wearable robot connection. After such a process is gone through, in the case where the wearable robot (200) of a member participating in the program and the administrator terminal (400) are not connected, the connection with the wearable robot can be completed.

As shown in FIG. 39, robot exercise according to the program can be started for a member who is not performing robot exercise among the plurality of members participating in the program. For example, the process of starting robot exercise according to the program for a member participating in the program may include a step of checking that the wearable robot (200) of the member 'Hong Gil-dong' is in the rest mode state (2935A) so that the member 'Hong Gil-dong' is not performing robot exercise in the program participating member information display area (2930) on the graphical user interface (2905) displaying the progress status information of the program, a step of selecting the area including the information (2933A) of the member 'Hong Gil-dong' and the rest mode state information (2935A) in order to start robot exercise according to the program for the member 'Hong Gil-dong'. After selecting the area displaying the information of the member 'Hong Gil-dong' to start robot exercise, a step in which a robot exercise start setting graphical user interface (3100) for the member appears, and a step of selecting Confirm (3112) in the robot exercise start status display area of the participating member (3110), which may include Confirm (3112) and Cancel (3114), on the robot exercise start setting graphical user interface (3100) for the member. After such a process is gone through, robot exercise according to the program can be started for a member who is not performing robot exercise.

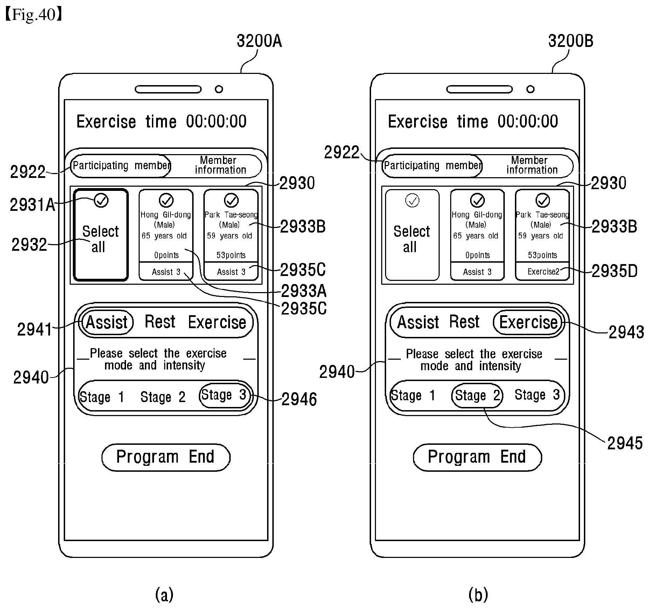

FIG. 40 is a diagram showing a graphical user interface of an administrator terminal in which the walking mode and torque intensity at the start of exercise of each member in a program for 1:N group exercise are set according to an embodiment of the present invention.

In the robot exercise setting area (840) on the robot exercise graphical user interface (800) in which the walking information of the user terminal (300) according to an embodiment of the present invention described in detail above is displayed, the wearable robot wearer can set at least one of the walking mode and torque intensity. Likewise, the administrator can also set at least one of the walking mode and torque intensity of the wearable robot (200) worn by the member performing robot exercise according to the program.

In addition, according to an embodiment of the present invention, the administrator can set at least one of the walking mode and torque intensity based on the comprehensive walking score of the member among the participating member information (2933) of the program participating member information display area (2930). For example, in the case where the comprehensive walking score is between 0 points and 79 points, it may be preferable to set the assistance mode. At this time, the torque intensity is not particularly limited, and can be flexibly set according to the robot exercise process. In the case where the comprehensive walking score is between 80 points and 86 points, it may be preferable to set the exercise mode and the 1st-level torque intensity. In the case where the comprehensive walking score is between 87 points and 93 points, it may be preferable to set the exercise mode and the 2nd-level torque intensity. In the case where the comprehensive walking score is between 94 points and 100 points, it may be preferable to set the exercise mode and the 3rd-level torque intensity. Such a walking mode and torque intensity setting criterion per comprehensive walking score is based on experimental values for 35 elderly people wearing wearable robots and standard data for walking ability measurement, this is merely a simple example, and the setting criterion may be changed as data of information regarding the walking of a greater number of wearable robot wearers is secured.
(a) of FIG. 40 is an exemplary diagram of a graphical user interface (3200A) in which, on the program participating member information display area (2930) on the graphical user interface (2905) displaying the progress status information of the program according to an embodiment of the present invention, Select All (2932) is selected, and the walking mode and torque intensity are set to assistance mode (2941) and 3rd-level (2946) in the robot exercise setting area (2940), while the status information of the wearable robots (2935) of the member 'Hong Gil-dong' and the member'Park Tae-seong' is each displayed as the assistance mode and 3rd-level intensity status information (2935C).
(b) of FIG. 40 is an exemplary diagram of a graphical user interface (3200B) in which the area including the information (2933A) of the member 'Park Tae-seong' and the rest mode state information (2935A) is selected in the program participating member information display area (2930), and the walking mode and torque intensity are set to exercise mode (2943) and 2nd-level (2945) in the robot exercise setting area (2940), while the status information of the wearable robot (2935) of the member 'Park Tae-seong' is each displayed as the exercise mode and 2nd-level intensity status information (2935D).

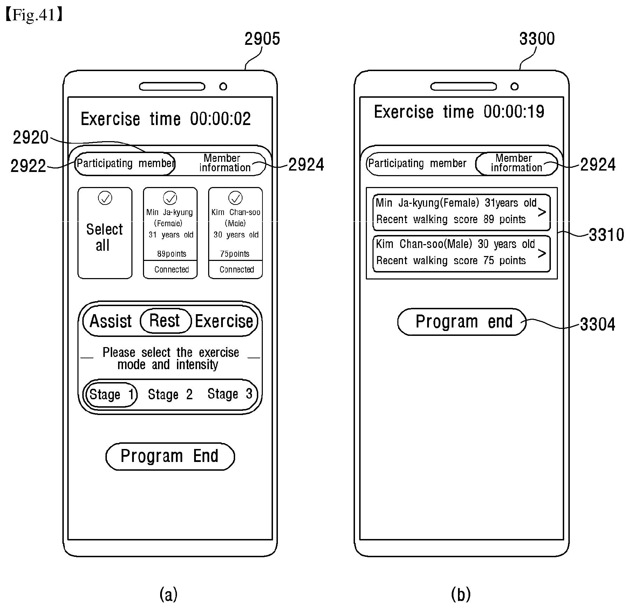

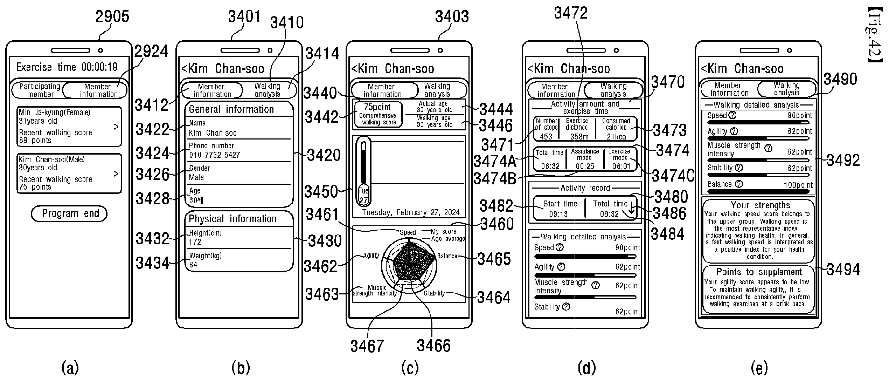

FIG. 41 is a diagram showing a graphical user interface that can check the walking information of a member participating in a program for 1:N group exercise through an administrator terminal according to an embodiment of the present invention. FIG. 42 is a diagram showing a graphical user interface that can check the basic information and walking analysis report of one of the members participating in a program for 1:N group exercise through an administrator terminal according to an embodiment of the present invention.

As shown in FIG. 41, when Member Information (2924) is selected in the category selection area (2920) on the graphical user interface (2905) displaying the progress status information of the program according to an embodiment of the present invention, a graphical user interface (3300) in which the information of the members participating in the program is displayed appears.

The graphical user interface (3300) in which the information of the members participating in the program is displayed according to an embodiment of the present invention may include the category selection area (2920), a program participating member information display area (3310), and Program End (3304). At this time, the program participating member information display area (3310) may include basic information such as the name, gender, etc., of the participating member and information regarding walking such as the comprehensive walking score, etc., and when the information display area of each member is selected, a graphical user interface displaying the detailed information about the selected member appears.

As shown in (a) and (b) of FIG. 42, the graphical user interface (3300) in which the information of the members participating in the program is displayed according to an embodiment of the present invention may include a program participating member information display area (3310). At this time, when one information display area among a plurality of member information display areas is selected, a graphical user interface (3401) displaying the detailed information about the member appears.

The graphical user interface (3401) displaying the detailed information about the member according to an embodiment of the present invention may include a category selection area (33410), a basic information display area (3420), and a physical information display area (3430). The category selection area (3410) may include Member Information (3412) displaying the detailed information of the member and Walking Analysis (3414) displaying the walking analysis report of the member. The basic information display area (3420) may include a phone number information display area (3422), a name information display area (3424), a gender information display area (3426), and a date-of-birth information display area (3428), and the physical information display area (3430) may include a height (cm) information display area (3432) and a weight (kg) information display area (3434). However, the detailed content included in the basic information display area (3420) and the physical information display area (3430) may be changed.

As shown in (c) to (e) of FIG. 42, when Walking Analysis (3414) is selected in the category selection area (3410), a graphical user interface (3403) displaying the walking analysis report for the selected member appears. The graphical user interface (3401) displaying the walking analysis report for the selected member may include at least one of a comprehensive walking score and age information display area (3440), a statistical information display area according to accumulated walking information (3450), a walking index schematization area (3460), an entire walking information display area of the selected date (3470), a per-walking record information display area of the selected date (3480), and a walking detailed analysis information display area (3490).

The comprehensive walking score and age information display area (3440) according to an embodiment of the present invention may include a comprehensive walking score display area of the wearer (3442), an actual age information display area (3444), and a walking age information display area (3446), and the information displayed in the comprehensive walking score display area of the wearer (3442) and the walking age information display area (3446) may be information generated based on statistical information according to the accumulation of real-time walking information and physical/exercise ability information according to the long-term use of the wearer.

The statistical information display area according to accumulated walking information (3450) according to an embodiment of the present invention may be represented by a bar or chart so that the statistical information according to accumulated walking information can be easily known, the bar or chart may include an average trend line representing the average of the statistical information according to accumulated walking information, and may have a plurality of areas according to dates. The plurality of areas according to dates appear in proportion to the statistical information according to accumulated walking information, and may have a relative size compared with other dates. The relative size may be displayed by a bar length, a gauge bar, a ratio, or a graph, etc., and is not limited to what is shown in (c) of FIG. 42, and may be replaced as long as it can be displayed so that the information displayed in the plurality of areas according to dates can be easily identified.

In addition, when one area indicating a certain date on the plurality of areas according to dates is selected, the selected area may be distinguished from the other plurality of areas by having a different color or being highlighted, etc., and information regarding walking according to the robot exercise process of the selected date may be displayed in another area on the graphical user interface. In addition, the statistical information according to accumulated walking information may represent statistical information about the accumulation regarding the per-date walking score, but is not limited thereto.

The walking index schematization area (3460) according to an embodiment of the present invention may include a score for each walking index, a walking index graph of the user (3466) connecting the scores for each walking index, and an age-average walking index graph (3467) connecting the scores for each walking index according to the age average. The above area is one that schematizes the score for each walking index of the wearer, and may represent the walking ability of the wearer with a figure so that the score for each walking index of the wearer and the score for each walking index according to the age average can be compared.

Specifically, methods of schematizing the score for each walking index of the wearer may include a bar graph, a line graph, a pie graph, a scatter plot, a histogram, a box plot, a heatmap, etc., and such methods are not limited as long as the score for each walking index of the wearer and the score for each walking index according to the age average can be easily compared. In addition, the score for each walking index may be a score of speed (3461), agility (3462), muscle strength intensity (3463), stability (3464), and balance (3465). However, if there is another factor that is important in measuring and analyzing the physical/exercise ability regarding the walking of the wearer, it may be added, changed, or deleted in addition to the five walking indices mentioned above.

The entire walking information display area of the selected date (3470) according to an embodiment of the present invention may include at least one of a number-of-steps information display area (3471), a distance information display area (3472), a consumed-calorie information display area (3473), and a walking-time information display area (3474). The number-of-steps information display area (3471) may display information related to the number of steps of the user based on the entire operation information of the wearable robot worn by the user on the selected date. The distance information display area (3472) may display information related to the exercise distance of the user based on the entire operation information of the wearable robot worn by the user on the selected date. The consumed-calorie information display area (3473) may display information related to the consumed calories of the user based on the entire operation information of the wearable robot worn by the user on the selected date. The walking-time information display area (3474) may include an entire-time information display area (3474A), an assistance-mode time information display area (3474B), and an exercise-mode time information display area (3474C).

In the walking-time information display area (3474) according to an embodiment of the present invention, the entire-time information display area (3474A) may display information related to the operating time of the user based on the entire operation information of the wearable robot worn by the user on the selected date, and the operating time may be the sum of the use times in the assistance mode, the rest mode, and the exercise mode. The assistance-mode time information display area (3474B) is an area for displaying the operating time of the wearable robot (200) in the assistance mode state out of the entire-time information, and the exercise-mode time information display area (3474C) is an area for displaying the operating time of the wearable robot (200) in the exercise mode state out of the entire-time information. However, the information included in the entire walking information display area of the selected date (3470) is exemplary, and it may also include information such as step length, step pattern, continuous walking time, walking rhythm, slope, number of steps when ascending or descending stairs, and number of floors, etc., based on the operation information of the wearable robot worn by the user.

The per-walking record information display area of the selected date (3480) according to an embodiment of the present invention may include a plurality of areas (3482, 3484) in which the per-walking records of the selected date are displayed. The number of the plurality of areas is determined by the number of walkings performed on the selected date, and the plurality of areas (3482, 3484) may include a start-time information display area (3482) and an entire-time information display area (3484). However, this is exemplary, and information regarding walking such as the number of steps, exercise distance, etc., may be included instead. In addition, in the per-walking record information display area of the selected date (3480), when an arrow (3486) that activates the detailed walking record information disposed for each area among the plurality of areas (3482, 3484) is selected, a per-walking detailed record information display area (not shown in the drawings) based on the arrow input information of one area appears, and each of the per-walking records including at least one of the number of steps, exercise distance, consumed calories, entire time, assistance-mode time, and exercise-mode time may be disposed in the per-walking detailed record information display area within the plurality of areas (3482, 3484). When an arrow (not shown in the drawings) meaning closing the detailed walking record disposed in the plurality of areas (3482, 3484) is selected, the per-walking detailed record information display area disappears.

The walking detailed analysis information display area (3490) according to an embodiment of the present invention may include at least one of a score-per-walking-index display area (3492) and a feedback display area according to the score for each walking index (3494). As mentioned above, the score for each walking index displayed in the score-per-walking-index display area (3492) is not limited to what is shown in (d) and (e) of FIG. 34, and the score for each index may be displayed only with numbers, or may be displayed in various ways, such as a bar graph or a linear graph, so that the wearable robot wearer can easily check his or her own score for each walking index. The feedback display area according to the score for each walking index (3494) may analyze the score for each walking index of the wearer to include feedback appropriate for the wearer, and specifically, the feedback of the wearer may include the strengths and points to be supplemented of the walking ability of the wearer, problems that may occur on the body if the walking ability of the wearer is maintained, or an introduction of a method or program for acquiring a walking ability suited to the age of the wearer, etc.

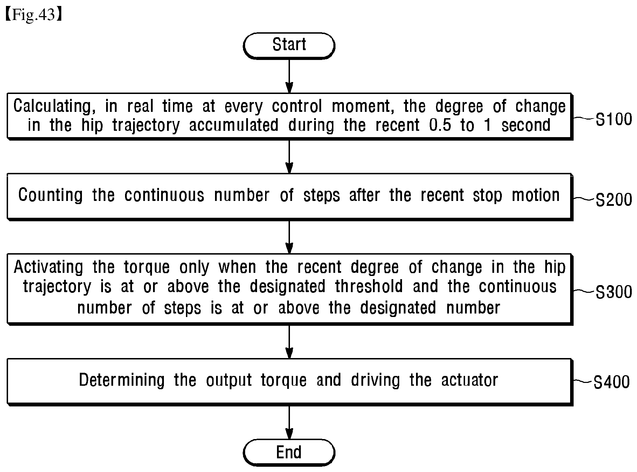

FIG. 43 is a diagram showing a device control method for safely performing various tasks other than walking during walking assistance of the wearer according to the present invention.

As shown, the device control method for safely performing various tasks other than walking during walking assistance of the wearer may include a step of calculating, in real time at every control moment, the degree of change in the hip trajectory accumulated during a predetermined time. Specifically, it may include a step (S100) of calculating, in real time at every control moment, the degree of change in the hip trajectory accumulated during a time of 0.5 seconds to 1 second or less.

According to the present invention, the wearable robot (200) includes a drive unit (220), and the drive unit (220) may be configured to calculate the degree of change of the state trajectory of the wearer accumulated during a predetermined time and to compute an output torque based thereon. Specifically, the drive unit (220) may be configured to calculate the degree of change of the state trajectory of the wearer accumulated during a time of 0.5 seconds or more and 1 second or less and to compute an output torque based thereon.

Hereinafter, in order to explain the step (S100) of calculating, in real time at every control moment, the degree of change in the hip trajectory accumulated during a time of 0.5 seconds to 1 second or less, the hip state trajectory memory buffer of FIG. 5a is explained.

The wearable robot (200) according to the present invention may estimate whether or not a walking motion is performed using the state variable history information stored in the state trajectory memory buffer (300). For example, in the case where a fast hip motion change is made for a short time to the wearable robot (200), the state trajectory moving distance (d0) value is calculated to be large, and during a stopping motion, the state trajectory moving distance (d0) value may be calculated to be a value close to 0 or a value of 0.

### Device torque deactivation

According to the present invention, in the case where the state trajectory moving distance (d0) value during a predetermined time is at or below a first threshold, the drive unit (220) may be configured to not compute an output torque or to compute it as a value at or below the threshold. As a specific example, in the case where the wearer of the wearable robot (200) stops in order to pick up trash during walking, the drive unit (220) can calculate, in real time, the degree of change of the hip state trajectory accumulated during a predetermined time within 1 second. In this case, since the calculated state trajectory moving distance (d0) value of the stopped wearer may be computed to be at or below the first threshold, the drive unit (220) may not compute the output torque or may compute it as a value at or below the threshold. Accordingly, since the wearable robot (200) does not compute an output torque while the wearer is stopped, the wearer, when performing a task motion that is not walking in the stopped state, does not receive a separate output torque, and thus may not feel inconvenience in the task motion.

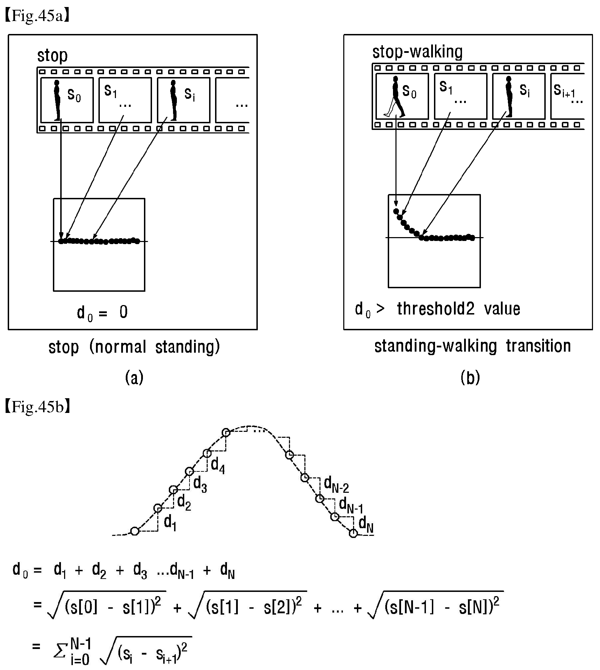

FIG. 45a is a diagram showing the state trajectory in the case where a walker stops and the case where a walker proceeds with walking after stopping according to an embodiment of the present invention.

As shown in (a) of FIG. 45a, in the case where the wearer of the wearable robot (200) is in a stopping motion, the state trajectory moving distance (d0) value may be calculated to be a value close to 0 or a value of 0.

### Device torque activation

As shown in (b) of FIG. 45a, there is a case where the wearer of the wearable robot (200) starts walking again after stopping. At this time, the drive unit (220) may be configured to, in the case where the state trajectory moving distance (d0) of the wearer during a predetermined time is at or above a second threshold, compute the output torque as a value at or above the threshold. As a specific example, in the case where the second threshold is 2.0, in the case where the wearer walks after stopping so that the state trajectory moving distance (d0) during a predetermined time is at or above 2.0, the drive unit (220) may be configured to compute the output torque as a value at or above the threshold. Even more specifically, in the case where the wearer walks after stopping so that the state trajectory moving distance (d0) during a time of 0.5 seconds or more and 1.0 second or less is at or above 2.0, the drive unit (220) may be configured to compute the output torque as a value at or above the threshold.

According to yet another embodiment of the present invention, the drive unit (220) may be configured to compute the output torque as a value at or above the threshold not only in the case where the state trajectory moving distance (d0) of the wearer during a predetermined time is at or above the second threshold, but also in the case where the wearer walks for at or above the continuous number of steps after stopping, that is, in the case where the state trajectory moving distance (d0) of the wearer is at or above the second threshold and the wearer walks for at or above the continuous number of steps after stopping.

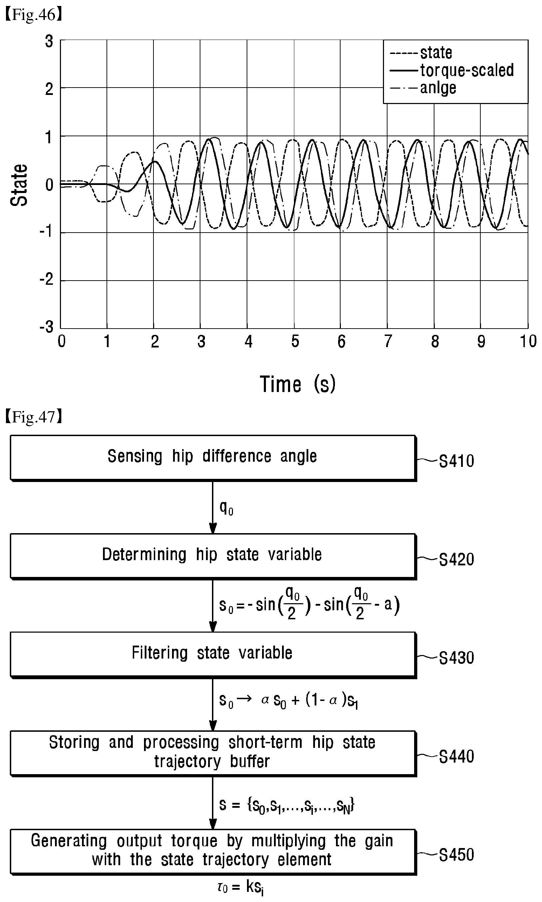

FIG. 46 is a diagram for explaining continuous number-of-steps counting according to an embodiment of the present invention.

As shown, the x-coordinate of FIG. 46 represents time and the y-coordinate represents torque (Nm), and the motion state value (hip state) of the wearer and the output torque (torque) during the walking of the wearer over time can be represented in a graph.

As shown in FIG. 43, the drive unit (220) may count (S200) the continuous number of steps from after the most recent stopping motion in order to compute the output torque as a value at or above the threshold. Specifically, the time point at which the motion state value (hip state) of the wearer during the walking of the wearer passes y=0 can be counted. That is, the number of steps of the wearer can be accurately counted from after the walking of the wearer is stopped.

According to an embodiment of the present invention, the time point at which the motion state value (hip state) of the wearer during the walking of the wearer passes y=0, that is, the zero cross point, may be the time point at which the one-side target body part and the other-side target body part of the wearer intersect. Alternatively, the number of steps of the wearer may be counted by designating a predetermined value other than the time point of passing y=0 and counting the event of passing through it.

According to an embodiment of the present invention, in the case where the wearer performs a task other than walking during walking, the hip movement may slow down or stop. As described above, in the case where the state trajectory moving distance (d0) value during a predetermined time is at or below the first threshold, the drive unit (220) may be configured to not compute the output torque or to compute it as a value at or below the threshold. (S100) In this case, the number of steps of the wearer can be reset to 0, and in the case where a walking motion is performed again after the task is completed, the continuous number of steps is counted, and in the case where the number of steps is at or above the threshold, the drive unit (220) can finally activate the device torque.

According to an embodiment of the present invention, in the case where the state trajectory moving distance (d0) of the wearer is at or above the second threshold and the wearer walks for at or above the continuous number of steps after stopping, the drive unit (220) may be configured to compute the output torque as a value at or above the threshold. As a specific example, in the case where the second threshold is set to 2.0 and the set continuous number of steps is 2 steps, in the case where the wearer starts walking again after stopping so that the state trajectory moving distance (d0) of the wearer is at or above 2.0 and the wearer walks 2 steps after stopping so that the counting at which the motion state value (hip state) of the wearer passes y=0 is performed 2 or more times, the drive unit (220) may be configured to compute the output torque as a value at or above the threshold.

### Torque intensity adjustment factor

According to the present invention, in the case where the wearer walks for at or above the continuous number of steps, the drive unit (220) may be configured to compute the output torque as a value at or above the threshold. In addition, the calculated state trajectory moving distance (d0) value of the stopped wearer is computed to be at or below the first threshold, so that the drive unit (220) may not compute the output torque or may compute it as a value at or below the threshold. In this case, the drive unit (220) may change the torque intensity adjustment parameter (k) value shown in FIG. 47 to a value at or below the threshold or change it to 0 in order to perform torque deactivation.

According to an embodiment of the present invention, the drive unit (220) may change the torque intensity adjustment parameter (k) value to a value of 2 or less or gradually change it to 0 in order to perform torque deactivation. Accordingly, when the wearer stops during walking, the provision of the output torque of the wearable robot (200) does not stop instantaneously, but the provision of the output torque gradually decreases, so that a smooth wearing sensation can be provided to the wearer.

According to an embodiment of the present invention, the drive unit (220) may gradually restore the torque intensity adjustment parameter (k) value to a set value in order to perform torque activation. As a specific example, in the case where the set torque intensity adjustment parameter (k) value is 2.0, in the case where the continuous number of steps of the wearer is detected after stopping and the state trajectory moving distance (d0) value is at or above the second threshold, the drive unit (220) gradually restores it to the set torque intensity adjustment parameter (k) value of 2.0 so that the provision of the output torque gradually increases, so that a smooth wearing sensation can be provided to the wearer.

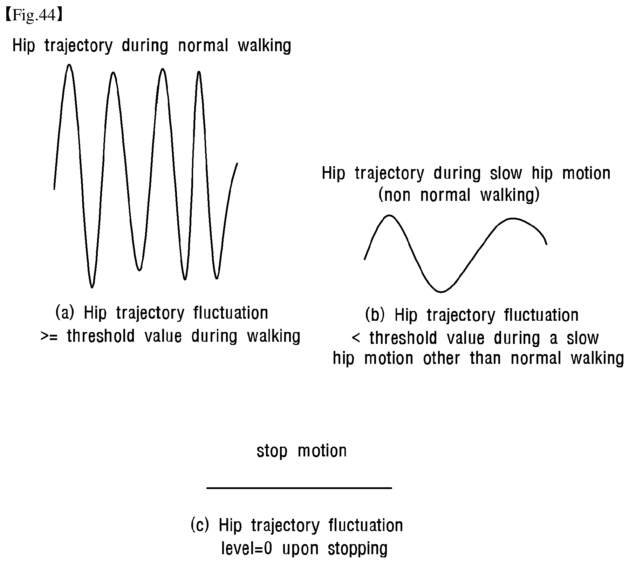

FIG. 44 is a diagram for explaining a comparison per hip state trajectory motion according to an embodiment of the present invention.

As shown in (a) of FIG. 44, in the case where the wearer walks with a normal step, the state trajectory moving distance (state trajectory fluctuation level, d0) may be greater than the threshold or equal to the threshold.

As shown in (b) of FIG. 44, in the case where the wearer performs a slow hip motion that is not a normal step, the state trajectory moving distance (state trajectory fluctuation level, d0) may be smaller than the threshold.

As shown in (c) of FIG. 44, in the case where the wearer stops, the state trajectory moving distance (state trajectory fluctuation level, d0) may represent a value of 0.

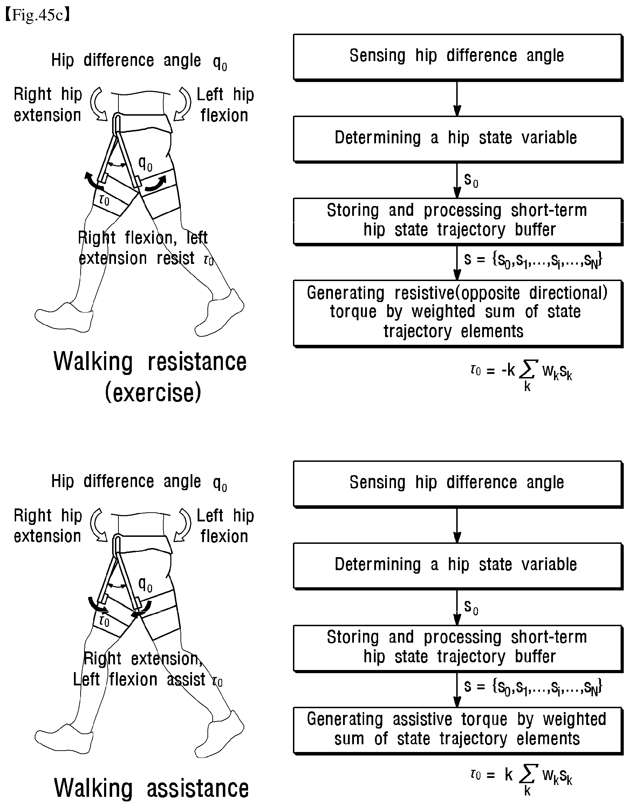

FIG. 45b is a diagram for explaining the state trajectory moving distance according to an embodiment of the present invention. FIG. 45c is a diagram for explaining the torque intensity adjustment factor according to an embodiment of the present invention. FIG. 47 is a diagram for explaining the walking assistance output algorithm according to the present invention.

As shown in FIG. 45b, the state trajectory moving distance (d0) may be expressed as the sum of several short state trajectory moving distances (d1, d2, ..., dN), and each state trajectory moving distance di may be the moving distance between consecutive states. That is, each state trajectory moving distance di may be the square root taken of the square of the difference between each motion state value (Si), and the state trajectory moving distance (d0) can be computed by summing them.

According to an embodiment of the present invention, in order to provide a driving force to the wearer of the wearable robot (200), a movement detection sensor (not shown) of the drive unit (220) may perform a step (S410) of sensing the hip angle of the wearer of the wearable robot (200). A processor unit (not shown) of the drive unit (220) may compute the angular velocity based on the sensed hip angle. Specifically, the movement detection sensor (not shown) senses the hip angle of the wearer, and the processor unit (not shown) of the drive unit (220) may compute the angular velocity by differencing and filtering the hip angle sensed by the movement detection sensor (not shown) at a speed of 1000 Hz or more. That is, according to the present invention, even without using a separate tachometer for sensing the angular velocity, the angular velocity can be computed through hip angle sensing. Even more specifically, the processor unit (not shown) performs real-time control, the processor unit (not shown) may be divided into a low-level processor and a high-level processor, and the angular velocity may be computed by differencing and filtering the hip angle sensed by the movement detection sensor (not shown) at a speed of 1000 Hz or more in the low-level processor that has a relatively small amount of computation.

As shown in FIG. 45c, the torque output in the step (S450) of outputting a torque for assisting the walking of the wearer of the wearable robot (200) or a torque for resisting may be a torque computed by computing (S440) a plurality of motion state values based on the angular velocity computed by the drive unit (220), selecting at least one motion state value among them, and multiplying the weighted sum of the selected motion state value by the torque intensity adjustment factor (k). At this time, the computed plurality of motion state values may be stored by repeating a shift operation through the state trajectory memory buffer (300). Hereinafter, the plurality of motion state values stored by repeating the shift operation through the state trajectory memory buffer (300) are described.

As shown in FIG. 47, in order to provide a driving force to the wearer of the wearable robot (200), a step (S450) of outputting, in the processor unit (not shown) of the drive unit (220), a torque for assisting the walking of the wearer or a torque for resisting may be performed. Specifically, the step (S450) of outputting a torque for assisting the walking of the wearer or a torque for resisting may include a step (S420) of determining a motion state value. In addition, it may further include a step (S430) of filtering the motion state value. In addition, it may further include a step (S440) of deleting the previously stored oldest motion state value from the memory.

According to the present invention, in the case where the wearer walks for at or above the continuous number of steps, the drive unit (220) may be configured to compute the output torque as a value at or above the threshold. In addition, the calculated state trajectory moving distance (d0) value of the stopped wearer is computed to be at or below the first threshold, so that the drive unit (220) may not compute the output torque or may compute it as a value at or below the threshold. In this case, the drive unit (220) may change the torque intensity adjustment parameter (k) value shown in FIG. 47 to a value at or below the threshold or change it to 0 in order to perform torque deactivation.

According to an embodiment of the present invention, the drive unit (220) may change the torque intensity adjustment parameter (k) value to a value of 2 or less or gradually change it to 0 in order to perform torque deactivation. Accordingly, when the wearer stops during walking, the provision of the output torque of the wearable robot (200) does not stop instantaneously, but the provision of the output torque gradually decreases, so that a smooth wearing sensation can be provided to the wearer.

According to an embodiment of the present invention, the drive unit (220) may gradually restore the torque intensity adjustment parameter (k) value to a set value in order to perform torque activation. As a specific example, in the case where the set torque intensity adjustment parameter (k) value is 2.0, in the case where the continuous number of steps of the wearer is detected after stopping and the state trajectory moving distance (d0) value is at or above the second threshold, the drive unit (220) gradually restores it to the set torque intensity adjustment parameter (k) value of 2.0 so that the provision of the output torque gradually increases, so that a smooth wearing sensation can be provided to the wearer.

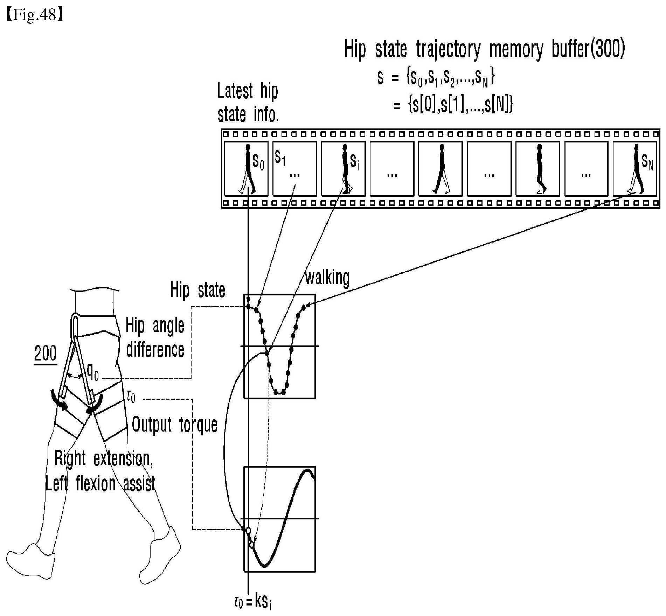

FIG. 48 is a diagram for explaining a state feedback control-based assistance method according to the present invention.

As shown in FIG. 48, the state trajectory memory buffer (300) is a storage device that sequentially stores the motion state values of the wearer. Specifically, when the first motion state value is input, it is stored in the first storage position of the memory array. Next, when a new motion state value is input, the motion state value that was stored in the first storage position of the memory array is moved to the second storage position of the memory array, and the newly input motion state value is stored in the first storage position of the memory array. Then, when a new motion state value is input, the motion state values that were respectively stored in the first storage position and the second storage position of the memory array are moved to the second storage position and the third storage position of the memory array, and the newly input motion state value is stored in the first storage position of the memory array.

When such a shift operation is repeated, N+1 motion state values are stored in all the storage positions of the memory array. Accordingly, when a new motion state value is input, the motion state values stored in the memory array are moved to the next position, but the motion state value stored in the last storage position (N), which has no more place to move, is deleted from the state trajectory memory buffer (300).

By storing the motion state values in such a FIFO (First In First Out) manner, only a preset number of motion state values regarding the recent movement of the wearer can be stored in the state trajectory memory buffer (300).

In addition, the motion state value (s0) stored in the state trajectory memory buffer (300) may be a sensing value (q0) estimated by a sensor or a converted value of a sensing value that can show the motion state of the wearer. As a specific example, the hip angle difference value (q0) may be the angle value (q0) between the one-side target body part and the other-side target body part of the wearer estimated by the sensor of the wearable robot (200). That is, as shown in FIG. 7, the step (S410) of sensing the hip angle difference value (q0) may be performed first. The angle value (q0) between the one-side target body part and the other-side target body part of the wearer estimated by the sensor of the wearable robot (200) may itself be the motion state value (s0).

According to an embodiment of the present invention, the motion state value stored in the state trajectory memory buffer (300) may represent the movement of the wearer during a recent short time (within several seconds, for example, within 1 to 2 seconds). For example, the motion state value stored in the state trajectory memory buffer (300) may include only one motion information of the wearer (a half-cycle of a repetitive movement). Even if the motion state value stored in the state trajectory memory buffer (300) includes only one motion of the wearer, that is, a half-cycle of a repetitive movement, the practice of the present invention is possible.

Preferably, as the motion state value stored in the state trajectory memory buffer (300), a motion state value for a movement including one cycle among the repetitive movements of the wearer may be stored. For example, in the case where the wearable robot (200) according to an embodiment of the present invention is worn on a lower-body part such as the waist or thigh, the one-cycle motion of the wearer may be a one-cycle walking motion of the wearer (e.g., a step motion, etc.).

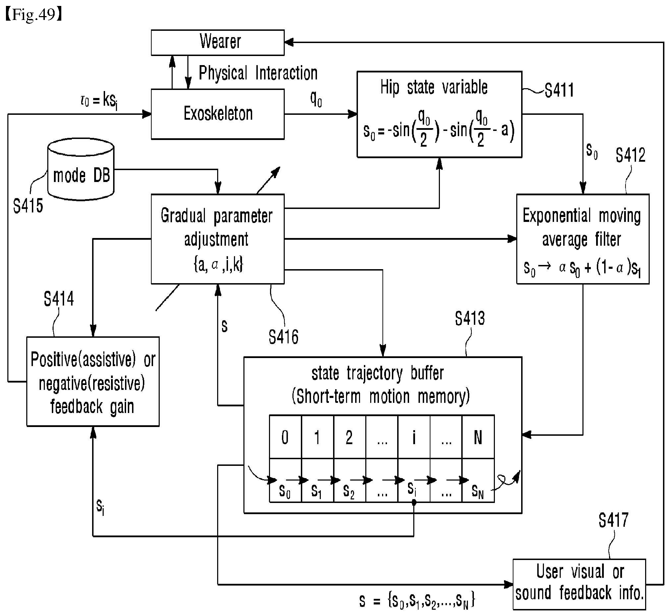

FIG. 49 is a diagram for explaining the output torque algorithm according to the present invention. Hereinafter, the output torque computation of the drive unit (220) of FIG. 47 is described in more detail.

As shown, the current hip angle information (q0) sensed from the Exoskeleton may be converted into the motion state value (S0), which is the hip state variable. (S411)

According to an embodiment of the present invention, the drive unit (220) may provide a difference of the output torque between the walking of the one-side target body part and the walking of the other-side target body part by using an asymmetry parameter a on the converted motion state value (S0). Specifically, if a=0, it means there is no degree of asymmetry, and the same torque may be provided symmetrically to the left and right motions. If the a value is negative, the assistance torque is increased for the motion of the left-side motion compared with the motion of the right-side motion. If the a value is positive, the assistance torque is increased for the motion of the right-side motion compared with the motion of the left-side motion. That is, the asymmetric assistance for the left-side or right-side motion can be adjusted with the single parameter a.

According to an embodiment of the present invention, the asymmetry of the left/right motion is determined by the sign of the a value, and the degree of asymmetry can be adjusted by the magnitude of the a value. For instance, in the case where a=0.20 compared with when a=0.10, the right-side asymmetric assistance is further strengthened. The a value may be within the range of -1.0 to 1.0, and a value of -0.5 to 0.5 is preferably used.

According to an embodiment of the present invention, the converted motion state value (S0) may pass through an Exponential moving average filter. (S412) That is, the drive unit (220) can adjust the cutoff frequency of the signal using a filter parameter. The -3 dB cutoff frequency (the frequency at which the signal power decreases by half, denoted by ) can be represented by the following [Mathematical Formula 8]. [Mathematical Formula 8] ${f}_{c} = \frac{{f}_{s}}{2 π} {cos}^{- 1} \left(1-\frac{{α}^{2}}{2 \left(1-α\right)}\right) , {f}_{s} = sampling frequency$

Here, fₛ represents the sampling frequency. According to the present invention, the filtered motion state value (S₀) may be stored and updated in the state trajectory memory buffer (300) (S440, S413), and at every control moment, the recent movement information of the hip state s0 is stored in a motion memory buffer of finite size and may be updated at every moment. Accordingly, the drive unit (220) may compute the output torque value based on the filtered motion state value (S₀). (S450)

According to the present invention, it is also possible to adaptively adjust, to the wearer's movement characteristics, a calculation control parameter (the parameter of S416) using the state trajectory moving distance (d₀) for the motion speed of the sensed signal. In addition, modes specialized for various workers/users may be stored in the form of a lookup table in the wearable robot (200), an external remote device, an external server, etc., and when selected through the interface of the user terminal (100), the stored parameters are received so that the drive unit (220) may compute the output torque according to the parameters of the designated mode.

According to the present invention, the parameter setting value by the changed mode may be reflected by gradually changing it at every control moment with the currently set parameter value as the starting point. (S416) Therefore, the setting corresponding to the selected exercise mode set in the mode database (S415) does not change immediately when the user changes to the selected exercise mode using the interface of the user terminal (100), but may be finally reflected to the drive unit (220) after a predetermined time. That is, the drive unit (220) can safely switch to the walking assistance mode or the walking resistance mode without stopping walking even during movement, through the state trajectory memory buffer (300) and the gradual parameter change (S416).

According to an embodiment of the present invention, the drive unit (220) can apply the switching to the walking assistance mode or the walking resistance mode as one integrated control rule without separate controller switching, by changing the sign of the torque intensity adjustment parameter (k). Accordingly, the wearable robot (200) can apply an output torque of a predictable (consistent) form to the wearer. Accordingly, the present invention can enable safe walking assistance/resistance for the wearer.

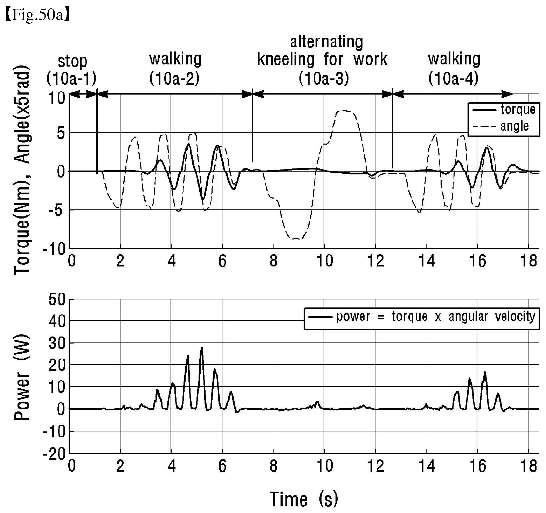

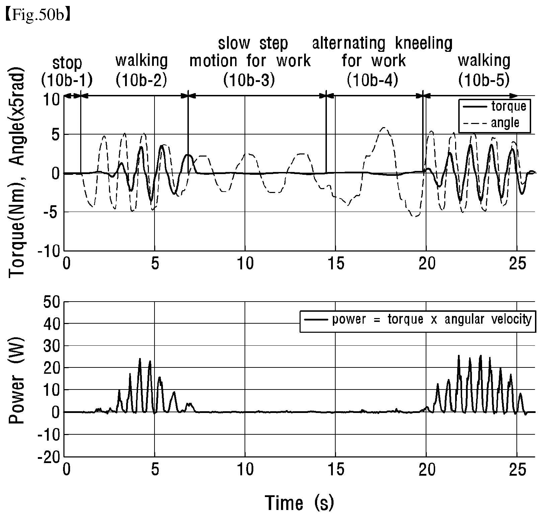

FIG. 50a is a diagram showing the change of the torque value when walking again after a task other than walking during walking according to the present invention. FIG. 50b is a diagram showing the change of the torque value when performing slow walking during walking, and walking again after a task other than walking according to the present invention.

As shown in FIG. 50a, in the state where the wearer has stopped (10a-1), the hip angle difference (angle) of the wearer does not occur and the output torque (torque) does not occur. Thereafter, in the case where the wearer starts walking (10a-2), the hip angle difference (angle) of the wearer begins to occur, but the output torque (torque) is not computed immediately according to the hip angle difference (angle) of the wearer, and the output torque (torque) may be gradually computed from after at or above the threshold number of steps. Thereafter, during a one-knee kneeling motion for a task (10a-3), since the state trajectory moving distance (d0) value during a predetermined time is at or below the threshold, the drive unit (220) can reduce the output torque (torque) by gradually decreasing the torque intensity adjustment parameter (k). Thereafter, in the case where the walking of the wearer starts again (10a-4), in the case where the continuous number of steps is at or above the threshold, the drive unit (220) can compute the output torque (torque) by gradually restoring the torque intensity adjustment parameter (k). Accordingly, since the power computed by the drive unit (220) can also be smoothly provided to the movement of the wearer, according to the present invention, the wearer can be provided with a smooth output torque.

As shown in FIG. 50b, in the state where the wearer has stopped (10b-1), the hip angle difference (angle) of the wearer does not occur and the output torque (torque) does not occur. Thereafter, in the case where the wearer starts walking (10b-2), the hip angle difference (angle) of the wearer begins to occur, but the output torque (torque) is not computed immediately according to the hip angle difference (angle) of the wearer, and the output torque (torque) may be gradually computed from after at or above the threshold number of steps.

Thereafter, the wearer can walk slowly (10b-3) in order to perform a task, and since the state trajectory moving distance (d0) value during a predetermined time is at or below the threshold, the drive unit (220) can reduce the output torque (torque) by gradually decreasing the torque intensity adjustment parameter (k). In addition, during a one-knee kneeling motion for a task after slow walking (10b-4), since the state trajectory moving distance (d0) value during a predetermined time is likewise at or below the threshold, the drive unit (220) can reduce the output torque (torque) by gradually decreasing the torque intensity adjustment parameter (k). Thereafter, in the case where the walking of the wearer starts again (10b-5), in the case where the continuous number of steps is at or above the threshold, the drive unit (220) can gradually compute the output torque (torque) by gradually restoring the torque intensity adjustment parameter (k). Accordingly, since the power computed by the drive unit (220) can also be smoothly provided to the movement of the wearer, according to the present invention, the wearer can be provided with a smooth output torque.

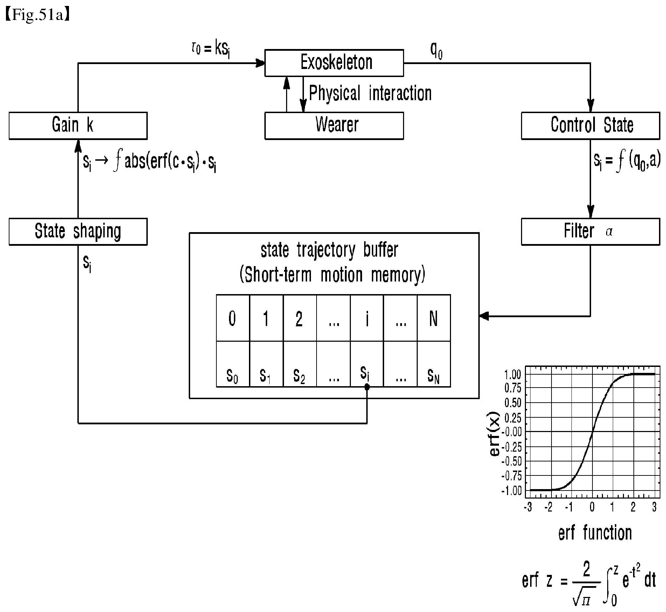

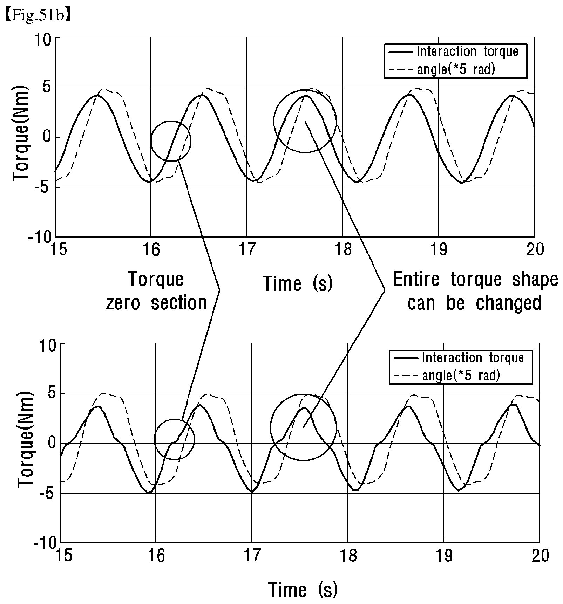

FIG. 51a is a diagram for explaining an embodiment that increases the torque deactivation section in the section where the device torque sign changes according to the present invention. FIG. 51b is a diagram for explaining, with a graph, an embodiment that increases the torque deactivation section in the section where the device torque sign changes according to the present invention.

According to the present invention, the drive unit (220) can increase the torque deactivation (torque zero) section in the section where the output torque sign changes, so as to provide, for a predetermined time, a time of providing the output torque as a value of 0 or at or below the threshold. Accordingly, the wearable robot (200) of the present invention can perform energy-efficient device driving by concentrating on the time point at which walking assistance is needed. In addition, the wearable robot (200) of the present invention enables performing natural walking assistance for the wearer, and can be effective for long-time tasks of the worker.

According to the present invention, the drive unit (220) can change the output torque form by additionally applying a Shaping operation to the motion state value (S0). As a specific example, in the case of finally applying the motion state value (S0) using an error function, as shown in FIG. 11b, the torque output zero section can be increased at the point where the output torque direction changes.

According to an embodiment of the present invention, the drive unit (220) can change the output torque form by additionally applying a Shaping operation to the motion state value (S0). Here, more various torque shape transformations may be applied by using exponential, sine, cosine functions, etc., other than the error function.

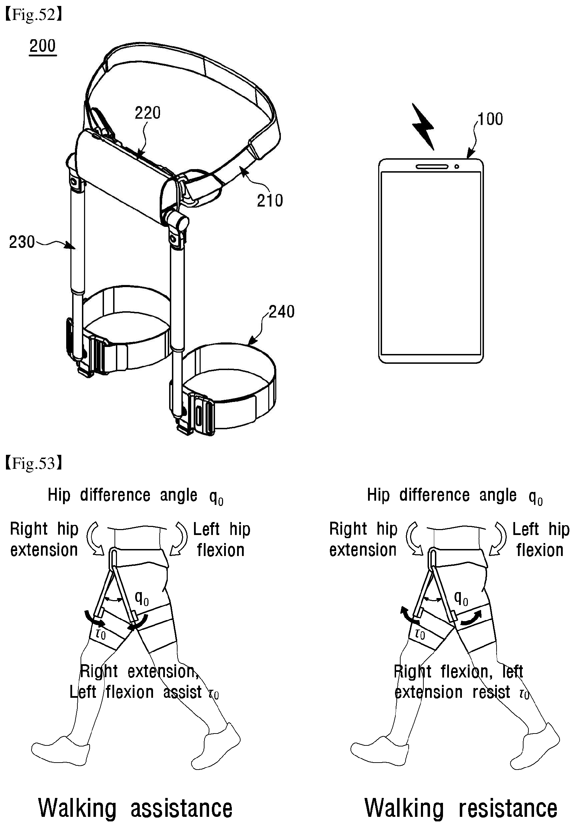

FIG. 52 is a diagram showing a wearable mobility system and a user terminal according to the present invention.

As shown in FIG. 52, the wearable robot (200) includes a waist wearing unit (210), a thigh wearing unit (240), a drive unit (220), and a connecting member (230). In addition, the waist wearing unit (210) and the thigh wearing unit (240) of the wearable robot (200) of the present invention may be worn on body parts. Specifically, the thigh wearing unit (240) and the waist wearing unit (210) may be mounted on the body parts of the wearer, and may correspond to the fixing unit in the present invention. In addition, the drive unit (220) may be mounted on the fixing unit.

For convenience of explanation, the front direction of the wearer wearing the wearable robot (200) is defined as the x-axis direction. In addition, both side directions of the wearer wearing the wearable robot (200) are defined as the y-axis direction, and the vertical direction is defined as the z-axis direction.

In an embodiment of the present invention, the waist wearing unit (210) may be mounted on a reference body part of the wearer, the thigh wearing unit (240) may be mounted on a target body part of the wearer, and the connecting member (230) connecting the drive unit (220) provided on the waist wearing unit (210) side and the thigh wearing unit (240) may rotate about the y-axis by the driving of the drive unit (220) to provide an assistance force to the movement of the body part centered on the joint. The connecting member (230) may be divided into a first connecting member and a second connecting member.

According to an embodiment of the present invention, the waist wearing unit (210) and the thigh wearing unit (240) are each mounted on a body part, and may be formed in a shape such as a band or belt that can wrap around the body part. In addition, fixing means such as a hook-and-loop fastener that can be mutually fixed may be provided at both ends.

According to an embodiment of the present invention, the drive unit (220) is provided on the waist wearing unit (210), may be configured of a motor or actuator, etc., for providing a rotational torque centered on the y-axis, and may include a reducer, etc., for increasing the torque.

The present invention can provide a walking assistance wearable robot (200) for assisting the walking exercise of the wearer, which receives target muscle information to be exercised from the user terminal (100). Specifically, the drive unit (220) may be configured to set a parameter so as to strengthen the target muscle received from the user terminal (100), and to compute an output torque based thereon.

The present invention may also provide a walking assistance wearable robot system for assisting the walking exercise of the wearer. The walking assistance wearable robot system for assisting the walking exercise of the wearer includes a user terminal (100) and a wearable robot (200), the wearable robot (200) can receive target muscle information to be exercised from the user terminal (100), set a parameter based on the received target muscle information, compute an output torque based thereon, and transmit information related to the walking exercise to the user terminal (100). At this time, the user terminal (100) can receive the transmitted information related to the walking exercise. In this case, the wearable robot (200) can compute an angular velocity based on the walking of the wearer, compute a motion state value based on the computed angular velocity, and compute an output torque based thereon.

According to the present invention, the user terminal (100) can provide a user-friendly interface so that the wearer of the wearable robot (200) can monitor his or her own movement data in real time. Specifically, the wearer can adjust the settings of the wearable robot (200), or can be provided with feedback on the walking performance from the user interface.

According to the present invention, the wearable robot (200) can receive target muscle information to be exercised from the user terminal (100), and can assist the walking exercise of the wearer based thereon.

According to the present invention, the user terminal (100) may also provide a function that can adjust settings such as the sensitivity, timing, intensity, walking assistance mode, walking resistance mode, or hiking mode, etc., of the wearable robot (200) according to personal preference.

According to the present invention, the resistance mode (Resist Program) gives resistance to the user's leg muscles while walking, making the muscles use more force, which is effective in strengthening muscle strength. In addition, the assistance mode (Assist Program) provides the effect of the wearable robot (200) assisting to help the user's walking, reducing muscle fatigue while maintaining exercise efficiency.

According to the present invention, the user terminal (100) may be mutually interconnected with the wearable robot (200) by Bluetooth, Wi-Fi, or another wireless communication technology, and can mutually exchange data through this. Specifically, the user terminal (100) can monitor the status information of the wearable robot (200) in real time, or control the operation of the wearable robot (200). In addition, the wearable robot (200) can transmit the data collected through sensors to the user terminal (100) in real time, and through this, the wearable robot (200) may also provide feedback on the walking of the wearer.

FIG. 53 is a diagram for explaining the walking assistance mode and the walking resistance mode according to the present invention.

As shown in FIG. 53, the angle value (q0, Hip difference angle) between the one-side target body part and the other-side target body part of the wearer may be the motion state value to be described later.

The present invention can provide a walking assistance wearable robot (200) for assisting the walking exercise of the wearer, which receives target muscle information to be exercised from the user terminal (100). Specifically, the drive unit (220) may be configured to set a parameter so as to strengthen the target muscle received from the user terminal (100), and to compute an output torque based thereon.

According to the present invention, the drive unit (220) can output a resistance torque, and can output an assistance torque. Specifically, in the case where the drive unit (220) outputs a resistance torque, it can be expressed as the resistance mode. In addition, in the case where the drive unit (220) outputs an assistance torque, it can be expressed as the assistance mode.

In the case of the walking assistance mode according to an embodiment of the present invention, by assisting the movement of the leg of the wearable robot wearer, the muscle strength consumption during walking can be reduced, or the wearer can be guided to a correct walking posture with fast and wide steps. With reference to FIG. 2, in the direction from right to left, a force that pushes (extension) the leg of the wearable robot wearer is applied, and with reference to FIG. 2, in the direction from left to right, a force that pulls (flexion) the leg of the wearable robot wearer is applied, so that as a result, the wearer can reduce the muscle strength consumption during walking.

In the case of the walking resistance (exercise) mode according to an embodiment of the present invention, it is possible to give an exercise effect by applying a reverse-direction torque (resistance torque) to the movement of the leg of the wearable robot wearer. With reference to FIG. 2, in the direction from right to left, a force that pulls (flexion) the leg of the wearable robot wearer is applied, and with reference to FIG. 2, in the direction from left to right, a force that pushes (extension) the leg of the wearable robot wearer is applied, so that as a result, the wearer consumes muscle strength during walking, and can have an exercise effect.

According to an embodiment of the present invention, in the case of the walking assistance mode, since the direction of the device hip angular velocity and the direction of the torque provided by the drive unit (220) are the same, transmission of power from the device to the wearable robot wearer occurs, and the power value may be calculated as positive. On the other hand, in the case of the walking resistance (exercise) mode, since the direction of the device hip angular velocity and the direction of the torque provided by the drive unit (220) are opposite, transmission of power from the wearer to the device occurs, so the power value may be calculated as negative. That is, during walking assistance, a positive power value may be mainly calculated, and during walking resistance, a negative power value may be mainly calculated.

According to the present invention, the drive unit (220) can compute an angular velocity based on the walking of the wearer. In addition, the drive unit (220) can compute a motion state value based on the computed angular velocity. In addition, the drive unit (220) can compute an output torque based on the computed motion state value. Details regarding the output torque computation of the drive unit (220) are described later.

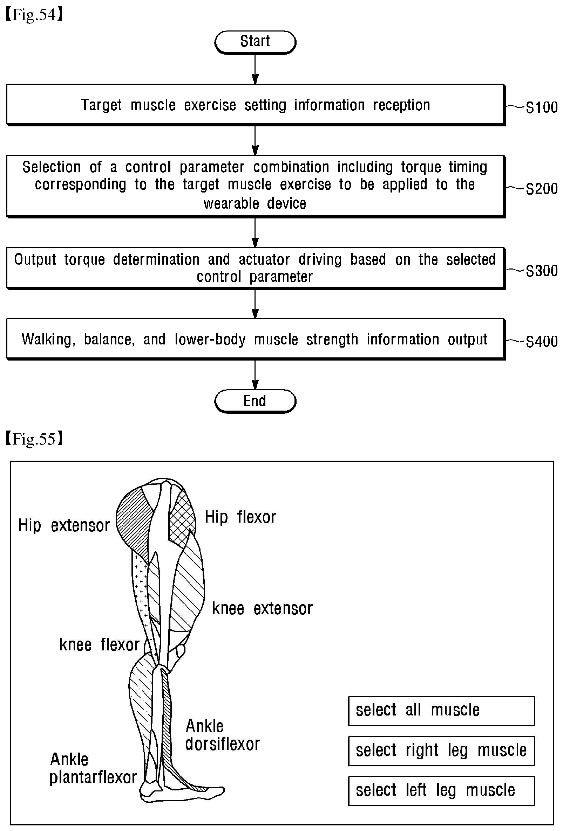

FIG. 54 is a diagram for explaining a method of supporting selective muscle strengthening exercise during walking exercise according to the present invention.

According to the present invention, the method of supporting selective muscle strengthening exercise during walking exercise may include a target muscle information reception step (S 100), a control parameter combination selection step including torque output timing information corresponding to the target muscle exercise to be applied to the wearable robot (200) (S200), a step of determining an output torque based on the selected control parameter and driving the actuator (S300), and a step of outputting walking, balance, and lower-body muscle strength information (S400).

The present invention can provide an output torque provision method using a wearable robot (200) for selective muscle strengthening. The output torque provision method using a wearable robot (200) for selective muscle strengthening may include a step in which the wearable robot (200) receives target muscle information from the user terminal (100), a selection step in which the wearable robot (200) selects a parameter based on the received target muscle information; and a computation step in which the wearable robot (200) computes an output torque based on the selected parameter. Specifically, the computation step may further include a step of computing an angular velocity based on the walking of the wearer of the wearable robot (200), a step of computing a motion state value based on the computed angular velocity, and a step of computing an output torque based on the computed motion state value. In addition, the computation step may further include a step of computing an output torque by multiplying the weighted sum of the computed motion state value by a torque intensity adjustment parameter. In addition, the selection step may include a step in which the wearable robot (200) stores a parameter according to the exercise mode as a lookup table, and a step in which the wearable robot (200) selects a parameter according to the target muscle information received from the user terminal (100) based on the stored lookup table.

FIG. 55 is a diagram showing a user interface for selecting a target muscle to be exercised according to an embodiment of the present invention.

According to the present invention, the method of supporting selective muscle strengthening exercise during walking exercise may include a target muscle information reception step (S100). Specifically, the target muscle information reception step (S100) may be accomplished by a method in which a user interface is provided on a display unit (not shown) of the user terminal (100), and the drive unit (220) receives the information input from the provided user interface.

As shown in FIG. 55, the display unit (not shown) of the user terminal (100) can provide a user interface so that the wearer can select a target muscle to be exercised. As a specific example, the wearer of the wearable robot (200) can select the target muscle to be exercised as Hip flexor, Knee flexor, etc. In addition, the wearer of the wearable robot (200) may also select a user interface in the form of a 'select all muscle' button in order to exercise all muscles. In this case, the user terminal (100) and the wearable robot (200) may be connected using Bluetooth, Wi-Fi, or another wireless communication technology, as shown in FIG. 1, and data may be mutually exchanged through such a wireless connection. Therefore, the wearable robot (200) can receive the target muscle information from the user terminal (100).

According to an embodiment of the present invention, the display unit (not shown) of the user terminal (100) can provide a user interface so that the wearer can select a target muscle to be exercised. In addition, the reception unit (not shown) of the user terminal (100) may also control the wearable robot (200) by receiving the voice data of the wearer so that the wearer can select a target muscle to be exercised, but the embodiment in which the wearable robot (200) receives the target muscle information from the user terminal (100) is not limited thereto.

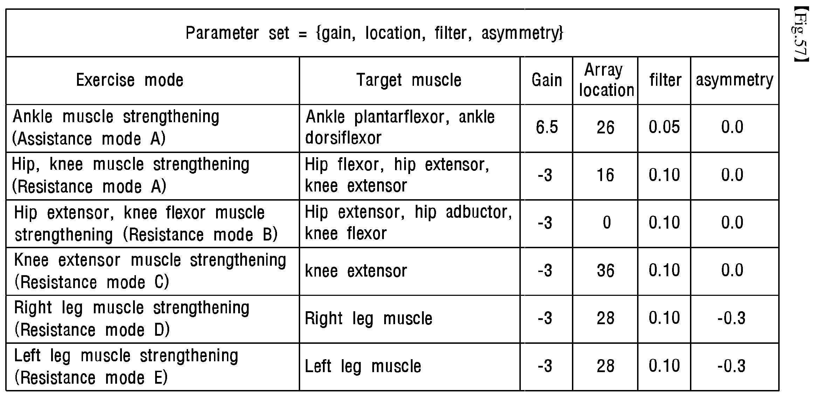

FIG. 57 is a diagram showing a lookup table of setting values matching the target muscle strength exercise mode according to an embodiment of the present invention.

According to the present invention, the method of supporting selective muscle strengthening exercise during walking exercise may include a control parameter combination selection step including torque output timing information corresponding to the target muscle exercise to be applied to the wearable robot (200) (S200).

According to the present invention, the wearable robot (200) may further include a memory unit (not shown). In addition, the drive unit (220) is configured to be able to compute an output torque according to the set exercise mode, and the memory unit (not shown) may be configured to store a parameter according to the set exercise mode as a lookup table. At this time, the set parameter may be configured as a parameter according to the target muscle information received from the user terminal (100) based on the lookup table. Specifically, the lookup table may be made of at least one of the output timing information of the computed torque, the intensity adjustment information of the computed torque, the filter information for computing the output torque, or the asymmetry information for computing the output torque, or at least one or a combination of at least two or more thereof.

According to an embodiment of the present invention, the drive unit (220) may also receive the parameter value itself from the user terminal (100). In this case, the drive unit (200) can compute an output torque based thereon. That is, the drive unit (200) may compute an output torque by a combination of parameters each having a value set by the wearer, rather than a parameter value by the lookup table.

According to the present invention, the wearable robot (200) may further include a memory unit (not shown). In addition, the memory unit (not shown) can store, in the form of a lookup table, an exercise mode based on the target muscle information received from the user terminal (100) and a parameter according to the exercise mode. In this case, the drive unit (220) can select an exercise mode based on the target muscle information received from the user terminal (100), and may be configured to compute an output torque based on the parameter according to the selected exercise mode.

According to the table shown in FIG. 57, the Ankle muscle strengthening mode (assistance mode A) is a muscle strengthening mode targeting the flexor and dorsiflexor of the ankle, the Hip, Knee muscle strengthening mode (resistance mode A) is a resistance exercise mode for strengthening the hip and knee muscles, the Hip extensor, Knee flexor muscle strengthening mode (resistance mode B) is a resistance exercise mode for strengthening the hip extensor and knee flexor, and the Knee extensor muscle strengthening (resistance mode C) mode is a resistance exercise mode for strengthening the muscle strength targeting the knee extensor. In addition, the Right leg muscle strengthening (resistance mode D) mode according to an embodiment of the present invention is an exercise mode for strengthening the right leg muscles, and the Left leg muscle strengthening (resistance mode E) mode is an exercise mode for strengthening the left leg muscles. Since the setting value lookup table matching the target muscle strength exercise mode shown in FIG. 57 is merely an embodiment, the scope of rights of the present invention is not limited thereto. However, although specific embodiments will be described below for each of the resistance modes described above, likewise the scope of rights of the present invention is not limited thereto.

According to an embodiment of the present invention, there is a case where the wearer selects 'Ankle plantarflexor' or 'Ankle dorsiflexor' as the target muscle to be exercised through the user interface of the user terminal (100). In this case, the wearable robot (200) can execute assistance mode A based on the lookup table of FIG. 57. Specifically, as assistance mode A is selected, the Target muscle can be Ankle plantarflexor and Ankle dorsiflexor, the Ankle plantarflexor is a muscle that serves to bend the ankle downward and can serve to exert force while walking or running to push the body forward. In addition, the Ankle dorsiflexor is a muscle that lifts the ankle upward, is involved in lifting the foot, and can serve to prevent the foot from touching the floor during walking or running.

According to the present invention, the drive unit (220) can set a parameter so as to strengthen the target muscle, and can compute an output torque based thereon. Specifically, the parameter set by the drive unit (220) may be a parameter representing at least one of the output timing information of the computed torque, the intensity adjustment information of the computed torque, the filter information for computing the output torque, or the asymmetry information for computing the output torque. As a specific example, the intensity adjustment information of the computed torque may be expressed by the Gain value, which is the torque intensity adjustment parameter according to the present invention.

According to an embodiment of the present invention, as assistance mode A is selected, it can be confirmed that the Gain value, which is the torque intensity adjustment parameter, is set to 6.5 by the lookup table of FIG. 57. Since the torque intensity adjustment parameter 6.5 is positive, it is used in the assistance mode, and can act in a manner of strengthening the force when the user uses the ankle muscles. That is, in assistance mode A, when the user bends or extends the ankle, the wearable robot (200) can apply a greater force to help the movement of the ankle muscles. That is, the intensity adjustment information of the torque computed by the drive unit (220) can be expressed by the torque intensity adjustment parameter, and the torque intensity adjustment parameter may be configured to be negative in the case where the drive unit (220) outputs a resistance torque, and positive in the case where the drive unit (220) outputs an assistance torque.

According to the present invention, the drive unit (220) can set a parameter so as to strengthen the target muscle, and can compute an output torque based thereon. Specifically, the parameter set by the drive unit (220) may be a parameter representing at least one of the output timing information of the computed torque, the intensity adjustment information of the computed torque, the filter information for computing the output torque, or the asymmetry information for computing the output torque. As a specific example, the output timing information of the computed torque may be expressed by the Array location value, which means the position of the motion state value according to the present invention.

According to the present invention, the output timing information of the computed torque may include the position information of the computed motion state value.

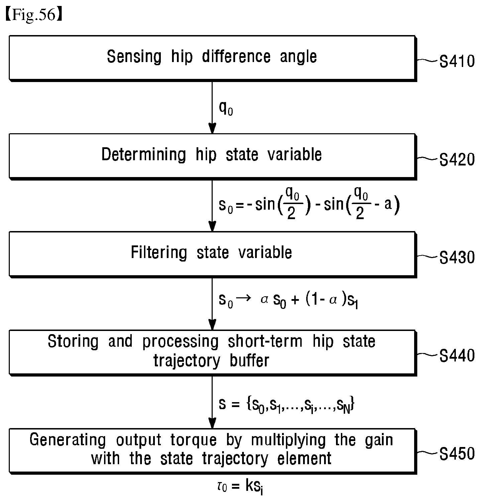

According to an embodiment of the present invention, as assistance mode A is selected, it can be confirmed that the Array location value is set to 26 by the lookup table of FIG. 57. Specifically, the Array location value according to the present invention may mean the position of the motion state value. In other words, the output timing information of the computed torque may include the position information of the computed motion state value. That is, the Array location value may be a parameter corresponding to i, which means a specific motion state value shown in FIG. 56. As a specific example, as assistance mode A is selected, since the Array location value is set to 26 by the lookup table of FIG. 57, the drive unit (220) can compute an output torque based on the parameter value set at the timing of the 26th motion state value. Accordingly, as assistance mode A is selected, the wearable robot (200) can provide an Ankle muscle strengthening effect to the wearer's Target muscle, Ankle plantarflexor and Ankle dorsiflexor.

According to the present invention, the drive unit (220) can set a parameter so as to strengthen the target muscle, and can compute an output torque based thereon. Specifically, the parameter set by the drive unit (220) may be a parameter representing at least one of the output timing information of the computed torque, the intensity adjustment information of the computed torque, the filter information for computing the output torque, or the asymmetry information for computing the output torque. As a specific example, the filter information for computing the output torque may be expressed by the Filter value according to the present invention.

According to an embodiment of the present invention, as assistance mode A is selected, it can be confirmed that the Filter value is set to 0.05 by the lookup table of FIG. 57. In this case, when the drive unit (220) computes the angular velocity by differencing and filtering the sensed hip angle in order to compute the output torque, it can perform filtering with the filter value of 0.05 to compute the output torque. The output torque computation is described in detail below.

According to the present invention, the drive unit (220) can set a parameter so as to strengthen the target muscle, and can compute an output torque based thereon. Specifically, the parameter set by the drive unit (220) may be a parameter representing at least one of the output timing information of the computed torque, the intensity adjustment information of the computed torque, the filter information for computing the output torque, or the asymmetry information for computing the output torque. As a specific example, the asymmetry information for computing the output torque may be expressed by the asymmetry value according to the present invention.

According to an embodiment of the present invention, as assistance mode A is selected, it can be confirmed that the asymmetry value is set to 0.0 by the lookup table of FIG. 57. The asymmetry value 0.0 may mean that the same stimulation is applied to both ankle muscles. That is, in assistance mode A, the same force is applied to the left and right ankle muscles so that balanced assistance can be achieved.

According to the present invention, the drive unit (220) may compute an output torque by applying a parameter value per predetermined exercise mode as shown in FIG. 57, but the wearer may also adjust each parameter through the user interface of the user terminal (100).

FIG. 56 is a diagram for explaining the walking assistance output algorithm according to the present invention.

According to an embodiment of the present invention, in order to provide a driving force to the wearer of the wearable robot (200), a movement detection sensor (not shown) of the drive unit (220) may perform a step (S410) of sensing the hip angle of the wearer of the wearable robot (200). A processor unit (not shown) of the drive unit (220) may compute the angular velocity based on the sensed hip angle. Specifically, the movement detection sensor (not shown) senses the hip angle of the wearer, and the processor unit (not shown) of the drive unit (220) may compute the angular velocity by differencing and filtering the hip angle sensed by the movement detection sensor (not shown) at a speed of 1000 Hz or more. That is, according to the present invention, even without using a separate tachometer for sensing the angular velocity, the angular velocity can be computed through hip angle sensing. Even more specifically, the processor unit (not shown) performs real-time control, the processor unit (not shown) may be divided into a low-level processor and a high-level processor, and the angular velocity may be computed by differencing and filtering the hip angle sensed by the movement detection sensor (not shown) at a speed of 1000 Hz or more in the low-level processor that has a relatively small amount of computation.

As shown, the torque output in the step (S450) of outputting a torque for assisting the walking of the wearer of the wearable robot (200) or a torque for resisting may be a torque computed by computing (S440) a plurality of motion state values based on the angular velocity computed by the drive unit (220), selecting at least one motion state value among them, and multiplying the weighted sum of the selected motion state value by the torque intensity adjustment factor (k). At this time, the computed plurality of motion state values may be stored by repeating a shift operation through the state trajectory memory buffer (300). Hereinafter, the plurality of motion state values stored by repeating the shift operation through the state trajectory memory buffer (300) are described.

As shown, in order to provide a driving force to the wearer of the wearable robot (200), a step (S450) of outputting, in the processor unit (not shown) of the drive unit (220), a torque for assisting the walking of the wearer or a torque for resisting may be performed. Specifically, the step (S450) of outputting a torque for assisting the walking of the wearer or a torque for resisting may include a step (S420) of determining a motion state value. In addition, it may further include a step (S430) of filtering the motion state value. In addition, it may further include a step (S440) of deleting the previously stored oldest motion state value from the memory.

As shown, the state trajectory memory buffer (300) is a storage device that sequentially stores the motion state values of the wearer. Specifically, when the first motion state value is input, it is stored in the first storage position of the memory array. Next, when a new motion state value is input, the motion state value that was stored in the first storage position of the memory array is moved to the second storage position of the memory array, and the newly input motion state value is stored in the first storage position of the memory array. Then, when a new motion state value is input, the motion state values that were respectively stored in the first storage position and the second storage position of the memory array are moved to the second storage position and the third storage position of the memory array, and the newly input motion state value is stored in the first storage position of the memory array.

When such a shift operation is repeated, N+1 motion state values are stored in all the storage positions of the memory array. Accordingly, when a new motion state value is input, the motion state values stored in the memory array are moved to the next position, but the motion state value stored in the last storage position (N), which has no more place to move, is deleted from the state trajectory memory buffer (300).

By using the above manner, asymmetric torque generation becomes possible even in a wearable robot using a single actuator. The asymmetric mode can be used for resolving the imbalance of the left/right range of motion and for posture improvement, and may be used for different purposes depending on the wearer or the application. For instance, when assisting the motion of a person who performs an asymmetric motion due to a disease such as a stroke, etc., the asymmetric mode may be usefully used. In addition, for example, in the case where the wearable robot according to an embodiment of the present invention is used for assisting a walking motion, the asymmetric mode may be utilized for walking navigation assistance.

A wayfinding assistance function may be possible, such as reducing the torque intensity to induce a speed reduction in a complex environment, direction guidance through asymmetric assistance at a curve, and increasing the speed on a straight distance, etc. In addition, if a vision sensor is used by a method such as mounting a smartphone on the exoskeleton device, etc., it may also be used for the walking movement of the visually impaired.

According to an embodiment of the present invention, after performing the step (S420) for determining the converted value (S0), the filtering step (S430) may be performed. The present invention can change the predetermined position for selecting the motion state value according to the motion state of the wearer in order to adaptively respond to the rapid motion change of the wearer. When the assistance force is determined by adaptively selecting the motion state value according to the motion change of the wearer, it is possible to respond more quickly to the motion change of the wearer than in the case where the motion state value selection position is fixed.

According to an embodiment of the present invention, the motion state value stored in the state trajectory memory buffer (300) may represent the movement of the wearer during a recent short time (within several seconds, for example, within 1 to 2 seconds). For example, the motion state value stored in the state trajectory memory buffer (300) may include only one motion information of the wearer (a half-cycle of a repetitive movement). Even if the motion state value stored in the state trajectory memory buffer (300) includes only one motion of the wearer, that is, a half-cycle of a repetitive movement, the practice of the present invention is possible.

Preferably, as the motion state value stored in the state trajectory memory buffer (300), a motion state value for a movement including one cycle among the repetitive movements of the wearer may be stored. For example, in the case where the wearable robot (200) according to an embodiment of the present invention is worn on a lower-body part such as the waist or thigh, the one-cycle motion of the wearer may be a one-cycle walking motion of the wearer (e.g., a step motion, etc.).

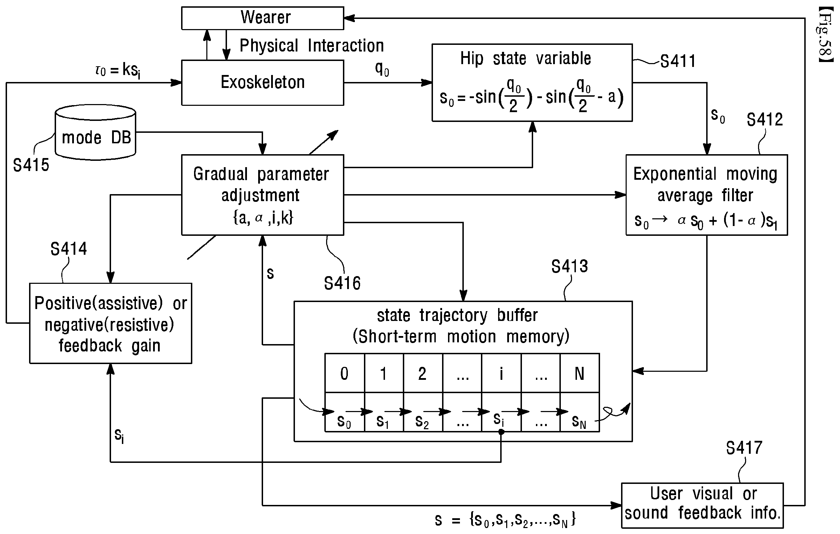

FIG. 58 is a diagram for explaining the output torque algorithm according to the present invention. The output torque computation of the drive unit (220) is described in more detail.

As shown, the current hip angle information (q0) sensed from the Exoskeleton may be converted into the motion state value (S0), which is the hip state variable. (S411)

According to an embodiment of the present invention, the drive unit (220) may provide a difference of the output torque between the walking of the one-side target body part and the walking of the other-side target body part by using an asymmetry parameter a on the converted motion state value (S0). Specifically, if a=0, it means there is no degree of asymmetry, and the same torque may be provided symmetrically to the left and right motions. If the a value is negative, the assistance torque is increased for the motion of the left-side motion compared with the motion of the right-side motion. If the a value is positive, the assistance torque is increased for the motion of the right-side motion compared with the motion of the left-side motion. That is, the asymmetric assistance for the left-side or right-side motion can be adjusted with the single parameter a.

According to an embodiment of the present invention, the asymmetry of the left/right motion is determined by the sign of the a value, and the degree of asymmetry can be adjusted by the magnitude of the a value. For instance, in the case where a=0.20 compared with when a=0.10, the right-side asymmetric assistance is further strengthened. The a value may be within the range of -1.0 to 1.0, and a value of -0.5 to 0.5 is preferably used.

According to an embodiment of the present invention, the converted motion state value (S0) may pass through an Exponential moving average filter. (S412) That is, the drive unit (220) can adjust the cutoff frequency of the signal using a filter parameter.

According to the present invention, the filtered motion state value (S0) may be stored in and updated to the state trajectory memory buffer (300) (S440, S413), and at every control instant, the recent movement information of the hip state may be stored in a motion memory buffer of finite size and updated at every instant. Accordingly, the drive unit (220) may calculate an output torque value based on the filtered motion state value (S0). (S450)

According to the present invention, the motion speed of the sensed signal may be calculated using the state trajectory movement distance (d0), and the control parameter (the parameter of S416) may be adaptively adjusted in accordance with the wearer's movement characteristics. In addition, modes specialized for various workers/users may be stored in the form of a lookup table in the wearable robot (200), an external remote device, or an external server, and when selected through the interface of the user terminal (100), the stored parameters may be received so that the drive unit (220) may calculate the output torque according to the parameters of the designated mode.

According to the present invention, the parameter setting value by the changed mode may be reflected by being gradually changed at every control instant starting from the currently set parameter value. (S416) Accordingly, the setting corresponding to the selected exercise mode set in the mode database (S415) does not change immediately when the user changes to the selected exercise mode using the interface of the user terminal (100), but may be finally reflected in the drive unit (220) after a predetermined time. That is, the drive unit (220) may, through the state trajectory memory buffer (300) and the gradual parameter change (S416), safely switch to the walking assistance mode or the walking resistance mode even during movement, without stopping walking.

According to an embodiment of the present invention, the drive unit (220) may, by changing the sign of the torque intensity adjustment parameter (k), apply the switching to the walking assistance mode or the walking resistance mode as one integrated control rule without a separate controller switching. Accordingly, the wearable robot (200) may apply an output torque of a predictable (consistent) form to the wearer. Accordingly, the present invention may enable safe walking assistance/resistance for the wearer.

The user audiovisual feedback information providing step (S417) of FIG. 58 is described below.

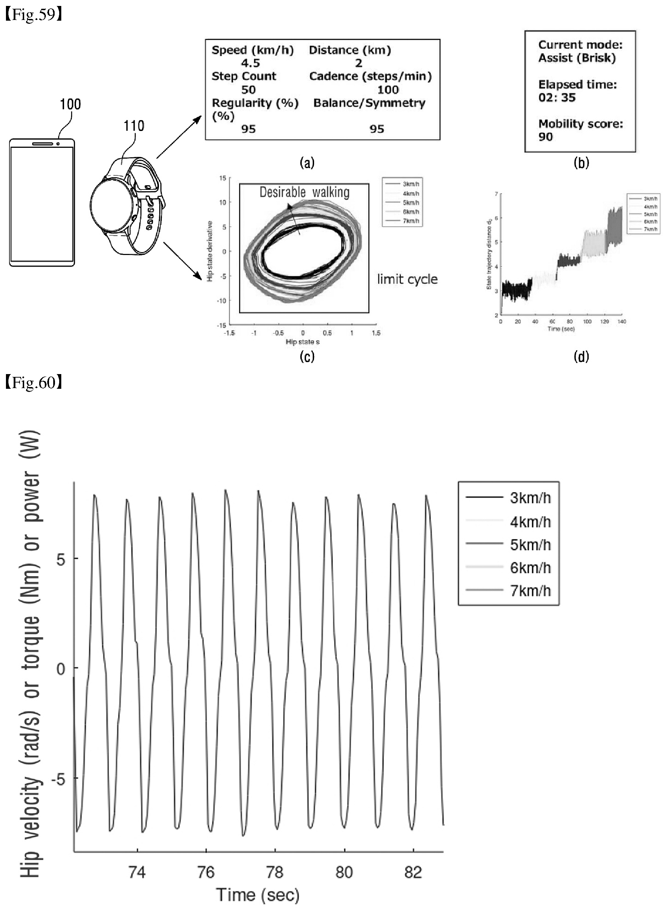

FIG. 59 is a diagram showing the provision of user feedback information according to an embodiment of the present invention.

The method of supporting selective muscle strengthening exercise during walking exercise of the present invention may further include a user audiovisual feedback information providing step (S417). Specifically, the wearable robot (200) may provide user audiovisual feedback information to the wearer through the user terminal (100) or a terminal in the form of a watch (110).

According to an embodiment of the present invention, the drive unit (220) may provide the wearer with information about walking exercise from the recent movement information of the wearer stored in the state trajectory memory buffer (300). In this case, the wearable robot (200) may provide audiovisual feedback information including the current wearer's walking performance information and exercise information through the user terminal (100) or a terminal in the form of a watch (110) that wirelessly communicates with the wearable robot (200). Specifically, the audiovisual feedback information includes walking performance information and exercise information, the walking performance information may include speed information, posture information, stride information, regularity information, asymmetry information, and walking score information, and the exercise information may include exercise elapsed time information per exercise mode, accumulated exercise time information, and current mode exercise elapsed time information.

According to the present invention, the method of supporting selective muscle strengthening exercise during walking exercise may include a target muscle information receiving step (S100), a control parameter combination selecting step (S200) including torque output timing information corresponding to the target muscle exercise to be applied to the wearable robot (200), a step (S300) of determining the output torque based on the selected control parameter and driving the actuator, and a step (S400) of outputting walking, balance, and lower-body muscle strength information. Specifically, the step (S400) of outputting walking, balance, and lower-body muscle strength information may include the user audiovisual feedback information providing step (S417). Accordingly, the present invention may provide the wearer with motivation to continue exercising, and has an effect of efficiently assisting exercise.

According to an embodiment of the present invention, the display unit (not shown) of the user terminal (100) may provide the audiovisual feedback information in the form of numbers or graphs, and specifically, may provide speed information, posture information, stride information, regularity information, asymmetry information and walking score information, exercise elapsed time information per exercise mode, accumulated exercise time information, and current mode exercise elapsed time information in the form of numbers or graphs.

According to an embodiment of the present invention, the wearable robot (200) may provide the walking index information shown in (a) of FIG. 59 through the user terminal (100) or a terminal in the form of a watch (110) that wirelessly communicates with the wearable robot (200). As shown in (a) of FIG. 59, as a specific example, the current wearer is wearing the wearable robot (200) and walking, and it can be confirmed that the current speed or average speed means the wearer's walking speed and is 4.5 km/h. In addition, it can be confirmed that distance means the total distance walked by the wearer and is 2 km. In addition, number of steps indicates the number of steps the wearer has walked, and it can be confirmed that 50 steps have been walked. cadence means the wearer's number of steps per minute, and since it is 100 steps/min, it can be confirmed that the wearer walks 100 steps per minute. regularity is an index indicating how regular the wearer's walking is, and it can be confirmed that a regularity of 95% is currently maintained. In addition, Balance/Symmetry is an index indicating the left-right balance or symmetry of the wearer when walking, and it can be confirmed that a symmetry of 95% is maintained.

According to an embodiment of the present invention, the wearable robot (200) may provide the current section exercise time information and applied exercise mode information shown in (b) of FIG. 59 through the user terminal (100) or a terminal in the form of a watch (110) that wirelessly communicates with the wearable robot (200). As shown in (b) of FIG. 59, as a specific example, Current mode: Assist (Brisk) may indicate the mode in which the wearable robot (200) is currently operating. That is, it can be confirmed that the wearable robot (200) is currently operating in the assistance mode, and it can be confirmed that it is supporting a brisk walk or a speedy walk. In addition, Elapsed time indicates the time elapsed after the user started walking, and it can be confirmed that 2 minutes and 35 seconds have elapsed. In addition, Mobility score is a score evaluating the wearer's mobility or walking performance, and it can be confirmed that the Mobility score was measured as 90 points.

According to an embodiment of the present invention, the Mobility score includes the wearer's overall walking performance, and the drive unit (220) or the user terminal (100) or the like may calculate the Mobility score based on the state trajectory movement distance (d0). That is, since the wearer's walking speed and the state trajectory movement distance (d0) have a positive correlation, when the wearer's walking speed increases, the Mobility score value may increase. Hereinafter, the Mobility score is further described based on the description of (c) of FIG. 59 and (d) of FIG. 59.

As shown in (c) of FIG. 59, the wearable robot (200) may provide walking limit cycles visual feedback information through the user terminal (100) or a terminal in the form of a watch (110) that wirelessly communicates with the wearable robot (200). As a specific example, the wearable robot (200) may visualize the wearer's walking state using a phase portrait, and may represent the relationship between two state variables, the motion state value s and the Hip state derivative.

As shown, it can be confirmed that the larger the limit cycle, the larger the wearer's stride and the faster the walking speed. In addition, as the speed increases (from 3 km/h to 7 km/h in the graph), the limit cycle becomes larger, and this may indicate a wide stride and a more active walking pattern. That is, when a larger limit cycle appears, it can be confirmed that the wearer's walking is more desirable, and this may indicate that the wearer's stride is large, the speed is fast, and the efficiency of walking has increased. In addition, the wearer may walk so as to increase the limit cycle through the graph shown in (c) of FIG. 59, and it can be understood that this has an effect of inducing the wearer to a more desirable walk according to the feedback provided in real time by the wearable robot (200).

As shown in (d) of FIG. 59, the wearable robot (200) may provide mobility score visual feedback information through the user terminal (100) or a terminal in the form of a watch (110) that wirelessly communicates with the wearable robot (200). As a specific example, the x-axis represents Time (sec), and this is for representing how the wearer's state trajectory movement distance (d0) changes as time progresses, and in (d) of FIG. 59, the wearer's walking time was measured from 0 seconds to 140 seconds.

In addition, the y-axis is the wearer's state trajectory movement distance (d0), and the state trajectory movement distance (d0) value increases when the walking speed increases, so it may be used to evaluate the mobility score, that is, the wearer's walking performance. It can be confirmed that the larger the y-axis value, the more the walking performance has increased. In addition, as shown, the wearer's state trajectory movement distance (d0) increases in a stepwise manner, and it can be confirmed that the wearer's walking state is maintained constant in each speed section, and that the state trajectory movement distance (d0) increases sharply each time the speed increases. That is, the wearer may confirm, through visual feedback via the user terminal (100) or the like, that walking at a higher speed further improves the walking performance. Accordingly, the present invention has an effect of serving to induce the wearer to maintain a better walking pattern or to achieve a goal.

FIG. 60 is a diagram showing the provision of hip joint visual feedback information according to an embodiment of the present invention.

As shown, since the state trajectory movement distance (d0) value does not have a constant value due to the wearer's movement at every instant when calculating the mobility score, the wearable robot (200) may use the average or median of the state trajectory movement distance (d0) values over a predetermined time. Accordingly, the average or median of the state trajectory movement distance (d0) values over a predetermined time may be normalized to a score of 100 points and provided to the wearer in the form of a mobility score value through the user terminal (100) or the like. In addition, the wearable robot (200) or the user terminal (100) or the like may receive literature statistical information or the like from a server device or the like, and may convert and represent the mobility score value as the wearer's walking age or walking rank based thereon. Accordingly, the present invention may have an effect of providing motivation for the wearer's exercise.

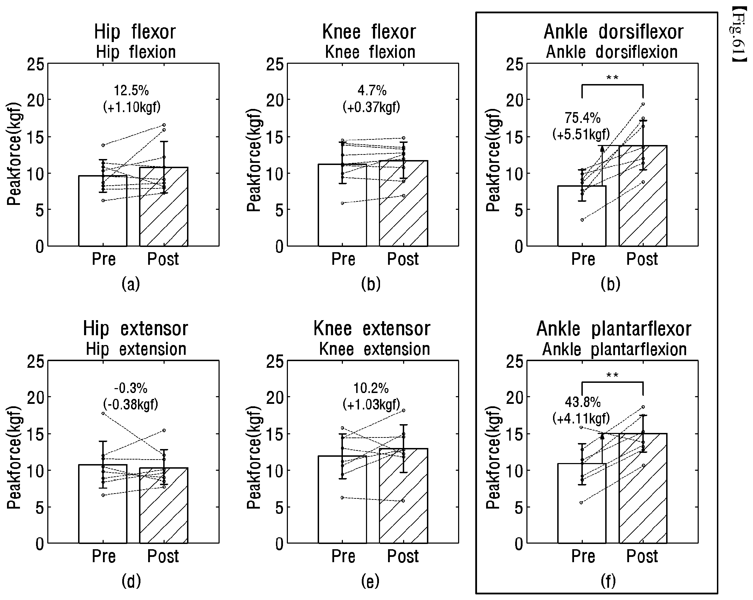

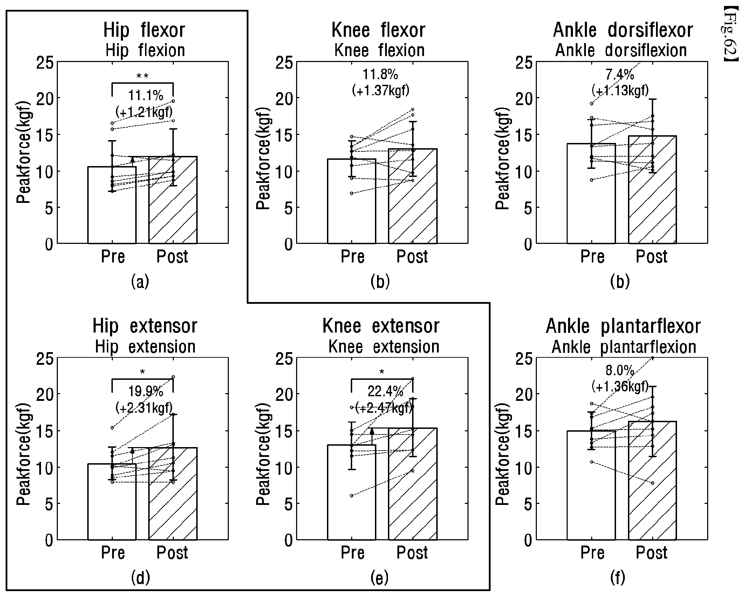

FIG. 61 is a diagram showing the muscle strengthening effect of assistance mode A according to an embodiment of the present invention. FIG. 62 is a diagram showing the muscle strengthening effect of resistance mode A according to an embodiment of the present invention.

Hereinafter, the experimental results according to the set parameter sets of each exercise mode shown in FIG. 57, namely assistance mode A, resistance mode A, resistance mode B, resistance mode C, resistance mode D, and resistance mode E, are described. As described above, since the setting value lookup table matched with the target muscle strength exercise mode is only one embodiment, yet another exercise effect according to the present invention may be expected by other parameters of an exercise mode other than the parameters of each of the modes described above.

According to an embodiment of the present invention, the wearable robot (200) may provide the wearer, in the assistance mode, with an effect of inducing a brisk walk in which the stride becomes larger and the speed becomes faster than usual. Accordingly, in the assistance mode, there is an effect that, compared to a walk with a small stride and slow speed, the lower-limb muscle stimulation becomes greater, and the upright posture of the upper body and the use of the core muscles also increase. In addition, in the assistance mode, there is an effect of inducing an upright and dynamic step for the wearer while simultaneously strengthening the core muscle strength and ankle muscle strength.

According to an embodiment of the present invention, the wearable robot (200) may, in the resistance mode, provide the wearer with an effect of directly applying resistance during walking, like walking in water, to strengthen the lower-body muscles and core muscles. Specifically, in the resistance mode, an effect of strengthening the hip joint and knee muscle strength together with the resistive ability to withstand external force may be provided to the wearer. In addition, when the wearer alternately uses the hip assistance mode and the resistance mode at intervals, various lower-body muscle strength and core muscle strength may be stimulated, so the wearable robot (200) may also provide the wearer with a corresponding synergy effect.

As shown, when an 8-week exercise program for 9 elderly subjects was conducted, as a result of conducting 4 weeks of assistance mode exercise, it could be confirmed that the ankle muscle strength generating propulsion when walking improved by 43.8%, and the ankle dorsiflexion muscle strength improved by 75.4%. Thereafter, as a result of conducting a 4-week resistance mode exercise program, it could be confirmed that the hip extensor/flexor improved by 19.9% and 11.7% respectively, and the knee extensor improved by 22.4%.

As shown in FIG. 61, in assistance mode A, a 12.5% increase (+1.10 kgf) in the Hip flexion was observed. This can confirm that the assistance mode exercise had a positive effect on the hip flexor. In addition, the muscle strength of the Knee flexion increased by 4.7% (+0.37 kgf). Although the increase is not large, it could be confirmed that the assistance mode gave a positive effect to the knee flexor. In addition, the Ankle dorsiflexion is the muscle in which the largest change appeared, with a 75.4% increase (+5.51 kgf), so that the muscle strength of the ankle dorsiflexor was greatly improved. This can be interpreted as the ankle being actively used through the assistance mode. In addition, the change of the Hip extension was slight, and a 0.3% decrease (-0.38 kgf) was confirmed. That is, it was confirmed that the Hip extension was not greatly affected by the assistance mode exercise. In addition, the muscle strength of the Knee extension was shown to increase by 10.2% (+1.03 kgf), and an effective result for strengthening the knee extensor was confirmed. In addition, a very large change was also observed in the Ankle plantarflexion, and the muscle strength of the ankle plantarflexor increased by 43.8% (+4.11 kgf). As a result, it can be confirmed that the ankle muscle strength and the knee and hip joint muscle strength were improved through the assistance mode. In particular, the muscle strength increase of the ankle dorsiflexor and the ankle plantarflexor is prominent, and it can be confirmed that this has an effect of contributing to strengthening the propulsion and balance of walking by making the ankle be actively used through the assistance mode.

As shown in FIG. 62, in resistance mode A, it can be confirmed that the Hip flexion muscle strength increased by 11.7% (+1.21 kgf) before and after the resistance mode exercise. That is, it indicates that the hip flexor was activated through the resistance mode. In addition, the Knee flexion increased by 11.8% (+1.37 kgf), and it can be confirmed that the resistance mode is also effective in strengthening the knee flexor. In addition, the Ankle dorsiflexion increased by 7.4% (+1.13 kgf), and it can be confirmed that there is a slight improvement through the resistance mode. That is, although the increase is small compared to the assistance mode, it indicates that there is still a positive effect on the ankle muscle strength. In addition, the Hip extension increased by 19.9% (+2.31 kgf), so it can be confirmed that it was greatly improved, and this result indicates that the resistance mode is very effective in strengthening the hip extensor. In addition, the Knee extension increased by 22.4% (+2.47 kgf), showing the largest increase after the resistance mode exercise, so it can be confirmed that the resistance mode is effective in strengthening the knee extensor. In addition, the Ankle plantarflexion increased by 8.0% (+1.36 kgf), and it can be confirmed that there is also an effect on the ankle muscle strength.

According to the present invention, the assistance mode induces a brisk walk and is mainly effective in improving the ankle muscle strength. Specifically, it can be confirmed that the ankle dorsiflexor and the ankle plantarflexor showed large increases of 75.4% and 43.8% respectively. That is, it can be seen that the assistance mode maintains a fast speed and a large stride through walking, and through this, makes the ankle and core muscles be used. On the other hand, the resistance mode is more effective in strengthening the hip joint and knee muscle strength, and has an effect of improving the resistance of the lower-body and core muscles through external resistance.

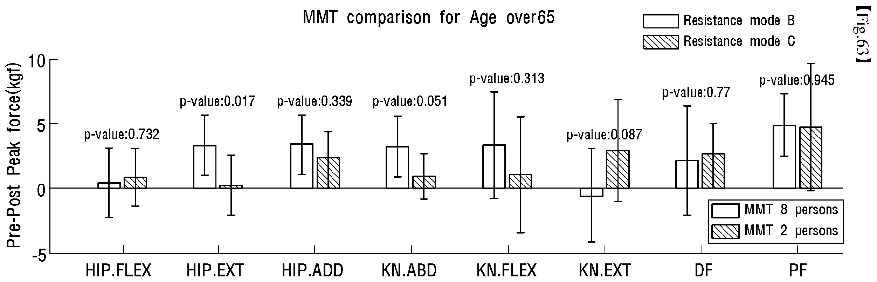

FIG. 63 is a diagram for comparing the difference in strengthened muscle strength between resistance mode B and resistance mode C according to the present invention.

As shown, it can be confirmed that the torque profile difference due to the timing difference between resistance mode B and C causes a difference in the muscle strengthening effect by lower-body part. Specifically, a 6-week exercise program was completed for 8 elderly subjects. Based on this, when comparing the strengthened muscle strength by lower-body part between the resistance mode B users and the resistance mode C users, it can be confirmed that, when resistance mode B is used, the muscle strengthening effects of the Hip extensor, Hip abductor, and Knee flexor are large, whereas, in the case of resistance mode C users, the muscle strengthening of the Knee extensor is large.

As shown, the x-axis represents HIP.FLEX (hip flexor), HIP.EXT (hip extensor), HIP.ADD (hip adductor), HIP.ABD (hip abductor), KN.FLEX (knee flexor), KN.EXT (knee extensor), DF (ankle dorsiflexor), and PF (ankle plantarflexor), and the y-axis represents the difference in muscle strength before and after exercise. That is, the larger the y-axis value, the greater the muscle strengthening effect can be indicated. In addition, the p-value for each muscle group is displayed in the graph, and the lower the p-value, the more statistically significant the difference can be indicated. Typically, when the p-value < 0.05, it can be regarded as a significant result. As shown, in the case of HIP.FLEX (hip flexor), there is no large difference in both groups, and the p-value is 0.732, indicating a non-significant difference.

In addition, the HIP.EXT (hip extensor) showed a larger muscle strength increase in resistance mode B than in resistance mode C, and the p-value is 0.017, indicating a significant difference. That is, it can be confirmed that resistance mode B is more effective in strengthening the hip extensor. In addition, the HIP.ADD (hip adductor) shows a larger muscle strength improvement in resistance mode B, but it can be confirmed that the p-value is 0.339, which is not significant. In addition, it can be confirmed that the HIP.ABD (hip abductor) shows a larger muscle strength increase in resistance mode B. In addition, the KN.FLEX (knee flexor) showed a larger muscle strength improvement in resistance mode B, but it can be confirmed that the p-value is 0.313, which is not significant. In addition, the KN.EXT (knee extensor) showed a larger muscle strength improvement in resistance mode C than in resistance mode B, and the p-value is 0.087, so it can be confirmed that resistance mode C is more effective in strengthening the knee extensor. In addition, the DF (ankle dorsiflexor) shows slight muscle strength change in both modes, and it can be confirmed that the p-value is 0.77, which is not significant. In addition, in the case of the PF (ankle plantarflexor), both modes showed similar muscle strength improvement, and it can be confirmed that the p-value is 0.945, which is not significant.

According to the present invention, resistance mode B has a larger muscle strength improvement effect in the hip extensor (HIP.EXT), hip abductor (HIP.ABD), and knee flexor (KN.FLEX), and in particular, it can be confirmed that there is a hip extensor strengthening effect. On the other hand, resistance mode C shows a larger muscle strength improvement in the knee extensor (KN.EXT), so it can be confirmed that it is more effective in strengthening the knee extensor.

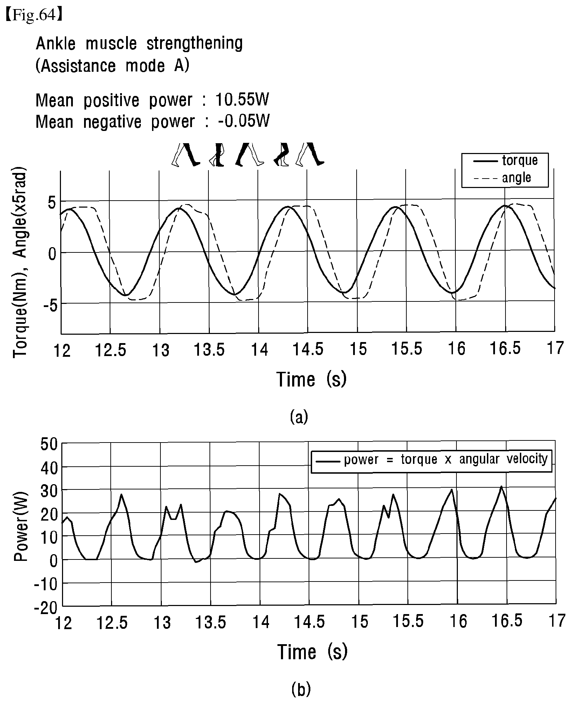

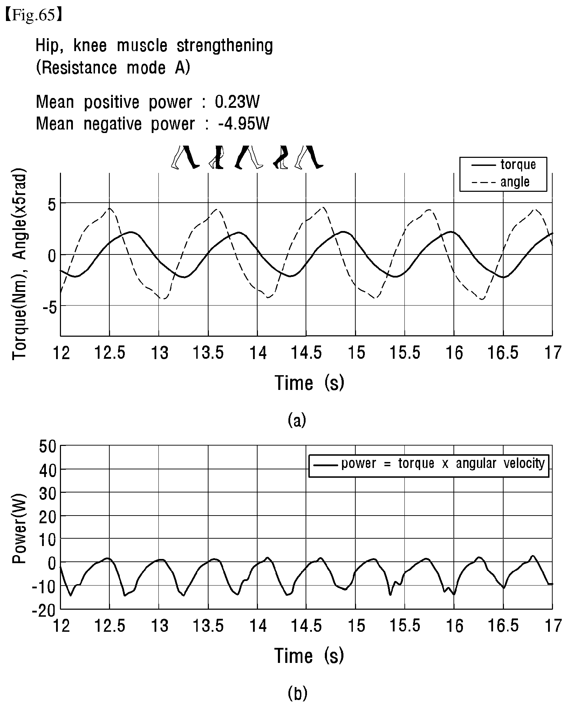

FIG. 64 is a diagram showing the torque and power trajectories of assistance mode A according to the present invention. FIG. 65 is a diagram showing the torque and power trajectories of resistance mode A according to the present invention.

As shown in FIG. 64, in assistance mode A, the y-axis torque and angle periodically change along the x-axis time, and since the waveform of the torque appears mainly in the positive region, it can be confirmed that mainly positive torque occurs when strengthening the ankle muscle strength. In addition, along the x-axis time, the y-axis Power (torque x angular velocity) also mainly produces positive power, and the average positive power can be confirmed as 10.55 W. This means that, in the assistance mode, the wearer is moving the ankle while emitting energy, and it can be confirmed that the average value of the negative power is -0.05 W, which is very small. This indicates that, in the assistance mode, the wearer's muscles mainly exert force and move the ankle.

As shown in FIG. 65, in resistance mode A, the y-axis torque and angle periodically change along the x-axis time, and the trajectory of the torque represents a trajectory that goes back and forth between positive values and negative values according to the change of the angle. This represents the characteristic that the wearer uses the leg muscles against resistance. In particular, relatively much negative torque occurs, and it can be confirmed that a movement of controlling the joint while receiving resistance is performed. In addition, along the x-axis time, in the y-axis Power (torque x angular velocity), negative power mainly occurs, and it can be confirmed that the average negative power is -4.95 W. This represents the characteristic that, in the resistance mode, the leg muscles absorb energy in response to resistance, and control and consume force. In this case, positive power hardly occurs, and it can be confirmed that the average value is 0.23 W, which is small. This indicates that, in the resistance mode, the main role of the wearer's muscles is to withstand and control resistance rather than to exert force.

According to the present invention, the assistance mode strengthens the ankle muscle strength and is effective in generating propulsion. In this mode, positive power mainly occurs, which is effective in the muscles exerting force, whereas it can be confirmed that the resistance mode applies resistance to the hip joint and knee muscles and is effective in developing the ability of the muscles to withstand and control resistance. In this mode, negative power mainly occurs, which has an effect of training the muscles to respond to external force.

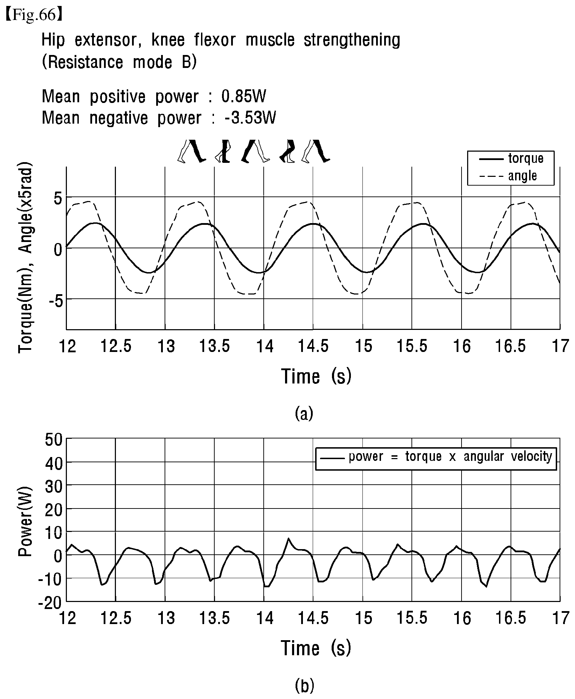

FIG. 66 is a diagram showing the torque and power trajectories of resistance mode B according to the present invention.

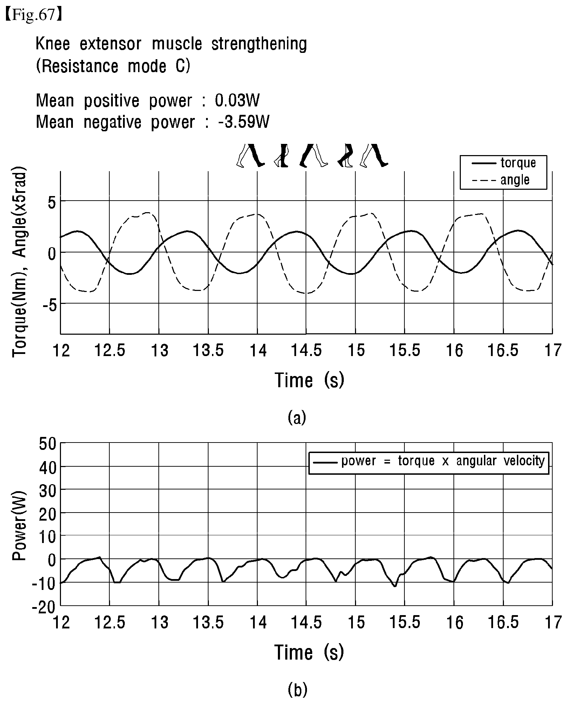

FIG. 67 is a diagram showing the torque and power trajectories of resistance mode C according to the present invention.

As shown in FIG. 66, in resistance mode B, the change between the y-axis torque and angle is periodically represented along the x-axis time. In addition, the waveform of the torque goes back and forth between positive values and negative values, and it can be confirmed that, in resistance mode B, both the hip extensor and the knee flexor are exercising while receiving resistance. In particular, negative torque occurs more than positive torque, and this may mean that the muscles operate in a manner of absorbing energy against resistance. In addition, along the x-axis time, in the y-axis Power (torque x angular velocity), negative power mainly occurs, and it can be confirmed that the average negative power is -3.53 W. This can confirm that, in resistance mode B, the muscles withstand resistance and absorb energy, and through this, the control ability of the muscles is strengthened. In addition, positive power also occurs slightly, and it can be confirmed that the average value is 0.85 W. This indicates that, in resistance mode B, the muscles not only receive resistance but some of them also operate by exerting force.

As shown in FIG. 67, in resistance mode C, the change between the y-axis torque and angle is periodically represented along the x-axis time. In resistance mode C, the Knee extensor is mainly used, the trajectory of the torque goes back and forth between positive values and negative values, and in particular, it can be confirmed that negative torque occurs more than positive torque. This may indicate that the knee extensor is controlling while absorbing energy in response to resistance. In addition, along the x-axis time, in the y-axis Power (torque x angular velocity), negative power mainly occurs, and it can be confirmed that the average negative power is -3.59 W. This can be understood as the knee extensor withstanding resistance, absorbing energy, and developing the ability to overcome resistance. In addition, positive power hardly occurs, and it can be confirmed that the average value is 0.03 W, which is low. This indicates that, in resistance mode C, the muscles are mainly training to withstand resistance.

According to the present invention, resistance mode B mainly affects the hip extensor (Hip extensor) and the knee flexor (Knee flexor). As shown, both positive power (0.85 W) and negative power (-3.53 W) occur, but it can be confirmed that the negative power occurs more largely, so that the muscles mainly play the role of withstanding and controlling resistance. That is, resistance mode B has an effect in which some muscles exert force while withstanding resistance.

According to the present invention, resistance mode C mainly affects the knee extensor (Knee extensor). As shown, positive power (0.03 W) hardly occurs, and negative power (-3.59 W) mainly occurs, so there is an effect of responding to and controlling resistance. In this mode, almost all of the exercise is concentrated on withstanding resistance, so it is effective in strengthening the control ability and resistance of the muscles.

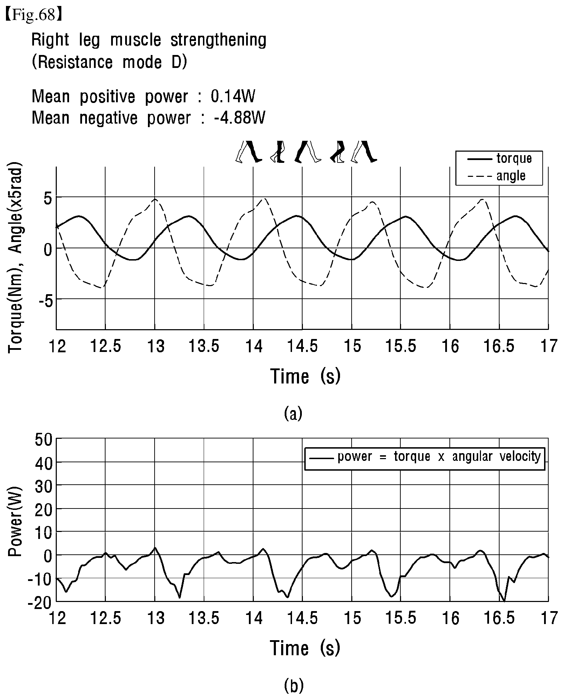

FIG. 68 is a diagram showing the torque and power trajectories of resistance mode D according to the present invention.

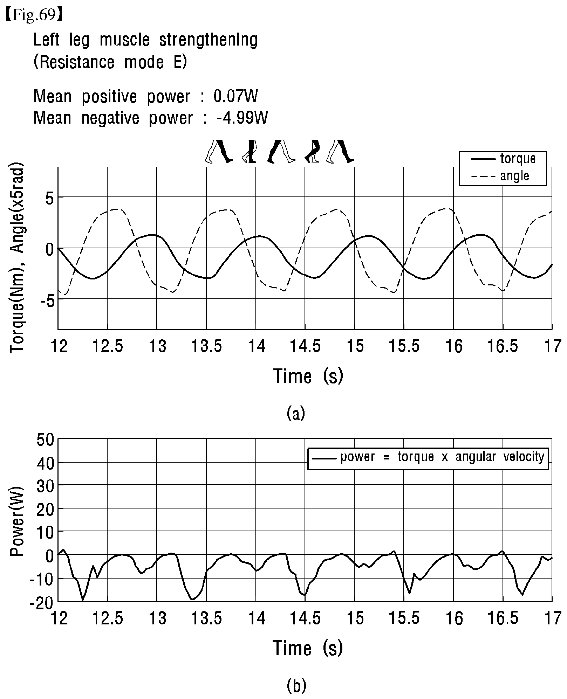

FIG. 69 is a diagram showing the torque and power trajectories of resistance mode E according to the present invention.

As shown in FIG. 68, in resistance mode D, the periodic change of the y-axis torque and angle is represented along the x-axis time. It can be confirmed that the muscles operate against resistance while the torque goes back and forth between positive values and negative values. That is, it can be confirmed that negative torque occurs more mainly in the right leg. In addition, in resistance mode D, along the x-axis time, in the y-axis Power (torque x angular velocity), negative power mainly occurs, and it can be confirmed that the average negative power is -4.88 W. In addition, the positive power is very small, with an average of 0.14 W, hardly occurring, and accordingly it can be confirmed that the muscles are performing an exercise in which energy is mainly consumed in responding to resistance.

As shown in FIG. 69, in resistance mode E, the periodic change of the y-axis torque and angle is represented along the x-axis time. That is, in resistance mode E, the change of the torque and angle in the left leg is represented. As with the right leg, the torque goes back and forth between positive values and negative values, and since negative torque mainly occurs, it indicates that the left leg also mainly controls torque in response to resistance. In addition, in resistance mode E, along the x-axis time, in the y-axis Power (torque x angular velocity), negative power mainly occurs, and it can be confirmed that the average negative power is -4.99 W. In addition, the positive power is very small, with an average of 0.07 W, hardly occurring, so it can be confirmed that resistance mode E is effective for muscle control and resistance-withstanding training.

According to the present invention, in resistance mode D (right leg), positive power (0.14 W) and negative power (-4.88 W) occur, but it can be confirmed that mainly the negative power occurs more largely. That is, in strengthening the right leg muscle strength, there is an effect that mainly an exercise responding to resistance is performed. In addition, in resistance mode E (left leg), positive power (0.07 W) and negative power (-4.99 W) occur, but it can likewise be confirmed that the negative power mainly occurs. That is, the strengthening of the left leg muscle strength also has, as with the right leg, an effect of mainly consuming energy in response to resistance.

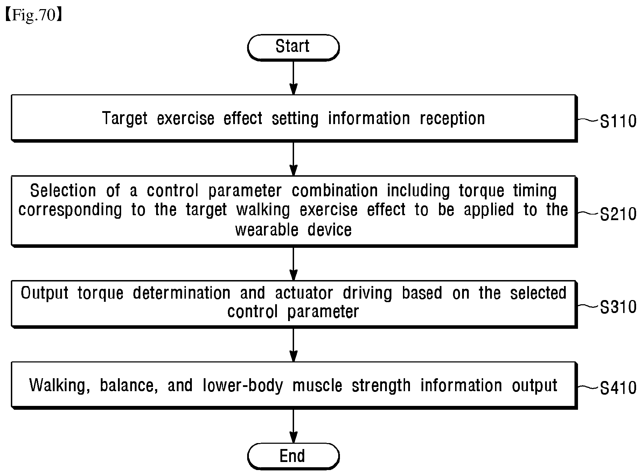

FIG. 70 is a diagram for explaining a method of supporting selective walking strengthening exercise during walking exercise according to an embodiment of the present invention. FIG. 21 is a diagram showing a setting value lookup table matched with a target motion exercise mode according to an embodiment of the present invention.

As shown in FIG. 70, according to an embodiment of the present invention, an embodiment in which a target exercise effect or a target walking ability strengthening effect, rather than a target muscle strength, is selectively set is also possible. Specifically, the method of supporting a target exercise effect or a target walking ability strengthening effect may include a target exercise effect setting information receiving step (S110), a control parameter combination selecting step (S210) including a torque timing corresponding to the target walking exercise effect to be applied to the wearable device, an output torque determination and actuator driving step (S310) based on the selected control parameter, and a walking, balance, and lower-body muscle strength information output step (S410).

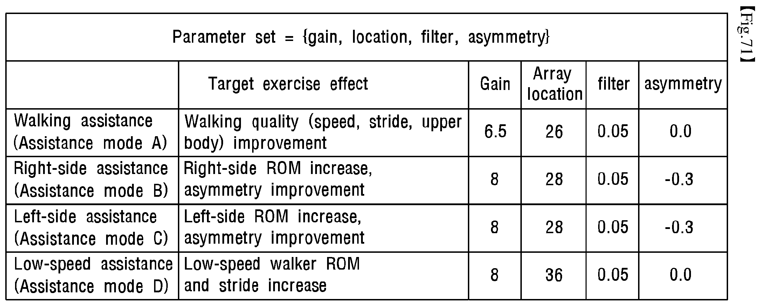

As shown in FIG. 71, a lookup table of each parameter setting value matched with the target motion exercise mode may be provided, but this is only an embodiment, so the scope of rights of the present invention is not limited thereto. Specifically, the Target exercise effect indicates the exercise effect provided by each mode, and this mode may be selected according to the exercise effect desired by the wearer. For example, when the wearer desires a walking assistance effect, assistance mode A may be executed, and this may be a mode that improves walking quality such as the speed, stride, and upper-body stability of walking.

In addition, when the wearer desires a right-side assistance effect, assistance mode B may be executed, and this may be a mode that increases the ROM (range of motion) of the right leg and improves asymmetry. In addition, when the wearer desires a left-side assistance effect, assistance mode C may be executed, and this may be a mode that increases the ROM of the left leg and improves asymmetry. In addition, when the wearer desires a low-speed assistance effect, assistance mode D may be executed, and this may be a mode that increases the ROM and stride of a low-speed walker.

According to an embodiment of the present invention, the torque intensity adjustment parameter (Gain value) may be a value that sets the output intensity occurring during exercise, and in assistance mode A it is set to 6.5 to provide an appropriate force necessary for improving walking, and in the other modes (B, C, D) it is set to 8 to provide a stronger output torque. In addition, in assistance mode A the output torque is calculated at the timing of the 26th position of the motion state value, in assistance modes B and C at the 28th position, and in assistance mode D at the 36th position, and each timing may be set in accordance with the purpose of the exercise. In addition, that a filter parameter and an asymmetry parameter may be set in each mode is as described above.

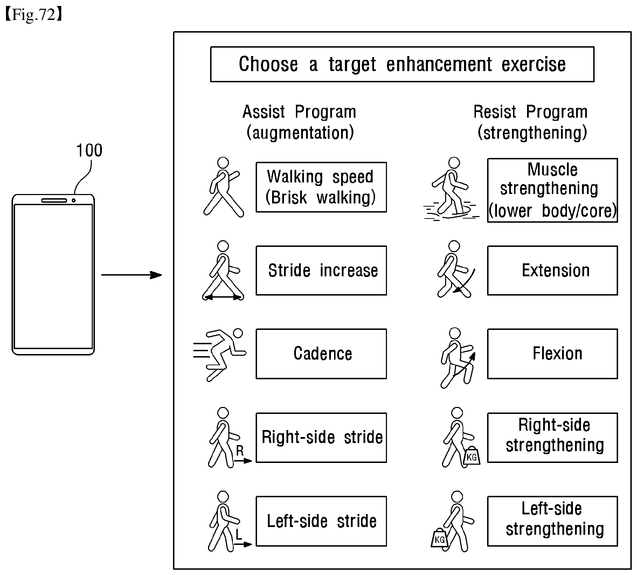

FIG. 72 is a diagram showing a target improvement motion setting user interface according to an embodiment of the present invention.

As shown, a target exercise setting user interface may be provided through the user terminal (100). In addition, the exercise mode mainly used by the wearer may be set/changed by a button (not shown) attached to the wearable robot (200), but the various target exercise modes as shown may be transmitted to the receiving unit (not shown) of the wearable robot (200) through the interface of the user terminal (100).

According to the present invention, the target exercise may be an exercise by motion item for improving a target walking function. Specifically, it may be divided into an Assist Program using assistance torque and a Resist Program using resistance torque, and each may be a walking-function strengthening exercise through mobility augmentation and muscle strengthening (strengthening). That is, by adjusting each parameter including the torque intensity adjustment parameter within each of the Assistive walking and Resistive walking exercise programs, an effect of giving more various exercise effects or strengthening a specific walking motion function may be provided.

According to an embodiment of the present invention, the Assist program using assistance torque may provide effects of walking posture improvement, correction, and increase of mobility motion (stride increase), and the Resist program using resistance torque may provide an effect of strengthening the muscle strength used during walking rather than a posture improvement effect, but it is not limited to the above-described effects. For example, when the wearer receives assistance torque and walks fast with a wide stride and an upright posture through a brisk walk, the lower-limb muscle stimulation becomes greater and also the upright posture of the upper body and the use of the core muscles increase, compared to non-wearing of the wearable robot (200) or slow walking. That is, even if the stride increase is not achieved by the resistance torque in the lower-limb/core mode, an effect in which the stride naturally increases when the device is not worn after resistance training may also be provided.

According to an embodiment of the present invention, when the wearer performs walking with a wide stride and a fast speed in the walking speed increase (Brisk walking) mode, a larger assistance force may be applied adaptively to the wearer compared to a narrow-stride, slow-speed walk. Accordingly, the wearable robot (200) induces the wearer to continue a wide-stride walk, that is, a brisk walk. In addition, experts recommend a brisk walk during walking exercise, and the wearable robot (200) has an effect of making it easy to continue a correct-style walking posture. In particular, in the case of the elderly or patients, when performing a brisk walk, they tend to become easily fatigued and perform an undesirable narrow-stride slow walk, and the wearable robot (200) of the present invention may also provide an effect of improving this.

According to an embodiment of the present invention, when the walking speed of the drive unit (220) is raised during walking exercise, the exercise intensity of the wearable robot (200) increases. That is, when the walking speed of the drive unit (220) is increased, there is an effect that the exercise intensity of the wearer increases, and in addition, when the speed of the drive unit (220) is increased, the resistance torque of the drive unit (220) increases, so there is an effect that the exercise intensity increases. In addition, when the walking speed increases, the plantar reaction force when the wearer lands on the ground increases, and this leads to an increase in the moment of each lower-limb joint portion (Hip/Knee/Ankle), which may lead to an increase in exercise intensity and strengthening of muscle stimulation. In addition, due to reasons such as a decline in lower-limb muscle strength caused by aging, in the case of the elderly, their usual walking speed falls by about 21 % compared to young people. The present invention may provide an effect of delaying or improving such a rate of decline in walking ability.

According to still another embodiment of the present invention, unlike the brisk walking mode in which the assistance timing is automatically adjusted adaptively in accordance with the wearer's movement, the wearer may also fix the assistance timing. That is, the stride increase mode may induce stride increase training by intentionally delaying the assistance timing so that the larger the wearer takes the walking stride, the smaller the resistance feeling due to the wearer-device mismatch becomes. For example, when the assistance timing is later than the appropriate assistance timing (when the position of the state array stored in the state trajectory memory buffer is moved to the latter part), an effect may be provided in which the resistance feeling is greatly felt when performing a narrow-stride walk in which the leg is lowered immediately after the hip swing, but the resistance feeling becomes smaller when performing a wide-stride walk in which the stride is enlarged after the hip swing and then the leg steps out.

According to an embodiment of the present invention, in order to appropriately assist a walking motion that increases the cadence (number of steps per minute), the present invention may adjust the filter parameter to raise the sensing sensitivity compared to slow walking, and move the state array position to the initial part. That is, the present invention may provide a cadence mode to a wearer who needs to intensively raise the cadence rather than the stride. In addition, the cadence mode may also be utilized when a cadence increase is needed during running exercise.

According to an embodiment of the present invention, the right-side stride increase mode may perform control that gives a larger assistance during right-step advance compared to left-step advance. Specifically, the control that gives a larger assistance during right-step advance compared to left-step advance may be handled by adjusting the asymmetry parameter a. In this case, if the value of a is negative, the assistance torque of the left step motion increases compared to the right step motion, and if the value of a is positive, the assistance torque of the right step motion may increase compared to the left step motion. That is, the drive unit (220) may adjust the left or right asymmetric assistance with one parameter. In other words, the left/right asymmetry is determined by the sign of the value of a, and the degree of asymmetry may be adjusted by the magnitude of the value of a. For example, in the case of a=0.20 rather than a=0.10, the drive unit (220) may provide the wearer with an effect of further strengthening the right-side asymmetric assistance. In addition, the value of a has a value between -1.0 and 1.0, and it is preferable that mainly a value of -0.5 to 0.5 is used.

According to an embodiment of the present invention, the lower-limb/core exercise mode may apply resistance torque to the wearer during walking, and this may be an exercise mode for strengthening the overall lower-limb muscle strength and the spinal core muscles. That is, by applying resistance torque during walking, an effect may be provided in which the wearable device applies a resistance force as if by water when walking in water. In addition, the lower-limb/core exercise mode may be performed by fixing the resistance timing, and by adjusting the resistance timing, that is, the position of the state variable array, an effect of making the main exercise muscle strengthening region different may be provided.

According to an embodiment of the present invention, in the case of the Extension mode, by setting the state variable array position to the initial part, an Extension motion strengthening exercise during walking may be performed. In addition, in the case of the Flexion mode, by setting the state variable array position to the latter part, a Flexion motion strengthening exercise during walking may be performed. Specifically, if the asymmetry parameter a value is used and the gain value is set to a negative number, an asymmetric resistance torque may be applied during walking. Therefore, in the case of the right-side strengthening mode, by giving a muscle load of right-leg Flexion and left-leg Extension, the motion during the right advance step may be strengthened. In addition, in the case of the left-side strengthening mode, conversely, by giving a muscle load of left-leg Flexion and right-leg Extension, the motion during the left advance step may be strengthened.

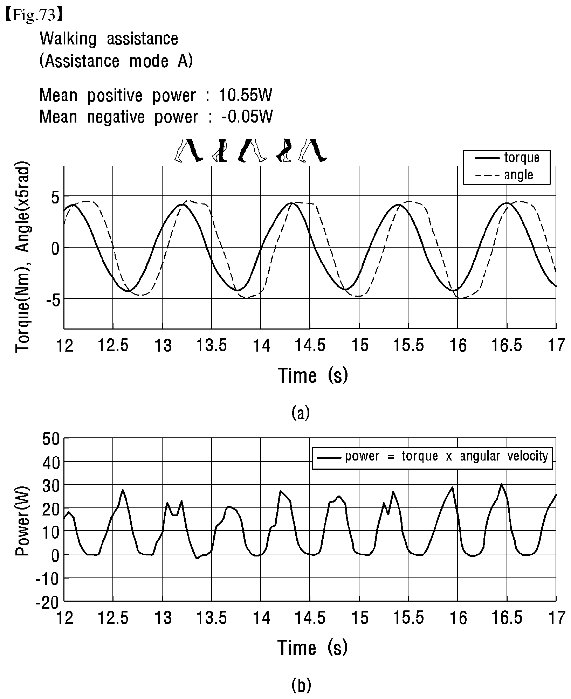

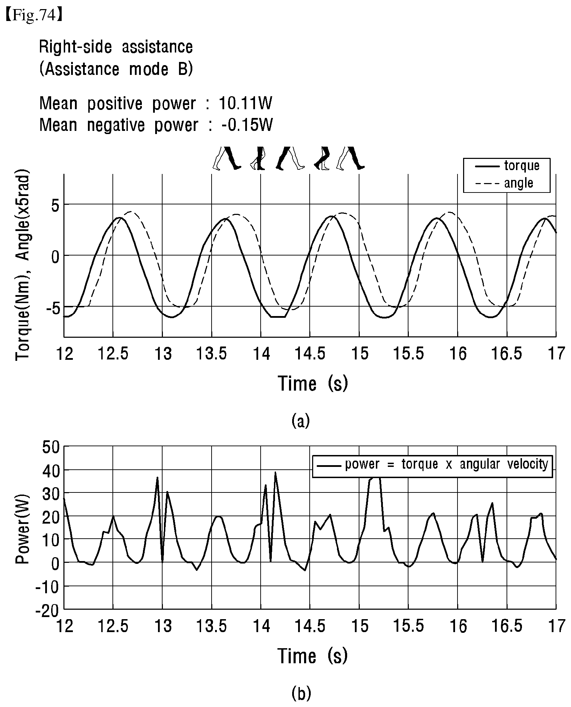

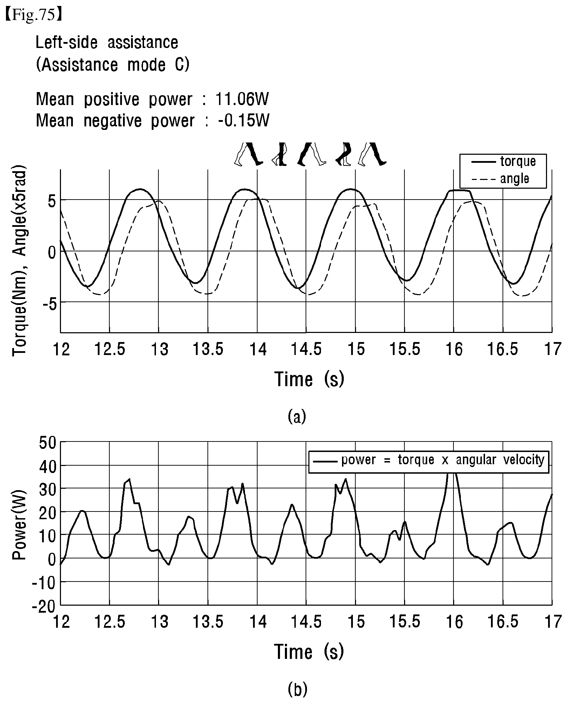

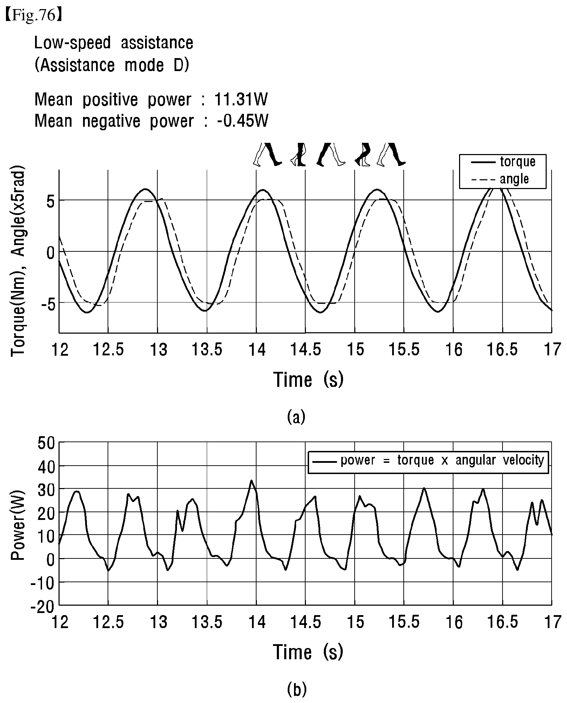

FIG. 73 is a diagram showing the torque and power profiles of assistance mode A according to an embodiment of the present invention. FIG. 74 is a diagram showing the torque and power profiles of assistance mode B according to an embodiment of the present invention. FIG. 75 is a diagram showing the torque and power profiles of assistance mode C according to an embodiment of the present invention. FIG. 76 is a diagram showing the torque and power profiles of assistance mode D according to an embodiment of the present invention.

As shown, when the wearer desires a walking assistance effect, the wearer may input information related to walking assistance through the user interface. In this case, the drive unit (220) may execute assistance mode A, and it can be confirmed that, for the y-axis torque and angle along the x-axis time, the torque (continuous line) and the angle (dotted line) periodically change. In addition, in assistance mode A, positive torque mainly occurs, and with the average positive power being 10.55 W and the negative power being -0.05 W, it can be confirmed that positive power mainly occurs and performs the role of assisting walking. In addition, for the y-axis Power (torque x angular velocity) along the x-axis time, positive power mainly occurs, and it can be confirmed that this occurs in the section where the wearer exerts force while receiving assistance. In this case, negative power is almost absent, and in assistance mode A, an exercise pattern of emitting force rather than absorbing energy can be confirmed.

As shown, when the wearer desires a right-side assistance effect, the wearer may input information related to right-side assistance through the user interface. In this case, the drive unit (220) may execute assistance mode B, and it can be confirmed that the y-axis torque and angle along the x-axis time periodically change. In the case of assistance mode B, it is effective in increasing the ROM (Range of Motion) of the right leg, and with the average positive power being 10.11 W and the negative power being -0.15 W, slightly more negative power occurs than in assistance mode A, but positive power mainly occurs. This indicates that assistance mode B has an effect of assisting the wearer so that the wearer can use the right leg more actively. In addition, for the y-axis Power (torque x angular velocity) along the x-axis time, positive power mainly occurs, and although the negative power is slightly larger, it can be confirmed that positive power is still dominant.

As shown, when the wearer desires a left-side assistance effect, the wearer may input information related to left-side assistance through the user interface. In this case, the drive unit (220) may execute assistance mode C, and it can be confirmed that the change of the y-axis torque and angle along the x-axis time occurs similarly. Assistance mode C is effective in improving the range of motion of the left leg, and with the average positive power being 11.06 W and the negative power being -0.15 W, it can be confirmed to be similar to assistance mode B. In addition, it can be confirmed that assistance mode C is effective in increasing the ROM of the left leg. In addition, for the y-axis Power (torque x angular velocity) along the x-axis time, positive power mainly occurs in the left leg, and the negative power is at the same level as in assistance mode B, so assistance mode C may provide an effect of making the wearer use the left leg more actively.

As shown, when the wearer desires a low-speed assistance effect, the wearer may input information related to low-speed assistance through the user interface. In this case, the drive unit (220) may execute assistance mode D, and it can be confirmed that the y-axis torque and angle along the x-axis time periodically change. Low-speed assistance mode D is effective in increasing the ROM and stride targeting low-speed walkers (walkers in which the distance between the two feet is short when stepping out with the other foot after stepping out with one foot), and the average positive power is shown to be 11.31 W and the negative power -0.45 W. That is, relatively more negative power occurs, so low-speed assistance has an effect of helping the control and resistance of the muscles. In addition, for the y-axis Power (torque x angular velocity) along the x-axis time, positive power mainly occurs, but the negative power can also be seen to be larger compared to the other modes. This indicates that the low-speed assistance mode is effective in helping walking while withstanding resistance.

According to the present invention, assistance mode A improves the overall walking quality, and positive power mainly occurs, so it is effective in actively assisting walking. In addition, assistance modes B and C aim to increase the ROM of the right and left legs, respectively, and positive power mainly occurs but some negative power also appears, so they are effective for muscle control and assistance. In addition, assistance mode D is a mode for low-speed walkers, and relatively more negative power occurs, so it is effective in developing the resistance of the muscles.

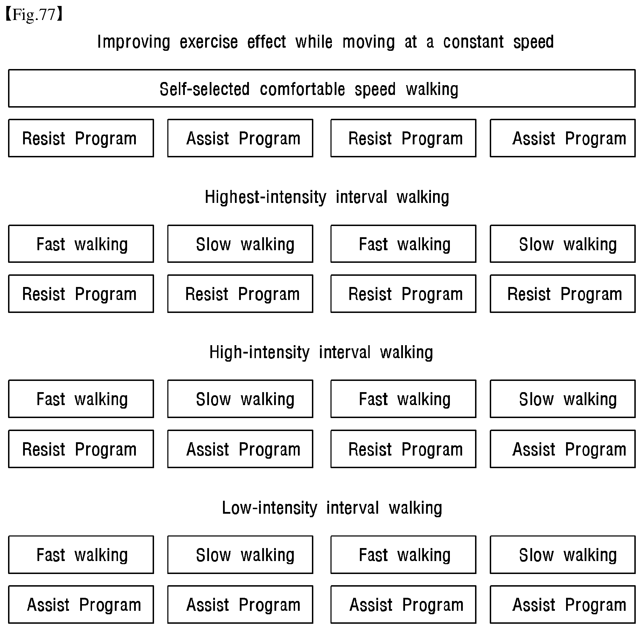

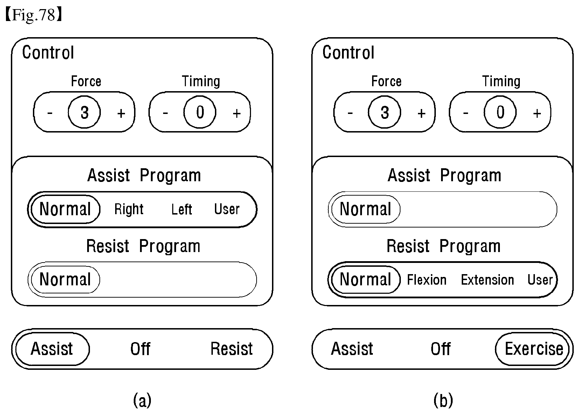

FIG. 77 is a diagram showing an interval walking exercise program according to an embodiment of the present invention. FIG. 78 is a diagram showing an assistance/resistance program user interface according to an embodiment of the present invention.

As shown, when performing interval walking that gives variation to the walking intensity, the exercise effect may increase by 2 to 3 times. In this case, using the wearable robot (200) of the present invention can further amplify the effect. This is because, by alternately performing the Assist Program and the Resist program during movement, the contraction and relaxation of the muscles can be more effectively performed. The wearer moves at the original speed, but an interval exercise effect can be given only by the Assist-resist switching. Alternatively, the exercise intensity may be further raised by using resist torque in a high-intensity (walking fast) section, or the muscle relaxation effect may be heightened by using assist torque in a low-intensity (walking slowly) section.

As shown in FIG. 77, the Assist program may be one of the assistance exercise modes belonging to the Assist program, and the Resist program may be one of the resistance exercise modes belonging to the Resist program. Specifically, in self-selected comfortable speed walking (Self-selected comfortable speed), the Resist Program and the Assist Program are applied alternately, and the wearer does walking exercise at a comfortable speed selected by oneself, and the resistance mode and the assistance mode are repeatedly applied, which has an effect of giving various stimuli to the muscles. In addition,

in the resistance mode, the leg muscles withstand resistance and can exert greater force, and in the assistance mode, there is an effect that the robot assists the muscles to make walking smooth and easy.

As shown, the highest-intensity interval walking alternates between walking fast and walking slowly, and has an effect of maximizing the exercise intensity. The Resist Program is applied in all phases, and it may proceed in a pattern of strengthening muscle strength while withstanding resistance during fast walking, and recovering the muscles during slow walking. This may mainly provide an effect of developing strong muscle strength and cardiorespiratory endurance.

As shown, high-intensity interval walking repeats walking fast and walking slowly, and the Resist Program and the Assist Program may be applied alternately. In the fast-walking section, the resistance mode is applied to strengthen muscle strength, and in the slow-walking section, the assistance mode is applied to recover muscle fatigue while still providing a certain exercise effect. This phase may also provide effects of muscle strength strengthening and improvement of walking efficiency.

As shown, low-intensity interval walking may apply the Assist Program in both the fast-walking and slow-walking sections. Through low-intensity exercise, walking ability can be maintained and improved through assistance without giving a large burden to the muscles. This may be mainly suitable for recovery exercise or for improving walking stability.

As shown in FIG. 78, the user may select assist, resist, or exercise through the user terminal (100), and as in (a) of FIG. 78, when the assist program is selected, a user interface may be provided so that the user can select the normal, right, left, and user buttons. In addition, a user interface may be provided so that the user can select force and timing, but it is not limited thereto. In addition, as in (b) of FIG. 78, when the user selects the exercise program, a user interface may be provided so that the user can select the normal, right, left, and user buttons. In addition, a user interface may be provided so that the user can select force and timing, but it is not limited thereto.

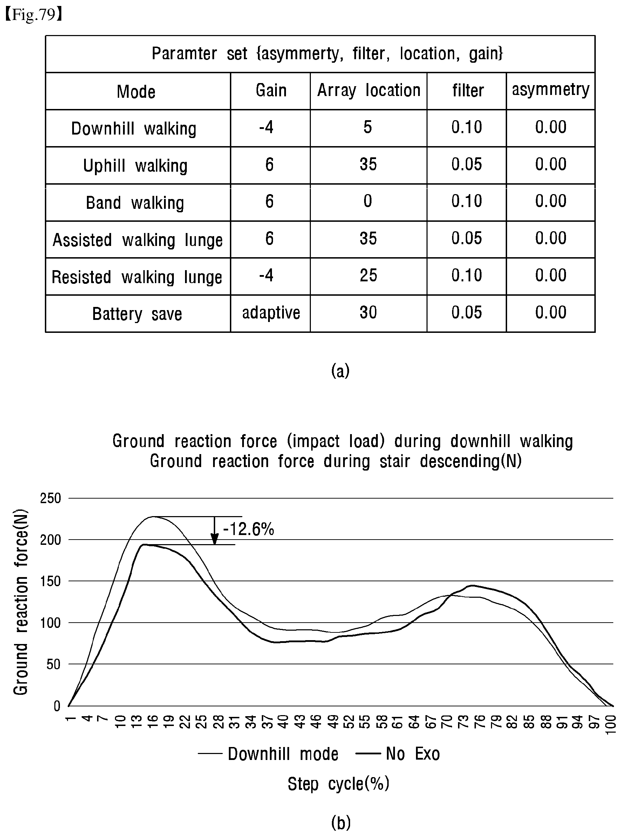

FIG. 79 is a diagram showing additional control modes suited to situations such as downhill and uphill walking according to an embodiment of the present invention.

As shown, just as an exercise mode capable of giving various exercise effects can be created through the parameter set setting as described above, an embodiment that provides an interaction torque suited to a specific environment and walking pattern by adjusting the parameters is also possible. For example, the wearable robot (200) may, for knee protection during downhill walking in mountain climbing, apply a supporting resistance force capable of reducing the foot-step impact amount with a hip torque when the foot lands. Alternatively, a mode that automatically lowers the torque intensity adjustment parameter (gain) setting in order to increase the device usage time is also possible as an embodiment, but it is not limited thereto.

As shown in (a) of FIG. 79, additional control modes suited to various environments and situations may be executed. For example, in the downhill walking mode, the torque intensity adjustment parameter (Gain) is -4, the Array location is the 5th position, the Filter is 0.10, and the Asymmetry is 0.00, which may represent the effect of a left-right symmetric walk. That is, this is a setting for reducing the ground reaction force and impact on a downhill, and the wearable robot (200) of the present invention may provide the exercise effect necessary therefor on a downhill.

As shown in (b) of FIG. 79, the x-axis is the Step cycle %, representing each section of the walking cycle from 0% to 100%. This may mean the time from the moment one foot is stepped out until one cycle ends. In addition, the y-axis is the Ground reaction force, representing the magnitude of the ground reaction force, and may be measured in units of N (Newton). It may represent the foot-step impact applied to the ground. Specifically, it can be confirmed that the ground reaction force in the case of No Exo, that is, when the robot is not used, reaches up to about 250 N, but when the wearable robot (200) of the present invention uses the downhill mode, the ground reaction force is reduced by 12.6% to down to about 190 N. That is, when the wearer uses the downhill mode, the wearable robot (200) may provide an effect of reducing the impact with the ground when the foot lands, thereby relieving the burden applied to the knee and joints.

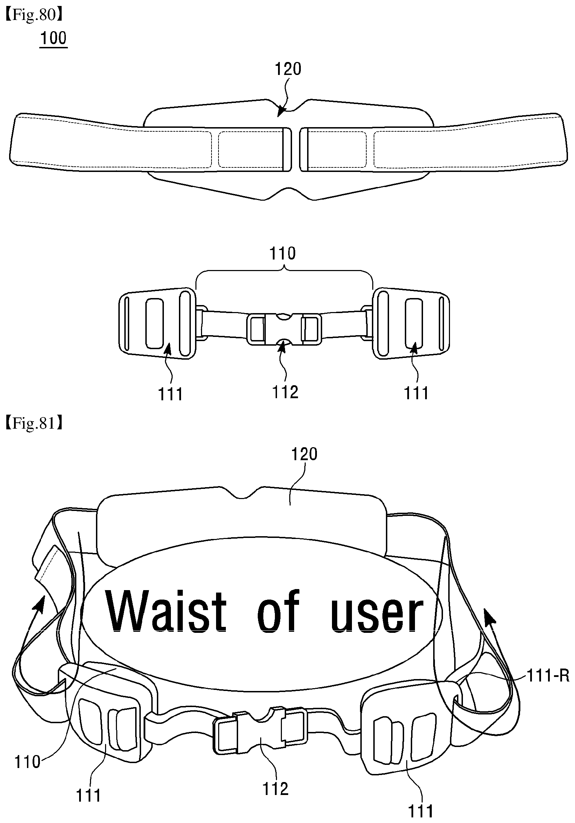

FIG. 80 is a diagram for explaining the waist wearing unit and the waist wearing buckle portion of the present invention.

As shown, the waist wearing unit (100) of the present invention may include a waist wearing buckle portion (110) and a waist wearing unit member (120). Specifically, the waist wearing unit member (120) may, when the wearer wears the wearable robot (10), perform the role of wrapping around the waist and allowing it to be mounted on the body, thereby supporting a part of the weight of the wearable robot (10). In addition, the waist wearing unit member (120) may be designed so as to maintain a fixed state even during movement while wrapping around the wearer's waist. In addition, the waist wearing unit member (120) may be in close contact so that the waist part does not shake when the wearer walks or moves, and may have a structure adjustable to fit the wearer's body shape. In addition, the waist wearing unit member (120) is designed to fit smoothly along the wearer's waist curve, so that the wearing feeling is comfortable and safe. For this purpose, an ergonomic material and structure may be used for the waist wearing unit member (120), and the burden may be minimized even during long-term wearing.

According to an embodiment of the present invention, since the waist wearing unit member (120) plays the role of supporting the wearer's motion, it may have strong durability and may be manufactured of a material capable of safely protecting the waist during wearing.

As shown, the waist wearing unit (100) of the present invention may include a waist wearing buckle portion (110), and the waist wearing buckle portion (110) may include a drive unit fixing member (111) and a buckle member (112). Specifically, the buckle member (112) may, independently of the drive unit fixing member (111), function as an additional auxiliary device that can enhance the fixity of the waist wearing unit (100) itself. That is, the buckle member (112) may perform the function of connecting the waist wearing unit at the center of the body. Although the two drive unit fixing members (111) each couple to the drive unit (200) to provide stability, when the buckle member (112) is present, the structural stability of the entire waist wearing unit may be further strengthened.

In particular, the structure wrapping around the waist becomes firm, and the wearer can use the robot more safely. In addition, when the buckle member (112) is present, the pressure that the drive unit fixing member (111) gives to the waist may be partially dispersed at the center. Therefore, the buckle member (112) may increase the comfort during wearing and reduce the force concentrated on the waist. In addition, if the wearer moves abruptly or a strong impact is applied to the waist side, since the buckle member (112) fixes the position of the waist wearing unit (100), the drive unit (200) may be maintained more precisely in place.

The drive unit fixing member (111) according to the present invention is described in detail below.

FIG. 81 is a diagram for explaining a Velcro fixing embodiment of the waist wearing unit and the waist wearing buckle portion of the present invention.

As shown, the waist wearing unit member (120) and the waist wearing buckle portion (110) are configured to be fixed by a band by the waist wearing unit member connection portion (111-R) of the drive unit fixing member (111) of the waist wearing buckle portion (110). In addition, the waist wearing unit member (120) and the waist wearing buckle portion (110) are configured to be passed through by the waist wearing unit member connection portion (111-R) of the drive unit fixing member (111) of the waist wearing buckle portion (110) and fixed by Velcro.

According to an embodiment of the present invention, the band may perform the function of adjusting the waist wearing unit member (120) to be in close contact with the wearer's waist. That is, the band may perform the role of wrapping around the entire waist wearing unit (100) so that the drive unit (200) is stably mounted on the wearer. In addition, the waist wearing unit (100) may wrap around the waist through the band, and may be adjusted to fit the wearer's body size. That is, the band structure of the waist wearing unit member (120) may provide flexibility to tighten the band to firmly fix the waist, or, when necessary, to adjust it more loosely. In addition, the band applies pressure uniformly to the wearer's waist to give a sense of stability so that the wearing unit does not shake, and may efficiently transmit the force generated by the drive unit (200).

According to an embodiment of the present invention, the Velcro may perform the role of fixing the band to the waist wearing unit (100). The wearer may easily wear the waist wearing unit (100) through the Velcro, and may, as needed, adjust the length of the band to fit it to the waist circumference. In addition, the Velcro has strong adhesion, so the waist wearing unit (100) does not easily come loose, and stability may be maintained. Since the waist wearing unit (100) can be quickly adhered or detached when fixing it, wearing and disassembly are very easy, so convenience may be provided to the wearer.

According to an embodiment of the present invention, the buckle member (112) may perform the role of fixing the waist wearing unit (100) at the center. That is, the buckle member (112) may connect both ends of the waist wearing unit (100) to provide a close-contact force so that the entire waist wearing unit (100) is in close contact with the wearer's waist. The wearer may firmly fix the waist wearing unit (100) to the wearer's waist through the buckle member (112), and through the connection with the drive unit (200), the wearable robot (10) can stably support the wearer's movement.

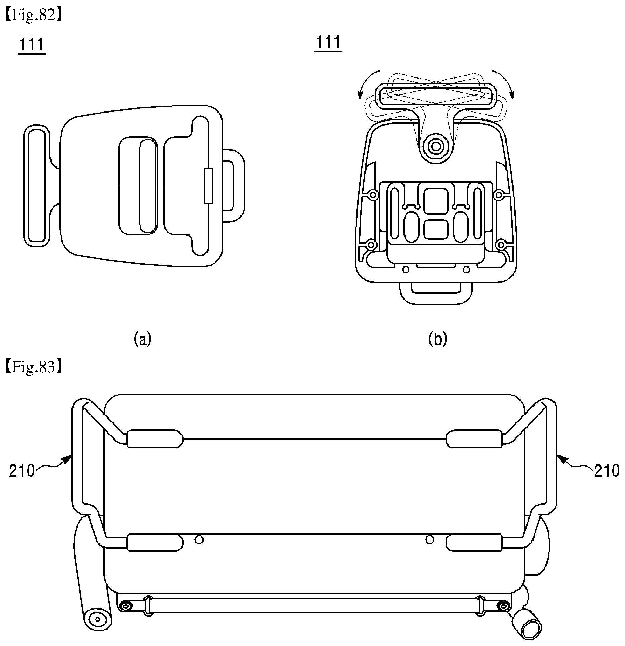

FIG. 82 is a diagram for explaining the drive unit fixing member according to the present invention.

As shown in (a) of FIG. 82, the drive unit fixing member (111) may include the waist wearing unit member connection portion (111-R) shown in FIG. 81, but may also include a separate swing member (111-S). The swing member (111-S) couples with the drive unit fixing member (111) to enable the waist wearing unit (100) to move finely in accordance with the wearer's movement. As in (b) of FIG. 82, the swing member (111-S) has a structure that can rotate at an angle of 0 degrees or more and 30 degrees or less to the left and right, so it may support the natural movement of the wearer's waist.

According to an embodiment of the present invention, the swing member (111-S) allows the coupling with the drive unit (200) not to be rigidly fixed in a state where the waist wearing unit (100) is fixed, but to be able to move flexibly within a certain angle. Through this, the wearer may comfortably turn or move the waist or upper body without restriction. Accordingly, the swing member (111-S) may provide the wearer with an effect of minimizing the pressure due to movement and reducing discomfort even when the wearer takes various postures.

Hereinafter, the role of the angle of the drive unit fixing frame (210) will be described in FIG. 83, but when the angle of the drive unit fixing frame (210) is 0 degrees, a flexible wearing feeling may be provided to the wearer due to the swing member (111-S). However, even in the case where the angle of the drive unit fixing frame (210) described below is more than 0 degrees and 30 degrees or less, an embodiment in which the drive unit fixing member (111) still includes the swing member (111-S) in order to provide an even more flexible wearing feeling to the wearer shall also be said to belong to the scope of rights of our invention.

FIG. 83 is a diagram for explaining the drive unit fixing frame according to the present invention.

As shown, the drive unit (200) may include a drive unit fixing frame (210) in order to be connected with the waist wearing unit (100). Specifically, the drive unit fixing frame (210) may be located on the rear surface of the drive unit (200). That is, when the wearer wears the wearable robot (10), it may be configured to be located on the front of the wearer. Hereinafter, the inclination of the drive unit fixing frame (210) is described.

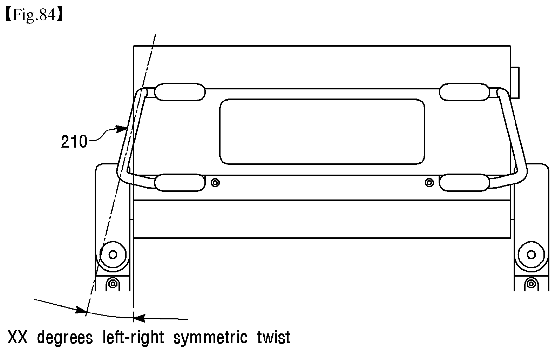

FIG. 84 is a diagram for explaining an embodiment related to the left-right symmetric twist angle of the drive unit fixing frame according to the present invention.

As shown, the drive unit fixing frame (210) may be configured to have an inclination of 0 degrees or more and 30 degrees or less with respect to the vertical plane of the drive unit (200). Specifically, the fact that the drive unit fixing frame (210) is inclined may provide an effect of being adjusted to fit the wearer's body curve when the wearable robot (10) is worn. Since the wearer's waist or upper body is not perfectly flat but has a curved shape, when the drive unit fixing frame (210) is inclined, it may be in even more natural close contact with the wearer's waist or back part.

According to an embodiment of the present invention, when the drive unit (200) is in close contact with the wearer's body, the operation efficiency of the wearable robot (10) may be increased. That is, the closer the drive unit (200) is to the body, the more effectively the operation of the wearable robot (10) can respond to the wearer's movement. Therefore, the force generated by the drive unit (200) is transmitted more directly through the wearer's waist or upper body, so the wearable robot (10) can respond more accurately and quickly. In addition, in a state where the drive unit (200) is in close contact, the movement of the wearable robot (10) and the wearer may be more naturally synchronized. That is, due to the inclined angle, the wearer may receive the feeling that the wearable robot (10) is accurately following one's own movement when moving the body.

According to an embodiment of the present invention, when the drive unit fixing frame (210) is inclined, the pressure with which the drive unit (200) contacts the wearer's body may be evenly distributed. Instead of the drive unit (200) applying pressure only to a specific part, through the inclined drive unit fixing frame (210), the force is divided into several parts so the robot can be worn more stably. Accordingly, since the pressure is distributed, the wearer does not feel excessive pressure on a specific part even when wearing the robot for a long time. In addition, since the risk of the robot shaking or coming off when the wearer moves is reduced, the present invention may provide an effect in stably maintaining the center of gravity of the wearable robot (10).

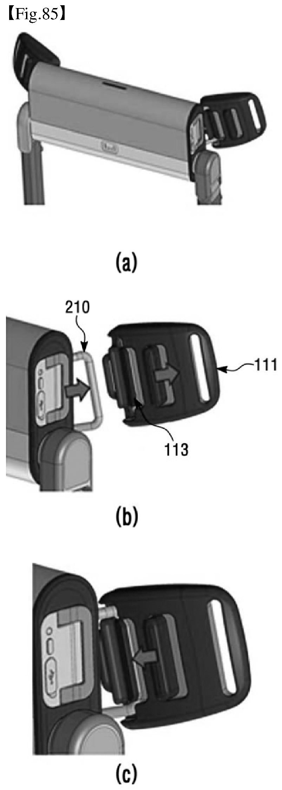

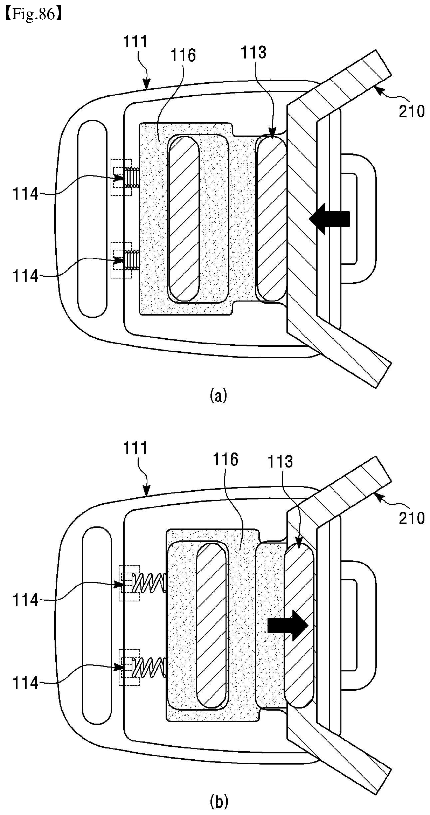

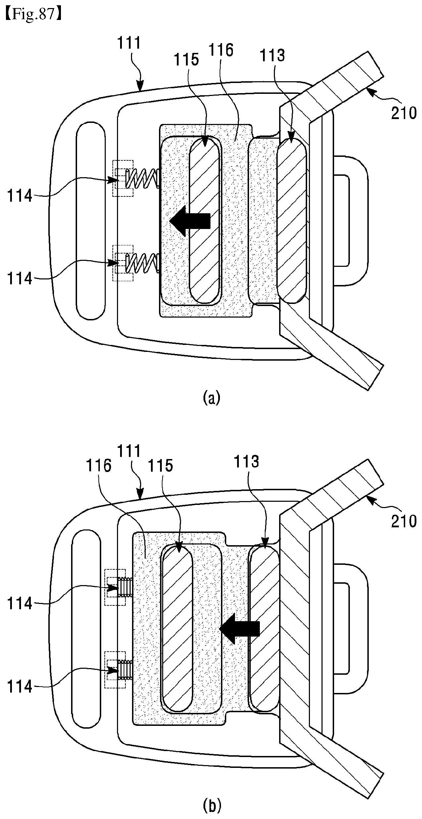

FIG. 85 is a diagram for explaining an embodiment of mounting the drive unit fixing member to the drive unit fixing frame according to the present invention. FIG. 86 is a diagram for explaining an embodiment related to the button member of the drive unit fixing member according to the present invention. FIG. 87 is a diagram for explaining an embodiment related to the button member handle portion of the drive unit fixing member according to the present invention.

As shown in FIG. 85, the drive unit fixing member (111) may be coupled to the drive unit fixing frame (210), and conversely, the drive unit fixing member (111) may be released from the drive unit fixing frame (210).

As shown in FIG. 86, the drive unit fixing member (111) may include an inner connection member (116), and may include a button member (113) located at the inner connection member and an elastic member (114) located inside the waist wearing buckle portion (110).

According to an embodiment of the present invention, in order to couple the drive unit fixing member (111) to the drive unit fixing frame (210), the drive unit fixing frame (210) may contact the button member (113). In this case, the inner connection member (116) and the button member (113) may move in the advancing direction of the drive unit fixing frame (210), and at this time the elastic member (114) may contract in the advancing direction of the drive unit fixing frame (210). Thereafter, the drive unit fixing frame (210) is coupled to the drive unit fixing member (111) by the button member (113), and the elastic member (114) relaxes so that the inner connection member (116) may move in the direction opposite to the advancing direction of the drive unit fixing frame (210). Accordingly, the drive unit fixing member (111) may perform the role of preventing the drive unit fixing frame (210) from coming off.

According to an embodiment of the present invention, the button member (113) may be configured to have an inclined portion. That is, when the drive unit fixing frame (210) contacts the button member (113), it may be configured such that the drive unit fixing frame (210) contacts the inclined portion of the button member (113) and moves in the advancing direction. That is, by the inclined surface of the button member (113), the operation is sufficiently possible with less force even when the button member (113) is pressed, and the direction in which the button member (113) moves while being pushed coincides with the operation direction desired by the user, so force can be transmitted efficiently. In addition, when there is no inclined surface, the surface of the button member (113) is flat so the finger may slip, but when there is an inclined surface, the finger is naturally fixed so slipping can be prevented.

As shown in FIG. 87, the drive unit fixing member (111) may include an inner connection member (116), and may include a handle member (115) located at the inner connection member, a button member (113) located at the inner connection member, and an elastic member (114) located inside the waist wearing buckle portion (110).

According to an embodiment of the present invention, in order to release the coupling of the drive unit fixing member (111) from the drive unit fixing frame (210), the handle member (115) may be configured to be movable in the advancing direction at the time of coupling of the drive unit fixing frame (210). In this case, the inner connection member (116) may move in the advancing direction at the time of coupling of the drive unit fixing frame (210), and the elastic member (114) contracts in the advancing direction at the time of coupling of the drive unit fixing frame (210). Accordingly, the drive unit fixing frame (210) may be released from contact with the button member (113).

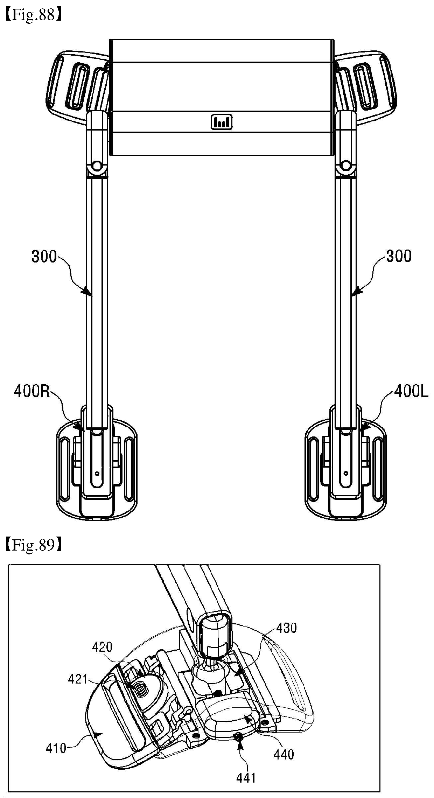

FIG. 88 is a diagram for explaining the detailed configuration of the thigh wearing unit according to the present invention.

According to an embodiment of the present invention, the thigh frame (300) is located on both thighs, and may connect the waist wearing unit (100) and the thigh wearing unit (400) of the wearable robot (10). Specifically, the thigh frame (300) may connect the waist wearing unit (100) and the thigh wearing unit (400) to transmit the output torque to the user. In addition, the thigh frame (300) may be mainly manufactured of metal or a lightweight composite material, and may have a length and rigidity suited to the user's body shape.

According to an embodiment of the present invention, the thigh wearing units (400R, 400L) are worn on the wearer's left and right thighs, and may be ergonomically designed in consideration of wearing feeling and stability. Specifically, the thigh wearing units (400R, 400L) may be composed of 400R (right) and 400L (left), and may each be connected to the end of the thigh frame (300). In addition, the inside of the thigh wearing units (400R, 400L) may have a soft padding or strap form that directly contacts the wearer's leg, and a mechanism (e.g., a hinge structure or a rotation axis) that follows the user in accordance with movement may be included.

FIG. 89 is a diagram for explaining the operation of the thigh wearing unit according to an embodiment of the present invention.

As shown, the thigh wearing unit (400) of the present invention includes a thigh buckle portion (410), a thigh buckle release button (420), a thigh buckle release button elastic member (421), a fixing clip portion (430), a thigh frame release button (440), and a thigh frame release button elastic member (441).

### Thigh buckle portion (410)

According to an embodiment of the present invention, the thigh wearing unit (400) may include a thigh buckle portion (410). Specifically, the thigh buckle portion (410) can adjust the length to fit the user's thigh circumference through the thigh band portion (470), so it may provide a customized wearing feeling suitable for users of various body shapes. In addition, the thigh buckle portion (410) performs tightening and fixing functions, and may be stably designed so as not to slip or loosen during use. In addition, the thigh buckle portion (410) operates in conjunction with the thigh buckle Release button (420), and when the thigh buckle Release button (420) is pressed, the internal fixing structure is released so that the thigh buckle portion (410) can be simply attached and detached. When the user releases the thigh buckle Release button (420), the thigh buckle Release button (420) and the fixing structure may be automatically restored to the original state by the thigh buckle Release button elastic member (421). Due to such an operating manner, the present invention may allow the user to easily operate the buckle even with one hand, thereby providing high convenience in the wearing and removal process.

According to an embodiment of the present invention, the thigh buckle portion (410) is generally manufactured of high-strength plastic or a metal alloy, so it has excellent durability and may maintain stability even during long-term use. In addition, through an ergonomic design, it may softly wrap around the user's thigh, not restricting movement and providing comfort. In addition, the internal locking mechanism stably maintains the tightened state, and may strengthen safety by preventing loosening or coming-off during wearing. In addition, the thigh buckle portion (410) may provide simplification of operation, flexibility according to body shape, and ease of repeated use.

### Thigh buckle Release button (420), thigh buckle Release button elastic member (421)

According to an embodiment of the present invention, the thigh buckle Release button (420) is a button designed to easily attach and detach the thigh buckle portion (410) attached to the thigh wearing unit (400), and can release the buckle with one simple operation. Specifically, when the thigh buckle Release button (420) is pressed, the internal fixing structure is released, and a state is reached in which the thigh wearing unit (400) can be easily removed. In addition, the operating manner of the thigh buckle Release button (420) is ergonomically designed, so a user-centered design may be applied so that it can operate quickly and simply even with one hand.

According to an embodiment of the present invention, the return force of the thigh buckle Release button (420) may be provided by the thigh buckle Release button elastic member (421). Specifically, the thigh buckle Release button elastic member (421) provides a restoring force so that the thigh buckle Release button (420) automatically returns to the original position after being pressed, and may maintain stable performance even when the thigh buckle Release button (420) is repeatedly used. In addition, the thigh buckle Release button elastic member (421) is manufactured of a highly durable material, so it may ensure stable operation without breakdown or performance degradation even during long-term use. In addition, the thigh wearing unit (400) is designed so that users of various body shapes can conveniently wear it, so length adjustment and attachment/detachment can be easily performed through the thigh buckle portion (410) and the thigh buckle Release button (420).

### Fixing clip portion (430)

According to an embodiment of the present invention, the thigh wearing unit (400) may include a fixing clip portion (430). Specifically, the fixing clip portion (430) may be a component designed to stably fix the thigh frame joint portion (450) in the thigh wearing unit (400) and to be easily separable when the wearer needs it. Specifically, the fixing clip portion (430) may maintain the connection between the thigh frame joint portion (450) and the thigh wearing unit (400), thereby providing stability so that the wearable robot (10) does not shake or loosen even during use. In addition, the fixing clip portion (430) may operate in conjunction with the thigh frame Release button (440), and when the user presses the thigh frame Release button (440), the fixing clip portion (430) is released so that the thigh frame (300) can be easily separated.

According to an embodiment of the present invention, the fixing clip portion (430) is manufactured of a high-strength material, so it operates stably even during repeated attachment/detachment processes and may maintain durability. In addition, the fixing clip portion (430) is combined with a Round-shaped joint structure, so it may allow the thigh frame (300) to move at various angles while also providing stable fixation. In addition, the user may stably fix the thigh frame (300) through the fixing clip portion (430) or, when necessary, simply release it. In particular, due to a design that prevents accidental release in the fixed state, high safety may be maintained even during operation. Top of form Bottom of form

### Thigh frame Release button (440), thigh frame Release button elastic member (441)

According to an embodiment of the present invention, the thigh wearing unit (400) may include a thigh frame Release button (440) for switching the thigh frame joint portion (450) from a fixed state to a released state. Specifically, the thigh frame Release button (440) is designed so that the user can easily attach and detach the thigh frame (300), and can switch the thigh frame from the fixed state to the released state with one simple operation. When the thigh frame Release button (440) is pressed, the fixing clip portion (430) is released so that the joint structure of the thigh frame (300) is easily separated, and due to this, the user can remove or reattach the frame with minimal effort.

According to an embodiment of the present invention, the thigh frame Release button (440) may include a thigh frame Release button elastic member (441). Specifically, the thigh frame Release button elastic member (441) may perform the role of providing a return force to the thigh frame Release button (440). Specifically, the thigh frame Release button elastic member (441), which helps the thigh frame Release button (440) automatically return to the original position after the user presses the thigh frame Release button (440), improves the convenience of button operation, and by the button accurately returning to its place, allows the fixing clip portion (430) to operate stably.

The present invention may provide a thigh wearing unit (400) of a walking assistance wearable robot (10) for assisting the wearer's walking exercise. Specifically, the walking assistance wearable robot (10) includes a waist wearing unit (100) that can be mounted on the wearer's waist, a thigh wearing unit (400) that can be mounted on the wearer's thigh, a drive unit (200) connected to the waist wearing unit (100), and a thigh frame (300) connecting the drive unit (200) and the thigh wearing unit (400), the thigh frame (300) includes a thigh frame joint portion (450), the thigh wearing unit (400) includes a fixing clip portion (430), and the fixing clip portion (430) is configured to be able to fix or separate the thigh frame joint portion (450).

According to an embodiment of the present invention, the thigh wearing unit (400) includes a thigh frame release button (440) for switching the thigh frame joint portion (450) from a fixed state to a released state.

According to an embodiment of the present invention, the thigh frame release button (440) further includes a thigh frame release elastic member (441), and the thigh frame release elastic member (441) is configured to provide a return force so that the thigh frame release button (440) can automatically return to the original position after operation.

According to an embodiment of the present invention, the thigh wearing unit (400) may further include a thigh band portion (470) and a thigh buckle portion (410). Specifically, the thigh band portion (470) is configured to be connected to the thigh buckle portion (410) so that the length can be adjusted to fit the wearer's thigh circumference.

According to an embodiment of the present invention, the thigh frame joint portion (450) is designed in a round shape so as to be rotatable, and is configured to flexibly rotate in accordance with the wearer's thigh movement.

According to an embodiment of the present invention, the thigh wearing unit (400) includes a thigh attachment/detachment frame (460), the thigh attachment/detachment frame (460) further includes a sliding rail member (320), and the thigh frame joint portion (450) is configured to be able to move along the sliding rail member (320).

According to an embodiment of the present invention, the thigh wearing unit (400) includes an impact prevention member, and the impact prevention member is located on the inner side of the thigh wearing unit (400) and is configured to be able to relieve the impact or vibration occurring during the sliding operation of the thigh frame (300).

According to an embodiment of the present invention, the sliding rail member (320) further includes a linear guide member (480) in the thigh attachment/detachment frame (460), and the linear guide member (480) is configured to support the sliding movement of the thigh frame joint portion (450). In addition, the linear guide member (480) is configured to guide the movement path of the thigh frame joint portion (450) so as to minimize the vibration and friction occurring during sliding. In addition, the impact prevention member is configured to absorb the impact energy occurring while the thigh frame joint portion (450) slides.

FIG. 4a is a diagram for explaining the thigh frame fixing joint structure according to the present invention.

### Thigh frame joint portion (450)

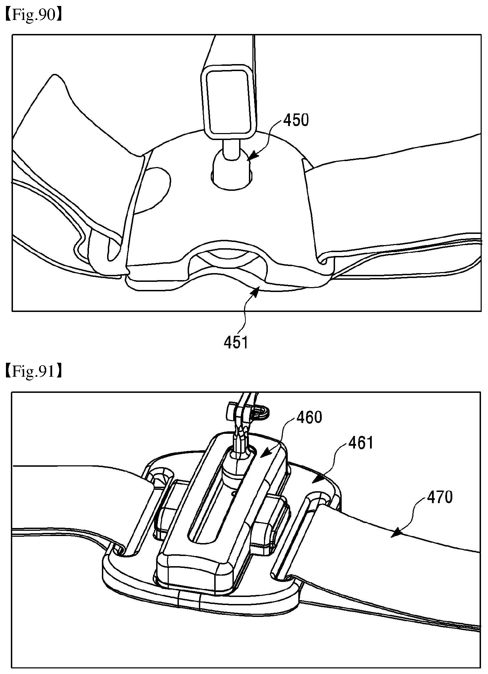

As shown in FIG. 90, the thigh frame joint portion (450) is a configuration that is designed in a round shape and connects the thigh frame (300) and the thigh wearing unit (400). Specifically, the thigh frame joint portion (450) provides flexibility so that the thigh frame (300) can rotate or move at various angles, and allows it to naturally respond to the user's movement. In particular, the thigh frame joint portion (450) performs the function of smoothly connecting the operation between the thigh frame (300) and the thigh wearing unit (400), thereby allowing the wearable robot (10) to adapt to the user's leg movement.

According to an embodiment of the present invention, since the thigh frame joint portion (450) has a round shape, it allows the thigh frame (300) to freely rotate or slide in accordance with the movement of the leg. In addition, the thigh frame joint portion (450) may be designed to operate in conjunction with the thigh frame Release button (440) and the fixing clip portion (430) so as to firmly fix the thigh frame (300) or, when necessary, easily release it.

### Thigh cushion (451)

According to an embodiment of the present invention, the thigh cushion (451) may be an ergonomic component for relieving the pressure that the user receives from the device over a long time during wearing, and for improving the wearing feeling. Specifically, the thigh cushion (451) disperses the pressure that the thigh wearing unit (400) applies to the skin or muscles, and may minimize the discomfort that may occur during wearing. In addition, the thigh cushion (451) is manufactured of a soft material, so it acts as a cushion between the user's thigh and the thigh wearing unit (400), and may protect the skin from friction or stimulation that may occur while the wearable robot (10) moves. In addition, the thigh cushion (451) helps the wearable robot (10) be in more stable close contact with the user's leg, so that the assistance force of the wearable robot (10) can be effectively transmitted.

According to an embodiment of the present invention, the Release state of the thigh frame Release button (440) may mean a state in which the user presses the thigh frame Release button (440) to release the fixing clip portion (430) from the thigh frame joint portion (450). In this state, the thigh frame joint portion (450) is not fixed, so the thigh frame (300) can be easily separated or adjusted. When the thigh frame Release button (440) is pressed, the fixing clip portion (430) moves left and right, so the force that was holding the thigh frame joint portion (450) is released, and a state is reached in which the thigh frame (300) can move freely. The Release state may be mainly used when removing or reattaching the thigh frame (300).

On the other hand, the Locking state of the thigh frame Release button (440) is a state in which the thigh frame Release button (440) is not pressed, and the fixing clip portion (430) is firmly fixing the thigh frame joint portion (450). In this state, the thigh frame (300) is stably fixed so that it does not shake or loosen during use, and allows the thigh frame (300) to effectively transmit the assistance force to the user. The fixing clip portion (430) automatically returns to its place when the thigh frame Release button (440) is not pressed, and may perform the role of firmly holding the thigh frame joint portion (450) from the left and right. In this process, the elastic member (441) may provide the return force of the thigh frame Release button (440) so as to maintain the Locking state.

According to an embodiment of the present invention, the switching between the Release and Locking states may be achieved by a simple operation of the thigh frame Release button (440). When the user presses the thigh frame Release button (440), it switches to the Release state so that the thigh frame joint portion (450) is released, and when the user takes the hand off the thigh frame Release button (440), it may automatically return to the Locking state by the return force of the elastic member. Such a design of the present invention simultaneously ensures the user's convenience and the stability of the device, and may maximize the usability of the wearable robot (10) through an intuitive and simple operating manner. Through the Release and Locking states, the thigh frame (300) maintains stability and reliability even during repeated use, and may operate effectively in various environments.

FIG. 91 is a diagram for explaining the thigh attachment/detachment frame according to the present invention.

### Thigh attachment/detachment frame (460)

As shown, the thigh attachment/detachment frame (460) is connected to the thigh frame joint structure (460) and is designed so as to naturally respond to the wearer's leg movement. The thigh attachment/detachment frame (460) combined with the round-shaped joint structure allows the thigh frame (300) to rotate at various angles or perform a sliding operation, so the frame can stably move while maintaining flexibility even during the user's walking or movement. Accordingly, the wearable robot (10) of the present invention transmits assistance force to the wearer while not obstructing the user's movement.

In addition, the thigh attachment/detachment frame (460) operates together with the thigh band portion (470) and the thigh frame lower member (461) so that the wearable robot (10) is stably attached to the wearer's thigh. In addition, the thigh attachment/detachment frame (460) utilizes the thigh frame Release button (440) and fixing clip portion (430) mechanism so that the thigh frame (300) can be easily separated from the device or reattached, and the thigh attachment/detachment frame (460) may be stably fixed as the thigh frame Release button (440) returns to the original position by the thigh frame Release button elastic member (441).

### Thigh frame lower member (461)

According to an embodiment of the present invention, the thigh frame lower member (461) is a configuration that connects the thigh attachment/detachment frame (460) and the thigh band portion (470) in the wearable robot (10), and connects the thigh attachment/detachment frame (460) and the thigh band portion (470) so that the other components of the wearable robot (10) can operate as an integrated structure. In addition, the thigh frame lower member (461) may perform the role of transmitting the assistance force generated in the thigh attachment/detachment frame (460) to the user's thigh.

According to an embodiment of the present invention, the thigh frame lower member (461) may also perform the role of preventing the wearable robot (10) from shaking or being misaligned in position during wearing. By fixing the thigh band portion (470) and the thigh attachment/detachment frame (460), the discomfort that may occur during movement may be minimized. In addition, the thigh frame lower member (461) is manufactured of a highly durable material, so it can operate stably even with repeated use and in various environments.

### Thigh band portion (470)

According to an embodiment of the present invention, the thigh wearing unit (400) may include a thigh band portion (470). Specifically, the thigh band portion (470) has a structure capable of adjusting the length to fit the user's thigh circumference, so it may provide a wearing feeling suitable for users of various body shapes. The adjustment mechanism of the thigh band portion (470) is generally constituted in a strap or buckle manner, designed so that the user can easily adjust and fix the length. Through this, the wearable robot (10) can be firmly fixed without giving excessive pressure to the user. In addition, the thigh band portion (470) is connected to the thigh attachment/detachment frame (460) and the thigh frame lower member (461) to stably fix the wearable robot (10), and may keep the device from slipping or being misaligned in position even during the user's movement. In addition, the thigh band portion (470) may be designed so that the wearable robot (10) is in close contact with the thigh to effectively transmit the assistance force.

According to an embodiment of the present invention, the thigh band portion (470) is a component that is in direct contact with the user's skin, and may be manufactured of a soft and flexible material in order to minimize the discomfort that may occur during wearing. Such a material reduces stimulation or friction to the skin, and makes the wearer feel less fatigue even when using the device for a long time. In particular, it is designed so that the band flexibly responds in accordance with the user's movement, so it can provide a natural wearing feeling while the device is stably fixed even during walking or movement.

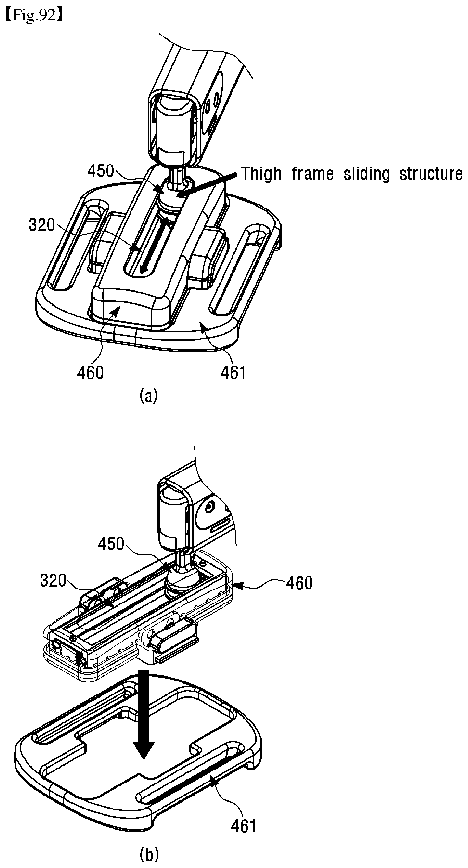

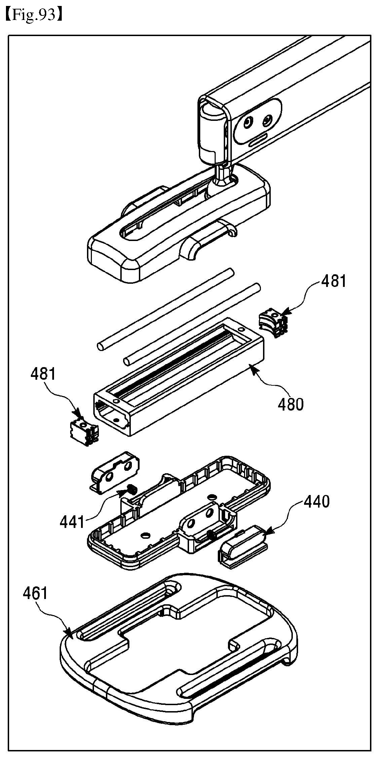

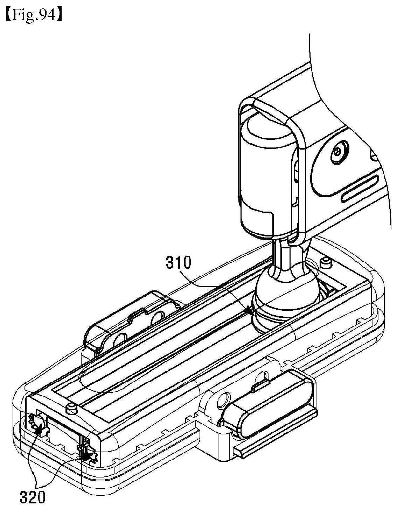

FIG. 92 is a diagram for explaining the attachment/detachment mechanism of the thigh wearing unit according to an embodiment of the present invention. FIG. 93 is a diagram for explaining the linear guide member according to an embodiment of the present invention. FIG. 94 is a diagram for explaining the sliding rail member according to an embodiment of the present invention.

As shown, the thigh wearing unit (400) is composed of the thigh frame (300) and the thigh attachment/detachment frame (460) connected with the thigh frame rotation guide member (310), and the sliding rail member (320) and the linear guide member (480) are included inside.

(a) of FIG. 92 shows a state in which the thigh frame (300) and the thigh wearing unit (400) are coupled by the sliding rail member (320) and the linear guide member (480). In this case, in accordance with the wearer's movement, the thigh wearing unit (400) slides and can operate smoothly at the contact surface with the leg. (b) of FIG. 92 shows a structure in which the thigh attachment/detachment frame (460) can be separated, and it may be designed so that attachment/detachment is easily possible using the thigh frame Release button (440).

### Sliding rail member (320), linear guide member (480)

According to an embodiment of the present invention, the thigh wearing unit (400) includes a thigh attachment/detachment frame (460), and the thigh attachment/detachment frame (460) may include a sliding rail member (320) and a linear guide member (480). In addition, the sliding rail member (320) further includes a linear guide member (480) in the thigh attachment/detachment frame (460), and the linear guide member (480) is configured to support the sliding movement of the thigh frame joint portion (450). Specifically, the inside of the thigh attachment/detachment frame (460) includes a sliding rail member (320) arranged left and right and a linear guide member (480) that stably fixes it. Specifically, the sliding rail member (320) can make the thigh wearing unit (400) move smoothly in the horizontal direction in accordance with the user's movement. That is, the thigh frame joint portion (450) can move along the sliding rail member (320). In addition, the linear guide member (480) keeps the sliding rail member (320) from shaking or being misaligned, so it may provide structural stability even during the sliding operation.

According to an embodiment of the present invention, the linear guide member (480) is located inside the thigh attachment/detachment frame (460), and may guide together with the sliding rail member (320) so that the thigh frame (300) can move smoothly. Specifically, the linear guide member (480) precisely controls the horizontal movement of the frame, and may prevent shaking during movement to maintain the stability of the wearing unit.

### Impact prevention member (481)

According to an embodiment of the present invention, the impact prevention member (481) may perform the role of absorbing the impact and vibration that may occur during the sliding and rotation operations, thereby minimizing the discomfort felt by the user. Specifically, the impact prevention member (481) relieves the impact occurring during wearing to increase the durability of the device, and may maintain performance even during repeated operations.

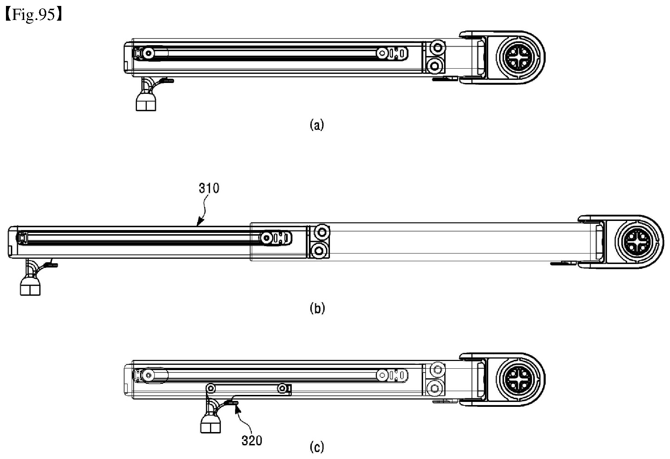

FIG. 95 is a diagram for explaining the operation of the 2nd stage and the 2.5th stage in the coaxial 2-bearing structure and the auxiliary 2-bearing structure of the thigh frame according to an embodiment of the present invention.
(a) of FIG. 95 shows a state in which the thigh frame (300) of the present invention does not generate any external force or assistance force in normal times. In such a state, the 2-stage sliding member (310) does not operate, so the thigh frame (300) may be maintained in a fixed form fitted to the wearer's leg length. Therefore, the thigh frame (300) may provide a structure that can be stably used in a general walking or stationary state.
(b) of FIG. 95 shows the case where the wearer of the wearable robot (10) walks while being provided with assistance force. The thigh frame (300) of the present invention includes a 2-stage sliding member (310). In such a state, the 2-stage sliding member (310) in the thigh frame (300) operates, showing a structure in which the thigh frame (300) lengthens. Specifically, while the wearer starts walking or proceeds with an up-and-down swing motion, the sliding mechanism is activated so that the thigh frame (300) dynamically fits the user's leg length. Through this, the present invention may provide the wearer with a more natural movement and assistance force.
(c) of FIG. 95 shows the time when the wearer of the wearable robot (10) performs a motion of climbing stairs, sitting on a chair, or squatting. The thigh frame (300) of the present invention includes a 2.5-stage sliding member (320). Specifically, when the wearer of the wearable robot (10) performs a motion of climbing stairs, sitting on a chair, or squatting, the 2.5-stage sliding member (320) is activated so that it can slide smoothly toward the inner side of the thigh frame (300). Accordingly, in the present invention, the thigh frame (300) is more finely adjusted in accordance with the movement of the wearer's leg, and an effect in which the wearing feeling is greatly improved may be provided. In particular, the present invention may support a natural movement without discomfort for the wearer even in a complex movement such as the motion of climbing stairs.

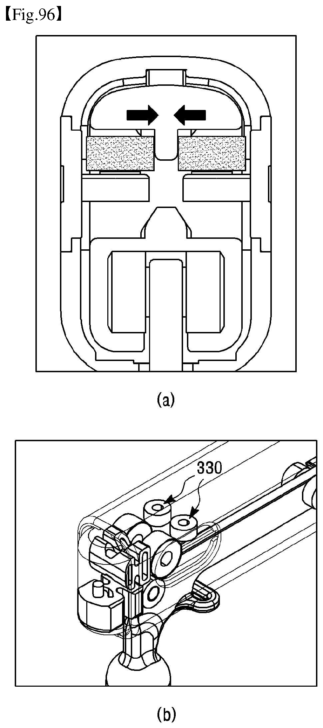

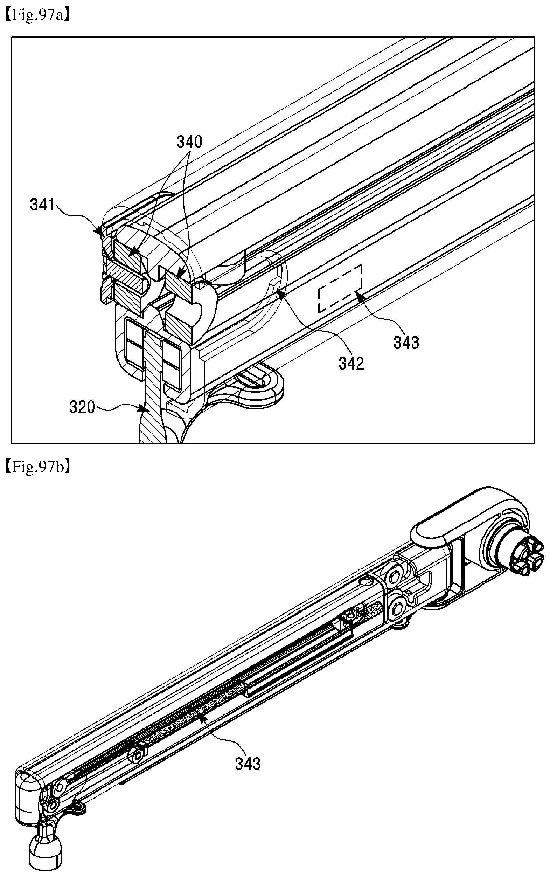

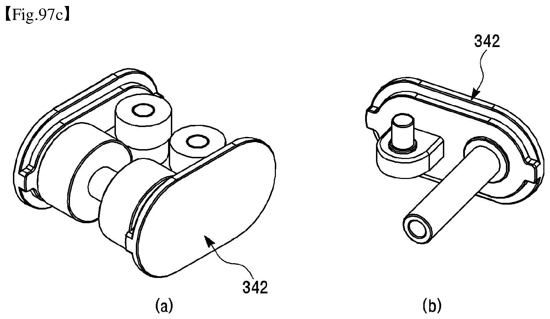

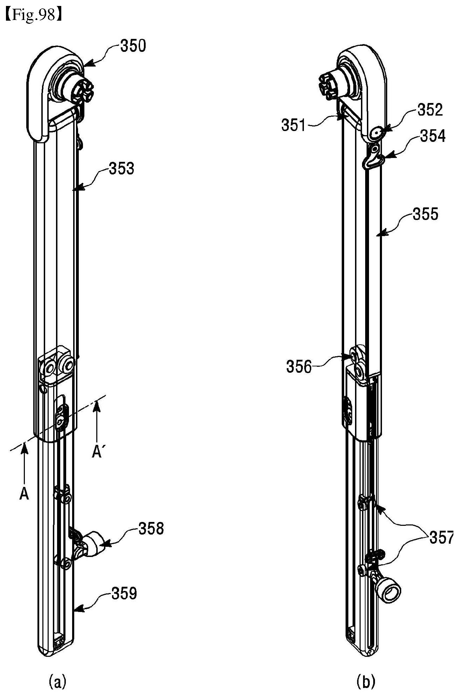

FIG. 96 is a diagram for explaining the 2-stage auxiliary bearing member of the thigh frame according to an embodiment of the present invention. FIG. 97a is a diagram for explaining the 2-stage bearing member according to an embodiment of the present invention. FIG. 97b is a diagram for explaining the role of the return elastic member being coupled to the 2.5-stage sliding member to help the return according to an embodiment of the present invention. FIG. 97c is a diagram for explaining the shape of the bearing shaft member according to an embodiment of the present invention.
(a) of FIG. 96 shows the front of the 2-stage auxiliary bearing member of the present invention. The thigh frame (300) of the present invention includes a 2-stage auxiliary bearing member (330). Specifically, the 2-stage auxiliary bearing member (330) may perform the role of maintaining the left-right balance while the thigh inner member (359) of FIG. 98(a) slides, and operating perpendicular to the sliding direction to provide stability inside the thigh frame (300). In addition, the 2-stage auxiliary bearing member (330) may perform the role of preventing the left-right tilting that may occur in the process of the thigh inner member (359) sliding, thereby reducing the discomfort that the wearer may feel or the wear of the device. In addition, the 2-stage auxiliary bearing member (330) may minimize the friction during sliding by keeping the gap (Gap) between the 2-stage sliding member (310) and the shaft cover member (341) constant.
(b) of FIG. 96 shows a structure in which the 2-stage auxiliary bearing member (330) is located perpendicular to the sliding direction to hold the left-right balance of the thigh inner member (359). In addition, the 2-stage auxiliary bearing member (330) may provide an effect of effectively distributing the load occurring from the wearer's movement, thereby increasing the durability of the device. In addition, the 2-stage auxiliary bearing member (330) may provide an effect of preventing the left-right tilting that may occur while the thigh inner member (359) slides, thereby increasing the stability and precision of the thigh frame. That is, the 2-stage auxiliary bearing member (330) may prevent the phenomenon in which the thigh inner member (359) tilts to one side while the 2-stage bearing member (340) rotates forward or backward. In addition, the 2-stage auxiliary bearing member (330) may, together with the Bearing END ASSY member (356) of FIG. 98, provide an effect of distributing the load occurring during use and increasing the stability of the device. In addition, the 2-stage auxiliary bearing member (330) may provide a comfortable wearing feeling by making the device operate smoothly even when the wearer performs various movements such as climbing stairs and squatting.

### 2-stage bearing member (340)

According to an embodiment of the present invention, the thigh frame (300) may include a 2-stage bearing member (340). Specifically, the 2-stage bearing member (340) operates perpendicular to the sliding direction, and may maintain the left-right balance while the thigh inner member (359) of FIG. 98 moves, thereby providing structural stability. In particular, the 2-stage bearing member (340) operates together with the Bearing END ASSY member (356) of FIG. 98, and may effectively prevent the left-right movement of the thigh inner member (359). Accordingly, the present invention may prevent the unnecessary tilting phenomenon that may occur during use of the wearable robot (10), and may provide an effect of strengthening the stability within the thigh frame (300).

According to an embodiment of the present invention, the 2-stage bearing member (340) is designed to maintain a constant gap (Gap) between the thigh inner member (359) and the shaft cover member (341), so it may minimize the frictional force that may occur during the sliding process and enable a smooth and seamless movement.

### Shaft cover member (341)

According to an embodiment of the present invention, the thigh frame (300) may include a shaft cover member (341). Specifically, the shaft cover member (341) may perform the role of protecting the 2-stage bearing member (340) and the bearing shaft member (342) from the external environment. Specifically, the shaft cover member (341) may prevent the internal components from being exposed to the outside. In particular, the shaft cover member (341) blocks dust, foreign substances, or contaminants of the external environment that may occur during the sliding operation of the thigh frame (300) from penetrating inside, so it may provide an environment in which the internal components can operate smoothly. Accordingly, the present invention may greatly improve the durability of the wearable robot (10), and may minimize the wear or damage of the internal components. In addition, the shaft cover member (341) may also together perform the role of maintaining the alignment of the internal components and helping a smooth and stable operation during the sliding process.

### Bearing shaft member (342)

FIG. 97c is a diagram for explaining the shape of the bearing shaft member according to an embodiment of the present invention.

According to an embodiment of the present invention, the thigh frame (300) may include a bearing shaft member (342). Specifically, the bearing shaft member (342) operates as the central axis of the 2-stage bearing member (340), and may support the sliding operation of the thigh inner member (359). In addition, the bearing shaft member (342) may be designed to operate in conjunction with the 2-stage bearing member (340) so that the sliding operation is performed accurately and smoothly. In particular, the bearing shaft member (342) may prevent the shaking or abnormal movement that may occur while the thigh inner member (359) slides, thereby maintaining the stability of the thigh frame (300). In addition, the bearing shaft member (342) may provide an effect of effectively distributing the load occurring during the sliding process, thereby increasing the durability of the thigh frame (300).

According to an embodiment of the present invention, the bearing shaft member (342), as a component of the thigh frame (300), may perform the role of combining with the 2-stage bearing member (340) to support the sliding operation of the thigh inner member (359) and to ensure stability. As shown in (a) of FIG. 5c, the bearing shaft member (342) operates as the central axis of the 2-stage bearing member (340), and is designed to be able to evenly distribute the load occurring during the sliding process. In addition, the bearing shaft member (342) maintains a precise position inside the thigh frame (300), and may perform the assistance so that the sliding operation is performed smoothly without shaking.

According to an embodiment of the present invention, as shown in (b) of FIG. 5c, the bearing shaft member (342) is precisely combined with the structure of the thigh inner member (359), and through this, may prevent the abnormal movement or instability that may occur during the sliding process. Such a design of the bearing shaft member (342) may contribute to increasing the overall durability and stability of the thigh frame (300). In addition, the bearing shaft member (342) operates in conjunction with the 2-stage bearing member (340) to minimize the vibration or friction occurring during the sliding process, and may provide a smooth and natural assistance operation to the user. That is, the bearing shaft member (342), as a component in the sliding mechanism of the thigh frame (300), may perform the role of supporting the smooth operation of the thigh inner member (359) and improving the durability of the device.

### Return elastic member (343)

FIG. 5b is a diagram for explaining the role of the return elastic member being coupled to the 2.5-stage sliding member to help the return according to an embodiment of the present invention.

According to an embodiment of the present invention, the thigh frame (300) may include a return elastic member (343). Specifically, the return elastic member (343) may perform the role of making the thigh inner member (359) quickly return to the original position after it is slid by the wearer's movement. In addition, the return elastic member (343) may, by utilizing the reaction occurring when the thigh inner member (359) moves, accelerate the process of the thigh inner member (359) returning to the initial position after sliding. Accordingly, the present invention may, even after the wearer performs various movements such as climbing stairs, squatting, or sitting on a chair, allow the thigh frame (300) to quickly return to the initial state to prepare for the next movement.

According to an embodiment of the present invention, the return elastic member (343) may perform the role of being coupled with the 2.5-stage sliding member (320) to assist returning to the original position after the sliding operation is completed. Specifically, the return elastic member (343) is designed in a long and flexible structure, so it does not restrict the movement of the 2.5-stage sliding member (320) while smoothly supporting the return operation, and through accurate position coupling, may prevent the trembling or abnormal movement that may occur during the return process.

According to an embodiment of the present invention, the return elastic member (343) may, after the user performs a motion of climbing stairs or sitting, return the expanded 2.5-stage sliding member (320) quickly and smoothly to the original state using elastic force. In addition, the return elastic member (320) is integrated with the 2.5-stage sliding mechanism to assist the movement of the wearable robot (10) while also performing the role of absorbing the reaction occurring during the return process and suppressing the vibration and noise inside the thigh frame (300).

According to an embodiment of the present invention, the return elastic member (343) provides stable return performance even during repeated sliding operations to increase the durability of the device, and being manufactured of an elastic material, it is strong against deformation and may maintain performance even during long-term use. Accordingly, the return elastic member (343) ensures the continuity between the sliding operation and the return operation, and may perform the role of helping to maintain consistent device performance even when the user performs various movements such as walking, sitting, and climbing stairs.

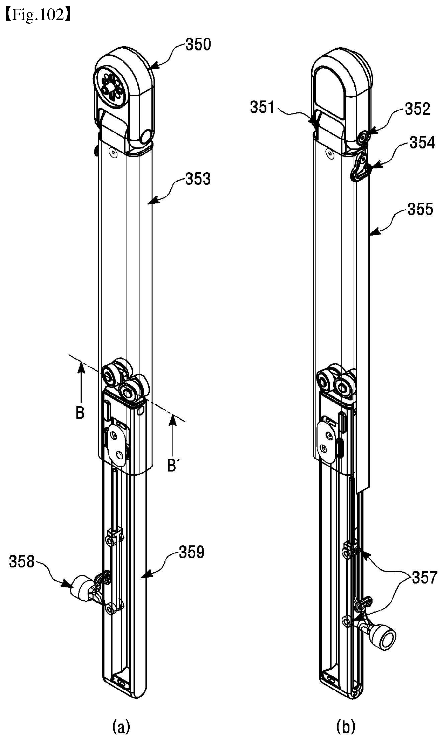

FIG. 98 is a diagram for explaining the joint member, the joint shaft member, the 2-stage return elastic band member, and the like according to an embodiment of the present invention. FIG. 102 is a diagram for explaining the joint member, the joint shaft member, the 2-stage return elastic band member, and the like in the urethane bearing structure according to an embodiment of the present invention.

### Joint member (350)

As shown, the thigh frame (300) may include a joint member (350). The joint member (350) of (a) of FIG. 98 may perform the role of transmitting the assistance force received from the drive unit (200) to the wearer. Specifically, the joint member (350) may be designed to smoothly transmit the force generated in the actuator of the drive unit (200) to the thigh frame (300) so that the wearer can receive the assistance force naturally and effectively. In addition, the joint member (350) forms a force transmission path between the drive unit (200) and the thigh frame (300), and may absorb the impact or load occurring during the movement process, thereby maintaining the stability and durability of the wearable robot (10). In addition, the joint member (350) may efficiently distribute the assistance force transmitted from the drive unit (200) in accordance with various movements of the wearer such as walking and climbing stairs.

According to an embodiment of the present invention, the joint member (350) includes a joint cap member (351) that performs the role of a cover of the joint member (350) and a joint shaft member (352) that performs the role of an axis of the joint member (350), and the joint member (350) is coupled with the joint cap member (351) and the joint shaft member (352) and is configured so that a swing motion coinciding with the wearer's walking direction and a swing motion in the direction perpendicular to the walking direction are possible.

### Joint cap member (351)

According to an embodiment of the present invention, the thigh frame (300) may include a joint member (350), and the joint member (350) may include a joint cap member (351). Specifically, the joint cap member (351) may perform the role of protecting the joint member (350) from external impact or wear. In addition, the joint cap member (351) may perform the role of a cover so that the joint member (350) is not damaged or worn from the external environment during use. In particular, in an environment where the thigh frame (300) receives various movements and loads, it prevents the damage of the joint member (350), so it may extend the lifespan of the component. In addition, the joint cap member (351) blocks the internal components of the joint member (350) from external factors such as dust, foreign substances, and moisture, so it may prevent the performance degradation of the wearable robot (10) and provide continuous stability.

### Joint shaft member (352)

According to an embodiment of the present invention, the thigh frame (300) may include a joint member (350), and the joint member (350) may include a joint shaft member (352). Specifically, the joint shaft member (352) may perform the role of an axis that transmits force between the joint member (350) and the drive unit (200). In addition, the joint shaft member (352) transmits the force generated in the drive unit (200) to the joint member (350) so that the thigh frame (300) can operate naturally. In addition, the joint shaft member (352), as a structural element that supports so that rotation and movement are possible within the thigh frame (300), may prevent the unnecessary shaking or twisting that may occur during operation, thereby maintaining the stability of the wearable robot (10). In addition, the joint shaft member (352) withstands and distributes the load occurring during the movement process, and at the same time may increase the mechanical performance and durability of the wearable robot (10).

### Thigh cover member (353)

According to an embodiment of the present invention, the thigh frame (300) may include a thigh cover member (353). Specifically, the thigh cover member (353) may, while covering the outer periphery of the thigh frame (300), effectively guide the assistance force in the direction of the wearer's movement. In addition, the thigh cover member (353) maintains the stability of the thigh frame (300), and may perform the role of a cover that protects the internal components. In addition, the thigh cover member (353) operates as a guide rail that supports the 2-stage sliding operation, so that the thigh inner member (359) can move smoothly. Accordingly, the present invention may allow the assistance force to be transmitted naturally and smoothly when the wearer performs various movements such as walking or climbing stairs.

### String end member (354)

According to an embodiment of the present invention, the thigh frame (300) may include a string end member (354). Specifically, the string end member (354) may maintain the tension within the thigh frame (300) and finish the connection part. In addition, the string end member (354) may be designed so that the internal tension of the thigh frame (300) is maintained constant. In addition, the string end member (354) may maintain the balance between the internal components when the wearer performs various movements such as walking or climbing stairs, and may prevent the unnecessary shaking or coming-off that may occur during operation.

### Elastic band member (355)

According to an embodiment of the present invention, the thigh frame (300) may include an elastic band member (355). Specifically, the elastic band member (355) allows the thigh inner member (359), after being slid by the wearer's movement, to quickly return to the original position by utilizing the force of reaction. In addition, the elastic band member (355) makes the thigh inner member (359) return to the initial state naturally and smoothly after performing an operation, and may perform the role of making the thigh inner member (359) return even after the wearer performs various movements such as climbing stairs or sitting on a chair. In addition, the elastic band member (355) may efficiently utilize the reaction energy occurring during the sliding operation to increase the return speed, and may prevent the unnecessary shaking or abnormal movement during the return process.

### Bearing end member (356)

According to an embodiment of the present invention, the thigh frame (300) may include a bearing end member (356). Specifically, the bearing end member (356) may prevent the thigh inner member (359) from tilting left and right when it performs the sliding operation, and may provide stable sliding. In addition, the bearing end member (356) supports and guides the movement of the thigh inner member (359), and may minimize the abnormal movement or shaking that may occur during the sliding process. In addition, the bearing end member (356) may effectively distribute the load occurring during the wearing of the wearable robot (10) of the present invention, thereby preventing excessive stress from being applied to a specific component within the wearable robot (10).

According to an embodiment of the present invention, the thigh frame (300) further includes a bearing end member (356), the thigh inner member (359) is coupled with the bearing end member (356), and the bearing end member (356) is configured to prevent left-right play during sliding of the thigh inner member (359), and to distribute the load applied to the thigh frame (300) when the assistance force of the drive unit (200) is generated.

### 2.5-stage bearing member (357)

According to an embodiment of the present invention, the thigh frame (300) may include a 2.5-stage bearing member (357). Specifically, the 2.5-stage bearing member (357) may, when the wearer performs a complex movement such as climbing stairs or squatting, widen the expansion range of the thigh inner member (359) to provide a more detailed assistance force. In addition, the 2.5-stage bearing member (357) assists the sliding operation of the thigh inner member (359), and enables a smooth and natural operation in accordance with the characteristics and demands of the wearer's movement. In particular, through an additional expansion function beyond the general 2-stage sliding operation, the 2.5-stage bearing member (357) may allow the wearer to receive comfortable and stable assistance even in a situation requiring a wider range of movement.

### End member (358)

According to an embodiment of the present invention, the thigh frame (300) may include an end member (358). Specifically, the end member (358) may, when the wearer performs a motion of climbing stairs, squatting, sitting on a chair, or the like, slide inward in accordance with the change in leg length to provide a comfortable wearing feeling to the wearer. When the wearer performs a motion of climbing stairs, squatting, sitting on a chair, or the like, if there is no change in leg length, the wearer feels considerable discomfort, so when the end member (358) slides inward in accordance with the change in leg length, it may provide a comfortable wearing feeling to the wearer.

According to an embodiment of the present invention, the end member (358) may resolve the discomfort that may occur when the length of the thigh frame (300) is fixed. That is, even if the leg length changes when the wearer performs an operation, the end member (358) naturally slides so that the wearable robot (10) can operate smoothly and seamlessly even at a position shorter than the thigh frame (300).

### Thigh inner member (359)

According to an embodiment of the present invention, the thigh frame (300) may include a thigh inner member (359). Specifically, the thigh inner member (359) may flexibly respond to the change in the user's leg length and perform a sliding operation of extending and contracting. Specifically, the thigh inner member (359) may, when the wearer performs various movements such as walking or climbing stairs, automatically detect the change in leg length and slide accordingly. In addition, the thigh inner member (359) may also perform the role of transmitting the assistance force generated in the drive unit (200) to the wearer through the sliding operation.

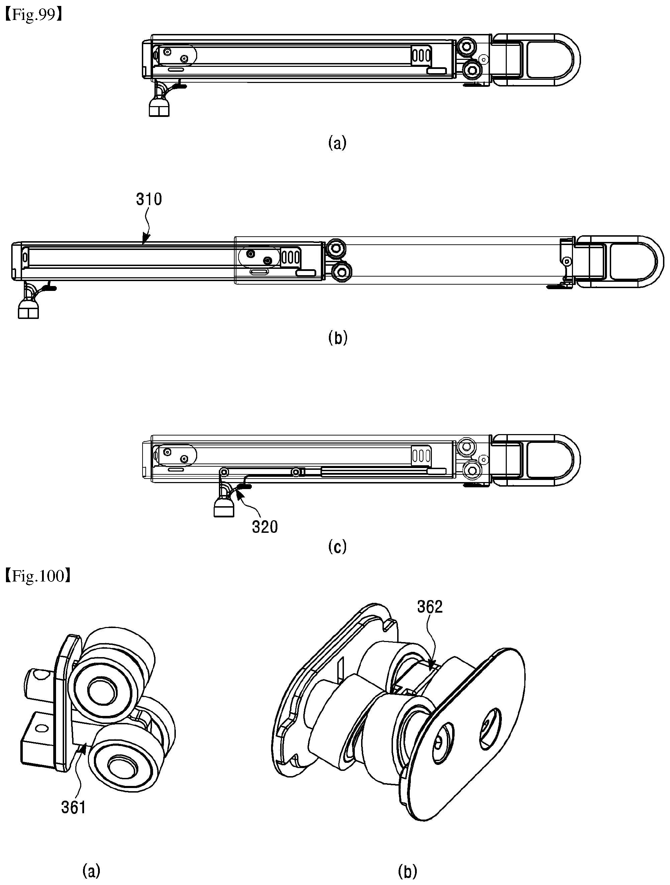

FIG. 99 is a diagram for explaining the operation of the thigh frame having a urethane bearing structure that slides on a rail having a certain angle according to the present invention.

As shown in (a) of FIG. 99, the normal-time state of the thigh frame (300) may maintain a basically fixed state in which the wearable robot (10) does not provide assistance force. In such a state, before the wearer starts movement, the thigh frame (300) is stably fixed at the initial position, and it is a state in which the 2-stage sliding or 2.5-stage sliding operation is not activated. As shown, the urethane-coated bearing may effectively suppress the vibration and noise that may occur inside the thigh frame (300), thereby maintaining a stable and quiet state.

(b) of FIG. 99 shows the case where the 2-stage sliding member (310) is activated so that the thigh frame (300) lengthens and is adjusted to fit the wearer's leg length. In (b) of FIG. 99, the thigh frame (300) is expanded to fit the wearer's leg length, and may be designed so as to be able to smoothly perform the up-and-down swing operation. As shown, the urethane bearing structure may minimize, with the urethane coating, the vibration and noise that may occur during the sliding process so that the user can feel a comfortable wearing feeling. In addition, the smooth movement of the urethane bearing may prevent the phenomenon in which the thigh frame (300) tilts left and right during sliding, thereby providing a stable operation.

As shown in (c) of FIG. 99, the state in which the 2.5-stage sliding member (320) is activated may, when the user performs a motion of climbing stairs or sitting, provide a more comfortable wearing feeling to the user as the 2.5-stage sliding member (320) is additionally activated so that the thigh frame (300) becomes shorter. The present invention may finely adjust the length of the thigh frame (300) in accordance with the wearer's movement to support a smooth and stable operation. Specifically, even in a complex movement such as climbing stairs or sitting, the thigh frame (300) is naturally adjusted to fit the user's demand, so it may provide a comfortable wearing experience. In addition, the urethane-coated bearing may effectively suppress the vibration and noise that may occur during the sliding process, and may maintain a stable sliding operation.

According to the present invention, the present invention may implement an angle left-right symmetrically in the thigh cover member (353) and the thigh inner member (359), thereby suppressing the left-right tilting phenomenon that may occur during sliding. In addition, the present invention may also minimize vibration and noise by applying a urethane coating to all sliding bearings.

FIG. 100 is a diagram for explaining the bearing end bracket and the bearing sliding bracket according to an embodiment of the present invention.

### Bearing end bracket (361)

As shown in (a) of FIG. 100, the thigh frame (300) may include a bearing end bracket (361). Specifically, the bearing end bracket (361) supports the bearing of the present invention so as to maintain an aligned state, and may prevent the left-right tilting or shaking that may occur during the sliding process. In addition, the bearing end bracket (361) performs the role of a guide so that the bearing can move smoothly along the rail, and may perform the role of effectively distributing the load occurring during sliding so that excessive pressure is not applied to the other components of the thigh frame (300). In addition, the bearing end bracket (361) is designed in a structure that supports the urethane-coated bearing, so it may minimize the vibration and noise that may occur during sliding, thereby providing a quiet and stable operating environment to the wearer.

### Bearing sliding bracket (362)

As shown in (b) of FIG. 100, the thigh frame (300) may include a bearing sliding bracket (362). Specifically, the bearing sliding bracket (362) maintains the bearing in an aligned state, and may prevent the shaking or left-right tilting that may occur during the sliding process. In addition, the bearing sliding bracket (362) performs the role of a guide so that the bearing can slide smoothly along the rail, thereby maintaining the stability of the sliding operation. In addition, the structure of the bearing sliding bracket (362) may provide an effect of effectively distributing the load occurring during sliding to reduce the pressure applied to the other components of the thigh frame (300). In addition, since the bearing sliding bracket (362) can suppress vibration and noise, it may provide a quiet and stable operating environment to the wearer.

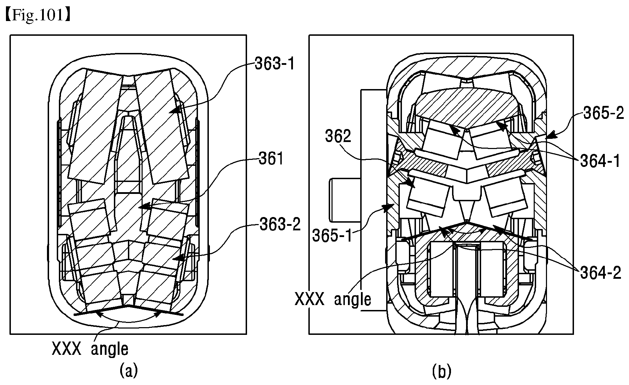

FIG. 101 is a diagram for explaining the urethane bearing according to an embodiment of the present invention.

### Upper urethane bearing (363-1) and lower urethane bearing (363-2)

As shown, the thigh frame (300) may include an upper urethane bearing (363-1) and a lower urethane bearing (363-2). Specifically, the upper urethane bearing (363-1) and the lower urethane bearing (363-2) may be located on the inner rail of the thigh cover member (353) to support the sliding operation. Since the upper urethane bearing (363-1) and the lower urethane bearing (363-2) have a urethane coating applied, they may effectively suppress the vibration and noise that may occur during the sliding process. In addition, the upper urethane bearing (363-1) and the lower urethane bearing (363-2) are each located at a certain angle, so they may prevent the left-right tilting or shaking that may occur during the sliding process. In particular, the upper urethane bearing (363-1) and the lower urethane bearing (363-2) balance each other to increase the structural stability of the thigh frame (300), and may maintain the smoothness of the sliding operation.

### Bearing cover (365)

As shown, the thigh frame (300) may include a bearing cover (365), a front urethane bearing (364-1), and a rear urethane bearing (364-2). Specifically, the bearing cover (365) may perform the role of fixing the bearing so that it does not deviate from its place. In addition, the bearing cover (365) may prevent the phenomenon in which the bearing moves or comes off due to the impact or vibration that may occur during sliding. In addition, the bearing cover (365) may protect the bearing from external environmental factors such as dust, foreign substances, and moisture. In addition, the bearing cover (365) prevents the bearing from being worn or its performance being degraded by the external environment, so it may improve the durability of the device. In addition, the bearing cover (365) may suppress the vibration and noise that may occur during sliding.

### Front urethane bearing (364-1), rear urethane bearing (364-2)

According to an embodiment of the present invention, the front urethane bearing (364-1) and the rear urethane bearing (364-2) are precisely combined with the inner rail of the thigh cover member (353) to suppress the vibration and noise that may occur during sliding, and to perform a smooth and quiet sliding operation. In particular, the thigh cover member (353) and the thigh inner member (359) are designed in a left-right symmetric structure, and such a structure interacts with the role of the front urethane bearing (364-1) and the rear urethane bearing (364-2) to effectively prevent the left-right tilting phenomenon during sliding. In addition, the front urethane bearing (364-1) and the rear urethane bearing (364-2) assist, through a rail structure having a left-right symmetric certain angle, so that the thigh inner member (359) can move stably without shaking during sliding, and this may provide an effect of greatly improving the precision and stability of the sliding process.

According to an embodiment of the present invention, the front urethane bearing (364-1) and the rear urethane bearing (364-2) operate integrated with the 2-stage and 2.5-stage sliding mechanisms, and may support the change in length of the thigh frame (300) when the user starts walking or performs an up-and-down swing motion. Specifically, the 2-stage sliding member (310) expands the thigh frame (300) to fit the leg length during the user's walking, and the 2.5-stage sliding member (320) additionally adjusts the length of the thigh frame (300) when the user performs a motion of climbing stairs or sitting, so it may provide a comfortable wearing feeling.

According to an embodiment of the present invention, the front urethane bearing (364-1) and the rear urethane bearing (364-2) effectively absorb the impact and vibration occurring during sliding so that the user can feel a smooth and stable assistance force. Such a design may contribute not only to user convenience through vibration and noise suppression, but also to increasing the durability of the device.

FIG. 102 is a diagram for explaining the joint member, the joint shaft member, the 2-stage return elastic band member, and the like in the urethane bearing structure according to an embodiment of the present invention.

As shown in (a) of FIG. 102, the joint member (350) is a component that receives the assistance force from the actuator and is connected to the thigh frame (359), and may be designed so that the user can perform a swing motion during walking. Specifically, the joint member (350) may transmit the assistance force between the actuator and the thigh frame (300) while at the same time providing a stable movement.

As shown, the joint cap member (351) may perform the role of protecting the joint member (350) from external impact or wear and ensuring the stability of the internal components. The joint shaft member (352) connects the joint member (350) and the thigh frame (300), and may provide a stable support structure when the thigh frame (300) slides or performs a swing motion.

According to an embodiment of the present invention, the thigh cover member (353) wraps around the thigh frame (359) and includes a sliding rail structure inside, so it may support the smooth and stable sliding operation of the thigh inner member (359). In addition, the thigh cover member (353) also performs the protection role of the entire thigh frame (300), so it may protect the structure from the external environment.

According to an embodiment of the present invention, the string end member (354) finishes the end part of the thigh frame structure, and may strengthen the connection of the internal components, thereby providing an effect of increasing the overall structural stability.

According to an embodiment of the present invention, the elastic band member (355) provides elasticity so that the thigh frame (300) can return to the original state after being expanded or contracted in accordance with the user's movement, and accordingly may assist the user's movement naturally and efficiently. In addition, the thigh inner member (359) slides inside the thigh frame (300), and may adjust its length to fit the user's leg length. Specifically, the thigh inner member (359) may perform the role of accurately transmitting the assistance force to the user.

According to an embodiment of the present invention, the 2.5-stage bearing member (357) supports the thigh inner member (359) so that it can be additionally expanded when performing a motion of climbing stairs or sitting, and may support a smooth and stable sliding operation.

According to an embodiment of the present invention, the end member (358) is located at the lower end part of the thigh frame (300), and may provide an effect of increasing structural completeness while at the same time effectively distributing the user's load. This strengthens the overall durability and stability of the thigh frame (300), and may provide a comfortable wearing feeling when the user performs various movements.

As shown in (b) of FIG. 102, the 2.5-stage bearing member (357) supports the additional sliding operation of the thigh inner member (359), and through this allows the user to smoothly and naturally perform various movements such as a motion of climbing stairs or sitting.

According to an embodiment of the present invention, the thigh cover member (353) and the thigh inner member (359) slide along a rail structure designed at a certain angle, and such a structure may prevent the left-right tilting phenomenon that may occur while the thigh inner member (359) moves. In addition, during the sliding process, the thigh inner member (359) is stably supported by the joint shaft member (352), and each bearing member (357) may effectively distribute the load to provide a precise and smooth operation.

### Embodiment of the connecting member

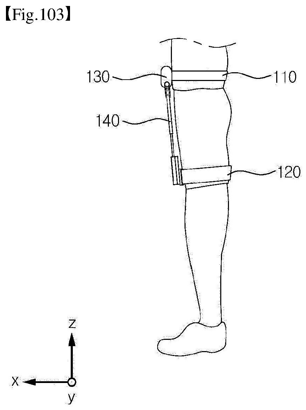

FIG. 103 to FIG. 108 are diagrams showing the operation state of the wearable robot according to an embodiment of the present invention.

As shown in FIG. 103, the first fixing unit (110) is fixed to the waist part located on the upper side of the hip joint, the second fixing unit (120) may be fixed to the thigh part located on the lower side of the hip joint, and the connecting member (140) may, in a state where it is arranged at the front part of the user, connect the drive unit (130) fixed to the first fixing unit (110) side and the second fixing unit (120). In this case, the connecting member (140) may, while rotating about the y-axis by the drive unit (130), provide assistance force in a direction of lifting up or lowering down the second fixing unit (120) fixed to the thigh part, so it may prevent the adduction, abduction, or twisting of the rotation center of the joint portion due to the provision of the assistance force, and even when the thigh part moves left and right in a state where it is lifted up by the assistance force, the connecting member (140) may comply therewith and rotate left and right, so it may not restrict the range of motion of the joint portion.

According to an embodiment of the present invention, the connecting member (140) is configured so that the length can be adjusted in correspondence to the interval between the drive unit (130) and the second fixing unit (120), and as the connecting member (140) is configured to expand and contract in multiple stages, it can provide a large stroke while minimizing the length of each member in the overlapped state. Therefore, it can transmit assistance force from a motion in which the range of motion of the joint portion is small, such as walking, to a motion in which the range of motion of the joint portion is large, such as sitting or stair walking.

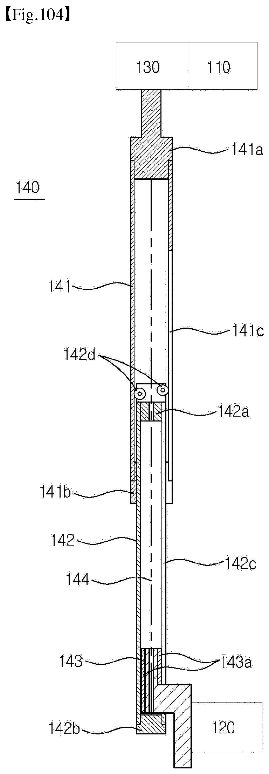

As shown in FIG. 104, the first member (141) of the connecting member (140) has one end portion fixed to the drive unit (130) provided in the first fixing unit (110), and the second member (142) is connected to the first member (141) in a state movable along the lengthwise direction. The third member (143) fixed to the second fixing unit (120) is connected to the second member (142) in a state movable along the lengthwise direction. Here, the elastic member (144) has both end portions fixed to the second member (142) and the first member (141), and provides an elastic force to the second member (142) in the direction in which the first member (141) and the second member (142) overlap. Here, the driving force for rotating about the y-axis by the drive unit (130) may be transmitted directly to the first member (141). Such driving force may be transmitted, through the second member (142) movable along the lengthwise direction on the first member (141) and subsequently the third member (143) movable along the lengthwise direction on the second member (142), to the second fixing unit (120) connected to the third member (143).

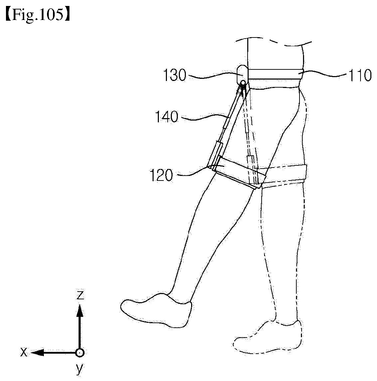

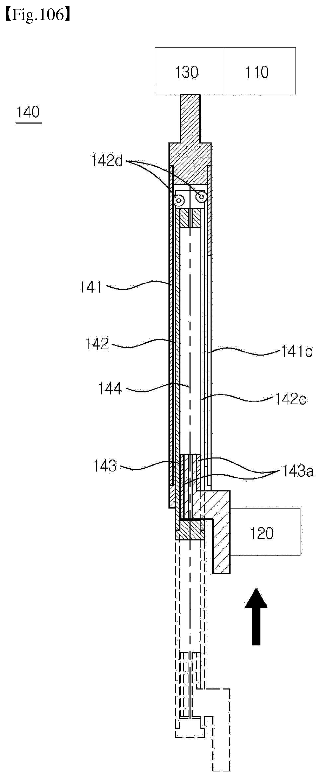

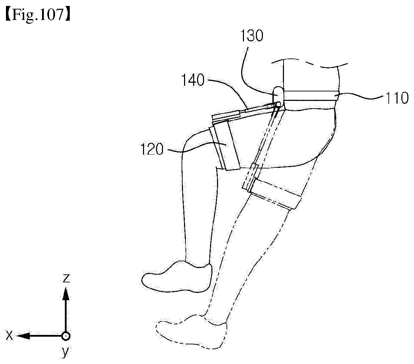

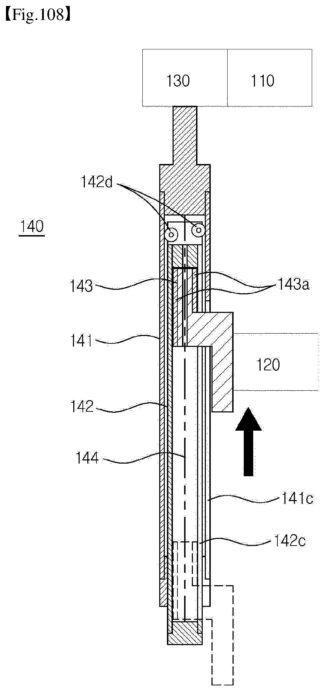

Here, the movement length of the second member (142) of the connecting member (140) is set so as to be able to correspond to the change in the interval between the drive unit (130) and the second fixing unit (120) due to a small motion of the joint portion, as in FIG. 105 and FIG. 106, and the movement length of the third member (143) is set so as to be able to correspond to the change in the interval between the drive unit (130) and the second fixing unit (120) due to a large motion of the joint portion, as in FIG. 107 and FIG. 108.

FIG. 103 shows a state in which the user wears the wearable robot according to an embodiment of the present invention and stands in place, and in such a state, the interval between the drive unit (130) arranged at the waist part on the upper side of the hip joint and the second fixing unit (120) fixed to the thigh part on the lower side becomes maximum. Accordingly, in the connecting member (140) arranged between the drive unit (130) and the second fixing unit (120), the second member (142) and the third member (143) each move in the expansion direction as in FIG. 104, and in this process the elastic member (144) becomes a state elastically tensioned by external force.

Subsequently, FIG. 105 shows a state in which the interval between the drive unit (130) and the second fixing unit (120) is changed by a small motion of the joint portion, and the range of motion of the joint portion in the walking process is limitedly made only within a first region that is a part of the range of motion, and the connecting member (140) is set so that the movement position of the second member (142) can be adjusted in correspondence to the change in the interval between the drive unit (130) and the second fixing unit (120) due to a small motion of the joint portion.

That is, as shown in FIG. 106, the connecting member (140) may, as the second member (142) moves in the contraction direction along the first member (141) in a state where it supports the third member (143), be adjusted to a length corresponding to the interval between the drive unit (130) and the second fixing unit (120) due to a small motion of the joint portion. Here, in correspondence to the interval between the drive unit (130) and the second fixing unit (120) being reduced, the second member (142) and the third member (143) may each move in the contraction direction, and since the second member (142) is in a state elastically supported in the direction of contracting with respect to the first member (141) by the elastic member (144), the second member (142) moves along the first member (141) in priority over the third member (143) moving along the second member (142). In addition, since the second member (142) becomes a state elastically supported in the contraction direction by the elastic member (144), it can prevent the second member (142) from arbitrarily moving in the expansion direction by its own weight in the state where it has moved in the contraction direction.

According to an embodiment of the present invention, the second member (142) is supported in a state capable of rolling motion with respect to the first member (141) through a plurality of rollers (142d), and the third member (143) is supported in a state capable of sliding motion with respect to the second member (142) through a sliding pad (143a), and the sliding pad (143a) is designed to have a relatively larger frictional force than the rollers (142d). Therefore, since the second member (142) can move with a smaller frictional force than the third member (143), the second member (142) can move along the first member (141) in a state where it supports the third member (143), in priority over the third member (143) moving along the second member (142).

Subsequently, FIG. 107 shows the change in the interval between the drive unit (130) and the second fixing unit (120) due to a large motion of the joint portion, and the range of motion of the joint portion in the sitting motion or the stair walking process expands to a second region exceeding the first region, and the connecting member (140) is set so that the movement position of the third member (143) together with the second member (142) can be adjusted in correspondence to the change in the interval between the drive unit (130) and the second fixing unit (120) due to a large motion of the joint portion. That is, the connecting member (140) may, in the state where the second member (142) has completely moved in the contraction direction along the first member (141) in a state where it supports the third member (143) as shown in FIG. 106, be adjusted to a length corresponding to the interval between the drive unit (130) and the second fixing unit (120) due to a large motion of the joint portion as the third member (143) moves in the contraction direction along the second member (142) as in FIG. 108.

At this time, the third member (143) moves in a state where a part of it is exposed to the outside through a second slit (142c) formed on one surface of the second member (142), and since a first slit (141c) communicating with the second slit (142c) is formed on one surface of the first member (141), the third member (143) can move in the contraction direction along the second member (142) even in a state where the second member (142) overlaps with the first member (141).

The wearable robot according to an embodiment of the present invention may, as in FIG. 103 to FIG. 108, provide assistance force to the movement of the joint portion by moving the thigh part upward while the connecting member rotates clockwise about the y-axis by the forward driving of the drive unit, and in this process, the connecting member (140) may contract in correspondence to the interval between the drive unit (130) and the second fixing unit (120).

Conversely, the connecting member (140) may provide assistance force to the movement of the joint portion by moving the thigh part in the downward direction while rotating counterclockwise about the y-axis by the reverse driving of the drive unit, and in this process, may expand in correspondence to the interval between the drive unit (130) and the second fixing unit (120). Since such an expansion process of the connecting member (140) is made in the reverse order of the above-described contraction process, a detailed description thereof is omitted.

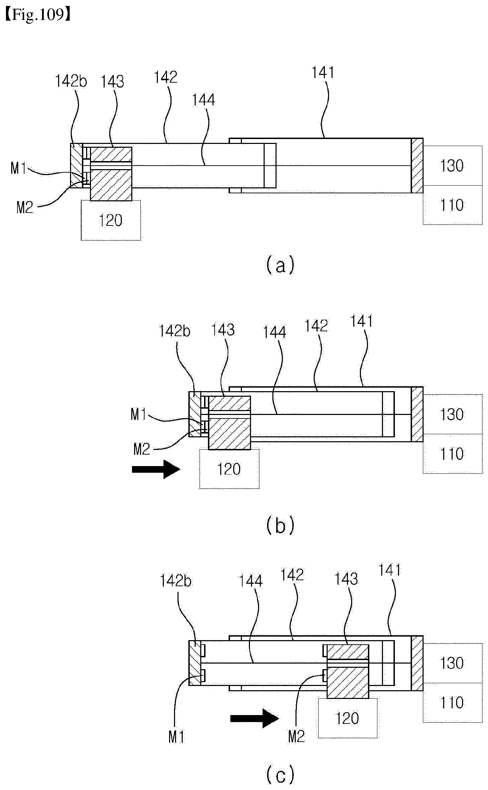

FIG. 109 is an operation diagram of the connecting member according to an embodiment of the wearable robot of the present invention.

As shown in FIG. 109, the connecting member (140) according to the present invention includes a first member (141), a second member (142), a third member (143), and an elastic member (144), and a first permanent magnet (M1) and a second permanent magnet (M2) are provided on the mutually facing surfaces of the second member (142) and the third member (143), so that when the third member (143) has completely moved in the expansion direction, the first permanent magnet (M1) and the second permanent magnet (M2) can stick to each other. Specifically, the first permanent magnet (M1) is arranged on the surface, facing the third member (143), of the 2-2 end cap (142b) that closes off the expansion-side end portion of the second member (142), and the second permanent magnet (M2) that can stick to the first permanent magnet (M1) is arranged on the surface, facing the 2-2 end cap (142b), of the third member (143). It is preferable that the fixing force by the magnetic force of the first permanent magnet (M1) and the second permanent magnet (M2) is set to a degree at which they cannot be arbitrarily separated by the elastic restoring force of the elastic member (144).

That is, as in (a) of FIG. 109, in a state where the first member (141), the second member (142), and the third member (143) have each moved in the expansion direction, the elastic member (144) connecting the first member (141) and the second member (142) is elastically tensioned, and the first permanent magnet (M1) of the second member (142) and the second permanent magnet (M2) of the third member (143) stick to each other by magnetic force.

Then, as in (b) of FIG. 109, when the interval between the drive unit (130) and the second fixing unit (120) is reduced by a small movement of the joint portion, the third member (143) connected to the second fixing unit (120) moves in the contraction direction. At this time, since the third member (143) is in a state fixed to the 2-2 end cap (142b) of the second member (142) by the first permanent magnet (M1) and the second permanent magnet (M2), and the second member (142) is in a state elastically supported in the contraction direction by the elastic member (144), the second member (142) moves in the contraction direction on the first member (141) in a state where it supports the third member (143).

Subsequently, as in (c) of FIG. 109, when the interval between the drive unit (130) and the second fixing unit (120) is reduced by a large movement of the joint portion, the third member (143) connected to the second fixing unit (120) additionally moves in the contraction direction. At this time, since additional movement is restricted in the state where the second member (142) has completely moved in the contraction direction with respect to the first member (141), the first permanent magnet (M1) and the second permanent magnet (M2) are separated by external force, and the third member (143) moves in the contraction direction on the second member (142).

Meanwhile, although the present embodiment has been described by way of example as using the pair of permanent magnets, it would also be possible to constitute either one of the first permanent magnet (M1) and the second permanent magnet (M2) as a magnetic body. In addition, a buffer (not shown) may be arranged between the first permanent magnet (M1) and the second permanent magnet (M2). The buffer may be arranged on one surface of one permanent magnet or on one surface of each permanent magnet. When the third member (143) moves from (c) to (b) of FIG. 109 within the second member (142), the first permanent magnet (M1) and the second permanent magnet (M2) combine. In this case, the buffer can prevent noise and breakage of the permanent magnets.

FIG. 100 is an operation diagram of the connecting member according to another embodiment of the wearable robot of the present invention.

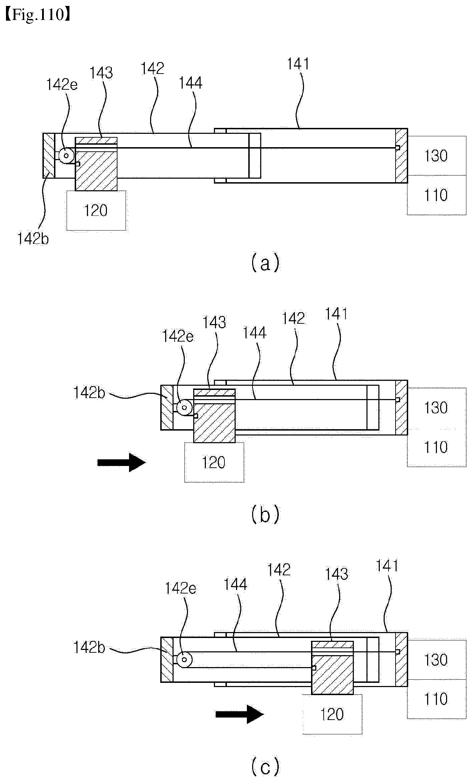

The connecting member (140) of the present invention includes a first member (141), a second member (142), a third member (143), and an elastic member (144) as shown in FIG. 110, but has a configuration difference from the above-described embodiment in that a first pulley (142e) capable of supporting the elastic member (144) in a movable state is arranged on the 2-2 end cap (142b) that closes off the expansion-side end portion of the second member (142), and the elastic member (144) is made in the form of an elastic cord and has one end portion fixed to the first member (141) and the other end portion fixed to the third member (143) in a state wound around the first pulley (142e).

As in (a) of FIG. 110, in a state where the first member (141), the second member (142), and the third member (143) have each moved in the expansion direction, the elastic member (144) becomes a state elastically deformed and tensioned, and the 2-2 end cap (142b) of the second member (142) may maintain a state elastically pressed toward the third member (143) by the elastic force of the elastic member (144).

Subsequently, as in (b) of FIG. 110, when the interval between the drive unit (130) and the second fixing unit (120) is reduced by a small movement of the joint portion, the third member (143) connected to the second fixing unit (120) moves in the contraction direction. At this time, since the second member (142) is pressed in the contraction direction on the first member (141) by the elastic force of the elastic member (144), and the third member (143) is in a state pressed in the expansion direction on the second member (142) by the elastic force of the elastic member (144), the second member (142) moves in the contraction direction on the first member (141) in a state where it supports the third member (143).

As in (c) of FIG. 110, when the interval between the drive unit (130) and the second fixing unit (120) is additionally reduced by a large movement of the joint portion, the third member (143) moves in the contraction direction on the second member (142) by the contraction-direction external force acting on the third member (143) through the second fixing unit (120). At this time, since the elastic member (144) has one end portion fixed to the first member (141) and the other end portion fixed to the third member (143) in a state supported on the first pulley (142e), it is elastically tensioned by the third member (143) moving in the contraction direction on the second member (142). That is, since the second member (142) becomes a state elastically supported in the contraction direction by the elastic member (144), it can prevent the second member (142) from arbitrarily moving in the expansion direction by its own weight.

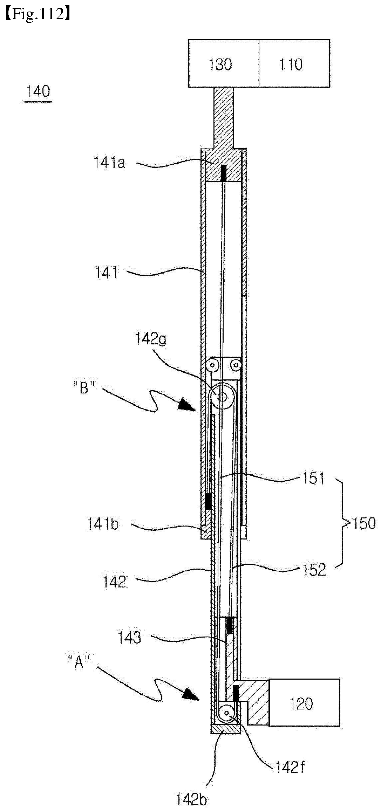

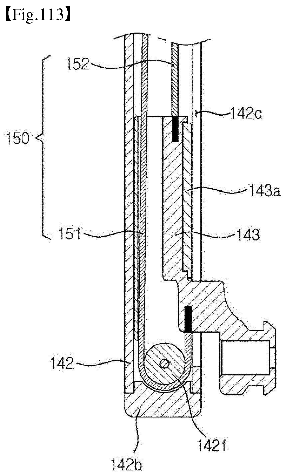

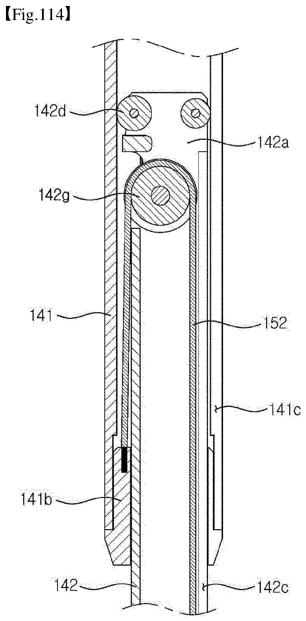

FIG. 112 is a cross-sectional view of the connecting member in the wearable robot according to an embodiment of the present invention, FIG. 113 is an enlarged view of part "A" of FIG. 112, and FIG. 114 is an enlarged view of part "B" of FIG. 112.

As shown in FIG. 103, the first fixing unit (110) is fixed to the waist part located on the upper side of the hip joint, the second fixing unit (120) may be fixed to the thigh part located on the lower side of the hip joint, and the connecting member (140) may, in a state where it is arranged at the front part of the user, connect the drive unit (130) fixed to the first fixing unit (110) side and the second fixing unit (120).

In such an arrangement structure, the connecting member (140) may, while rotating up and down about the y-axis by the drive unit (130), provide assistance force in a direction of lifting up or lowering down the second fixing unit (120) fixed to the thigh part, so it may prevent the adduction, abduction, or twisting of the rotation center of the joint portion due to the provision of the assistance force, and even when the thigh part moves left and right in a state where it has moved by the assistance force, the connecting member (140) may comply therewith and rotate, so it may not restrict the range of motion of the joint portion.

In addition, the connecting member (140) is configured so that the length can be adjusted in correspondence to the interval between the drive unit (130) and the second fixing unit (120), and as the connecting member (140) is configured to expand and contract in multiple stages, it can provide a large stroke while minimizing the length in the contracted state. Therefore, it can transmit assistance force from a motion in which the range of motion of the joint portion is small, such as walking, to a motion in which the range of motion of the joint portion is large, such as sitting or stair walking.

As shown in FIG. 112 to FIG. 114, the first member (141) of the connecting member (140) has one end portion connected to the drive unit (130) provided in the first fixing unit (110), and the second member (142) is coupled, in a state inserted into the inner side of the first member (141), in a state movable along the lengthwise direction of the first member (141). In addition, the third member (143) connected to the second fixing unit (120) is coupled, in a state inserted into the inner side of the second member (142), in a state movable along the lengthwise direction of the second member (142), and the interlocking unit (150) makes the second member (142) and the third member (143) interlock with each other. In addition, the elastic member (144) provides an elastic force to the second member (142) in the contraction direction in a state where both end portions are fixed to the first member (141) and the second member (142). Here, the driving force for rotating about the y-axis by the drive unit (130) may be transmitted directly to the first member (141). Such driving force may be transmitted, through the second member (142) movable along the lengthwise direction on the first member (141) and subsequently the third member (143) movable along the lengthwise direction on the second member (142), to the second fixing unit (120) connected to the third member (143).

Here, the first cable (151) constituting the interlocking unit (150) has one end portion fixed to the 1-1 end cap (141a) of the first member (141) and the other end portion fixed to the third member (143) via a second pulley (142f) assembled to the 2-2 end cap (142b) of the second member (142), and the second cable (152) has one end portion fixed to the 1-2 end cap (141b) of the first member (141) and the other end portion fixed to the third member (143) via a third pulley (142g) assembled to the 2-1 end cap (142a) of the second member (142).

That is, with respect to the contraction-direction movement of the third member (143), as the first cable (151) connected to the third member (143) moves the second pulley (142f) coupled to the expansion-side end portion of the second member (142) in the contraction direction, the second member (142) moves in the contraction direction, and conversely, with respect to the expansion-direction movement of the third member (143), as the second cable (152) connected to the third member (143) moves the third pulley (142g) coupled to the contraction-side end portion of the second member (142) in the expansion direction, the second member (142) moves in the expansion direction.

In addition, the second member (142) can minimize the frictional force with respect to the first member (141) as the rollers (142d) arranged respectively on the upper side and the lower side of the contraction-side end portion roll along the inner surface of the first member (141), and the third member (143) can minimize the frictional force with respect to the second member (142) as the sliding pad (143a) arranged in a form wrapping the outer surface slides along the inner surface of the second member (142).

FIG. 103 shows a state in which the user wears the wearable robot according to an embodiment of the present invention and stands in place, and in such a state, the interval between the drive unit (130) arranged at the waist part on the upper side of the hip joint and the second fixing unit (120) fixed to the thigh part on the lower side becomes maximum. Accordingly, in the connecting member (140) arranged between the drive unit (130) and the second fixing unit (120), the third member (143) connected to the second fixing unit (120) moves in the expansion direction as in FIG. 112, and in this process the second member (142) connected to the third member (143) through the second cable (152) of the interlocking unit (150) moves in the expansion direction in conjunction together with the third member (143), and in this process the elastic member (144) connecting the first member (141) and the second member (142) becomes a state elastically tensioned by external force.

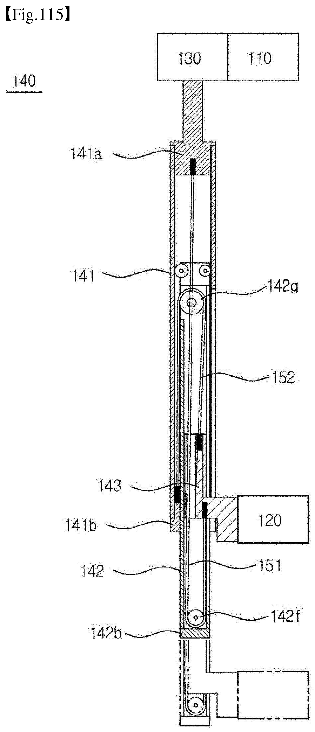

Subsequently, FIG. 105 and FIG. 115 show a state in which the interval between the drive unit (130) and the second fixing unit (120) is changed by a small range of movement such as a walking motion, and the connecting member (140) may, while rotating by the driving of the drive unit (130), lift the second fixing unit (120) upward to provide assistance force to the user's walking motion. At this time, since the connection position of the drive unit (130) and the connecting member (140) does not coincide with the rotation center of the joint portion, in the process in which the thigh part rotates in the upward direction, the interval between the second fixing unit (120) and the drive unit (130) changes, and the length of the connecting member (140) may be adjusted in correspondence to such a change in the interval.

Specifically, when the thigh part rotates in the upward direction about the hip joint as in FIG. 105, the interval between the second fixing unit (120) located on the thigh part and the drive unit (130) located on the waist part is reduced, and in this process, an external force in the contraction direction acts on the connecting member (140) connected to the second fixing unit (120).

That is, as shown in FIG. 115, when the third member (143) arranged movably along the lengthwise direction on the second member (142) moves in the contraction direction by external force, the end portion of the first cable (151) connected to the third member (143) is pulled in the contraction direction, and in this process, as force acts in the contraction direction on the second pulley (142f) supporting the first cable (151), the second member (142) arranged movably along the first member (141) moves in the contraction direction together with the third member (143).

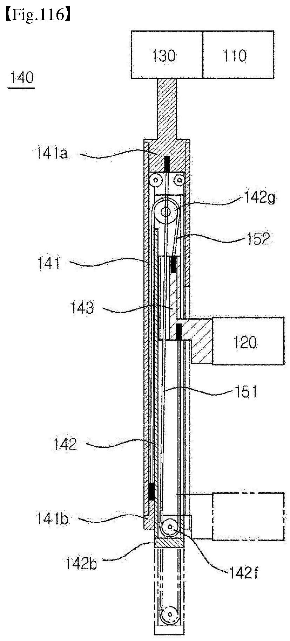

Subsequently, FIG. 107 and FIG. 116 show a state in which the interval between the drive unit (130) and the second fixing unit (120) is changed by a large range of movement such as a sitting motion or stair walking, and the connecting member (140) may, while rotating by the driving of the drive unit (130), lift the second fixing unit (120) upward to provide assistance force to a motion such as the user's sitting motion or stair walking, and since it is configured in multiple stages so that it has a short length in the contracted state while at the same time providing a large stroke, it can effectively cope even with a large range of motion of the joint portion.

Specifically, the third member (143) of the connecting member (140) moves in the contraction direction by the external force applied through the second fixing unit (120) in the process in which the thigh part rotates in the upward direction about the hip joint, and in this process, as the end portion of the first cable (151) connected to the third member (143) is pulled in the contraction direction so that force acts in the contraction direction on the second pulley (142f) supporting the first cable (151), the second member (142) arranged movably along the first member (141) moves in the contraction direction together with the third member (143).

Here, the third member (143) moves in a state where a part of it is exposed to the outside through a second slit (142c) formed on one surface of the second member (142), and since a first slit (141c) communicating with the second slit (142c) is formed on one surface of the first member (141), the third member (143) can move in the contraction direction along the second member (142) even in a state where the second member (142) overlaps with the first member (141).

In addition, the second member (142) may, in the state where it has moved in the contraction direction, have its additional movement in the contraction direction restricted by the contraction-side end portion contacting the 1-1 end cap (141a) of the first member (141), and in such a state, the movement in the contraction direction of the third member (143) connected to the second member (142) through the first cable (151) and the second cable (152) may also be restricted.

Meanwhile, since the second member (142) is configured to interlock with respect to the bidirectional movement of the third member (143) by the first cable (151) and the second cable (152) connecting the first member (141) and the third member (143), it can be prevented from arbitrarily moving in the expansion direction by its own weight in the state where it has moved in the contraction direction.

The wearable robot according to an embodiment of the present invention may, as in FIG. 105, 207, 115, and 116, provide assistance force to the movement of the joint portion by moving the thigh part upward while the connecting member rotates clockwise about the y-axis by the forward driving of the drive unit, and in this process, the connecting member (140) may contract in correspondence to the interval between the drive unit (130) and the second fixing unit (120).

Conversely, the connecting member (140) may provide assistance force to the movement of the joint portion by moving the thigh part in the downward direction while rotating counterclockwise about the y-axis by the reverse driving of the drive unit, and in this process, may expand in correspondence to the interval between the drive unit (130) and the second fixing unit (120).

That is, the third member (143) of the connecting member (140) moves in the expansion direction by the external force applied through the second fixing unit (120) in the process in which the thigh part rotates in the downward direction about the hip joint. At this time, the end portion of the second cable (152) connected to the third member (143) is pulled in the expansion direction, and in this process, as force acts in the expansion direction on the third pulley (142g) supporting the second cable (152), the second member (142) arranged movably along the first member (141) can move in the expansion direction together with the third member (143).

According to the present embodiment, through the first cable (151) of the interlocking unit (150), the second member (142) can be moved in the contraction direction with respect to the contraction-direction movement of the third member (143), and through the second cable (152) of the interlocking unit (150), the second member (142) can be moved in the expansion direction with respect to the expansion-direction movement of the third member (143). By thus making the second member (142) and the third member (143) move in conjunction with each other by the interlocking unit (150), it is possible not only to provide elastic force respectively to the second member (142) and the third member (143) using a single elastic member (144), but also to prevent some members among the plurality of members constituting the connecting member (140) from arbitrarily operating.

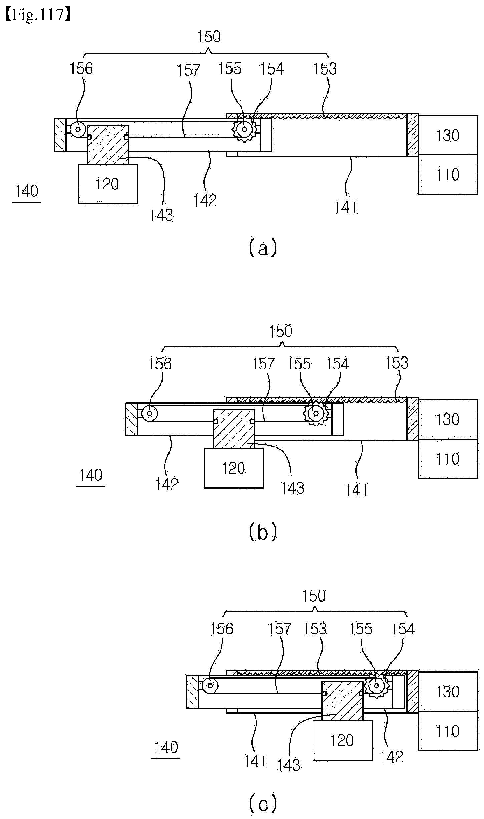

FIG. 117 is an operation diagram of the connecting member according to another embodiment of the wearable robot of the present invention.

The connecting member (140) of the wearable robot according to an embodiment of the present invention includes, as shown in FIG. 117, a first member (141), a second member (142), a third member (143), an interlocking unit (150), and an elastic member (144), and the interlocking unit (150) includes a rack (153) arranged along the lengthwise direction on the inner surface of the first member (141), a pinion (154) arranged at the contraction-side end portion of the second member (142) and meshing with the rack (153), a first pulley (155) rotating together with the pinion (154), a second pulley (156) arranged at the expansion-side end portion of the second member (142), and a belt (157) fixed at both end portions to the third member (143) in a state wound around the first pulley (155) and the second pulley (156).

As in (a) of FIG. 117, in a state where the first member (141), the second member (142), and the third member (143) have each moved in the expansion direction, the pinion (154) coupled to the contraction-side end portion of the second member (142) meshes with the rack (153) provided on the inner surface of the first member (141), the third member (143) is fixed to the belt (157) wound around the first pulley (155) and the second pulley (156) of the second member (142), and the first pulley (155) is configured to rotate together with the pinion (154).

In such a state, as shown in (b) and (c) of FIG. 117, in the process in which the third member (143) moves in the contraction direction by external force, the first pulley (155) and the second pulley (156) each rotate counterclockwise by the belt (157) fixed to the third member (143). At this time, as the pinion (154) supported on the second member (142) and meshed with the rack (153) of the first member (141) rotates counterclockwise together with the first pulley (155), the second member (142) moves in the contraction direction in conjunction with the contraction-direction movement of the third member (143).

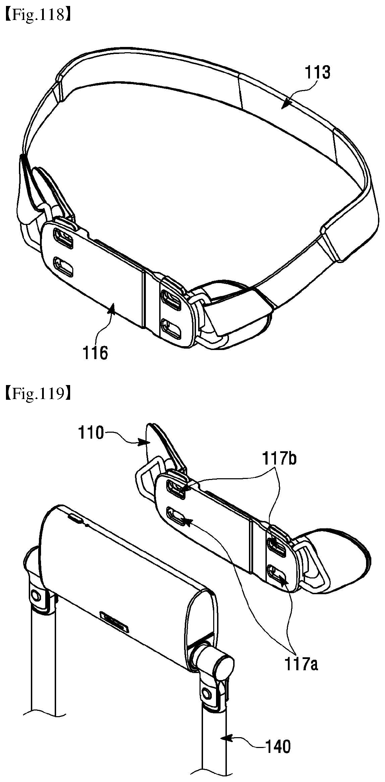

FIG. 118 is a perspective view of a state in which the waist belt (113) and the waist wearing frame (116) are coupled, and FIG. 119 to FIG. 121 are diagrams for showing the process of attaching/detaching the waist wearing unit (110) of the wearable robot.

The waist wearing unit (110) according to an embodiment of the present invention may include a waist belt (113) and a waist wearing frame (116). In addition, both ends of the waist belt (113) may be coupled with both ends of the waist wearing frame (116). The waist belt (113) may have its length adjusted according to the size of the wearer's waist.

According to the present invention, the waist wearing frame (116) may include a lower mechanism portion (117a), an upper mechanism portion (117b), and an attachment/detachment button (118). A lower hook (134a) and an upper hook (134b) may be included on one surface of the main body housing (134) of the drive unit (130). The lower mechanism portion (117a) couples with the lower hook (134a) and the upper mechanism portion (117b) couples with the upper hook (134b), so that the waist wearing unit (110) and the drive unit (130) can couple. When the attachment/detachment button (118) is pressed, the upper mechanism portion (117b) and the upper hook (134b) are separated, and accordingly the lower mechanism portion (117a) and the lower hook (134a) can be easily separated, so that the drive unit (130) and the waist wearing unit (110) can be easily attached/detached.

Hereinafter, the operation of the waist wearing unit (110) at the time of attachment/detachment of the drive unit (130) according to an embodiment of the present invention will be described.

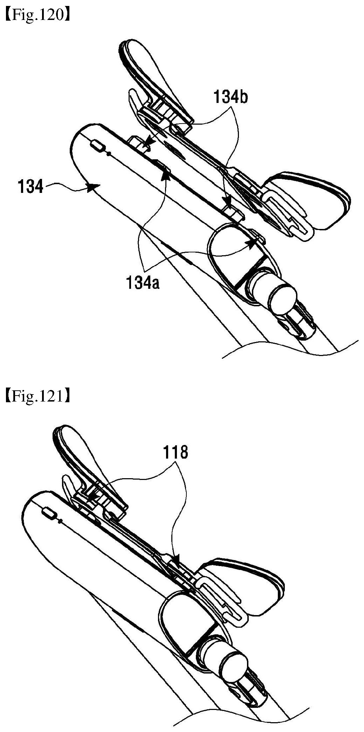

In the case of wearing the wearable robot, the wearable robot wearing method includes a step of wearing the waist wearing unit (110) on the wearer's waist and bringing the drive unit (130) near the waist wearing unit (110), as in FIG. 119. The wearable robot wearing method according to an embodiment of the present invention includes a step of hooking the lower hook (134a) on the inner side of the main body housing (134) onto the mechanism portion lower end (117a) of the waist wearing frame (116), as in FIG. 120.

The wearable robot wearing method includes a step of tilting the drive unit (130) to hook the upper hook (134b) on the inner side of the main body housing (134) onto the mechanism portion upper end (117b) of the waist wearing frame (116), as in FIG. 121.

At this time, there is a fastener at the mechanism portion upper end (117b), so the mechanism portion upper end (117b) and the upper hook (134b) are automatically fastened, and the main body housing (134) and the waist wearing frame (116) couple. Accordingly, the drive unit (130) and the waist wearing unit (110) couple.

In the case of detaching the wearable robot, the wearable robot detachment method includes a step of separating the waist wearing unit from the wearer's waist and pressing the attachment/detachment button (118) on the waist wearing frame (116) of the waist wearing unit (110), as in FIG. 121. The wearable robot detachment method according to an embodiment of the present invention includes a step in which, in a state where the lower hook (134a) is hooked on the mechanism portion lower end (117a), the upper end of the drive unit (130) tilts to the front of the wearer by the own weight of the drive unit (130), as in FIG. 120. The wearable robot detachment method according to an embodiment of the present invention includes a step of slightly lifting the drive unit (130) and moving it forward to separate it from the waist wearing unit (110), as in FIG. 119.

According to an embodiment of the present invention, the drive unit (130) and the waist wearing unit (110) can be separated, so the volume of the wearable robot can be reduced. In addition, storage of each of the separated drive unit (130) and waist wearing unit (110) is easy. In addition, by wearing the waist wearing unit (110) separately in advance, the wearing and separation time of the wearable robot can be shortened. In addition, by making the waist wearing unit (110) similar to a belt shape, usability and wearing convenience can be secured.

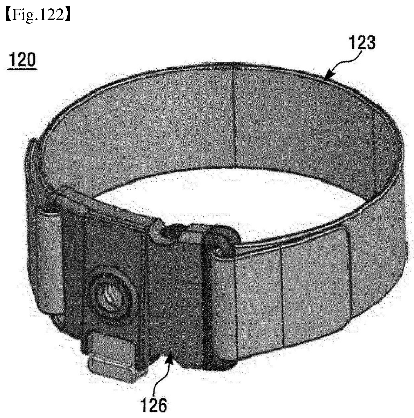

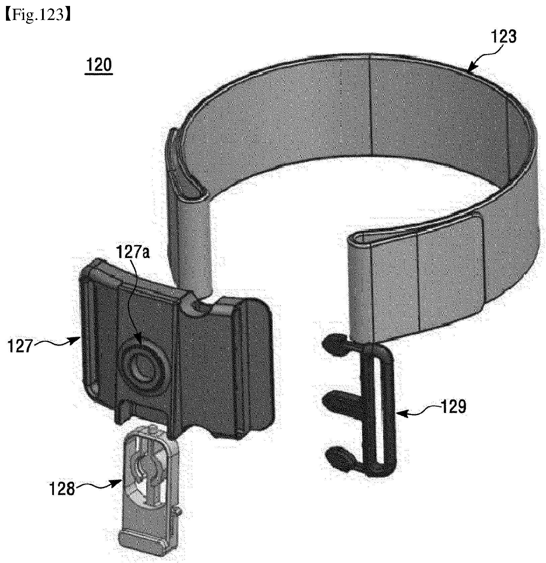

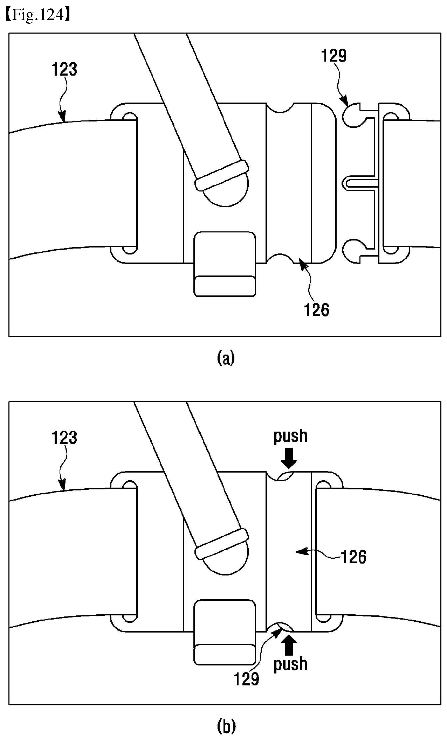

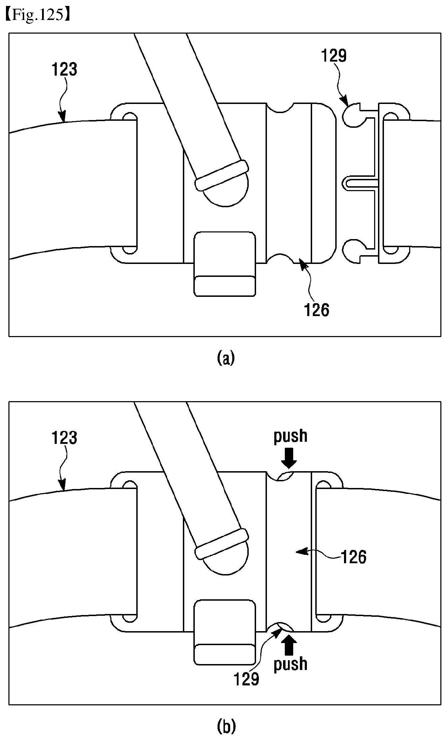

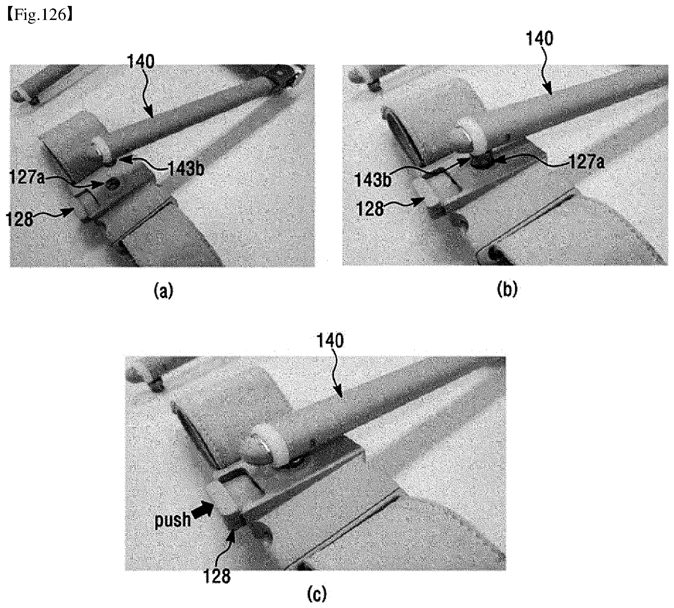

FIG. 122 is a perspective view of the thigh wearing unit in the wearable robot according to an embodiment of the present invention, and FIG. 123 is an exploded perspective view of the thigh wearing unit shown in FIG. 122. FIG. 124 is a diagram for showing the process of the plate and the strap portion separating/coupling, FIG. 125 is a diagram showing the method by which the first button operates within the plate, and FIG. 126 is a diagram for showing the process of the plate and the connecting member separating/coupling.

The thigh wearing unit (120) according to an embodiment of the present invention may include a strap portion (123) and a plate (126). The strap portion (123) may include a second button (129) that couples to one end of the strap portion (123). The second button (129) may be constituted in a simple buckle form, but may also be constituted in a form in which a hook-spring element is combined. Both ends of the strap portion (123) may couple with both ends of the plate (126). The strap portion (123) may have its length adjusted according to the size of the wearer's thigh.

According to an embodiment of the present invention, the length adjustment or separation of the strap portion (123) may be made at both ends where the strap portion (123) and the plate (126) couple. In addition, one of the coupling both ends may be fixed, and the strap portion (123) may be length-adjusted and separated only at the other end. In addition, the strap portion (123) may be constituted of a single elastic material or non-elastic material, or by mixing each, and may also be constituted of heterogeneous elastic materials.

The coupling method between the plate (126) and the strap portion (123) according to an embodiment of the present invention includes a step of contacting one end of the plate (126) and the second button (129) and pushing the second button (129) in the direction of the plate (126) with a predetermined force, as in (a) of FIG. 124. The coupling method between the plate (126) and the strap portion (123) according to an embodiment of the present invention includes a step in which the groove (not shown) of the second button (129) is caught on the plate (126) and couples, as in (b) of FIG. 124.

The separation method between the plate (126) and the strap portion (123) according to an embodiment of the present invention includes a step of pressing the second button (129) of the strap portion (123), as in (b) of FIG. 124. The separation method between the plate (126) and the strap portion (123) includes a step in which the second button (129) is pushed out from the plate (126) and separated, as in (a) of FIG. 124. In this way, the plate (126) and the strap portion (123) can be easily separated, so the wearable robot can be easily attached/detached.

As shown in FIG. 123, the plate (126) may include a plate frame (127) and a first button (128). The plate frame (127) may include an opening (127a). In addition, the third member (143) may include a protrusion (143b).

As shown in (a) of FIG. 125, in a state where the first button (128) is not pressed, the opening (127a) is in an open state. Therefore, the protrusion (143b) of the third member (143) can couple with the opening (127a). On the other hand, in the case where the first button (128) is pressed, as shown in (b) of FIG. 125, the first button (128) can push out the protrusion (143b) coupled to the opening (127a).

The coupling method between the plate (126) and the connecting member (140) according to an embodiment of the present invention may include a step in which the protrusion (143b) of the connecting member (140) contacts the opening (127a), as in (b) of FIG. 125. The coupling method between the plate (126) and the connecting member (140) includes a step in which, by applying a predetermined force to the connecting member (140), the protrusion (143b) is completely inserted into the opening (127a), as in (c) of FIG. 126.

The separation method between the plate (126) and the connecting member (140) according to an embodiment of the present invention includes a step of pressing the first button (128), as in (c) of FIG. 126. The separation method between the plate (126) and the connecting member (140) includes a step in which the protrusion (143b) protrudes out from the opening (127a), so that a state is reached in which the connecting member (140) can be easily separated from the plate (126), as in (b) of FIG. 126. In this way, the plate (126) and the connecting member (140) can be easily separated, so the wearable robot can be easily attached/detached.

According to an embodiment of the present invention, when the plate (126) and the connecting member (140), and the plate (126) and the strap portion (123) couple, the rotational output produced by the actuator (131) built into the drive unit (130) can be transmitted to the plate (126) and the strap portion (123) through the connecting member (140) and the third member (143). Through this, the wearable robot can transmit the assistance force or resistance force generated by the drive unit (130) to the wearer's thigh, thereby lifting or lowering the thigh. That is, the action sites at which the wearer receives the assistance force or resistance force are the plate (126) and the strap portion (123).

In addition, one or more degrees of freedom may be imparted between the plate (126) and the connecting member (140). For example, as an embodiment of the present invention, the protrusion (143b) may form a spherical shape, and when the protrusion (143b) couples with the opening (127a), the connecting member (140) is rotatable without coming out from the plate (126). That is, a configuration that functions as a joint portion in the form of a pivot or joint between the plate (126) and the connecting member (140) imparts the degree of freedom. Meanwhile, the protrusion (143b) is not necessarily limited to a spherical shape. In addition, a degree of freedom in another axial direction may be imparted in the protrusion (143b) or another configuration, and in this case, a new degree of freedom is added to the rotational degree of freedom that existed in the existing coupling. Through such degrees of freedom, the wearer can take various motions even while wearing the wearable robot, and can minimize the resistance feeling when taking various motions.

According to an embodiment of the present invention, the wearable robot and the thigh wearing unit (120) can be separated, so the volume of the wearable robot can be reduced. In addition, storage of each of the separated wearable robot and thigh wearing unit (120) is easy. In addition, by wearing the thigh wearing unit (120) separately in advance, the wearing and separation time of the wearable robot can be shortened. In addition, by appropriately using a non-elastic material and an elastic material in the strap portion (123), the usability and wearing convenience of the wearable robot can be secured.

The configuration of the present invention is not limited to the above-mentioned embodiments. For example, the configuration of the thigh wearing unit (120) may be merged into clothing so that the function of the thigh wearing unit (120) can be performed by clothing alone. Thus, even without a separate thigh wearing unit (120), the usability and wearing convenience of the wearable robot can be maintained.

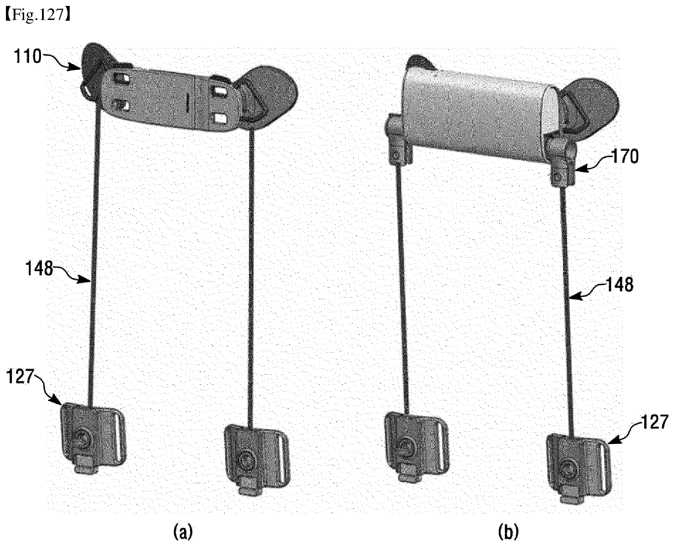

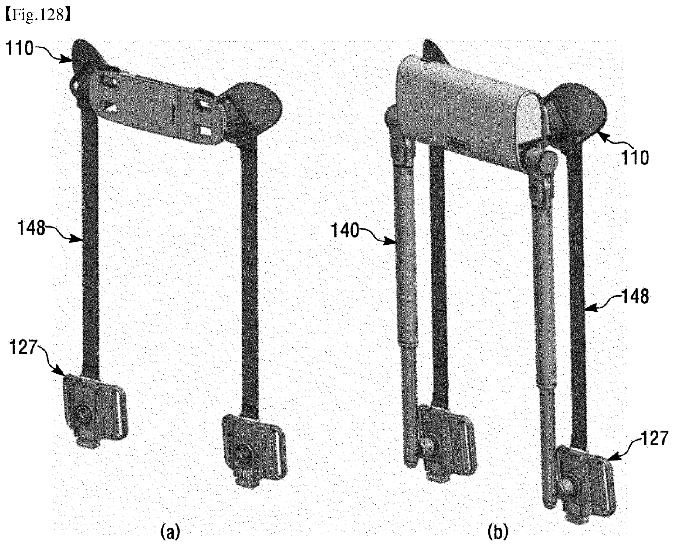

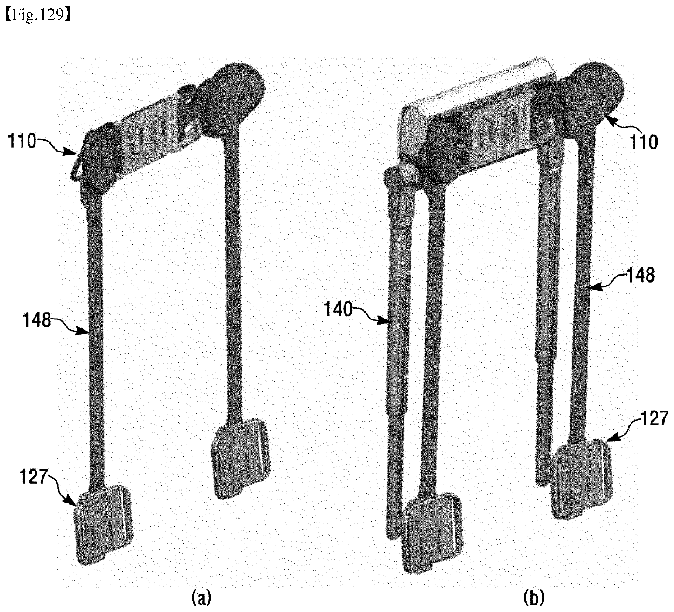

Meanwhile, as shown in FIG. 127 to FIG. 129, a wearing unit elastic member (148) may be arranged between the waist wearing unit (110) and the thigh wearing unit (120). In this case, a ring (not shown) or an opening (not shown) of the waist wearing unit (110) may couple with one end of the wearing unit elastic member (148), and a ring (not shown) or an opening (not shown) of the plate frame (127) in the thigh wearing unit (120) may couple with the other end of the wearing unit elastic member (148). Such coupling may be implemented by various methods as well as a ring or an opening.

In addition, as shown in (b) of FIG. 127, one end of the wearing unit elastic member (148) may couple with the rotation joint portion (170) and the other end may couple with the thigh wearing unit (120). In the coupling state as above, the wearing unit elastic member (148) is length-adjustable. Meanwhile, due to the tension occurring in the wearing unit elastic member (148), the wearing unit elastic member (148) can perform the function of the elastic member (144) in the connecting member (140) instead. Accordingly, implementation of the wearable robot is possible with the elastic member (144) within the connecting member (140) omitted.

According to an embodiment of the present invention, in the case of wearing the wearable robot including the wearing unit elastic member (148), the wearable robot wearing method includes a step of wearing the waist wearing unit (110) and the thigh wearing unit (120) and adjusting the length of the wearing unit elastic member (148). The wearable robot wearing method according to an embodiment of the present invention includes a step of coupling the drive unit (130) and the waist wearing unit (110), and coupling the connecting member (140) and the thigh wearing unit (120).

According to an embodiment of the present invention, in the case where the wearing unit elastic member (148) connects between the waist wearing unit (110) and the thigh wearing unit (120), the sliding-down of the thigh wearing unit (120) can be prevented. In addition, when the length of the wearing unit elastic member (148) is adjusted to fit the wearer's body shape, the tension of the wearing unit elastic member (148) can be minimized. Accordingly, the force by which the thigh wearing unit (120) pulls the waist wearing unit (110) downward can be minimized, so that the sense of weight felt by the wearer can be minimized. In addition, the elastic force of the elastic member (144) within the connecting member (140) can be minimized. In addition, by omitting the elastic member (144) configuration in the connecting member (140), the structure of the connecting member (140) can be simplified.

The scope of rights of the present invention is not limited to the above-described embodiments, but may be implemented in various forms of embodiments within the scope of the appended claims. Anyone having ordinary knowledge in the technical field to which the present invention pertains shall, without departing from the gist of the present invention claimed in the claims, regard even the various ranges modifiable thereby as being within the scope of the description of the claims of the present invention.

## Claims

1. A thigh frame of a walking assistance wearable robot for assisting a walking exercise of a wearer, the thigh frame comprising:
a waist wearing unit mountable on a waist of the wearer;
a thigh wearing unit mountable on a thigh of the wearer;
a drive unit connected to the waist wearing unit and comprising an actuator; and
a thigh frame connecting the drive unit and the thigh wearing unit,
wherein the thigh frame comprises: a joint member connecting the drive unit and the thigh frame; a thigh cover member connected to the joint member; and a thigh inner member sliding inside the thigh cover member according to the walking exercise of the wearer,
wherein the joint member is connected in series with the actuator and is configured to transmit an assistance force generated from the actuator to the thigh frame, and
wherein the thigh cover member is configured to comprise a rail structure having a predetermined angle and formed left-right symmetrically inside the thigh cover member.

2. The thigh frame of Claim 1, further comprising a bearing end member,
wherein the thigh inner member is coupled with the bearing end member, and
wherein the bearing end member is configured to prevent left-right play during sliding of the thigh inner member and to distribute a load applied to the thigh frame when the assistance force of the actuator is generated.

3. The thigh frame of Claim 2,
wherein the thigh inner member further comprises a two-stage auxiliary bearing member, and
wherein the two-stage auxiliary bearing member is configured to, together with the two-stage bearing member, prevent left-right movement of the thigh inner member and maintain a constant gap during sliding.

4. A thigh wearing unit of a walking assistance wearable robot for assisting a walking exercise of a wearer,
wherein the walking assistance wearable robot comprises: a waist wearing unit mountable on a waist of the wearer; a thigh wearing unit mountable on a thigh of the wearer; a drive unit connected to the waist wearing unit; and a thigh frame connecting the drive unit and the thigh wearing unit,
wherein the thigh frame comprises a thigh frame joint portion, wherein the thigh wearing unit comprises a fixing clip unit,
wherein the fixing clip unit is configured to fix or detach the thigh frame joint portion, and wherein the thigh wearing unit comprises a thigh frame release button for switching the thigh frame joint portion from a fixed state to a released state.

5. The thigh wearing unit of Claim 1,
wherein the thigh wearing unit further comprises a thigh band unit and a thigh buckle unit,
wherein the thigh band unit is connected to the thigh buckle unit and is configured to be length-adjustable to fit a circumference of the thigh of the wearer, and wherein the thigh frame joint portion is designed in a round shape to be rotatable and is configured to rotate flexibly according to a movement of the thigh of the wearer.

6. The thigh wearing unit of Claim 1,
wherein the thigh wearing unit comprises a shock prevention member and a thigh detachable frame, wherein the thigh detachable frame further comprises a sliding rail member,
wherein the thigh frame joint portion is configured to move along the sliding rail member, and wherein the shock prevention member is positioned on an inner side of the thigh wearing unit and is configured to alleviate an impact or vibration occurring during a sliding motion of the thigh frame.

7. A waist wearing unit of a walking assistance wearable robot for assisting a walking exercise of a wearer, comprising:
a waist wearing unit mountable on a waist of the wearer; and
a drive unit mountable on the waist wearing unit,
wherein the waist wearing unit comprises: a waist wearing unit member; and a waist wearing buckle unit connected to the waist wearing unit member,
wherein the drive unit comprises a drive unit fixing frame positioned on both sides of the drive unit, wherein the waist wearing buckle unit is configured to be engageable with and disengageable from the drive unit fixing frame, and
wherein the drive unit fixing frame is configured to have an inclination of 0 degrees or more and 30 degrees or less with respect to a vertical plane of the drive unit.

8. The waist wearing unit of Claim 7,
wherein the waist wearing buckle unit comprises a drive unit fixing member, wherein the drive unit fixing member further comprises an inner connection member,
wherein the drive unit fixing member further comprises a button member positioned on the inner connection member and an elastic member positioned inside the waist wearing buckle unit,
wherein, when the waist wearing buckle unit and the drive unit fixing frame are engaged, the button member is configured to move in an advancing direction of the drive unit fixing frame, whereby the elastic member is configured to contract, and wherein, when the button member and the drive unit fixing frame are engaged, the elastic member relaxes to prevent the drive unit fixing frame from escaping toward the button member.

9. A wearable robot capable of estimating a physical ability of a wearer during a walking exercise, comprising:
a fixing unit mountable on a body part; and
a drive unit mountable on the fixing unit,
wherein the drive unit is configured to: estimate the physical ability of the wearer based on a power of the drive unit resulting from the walking exercise of the wearer;
estimate the physical ability of the wearer based on a value obtained by normalizing the power of the drive unit by a torque; and calculate a ratio of the power of the drive unit to the torque by dividing the power of the drive unit by an RMS (Root Mean Square) torque of the drive unit.

10. The wearable robot of Claim 9,
wherein the drive unit is configured to sense a hip angle of the wearer and calculate an angular velocity based on the sensed hip angle, and to calculate the power of the drive unit by a product of the calculated angular velocity and the torque, and
wherein the calculated angular velocity is calculated based on a walking of the wearer in a case where the wearer uses a backdrivable actuator.

11. The wearable robot of Claim 10,
wherein the torque is calculated, when the drive unit calculates a plurality of motion state values based on the calculated angular velocity, as a weighted sum of at least one motion state value selected from among the calculated plurality of motion state values.

12. A server device for managing an exercise of a wearer wearing a wearable robot, the server device being configured to:
receive, from a first user terminal, walking information of the wearer wearing the wearable robot linked with the first user terminal;
transmit the received walking information of the wearer to a second user terminal; and
receive, from the second user terminal, feedback information based on the walking information of the wearer received by the second user terminal,
wherein the feedback information based on the walking information of the wearer received by the second user terminal comprises an exercise intensity based on the walking information of the wearer, and
wherein the feedback information based on the walking information of the wearer received by the second user terminal comprises information for controlling, in real time, the exercise intensity based on the walking information of the wearer.

13. A system for managing an exercise of a wearer wearing a wearable robot capable of estimating a physical ability of the wearer during a walking exercise, comprising:
a first user terminal configured to output analysis information on the exercise of the wearer;
a server device configured to receive the analysis information on the exercise of the wearer output by the first user terminal; and
a second user terminal configured to receive the analysis information on the exercise of the wearer transmitted by the server device,
wherein the server device is configured to: receive, from the second user terminal, feedback information based on the information on the exercise of the wearer transmitted to the second user terminal; and transmit the received feedback information to the first user terminal,
wherein the feedback information received by the server device comprises an exercise intensity based on walking information of the wearer.

14. The system of Claim 13,
wherein the analysis information on the exercise of the wearer output by the first user terminal is based on a ratio of power to torque calculated by the wearable robot, and is at least one of an agility or a lower-body muscle strength intensity of the wearer.

15. A walking assistance wearable robot system for assisting a walking exercise of a wearer,
wherein the system comprises a user terminal and the wearable robot,
wherein the wearable robot is configured to: receive target muscle information to be exercised from the user terminal; set a parameter based on the received target muscle information and calculate an output torque based thereon; and transmit information related to the walking exercise to the user terminal,
wherein the user terminal is configured to receive the transmitted information related to the walking exercise, and wherein the wearable robot is configured to calculate an angular velocity based on a walking of the wearer, calculate a motion state value based on the calculated angular velocity, and calculate the output torque based thereon.

16. The walking assistance wearable robot system of Claim 15,
wherein the set parameter is a parameter representing at least one of output timing information of the calculated torque, intensity adjustment information of the calculated torque, filter information for calculating the output torque, or asymmetry information for calculating the output torque, and
wherein the output timing information of the calculated torque comprises position information of the calculated motion state value.

17. The walking assistance wearable robot system of Claim 16, further comprising a memory unit,
wherein the drive unit may be configured to calculate an output torque according to a set exercise mode, wherein the memory unit may be configured to store a parameter according to the exercise mode as a lookup table,
wherein the set parameter is configured as a parameter according to the target muscle information received from the user terminal based on the lookup table, and wherein the lookup table is configured to consist of a combination of at least one of the output timing information of the calculated torque, the intensity adjustment information of the calculated torque, the filter information for calculating the output torque, or the asymmetry information for calculating the output torque.

18. A walking assistance wearable robot for assisting a walking exercise of a wearer, comprising:
a fixing unit mountable on a body part of the wearer; and
a drive unit mountable on the fixing unit,
wherein the drive unit is configured to: calculate a degree of change in a state trajectory of the wearer and calculate an output torque based thereon; and calculate, in a range at or below a threshold including a time point at which a sign of the calculated output torque changes, the output torque as a value at or below the threshold for a predetermined time,
wherein the predetermined time is 0.5 seconds or more and 1 second or less.

19. The walking assistance wearable robot of Claim 18,
wherein the drive unit may be configured not to calculate the output torque or to calculate the output torque as a value at or below a threshold when a state trajectory movement distance of the wearer during the predetermined time is equal to or less than a first threshold, and
wherein the drive unit is configured to calculate the output torque as a value at or above a threshold when the state trajectory movement distance of the wearer during the predetermined time is equal to or greater than a second threshold.

20. The walking assistance wearable robot of Claim 19,
wherein the first threshold and the second threshold are configured to be the same value, or the second threshold is configured to be a value greater than the first threshold,
wherein the drive unit is configured to, when the state trajectory movement distance of the wearer during the predetermined time is equal to or less than the first threshold, change a value of a torque intensity adjustment parameter for calculating the output torque to a value equal to or less than a threshold value or to 0, and wherein the value of the torque intensity adjustment parameter for calculating the output torque is configured to be gradually changed to a value equal to or less than the threshold value or to 0.
